# EUROPEAN PATENT APPLICATION

(11) **EP 4 596 557 A1**
(43) Date of publication of application: **06.08.2025**
(21) Application number: 23870903.4
(22) Date of filing: 27.09.2023
(51) Int. Cl.: C07D 495/10, C07D 237/24, A61K 31/506, A61K 31/381, A61P 35/00

(54) **COMPOUND AS INHIBITOR FOR SUMO ACTIVATING ENZYME**

(30) Priority: 30.09.2022 CN 202211212103; 15.08.2023 CN 202311026791
(71) Applicant: Wigen Biomedicine Technology (Shanghai) Co., Ltd., Shanghai 201203 (CN)
(72) Inventor: XIE, Yuli, Shanghai 201203 (CN); WU, Yingming, Shanghai 201203 (CN); QIAN, Lihui, Shanghai 201203 (CN)
(74) Representative: Dr. Langfinger & Partner
(86) International application number: PCT/CN2023/121932
(87) International publication number: WO 2024/067676

(57) **Abstract**

Disclosed in the present disclosure are compounds as SUMO activating enzyme inhibitors. Specifically, the present disclosure relates to a compound of general formula (1), a method for preparing same, and use of the compound of general formula (1) and isomers, crystalline forms, pharmaceutically acceptable salts, hydrates or solvates thereof as SAE inhibitors. The compound and the isomer thereof, the crystalline form thereof, the pharmaceutically acceptable salt thereof, the hydrate thereof, or the solvate thereof of the present disclosure can be used in preparing a medicament for treating or preventing a disease related to SAE protein.

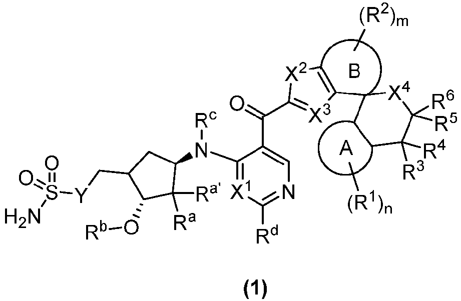

## Description

The present application claims priority to Chinese Patent Application No. 202211212103.5 filed on September 30, 2022 and Chinese Patent Application No. 202311026791.0 filed on August 15, 2023, which are incorporated herein by reference in their entirety.

### TECHNICAL FIELD

The present disclosure relates to the field of pharmaceutical chemistry, and particularly relates to a class of compounds with an inhibitory effect on SAE protein, a preparation method therefor, and use of the compounds in preparing a medicament for treating or preventing a related disease mediated by SAE.

### BACKGROUND

Small ubiquitin-like modifiers (SUMOs) are a family of ubiquitin-like proteins that function as reversible posttranslational modifiers within cells. Mammalian cells express three types of SUMO family proteins: SUMO1, SUMO2, and SUMO3. SUMO2 shares about 95% amino acid sequence homology with SUMO3 and primarily forms oligomeric chains upon protein modification. SUMO1 shares about 45% sequence homology with SUMO2 and SUMO3, and primarily modifies proteins in a monomer form. Sumoylation of a target protein involves a three-step enzyme-catalyzed reaction that activates, transfers, and ultimately conjugates the SUMO protein to the lysine residue of the target protein. The first step is catalyzed by SAE (SUMO activating enzyme), which is a class of activating enzymes known as E1 enzymes and functions as a heterodimer composed of SAE1 and SAE2/UBA2. SAE uses ATP to adenylate the C-terminal glycine residue of SUMO, leading to the formation of a thioester intermediate between the C-terminal glycine of SUMO and a cysteine residue in SAE2. Subsequently, the SUMO protein is transferred from E1 to the SUMO specific conjugating enzyme, collectively known as E2, via thioester bond exchange. Finally, under the action of a SUMO specific E3 protein ligase, the SUMO protein is ultimately conjugated to the lysine residue of the target protein, forming an oligomeric chain. Sumoylation of proteins affects catalytic activity, intracellular localization, and interactions with other proteins. In addition, recent studies have shown that sumoylation of proteins plays a significant role in various cellular signaling pathways, including cell division, DNA repair, chromosome segregation, nuclear transport, gene transcription, and immune regulation. Overexpression of proteins related to the SUMO signaling pathway is associated with poor prognosis in certain cancer patients. Knockdown of SAE has shown synthetic lethality in certain MYC-overexpressing tumor cells. In addition, sumoylation can also modulate innate immune responses. Inhibition of sumoylation can enhance type 1 interferon (IFN) expression. In summary, there is an urgent need to study and identify compounds with potent activity targeting SAE.

### SUMMARY

The present disclosure provides a compound of general formula (1) or an isomer thereof, a crystalline form thereof, a pharmaceutically acceptable salt thereof, a hydrate thereof, or a solvate thereof: 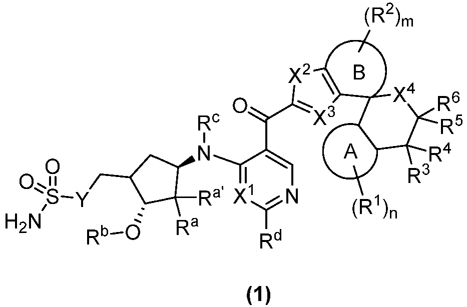 wherein in general formula (1):
Y is -O-, -CH₂-, or -N(H)-;
R^{a} is -H, -F, -NH₂, or -OH;
R^{a'} is -H or -F, and when R^{a} is -NH₂ or -OH, R^{a'} is -H;
R^{b} is -H or (C1-C4) alkyl;
R^{c} is -H or (C1-C4) alkyl;
R^{d} is -H, halogen, -CF₃, or (C1-C4) alkyl;
X¹ is C(H), C(F), or N;
X² is S or O;
X³ is C(R^{x3}) or N;
R^{x3} is -H, halogen, or -CH₃;
X⁴ is S, O, C(R^{x41})(R^{x41'}), or N(R^{x42});
R^{x42} is -H, (C1-C4) alkyl or (C3-C5) cycloalkyl;
R^{x41} and R^{x41'} are each independently and optionally -H, halogen, -OH, -OR^{x411}, -N(R^{x411})(R^{x412}), -CN, (C1-C6) alkyl, (C1-C6) haloalkyl, (C3-C9) cycloalkyl, or (C1-C6) alkoxy;
R^{x411} and R^{x412} are each independently and optionally -H, (C1-C4) alkyl, or (C3-C5) cycloalkyl, or R^{x411} and R^{x412} on a same nitrogen atom, together with the N atom to which they are attached, can form (3- to 6-membered) heterocycloalkyl, wherein the (3- to 6-membered) heterocycloalkyl may be optionally substituted with 1, 2, 3, or 4 of the following groups: -H or halogen;
R³ and R⁴ are each independently and optionally -H, -D, -OH, -NH₂, -CN, (C1-C6) alkyl, (C1-C6) haloalkyl, (C2-C6) alkenyl, (C2-C6) alkynyl, (C3-C9) cycloalkyl, (C1-C6) alkoxy, (C6-C14) aryl, (3- to 11-membered) heterocycloalkyl, or (5- to 11-membered) heteroaryl, wherein the (C1-C6) alkyl, (C1-C6) haloalkyl, (C2-C6) alkenyl, (C2-C6) alkynyl, (C3-C9) cycloalkyl, (C1-C6) alkoxy, (C6-C14) aryl, (3- to 11-membered) heterocycloalkyl, or (5- to 11-membered) heteroaryl may be each independently and optionally substituted with 1, 2, 3, or 4 of the following groups: -H, halogen, -OH, -(CH₂)ᵣOR³¹, -(CH₂)ᵣNR³¹R³², -OR³¹, -NR³¹R³², -CN, -C(O)NR³¹R³², -NR³²C(O)R³¹, -NR³²S(O)₂R³¹, -S(O)ₚR³¹, and -S(O)₂NR³¹R³²; or R³ and R⁴, together with the carbon atom to which they are attached, can form a (4- to 7-membered) heterocycloalkyl or (C3-C6) cycloalkyl, wherein the (4- to 7-membered) heterocycloalkyl or (C3-C6) cycloalkyl may be optionally substituted with 1, 2, 3, or 4 of the following groups: -H, halogen, (C1-C6) alkyl, or (C1-C6) alkoxy; or R³ and the adjacent R⁵, together with the atoms to which they are attached, can form a (C3-C9) cycloalkyl or (3-to 11-membered) heterocycloalkyl, wherein the (C3-C9) cycloalkyl or (3- to 11-membered) heterocycloalkyl may be each independently and optionally substituted with 1, 2, 3, or 4 of the following groups: -H, halogen, (C1-C6) alkyl, or (C1-C6) alkoxy; or when R³ and the adjacent R⁵ are both absent, an endocyclic double bond is formed; or R³ and R⁴ together form an oxo;
R⁵ and R⁶ are each independently and optionally -H, -D, -OH, -NH₂, -CN, (C1-C6) alkyl, (C1-C6) haloalkyl, (C2-C6) alkenyl, (C2-C6) alkynyl, (C3-C9) cycloalkyl, (C1-C6) alkoxy, (C6-C14) aryl, (3- to 11-membered) heterocycloalkyl, or (5- to 11-membered) heteroaryl, wherein the (C1-C6) alkyl, (C1-C6) haloalkyl, (C2-C6) alkenyl, (C2-C6) alkynyl, (C3-C9) cycloalkyl, (C1-C6) alkoxy, (C6-C14) aryl, (3- to 11-membered) heterocycloalkyl, or (5- to 11-membered) heteroaryl may be each independently and optionally substituted with 1, 2, 3, or 4 of the following groups: -H, halogen, -OH, -(CH₂)ᵣOR³¹, -(CH₂)ᵣNR³¹R³², -OR³¹, -NR³¹R³², -CN, -C(O)NR³¹R³², -NR³²C(O)R³¹, -NR³²S(O)₂R³¹, -S(O)ₚR³¹, and -S(O)₂NR³¹R³²; or R⁵ and R⁶, together with the carbon atom to which they are attached, can form a (4- to 7-membered) heterocycloalkyl or (C3-C6) cycloalkyl, wherein the (4- to 7-membered) heterocycloalkyl or (C3-C6) cycloalkyl may be optionally substituted with 1, 2, 3, or 4 of the following groups: -H, halogen, (C1-C6) alkyl, and (C1-C6) alkoxy; or R⁵ and R⁶ together form an oxo;
ring A is (C6-C10) aryl or (5- to 10-membered) heteroaryl;
each R¹ is independently and optionally: -H, halogen, -OH, -NO₂, -NR³¹R³², -(CH₂)ᵣOR³¹, -(CH₂)ᵣNR³¹R³², - CN, (C1-C6) alkyl, (C1-C6) haloalkyl, (C1-C6) alkoxy, (C2-C6) alkenyl, (C2-C6) alkynyl, (C3-C8) cycloalkyl, -C(O)NR³¹R³², -NR³²C(O)R³¹, -NR³²S(O)₂R³¹, -S(O)ₚR³¹, or -S(O)₂NR³¹R³², wherein the (C1-C6) alkyl, (C1-C6) haloalkyl, (C1-C6) alkoxy, (C2-C6) alkenyl, (C2-C6) alkynyl, or (C3-C8) cycloalkyl may be each independently and optionally substituted with 1, 2, 3, or 4 of the following groups: -H, halogen, -OH, - (CH₂)ᵣOR³¹, -(CH₂)ᵣNR³¹R³², -OR³¹, -NR³¹R³², -CN, and (C1-C6) alkyl;
ring B is (C5-C7) cycloalkyl or (5- to 7-membered) heterocycloalkyl;
each R² is independently and optionally: -H, halogen, -OH, -NR³¹R³², -CN, (C1-C6) alkyl, (C1-C6) haloalkyl, (C1-C6) alkoxy, (C2-C6) alkenyl, (C2-C6) alkynyl, or (C3-C8) cycloalkyl; or two R² on a same carbon atom, together with the carbon atom to which they are attached, can form (4- to 6-membered) heterocycloalkyl or (C3-C6) cycloalkyl, wherein the (4- to 6-membered) heterocycloalkyl or (C3-C6) cycloalkyl may be optionally substituted with 1, 2, 3, or 4 of the following groups: -H, halogen, (C1-C6) alkyl, and (C1-C6) alkoxy; or two R² on the same carbon atom together form an oxo;
R³¹ and R³² are each independently and optionally -H, (C1-C4) alkyl, or (C3-C5) cycloalkyl, or R³¹ and R³² on a same nitrogen atom, together with the N atom to which they are attached, can form (3- to 6-membered) heterocycloalkyl, wherein the (3- to 6-membered) heterocycloalkyl may be optionally substituted with 1, 2, 3, or 4 of the following groups: -H and halogen; and
n is an integer selected from 0, 1, 2, 3, or 4, m is an integer selected from 0, 1, 2, 3, or 4, r is an integer selected from 0, 1, or 2, and p is an integer selected from 0, 1, or 2.

In another preferred embodiment, in the general formula (1), Y is -O- or -N(H)-, preferably -O-.

In another preferred embodiment, in the general formula (1), R^{d} is -H, -F, -CF₃, or -CH₃.

In another preferred embodiment, in the general formula (1), R^{x42} is -H, (C1-C3) alkyl, or (C3-C5) cycloalkyl.

In another preferred embodiment, in the general formula (1), R^{x42} is -H,

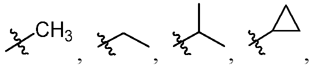

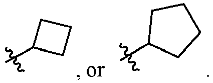

In another preferred embodiment, in the general formula (1), R^{x41} and R^{x41}, are each independently and optionally -H, -F, -OH, -OCH₃, -N(CH₃)₂, -NH₂, -CN,

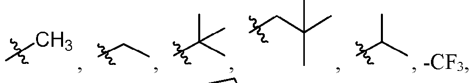

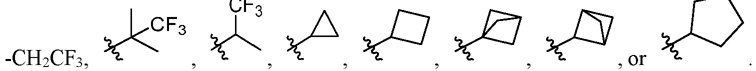

In another preferred embodiment, in the general formula (1), R³ and R⁴ are each independently and optionally -H, -D, -OH, -NH₂, -CN, (C1-C3) alkyl, (C1-C3) haloalkyl, (C2-C4) alkenyl, (C2-C4) alkynyl, (C3-C5) cycloalkyl, (C1-C3) alkoxy, phenyl, (4- to 6-membered) heterocycloalkyl, or (5- to 6-membered) heteroaryl, wherein the (C1-C3) alkyl, (C1-C3) haloalkyl, (C2-C4) alkenyl, (C2-C4) alkynyl, (C3-C5) cycloalkyl, (C1-C3) alkoxy, phenyl, (4- to 6-membered) heterocycloalkyl, or (5- to 6-membered) heteroaryl may be each independently and optionally substituted with 1, 2, 3, or 4 of the following groups: -H, -F, -OH, -CH₂OCH₃, - CH₂N(CH₃)₂, -OCH₃, -N(CH₃)₂, -CN, -C(O)N(CH₃)₂, -NCH₃C(O)CH₃, -NHC(O)CH₃, -NCH₃S(O)₂CH₃, - NHS(O)₂CH₃, -SCH₃, -S(O)₂CH₃, -S(O)₂NH₂, and -S(O)₂N(CH₃)₂; or R³ and R⁴, together with the carbon atom to which they are attached, can form (4- to 6-membered) heterocycloalkyl or (C3-C4) cycloalkyl, wherein the (4- to 6-membered) heterocycloalkyl or (C3-C4) cycloalkyl may be optionally substituted with 1, 2, 3, or 4 of the following groups: -H, -F, 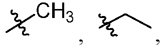 or -OCH₃; or R³ and the adjacent R⁵, together with the atoms to which they are attached, can form a (C3-C6) cycloalkyl or (3- to 6-membered) heterocycloalkyl, wherein the (C3-C6) cycloalkyl or (3- to 6-membered) heterocycloalkyl may be each independently and optionally substituted with 1, 2, 3, or 4 of the following groups: -H, -F, 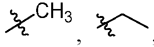 , or -OCH₃; or when R³ and the adjacent R⁵ are both absent, an endocyclic double bond is formed; or R³ and R⁴ together form an oxo.

In another preferred embodiment, in the general formula (1), R⁵ and R⁶ are each independently and optionally -H, -D, -OH, -NH₂, -CN, (C1-C3) alkyl, (C1-C3) haloalkyl, (C2-C4) alkenyl, (C2-C4) alkynyl, (C3-C5) cycloalkyl, (C1-C3) alkoxy, phenyl, (4- to 6-membered) heterocycloalkyl, or (5- to 6-membered) heteroaryl, wherein the (C1-C3) alkyl, (C1-C3) haloalkyl, (C2-C4) alkenyl, (C2-C4) alkynyl, (C3-C5) cycloalkyl, (C1-C3) alkoxy, phenyl, (4- to 6-membered) heterocycloalkyl, or (5- to 6-membered) heteroaryl may be each independently and optionally substituted with 1, 2, 3, or 4 of the following groups: -H, -F, -OH, -CH₂OCH₃, - CH₂N(CH₃)₂, -OCH₃, -N(CH₃)₂, -NH₂, -CN, -C(O)N(CH₃)₂, -NCH₃C(O)CH₃, -NHC(O)CH₃, - NCH₃S(O)₂CH₃, -NHS(O)₂CH₃, -SCH₃, -S(O)₂CH₃, -S(O)₂NH₂, and -S(O)₂N(CH₃)₂; or R⁵ and R⁶, together with the carbon atom to which they are attached, can form a (4- to 6-membered) heterocycloalkyl or (C3-C4) cycloalkyl, wherein the (4- to 6-membered) heterocycloalkyl or (C3-C4) cycloalkyl may be optionally substituted with 1, 2, 3, or 4 of the following groups: -H, -F, 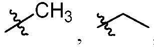 , or -OCH₃; or R⁵ and R⁶ together form an oxo.

In another preferred embodiment, in the general formula (1), ring A is phenyl or (5- to 6-membered) heteroaryl.

In another preferred embodiment, in the general formula (1), ring A is phenyl, pyridinyl, furanyl, thienyl, thiazolyl, imidazolyl, or oxazolyl, preferably phenyl and pyridinyl.

In another preferred embodiment, in the general formula (1), ring A is:

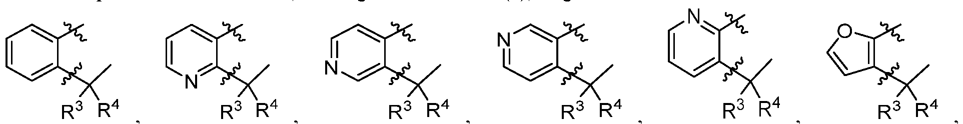

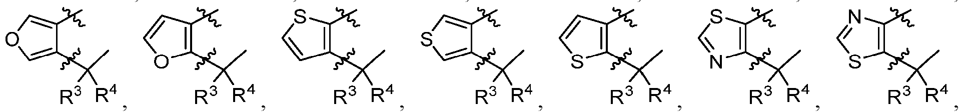

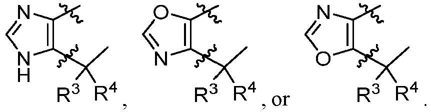

In another preferred embodiment, in the general formula (1), each R¹ is independently and optionally: -H, -F, -Cl, -Br, -I, -OH, -NO₂, -N(CH₃)₂, -NH₂, -CH₂OCH₃, -CH₂N(CH₃)₂, -CN, (C1-C3) alkyl, (C1-C3) haloalkyl, (C1-C3) alkoxy, (C2-C4) alkenyl, (C2-C4) alkynyl, (C3-C6) cycloalkyl, -C(O)N(CH₃)₂, -NCH₃C(O)CH₃, - NHC(O)CH₃, -NCH₃S(O)₂CH₃, -NHS(O)₂CH₃, -SCH₃, -S(O)₂CH₃, -S(O)₂NH₂, and -S(O)₂N(CH₃)₂, wherein the (C1-C3) alkyl, (C1-C3) haloalkyl, (C1-C3) alkoxy, (C2-C4) alkenyl, (C2-C4) alkynyl, or (C3-C6) cycloalkyl may be each independently and optionally substituted with 1, 2, 3, or 4 of the following groups: - H, -F, -OH, -CH₂OCH₃, -CH₂N(CH₃)₂, -OCH₃, -N(CH₃)₂, -NH₂, -CN,

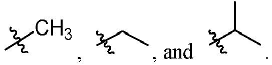

In another preferred embodiment, in the general formula (1), each R¹ is independently: -H, -F, -Cl, -Br, -I, - OH, -NO₂, -N(CH₃)₂, -NH₂, -CH₂OCH₃, -CH₂N(CH₃)₂, -CN, -C(O)N(CH₃)₂, -NCH₃C(O)CH₃, -NHC(O)CH₃, -NCH₃S(O)₂CH₃, -NHS(O)₂CH₃, -SCH₃, -S(O)₂CH₃, -S(O)₂NH₂, -S(O)₂N(CH₃)₂, 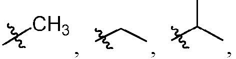 -CF₃, -CH₂CF₃, 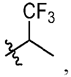 -OCH₃, -OCH₂CH₃, -OCH(CH₃)₂,

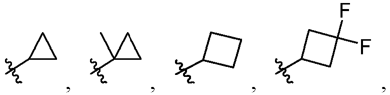

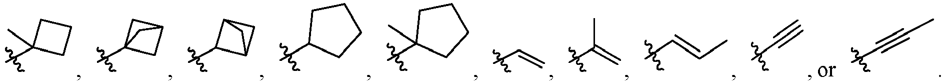

In another preferred embodiment, in the general formula (1), ring B is (C5-C6) cycloalkyl or (5- to 6-membered) heterocycloalkyl.

In another preferred embodiment, in the general formula (1), ring B is (C5-C6) cycloalkyl or (5- to 6-membered) heterocycloalkyl containing 1 ring heteroatom independently selected from nitrogen, oxygen, or sulfur.

In another preferred embodiment, in the general formula (1), the structural unit 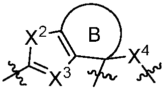 is: 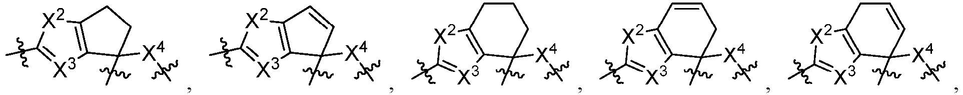 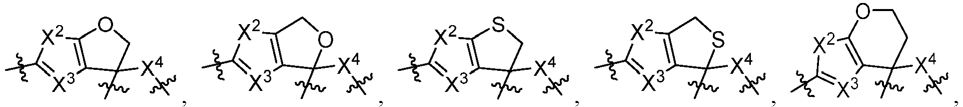 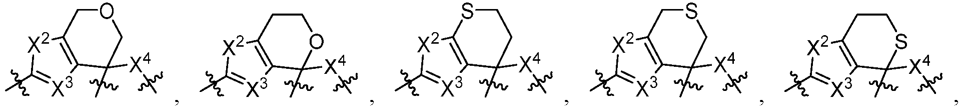 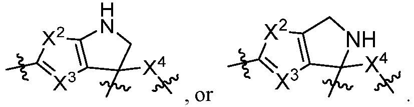 In another preferred embodiment, in the general formula (1), the structural unit 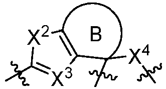 is: 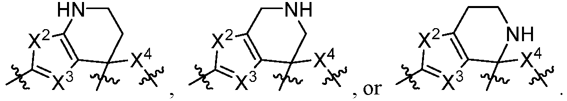 In another preferred embodiment, in the general formula (1), each R² is independently and optionally: -H, -F, -Cl, -Br, -I, -OH, -N(CH₃)₂, -NH₂, -CN, (C1-C3) alkyl, (C1-C3) haloalkyl, (C1-C3) alkoxy, (C2-C4) alkenyl, (C2-C4) alkynyl, or (C3-C5) cycloalkyl; or two R² on a same carbon atom, together with the carbon atom to which they are attached, can form (4- to 5-membered) heterocycloalkyl or (C3-C5) cycloalkyl, wherein the (4- to 5-membered) heterocycloalkyl or (C3-C5) cycloalkyl may be optionally substituted with 1, 2, 3, or 4 of the following groups: -H, -F, 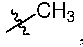 and -OCH₃; or two R² on a same carbon atom together form an oxo. In another preferred embodiment, in the general formula (1), each R² is independently and optionally: -H, -F, -Cl, -Br, -I, -OH, -N(CH₃)₂, -NH₂, -CN, 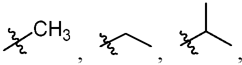 -CF₃, -CH₂CF₃, 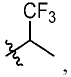 -OCH₃, -

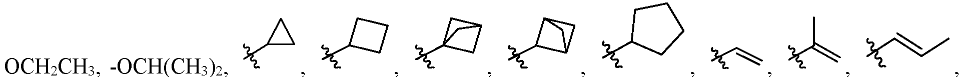

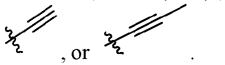

In another preferred embodiment, in the general formula (1), the structural unit 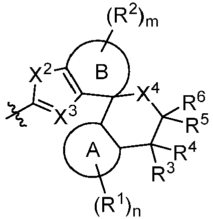 is:

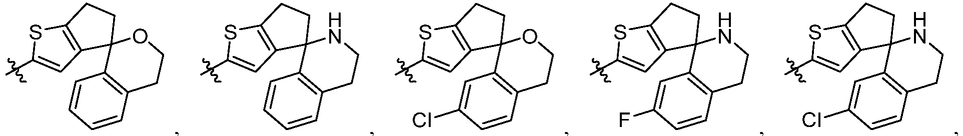

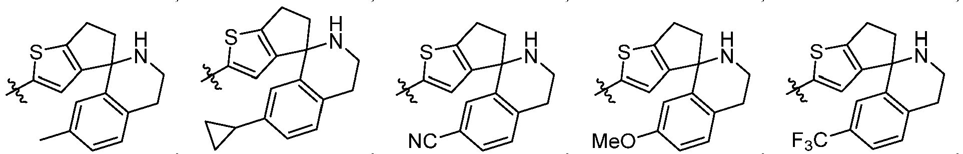

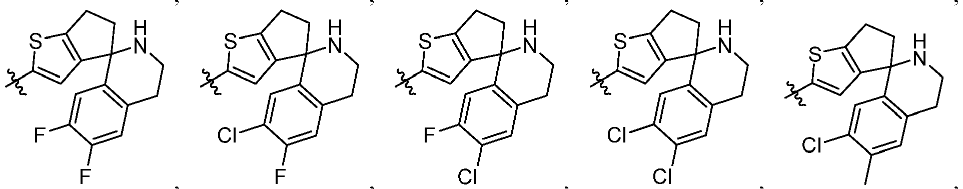

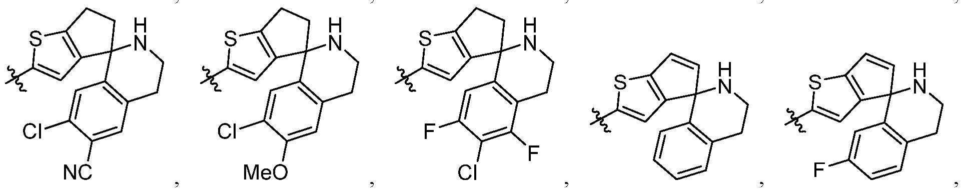

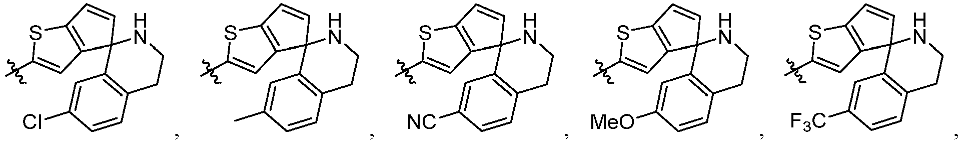

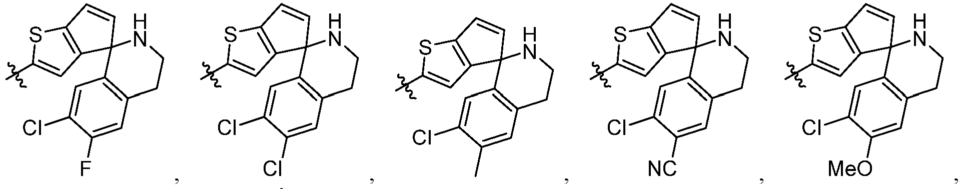

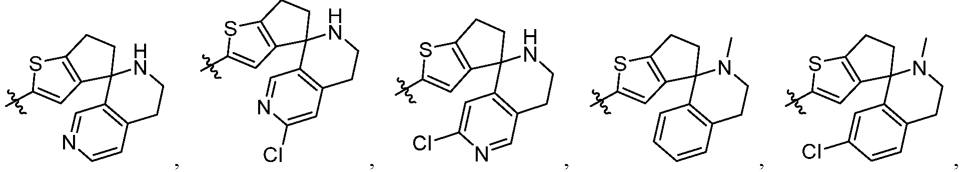

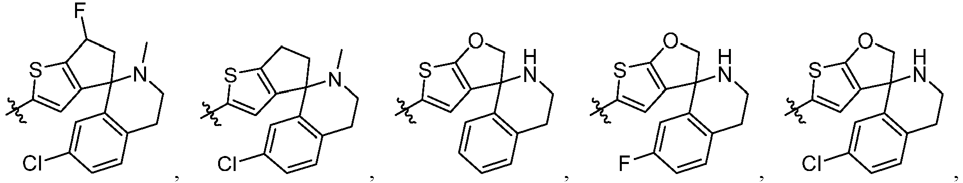

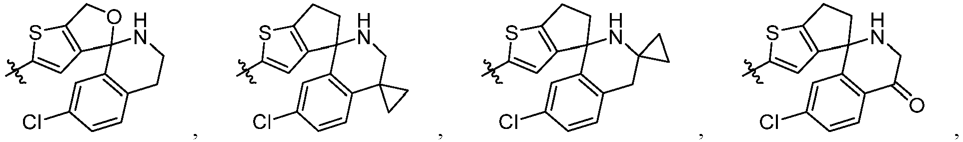

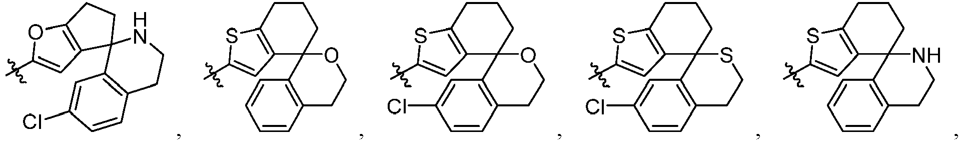

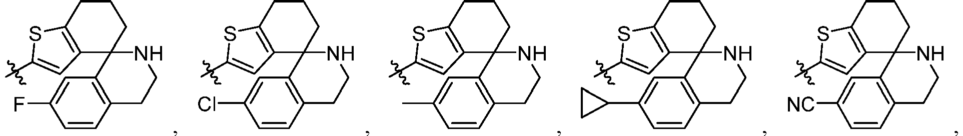

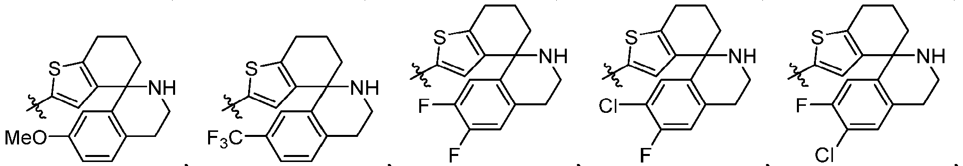

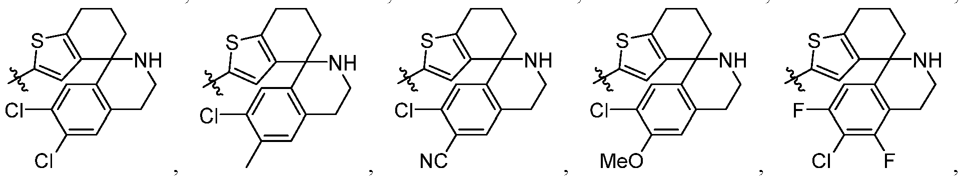

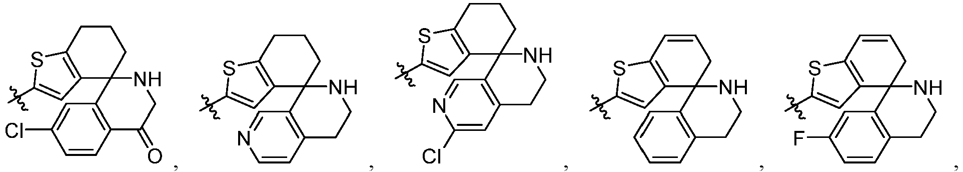

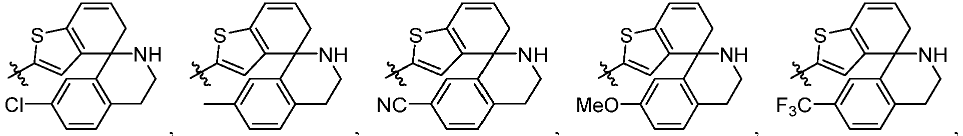

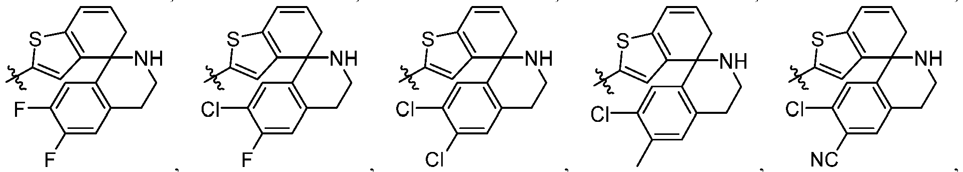

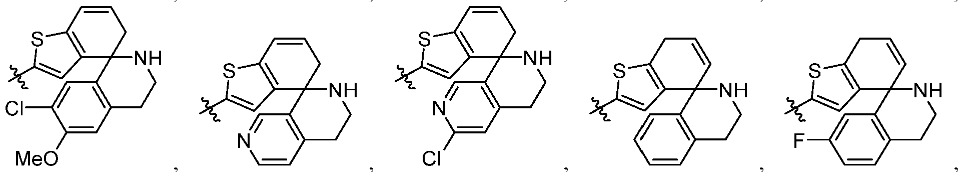

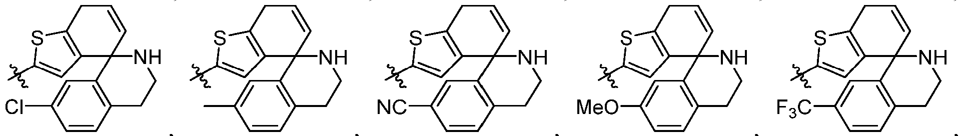

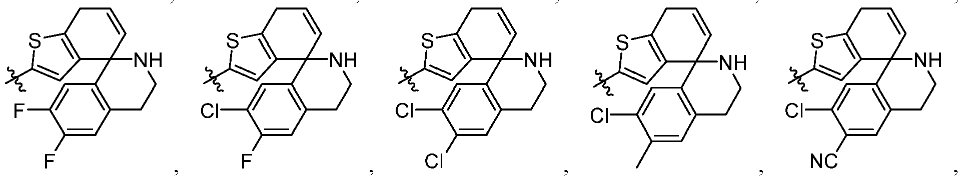

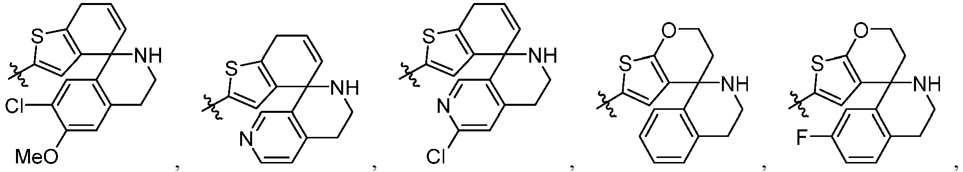

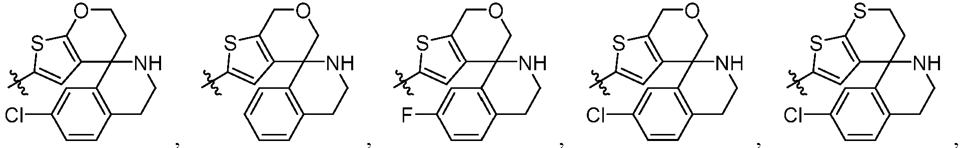

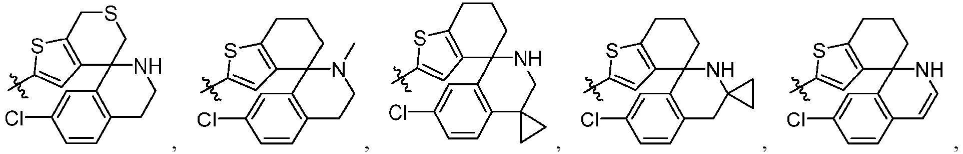

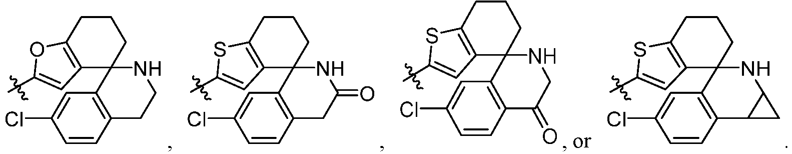

In another preferred embodiment, in the general formula (1), the structural unit 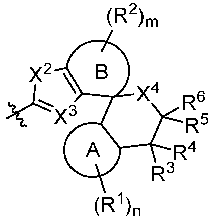 is:

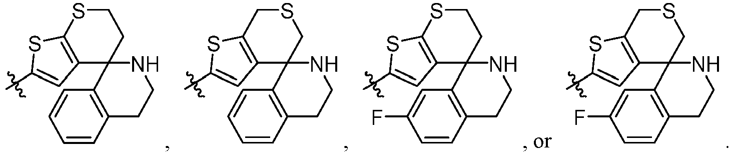

In another preferred embodiment, in the general formula (1), the structural unit 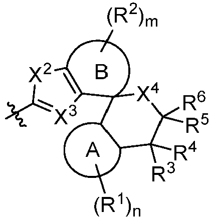 is:

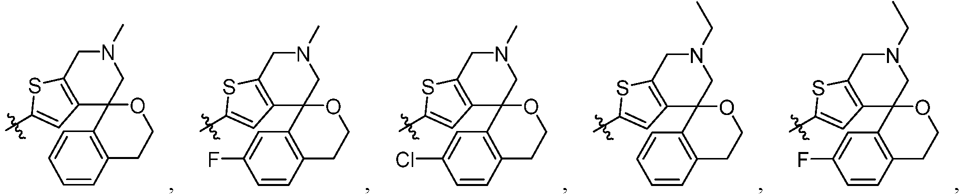

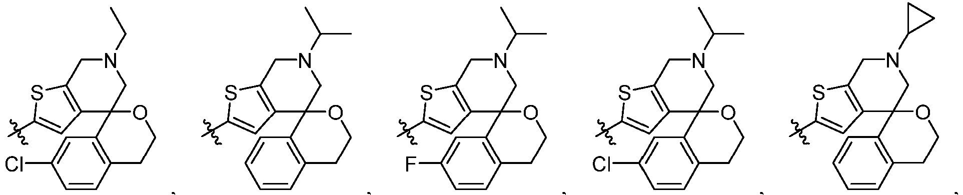

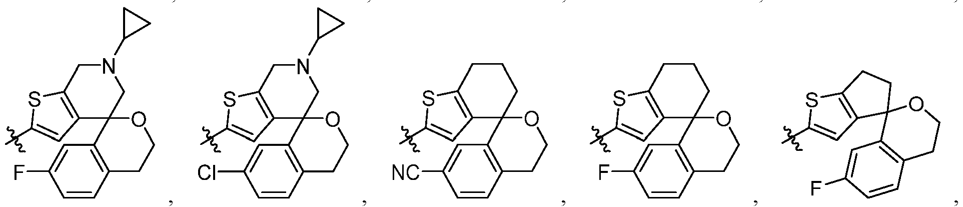

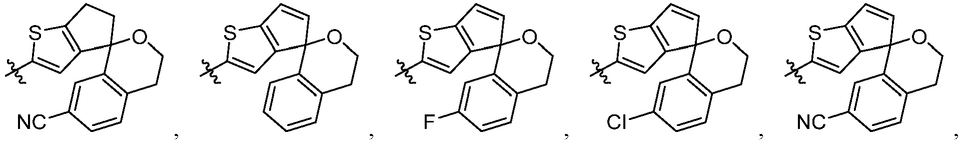

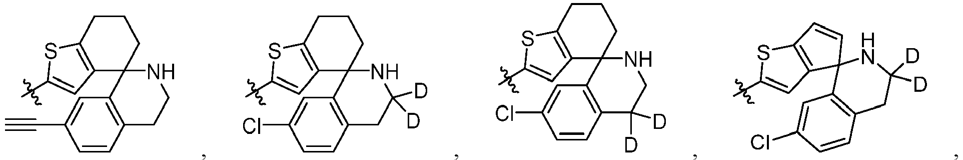

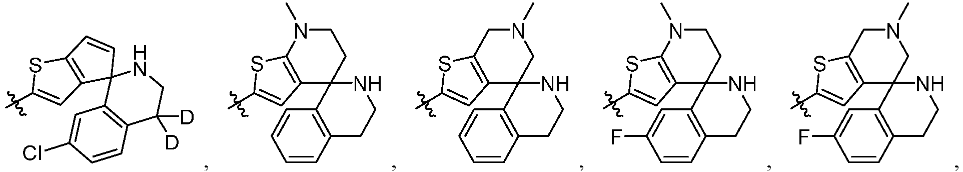

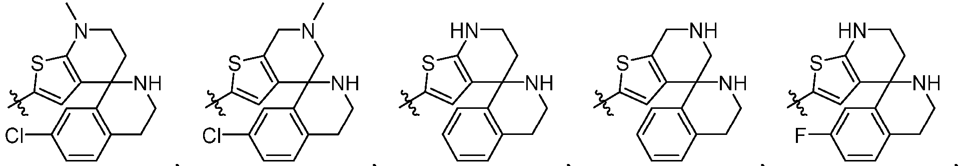

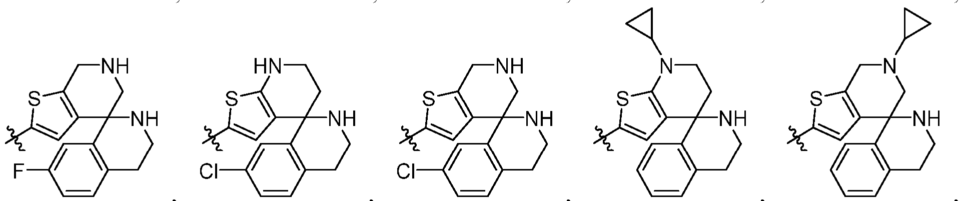

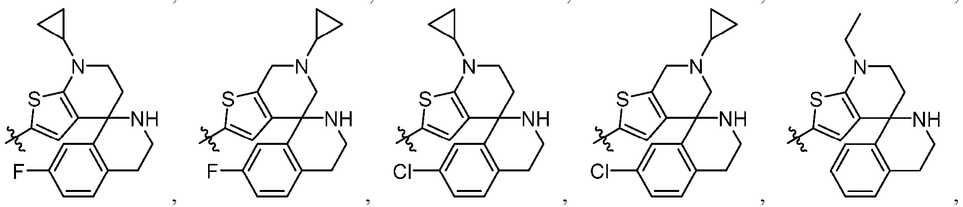

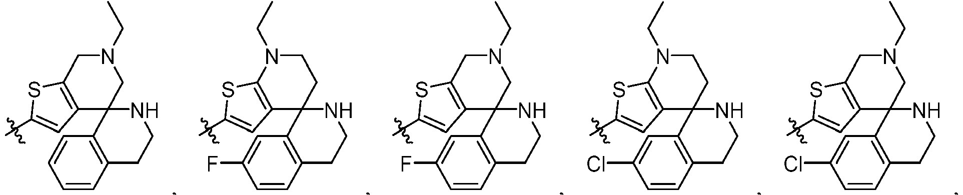

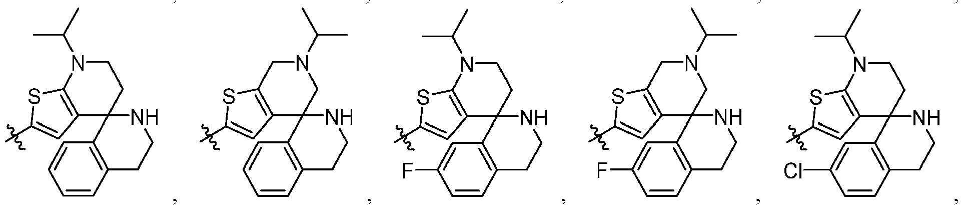

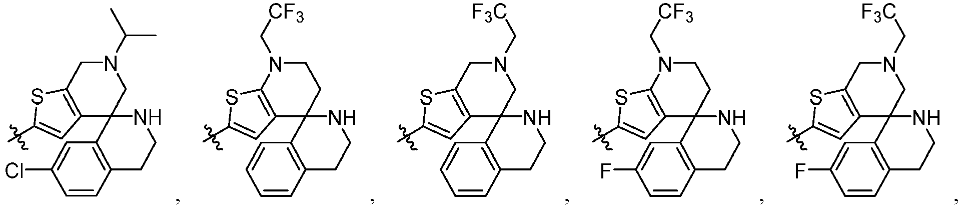

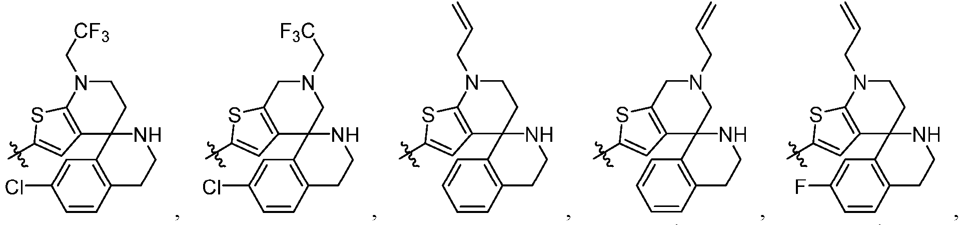

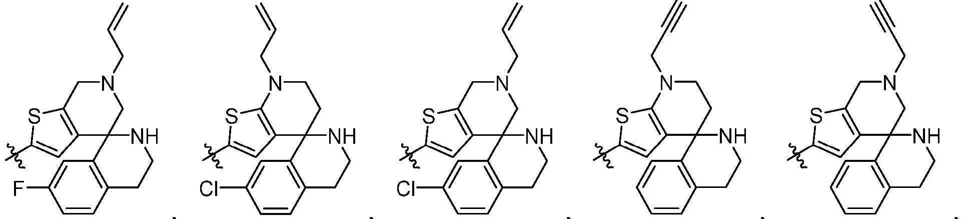

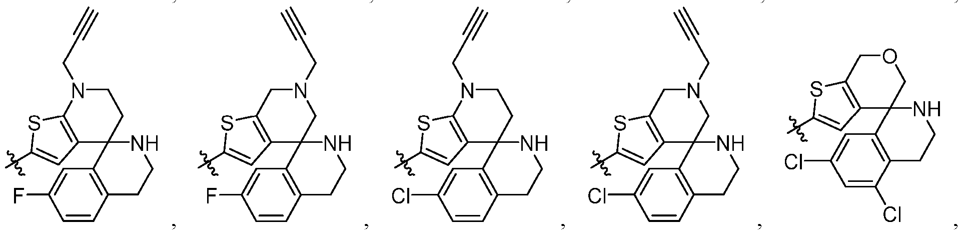

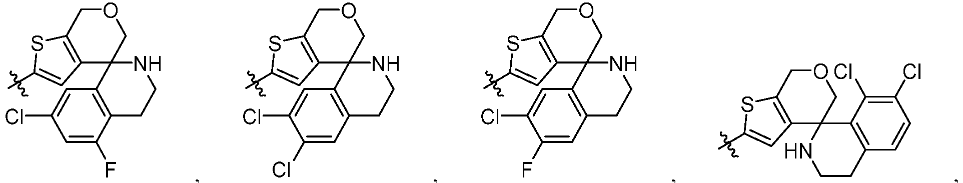

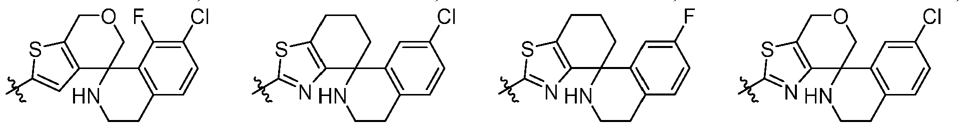

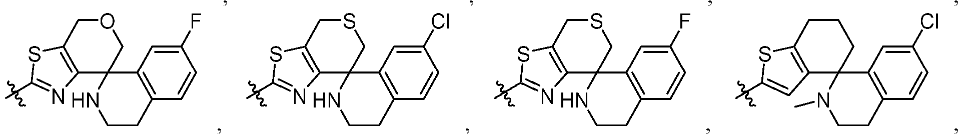

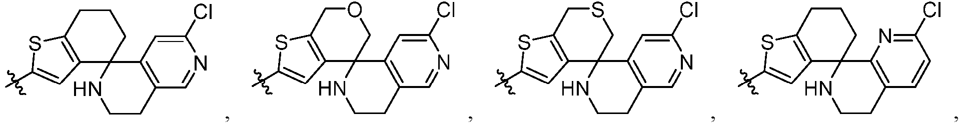

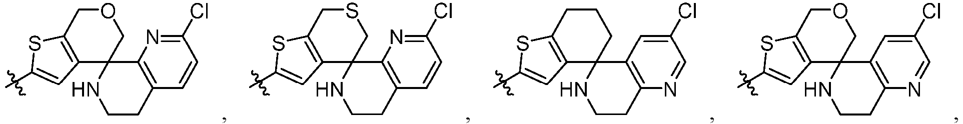

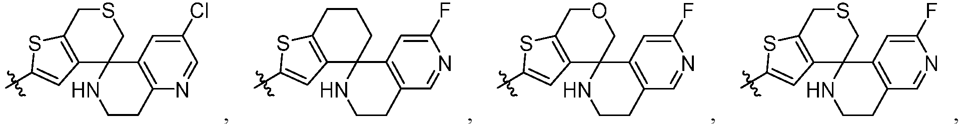

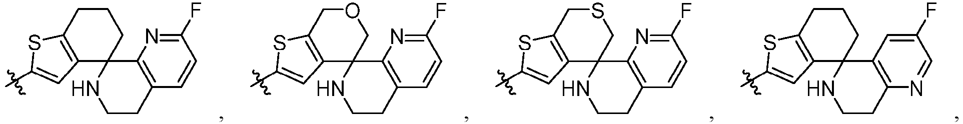

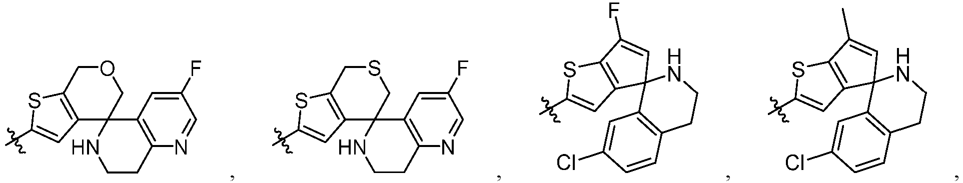

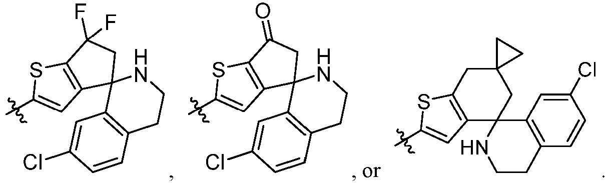

In another preferred embodiment, in the general formula (1), the general formula (1) has a structure as shown in the general formula (1a): 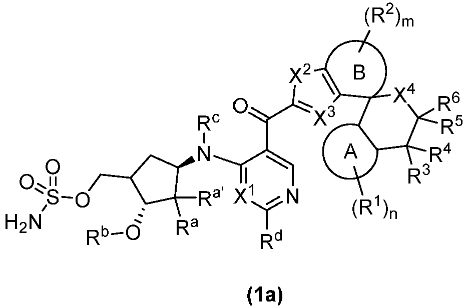 wherein ring A, ring B, R¹, R², R³, R⁴, R⁵, R⁶, R^{a}, R^{a'}, R^{b}, R^{c}, R^{d}, X¹, X², X³, X⁴, m, and n are as previously defined and exemplified in the detailed examples.

In another preferred embodiment, in the general formula (1), the general formula (1) has a structure as shown in the general formula (2a): 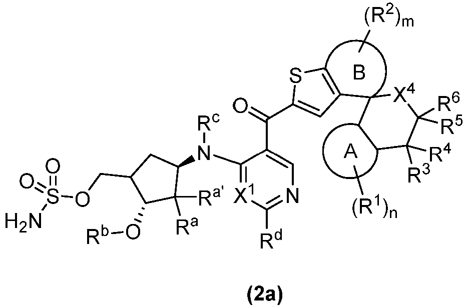 wherein ring A, ring B, R¹, R², R³, R⁴, R⁵, R⁶, R^{a}, R^{a'}, R^{b}, R^{c}, R^{d}, X¹, X⁴, m, and n are as previously defined and exemplified in the detailed examples.

In another preferred embodiment, in the general formula (1), the general formula (1) has a structure as shown in the general formula (3a): 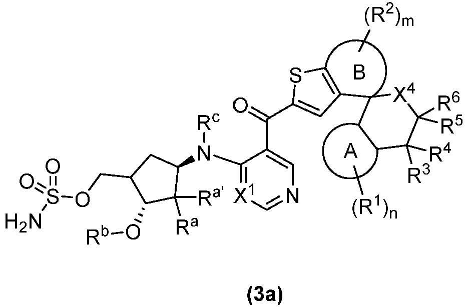 wherein ring A, ring B, R¹, R², R³, R⁴, R⁵, R⁶, R^{a}, R^{a'}, R^{b}, R^{c}, X¹, X⁴, m, and n are as previously defined and exemplified in the detailed examples.

In another preferred embodiment, in the general formula (1), the general formula (1) has a structure as shown in the general formula (4a): 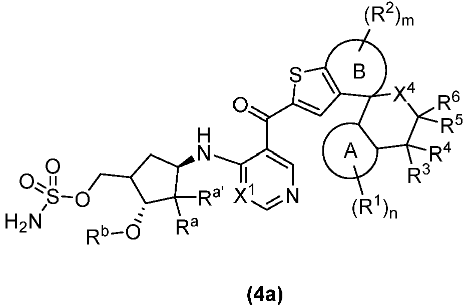 wherein ring A, ring B, R¹, R², R³, R⁴, R⁵, R⁶, R^{a}, R^{a'}, R^{b}, X¹, X⁴, m, and n are as previously defined and exemplified in the detailed examples.

In another preferred embodiment, in the general formula (1), the general formula (1) has a structure as shown in the general formula (5a): 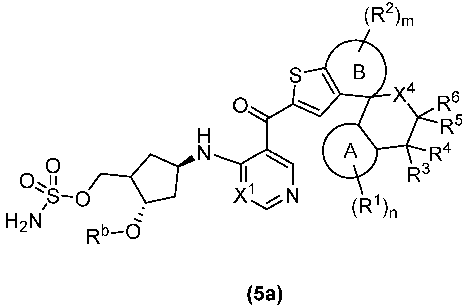 wherein ring A, ring B, R¹, R², R³, R⁴, R⁵, R⁶, R^{b}, X¹, X⁴, m, and n are as previously defined and exemplified in the detailed examples.

In another preferred embodiment, in the general formula (1), the general formula (1) has a structure as shown in the general formula (6a): 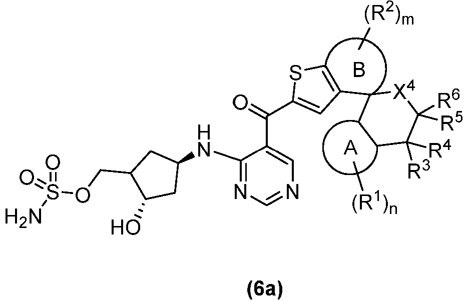 wherein ring A, ring B, R¹, R², R³, R⁴, R⁵, R⁶, X⁴, m, and n are as previously defined and exemplified in the detailed examples.

In another preferred embodiment, in the general formula (1), the general formula (1) has a structure as shown in the general formula (7a) or (7b): 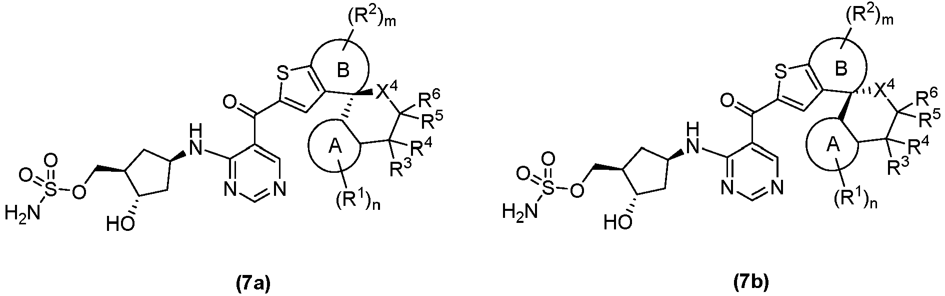 wherein ring A, ring B, R¹, R², R³, R⁴, R⁵, R⁶, X⁴, m, and n are as previously defined and exemplified in the detailed examples.

In another specific embodiment of the present disclosure, the compound of general formula (1) has one of the following structures:

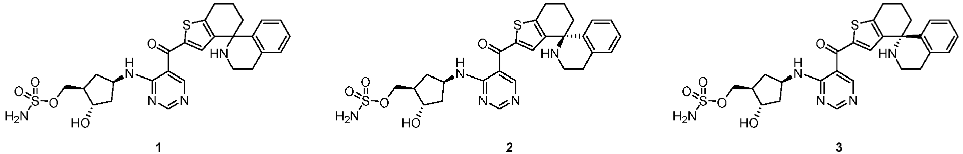

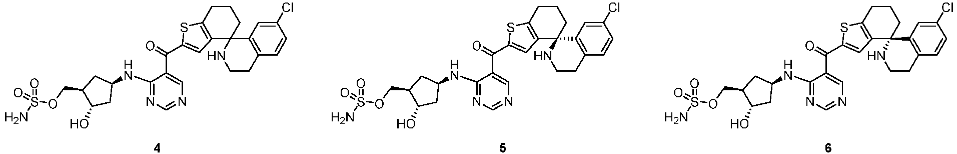

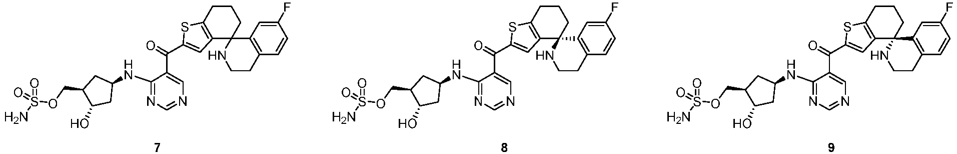

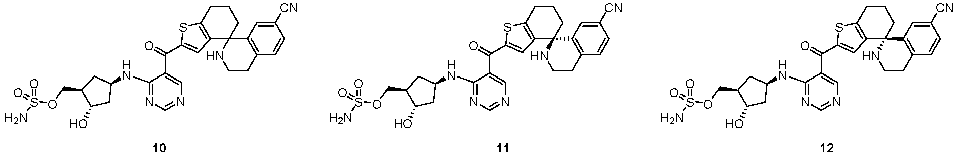

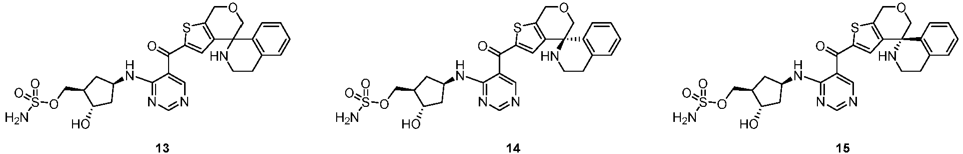

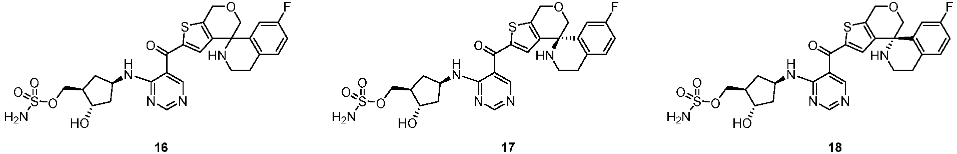

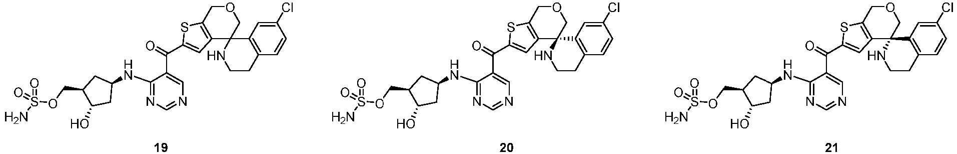

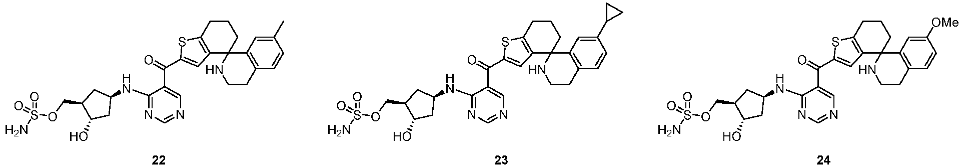

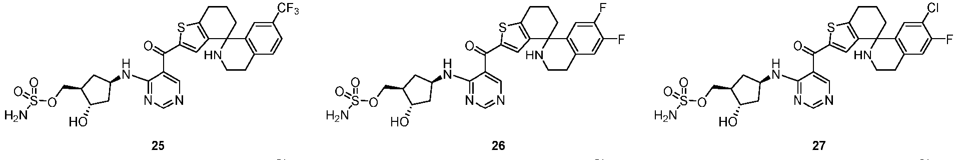

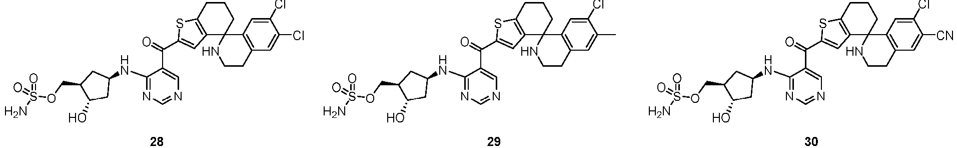

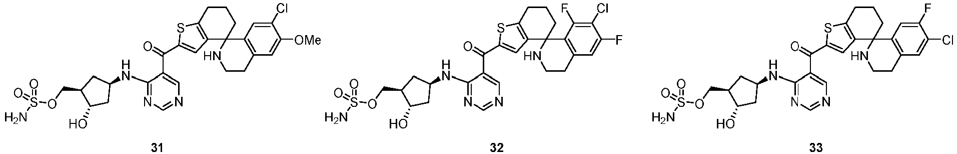

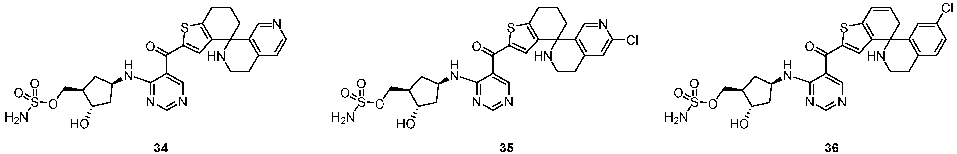

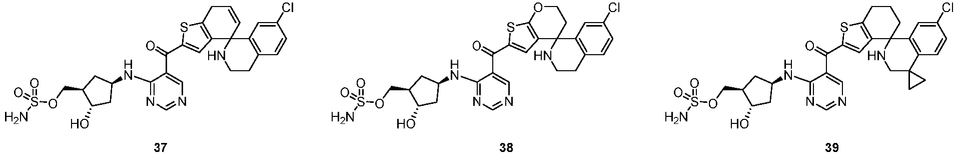

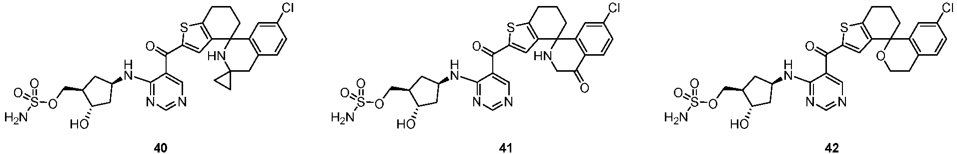

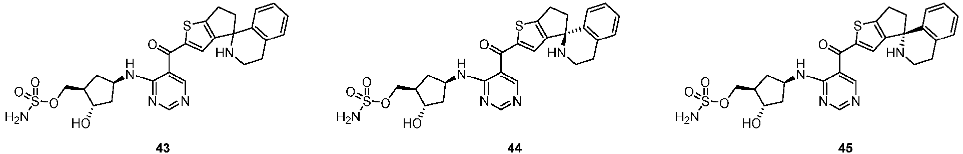

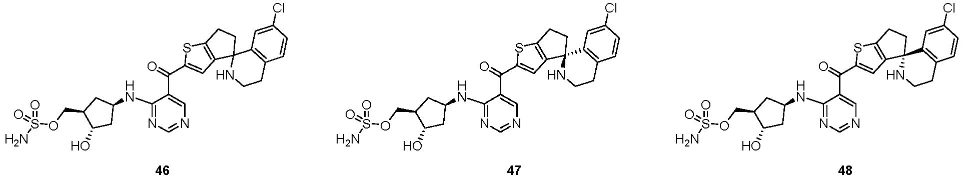

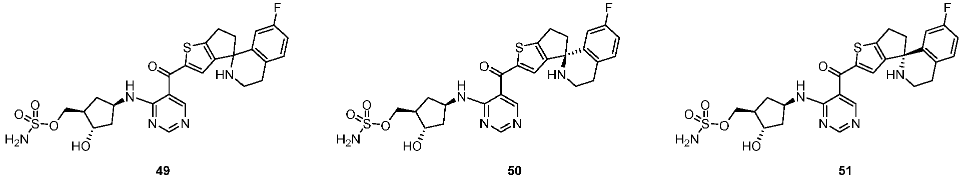

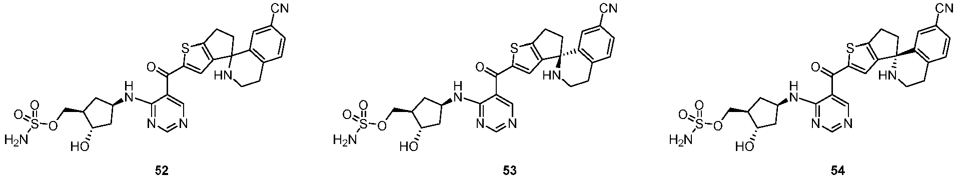

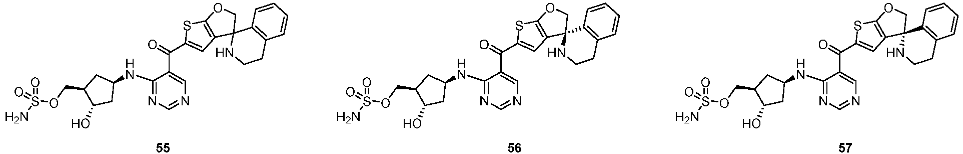

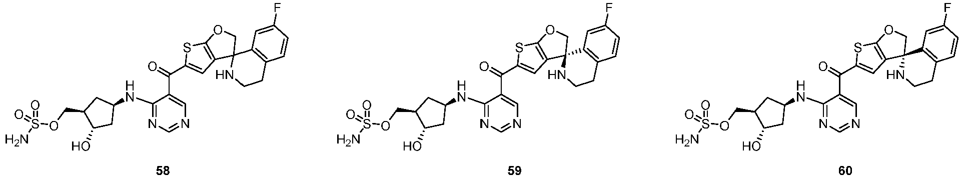

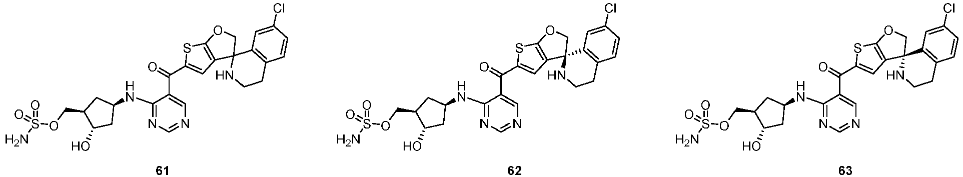

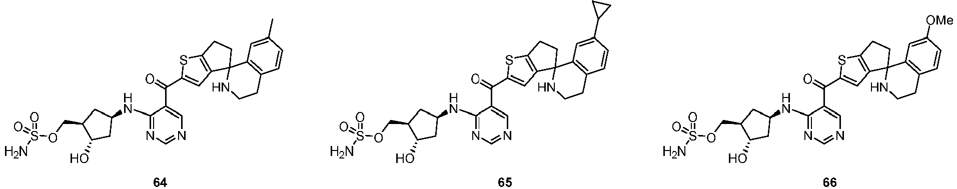

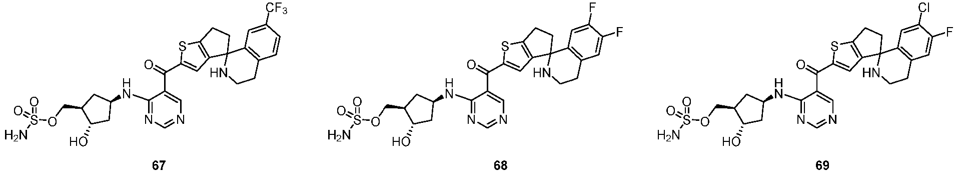

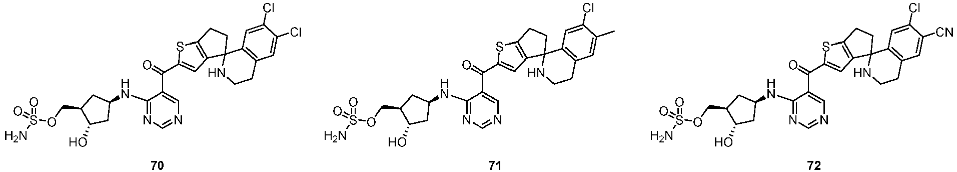

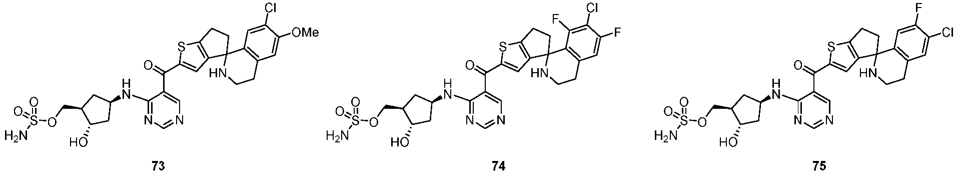

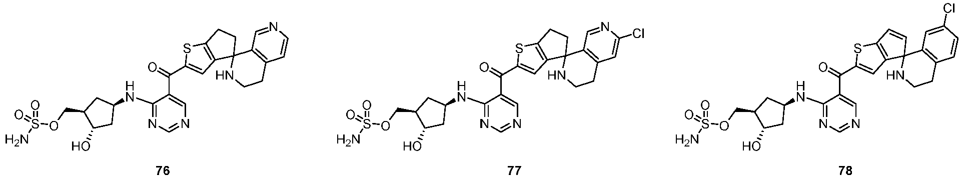

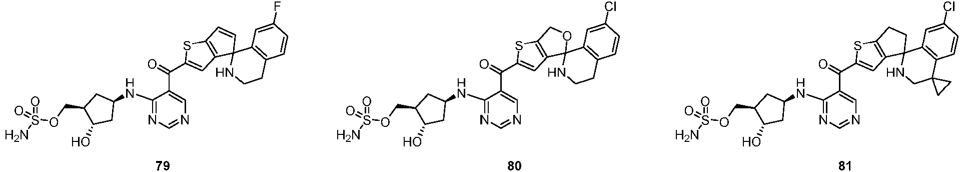

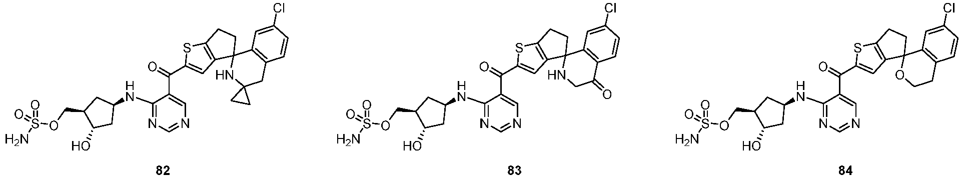

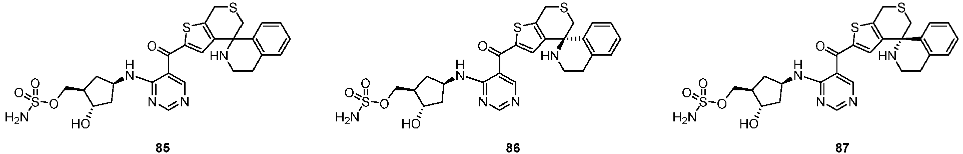

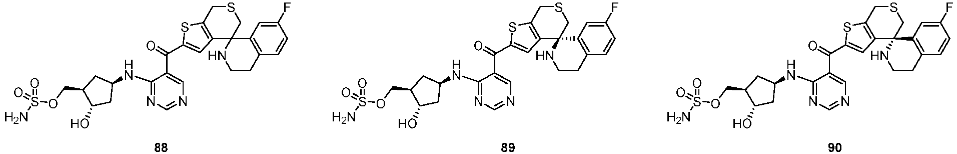

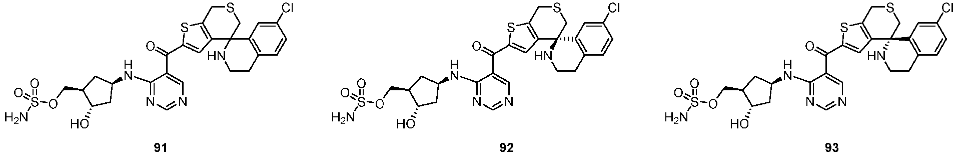

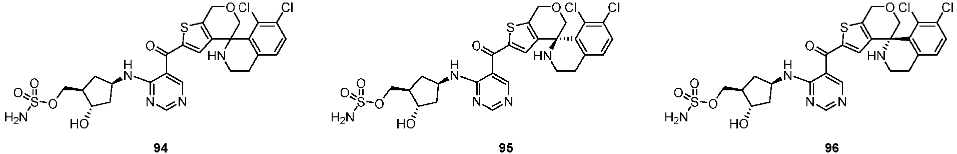

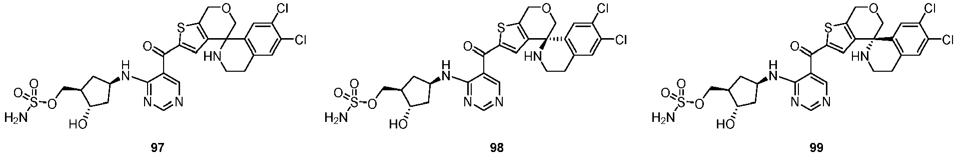

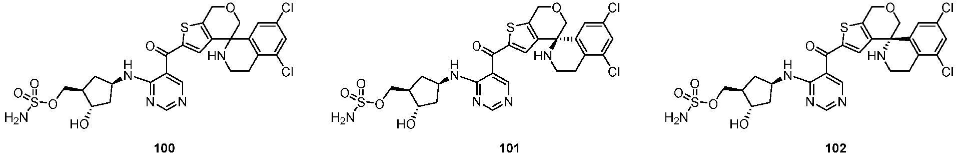

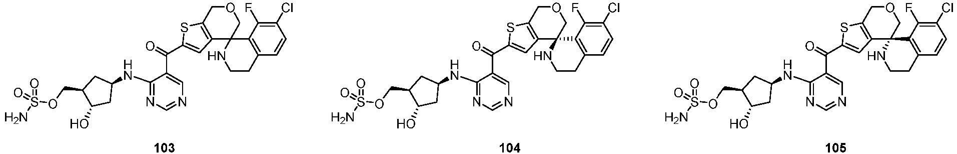

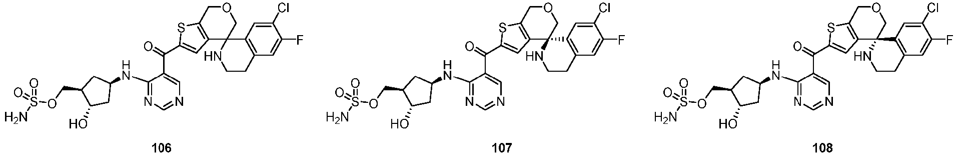

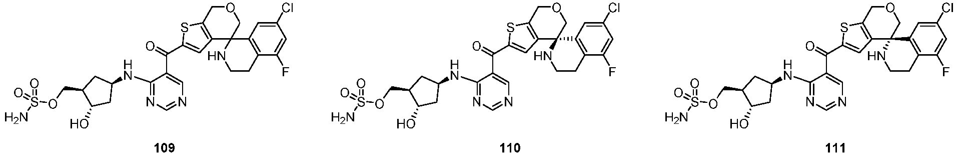

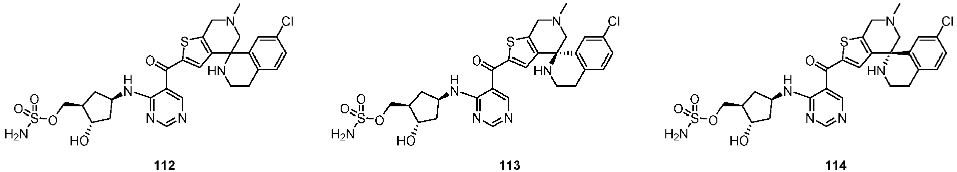

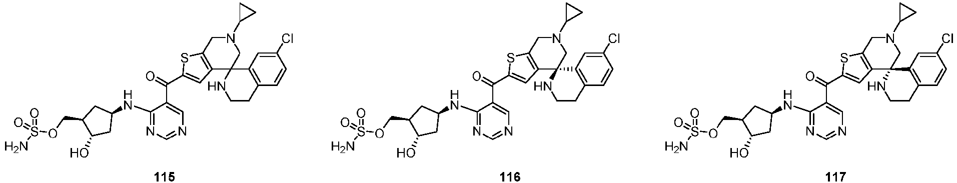

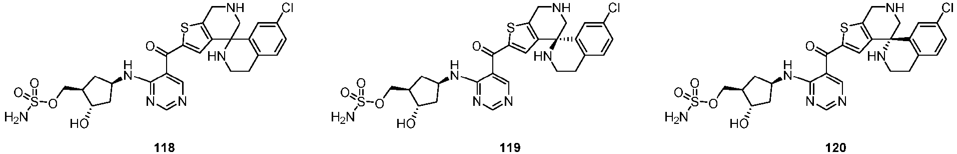

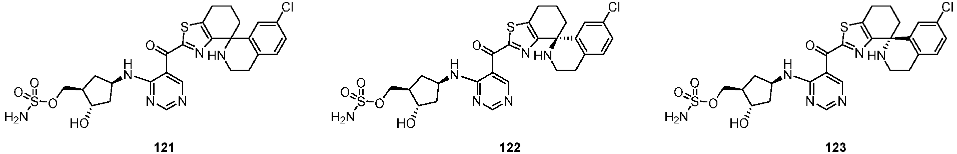

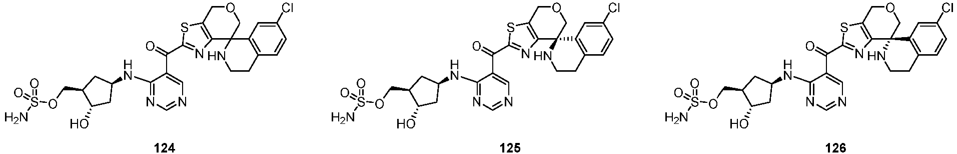

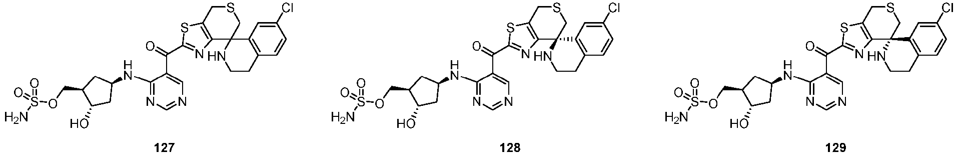

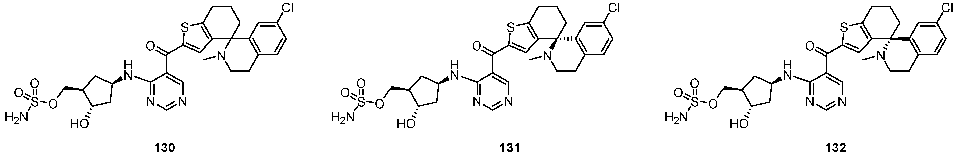

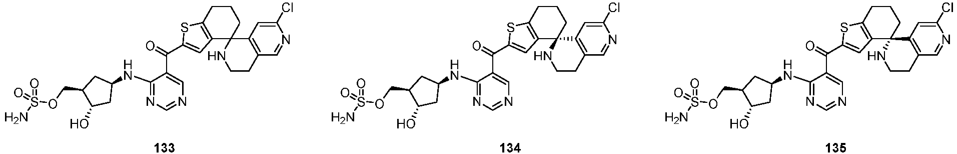

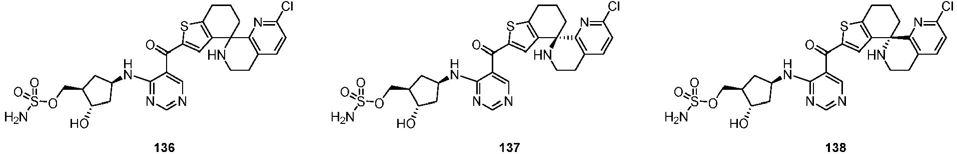

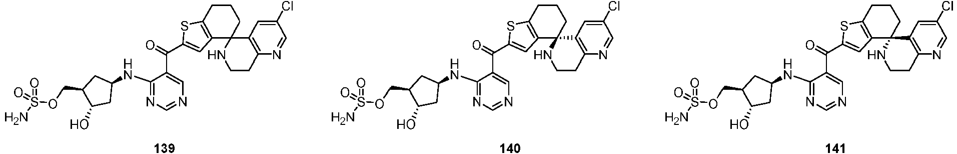

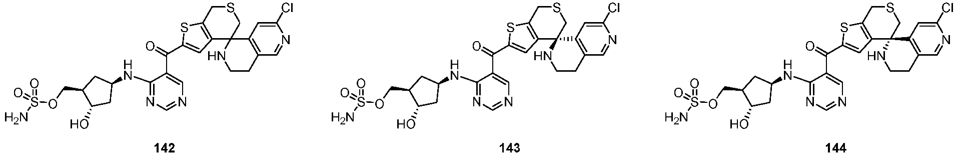

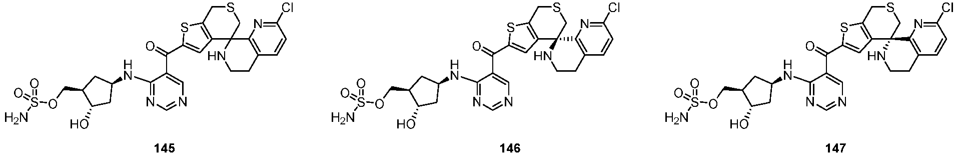

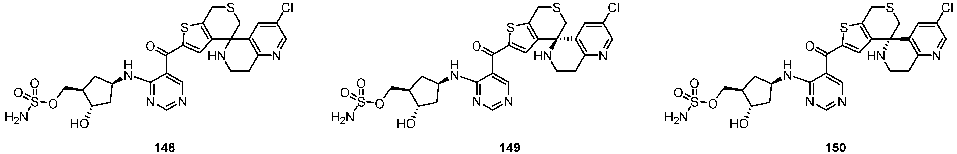

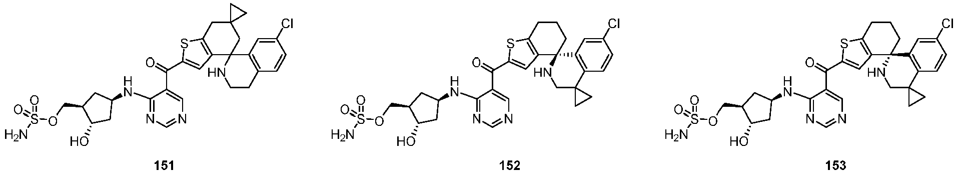

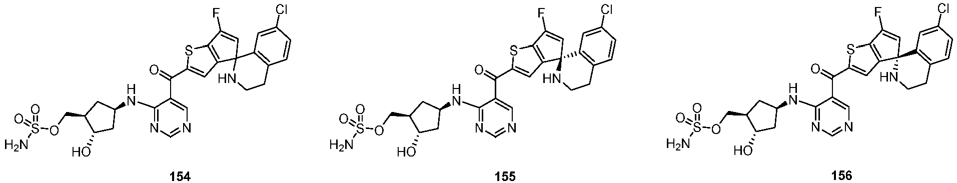

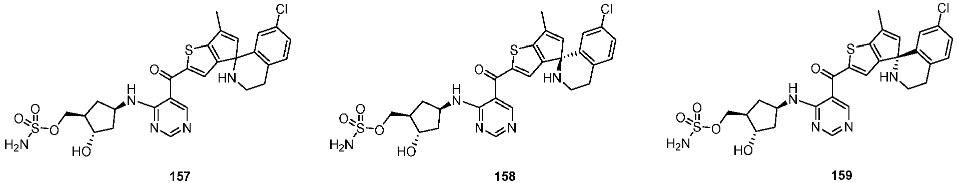

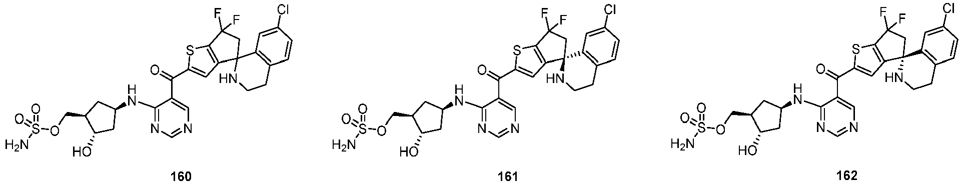

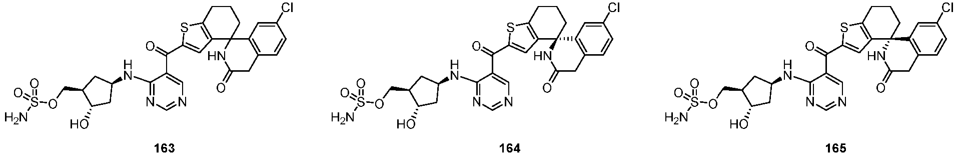

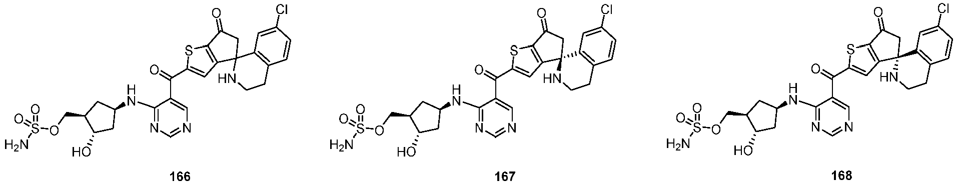

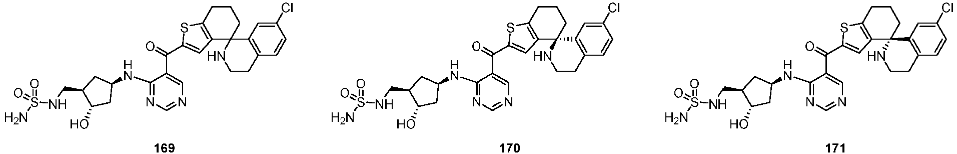

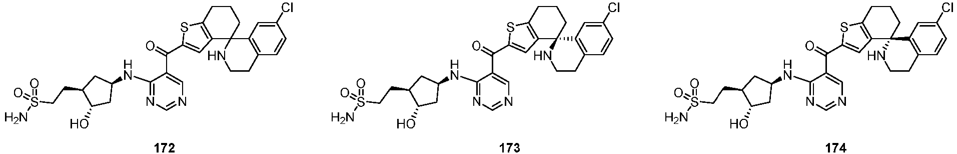

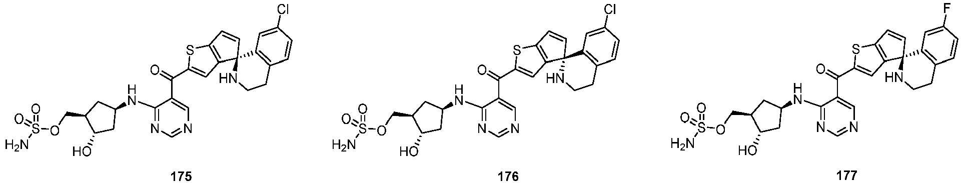

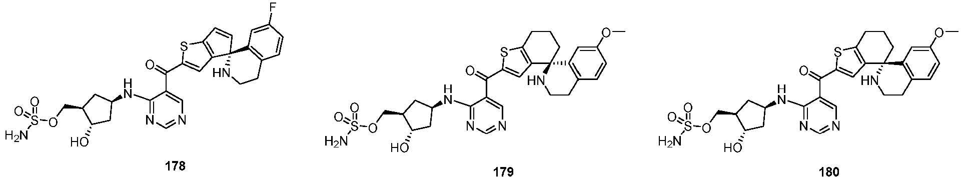

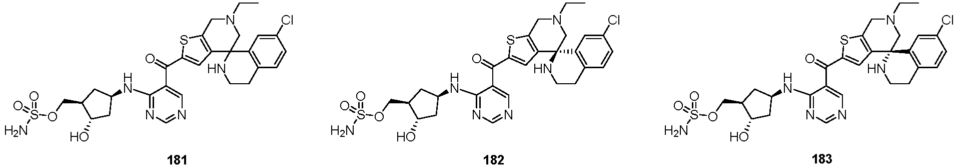

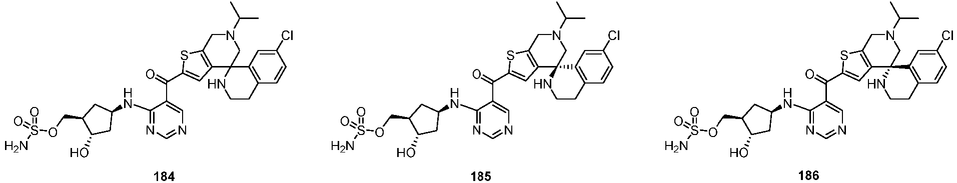

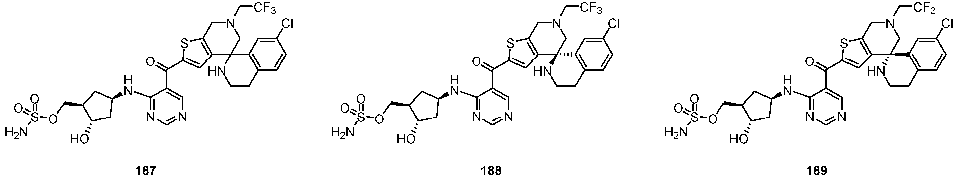

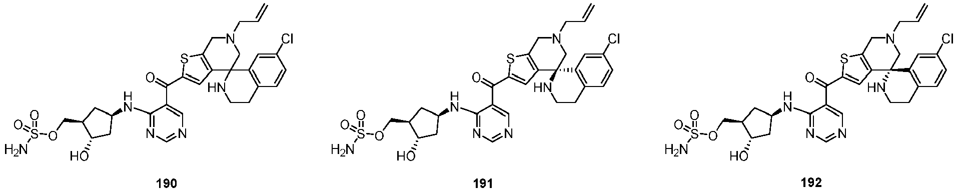

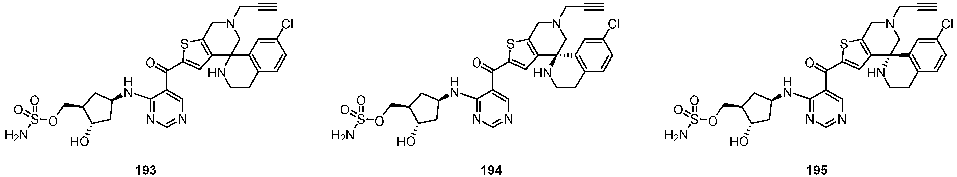

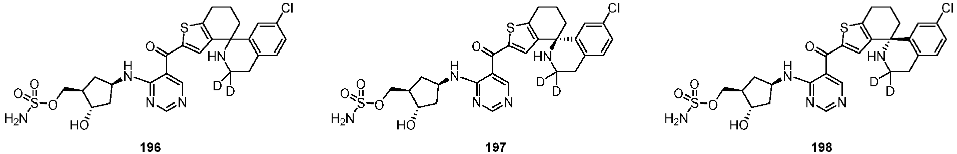

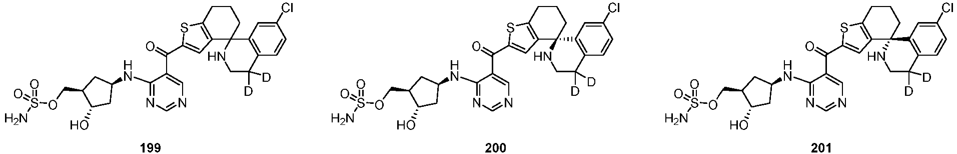

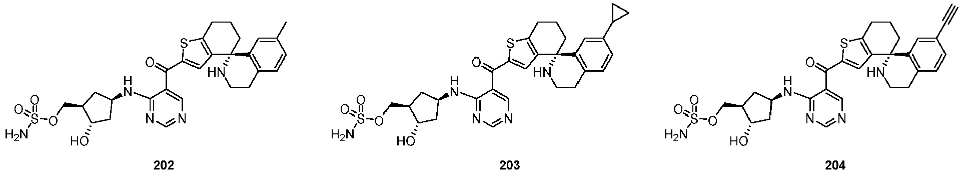

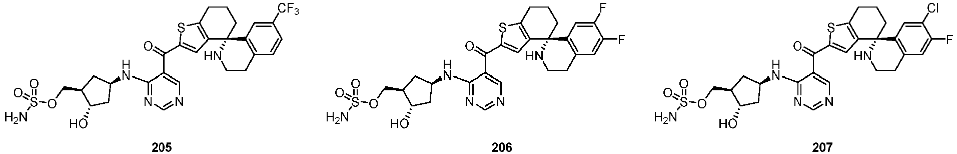

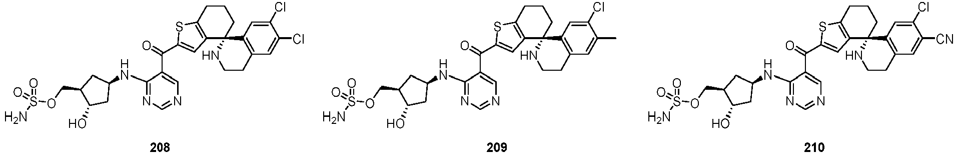

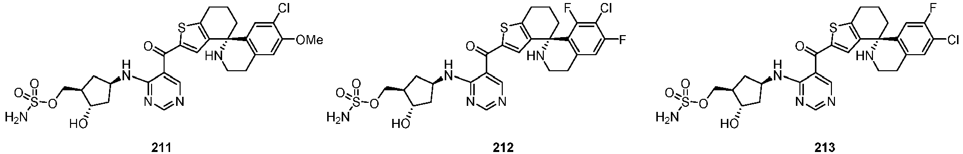

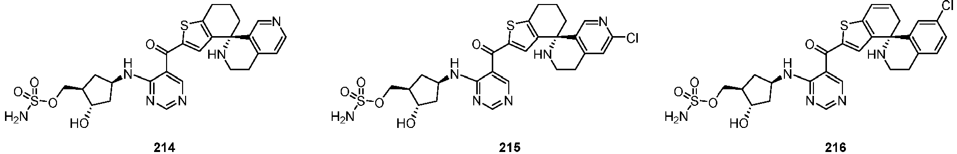

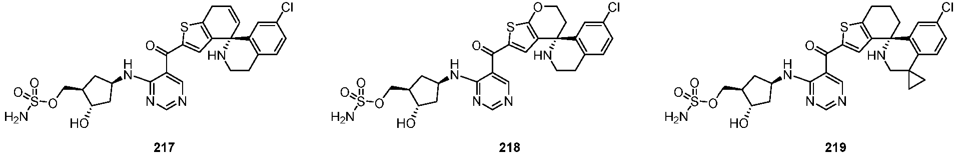

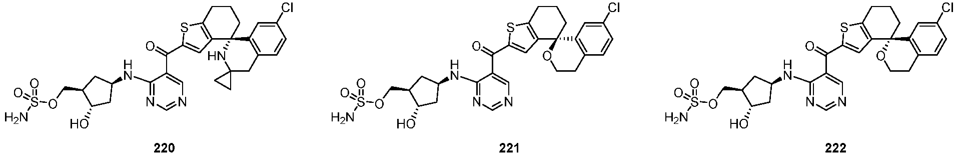

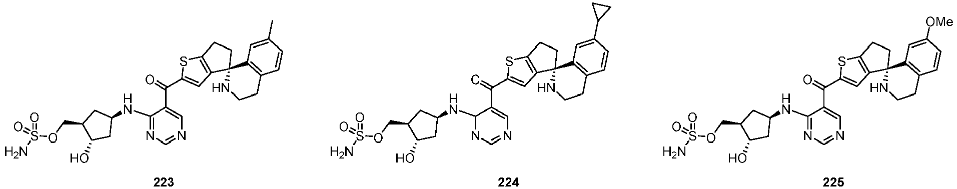

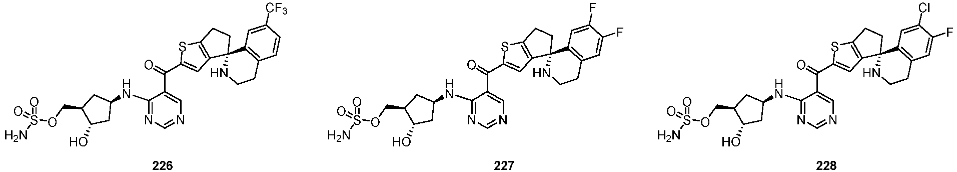

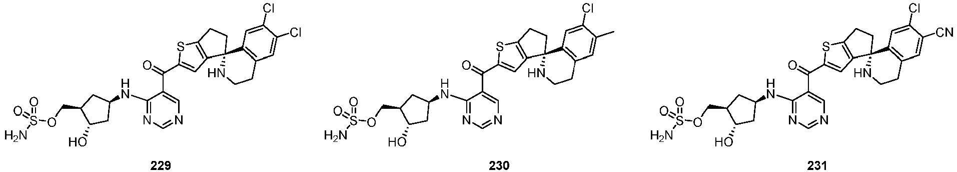

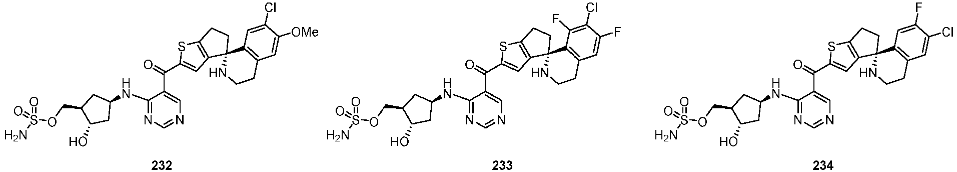

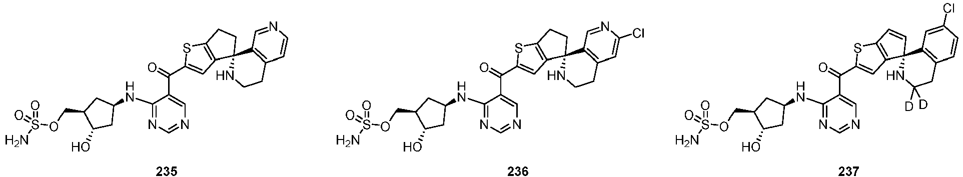

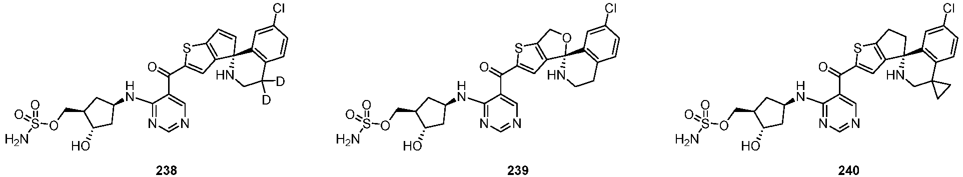

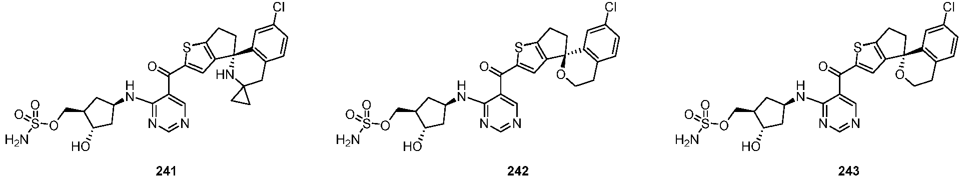

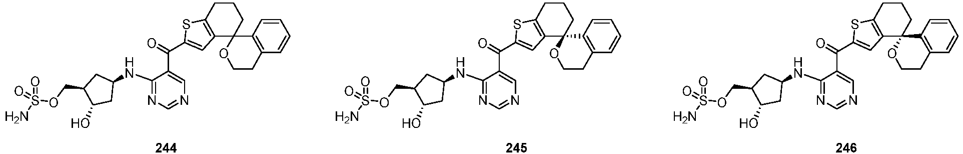

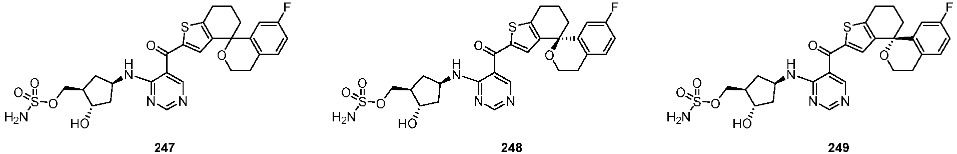

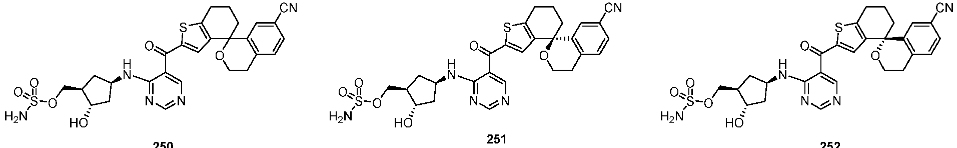

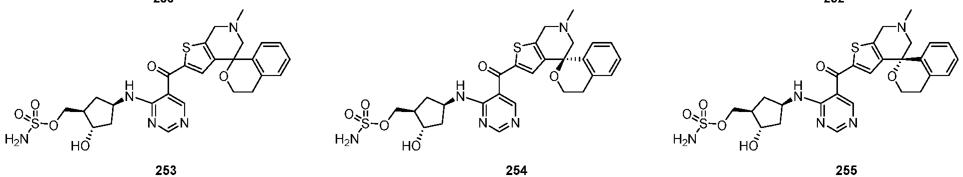

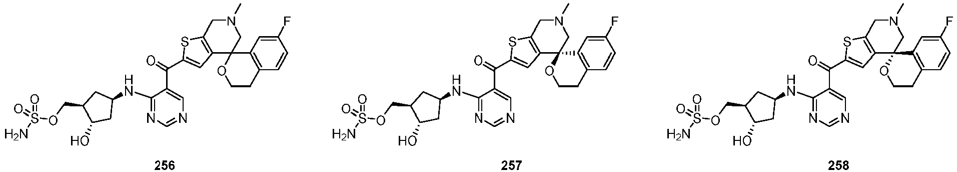

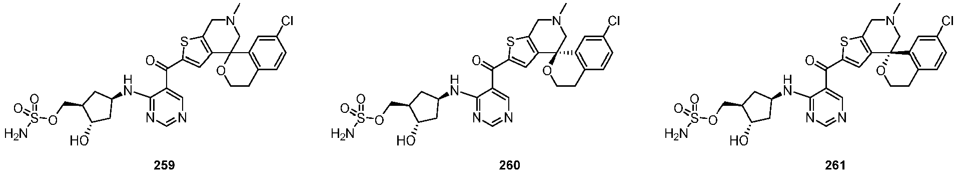

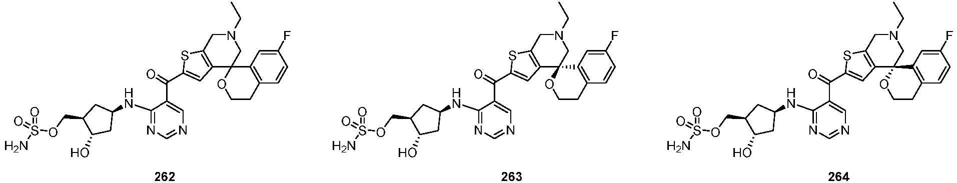

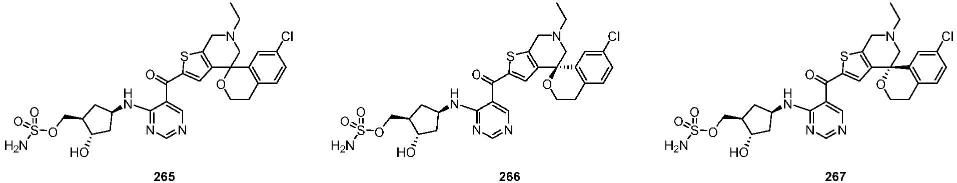

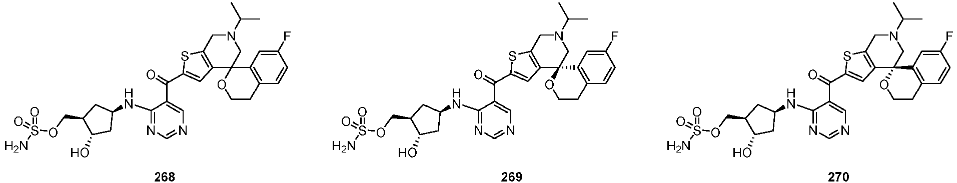

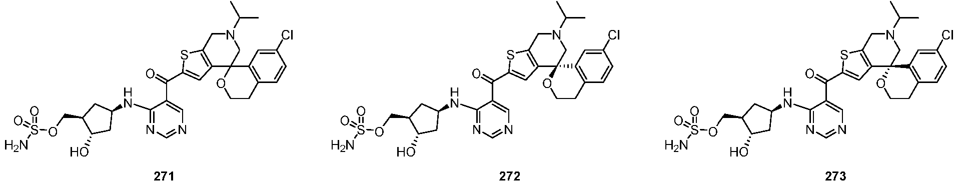

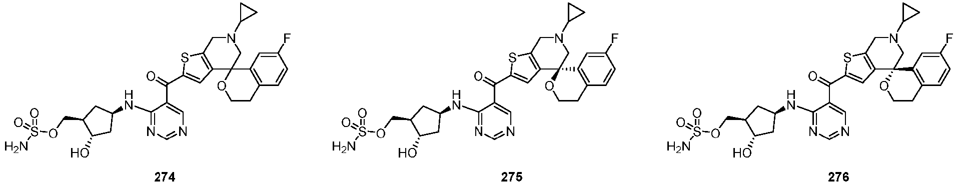

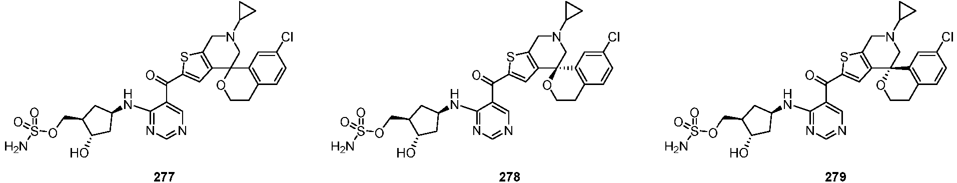

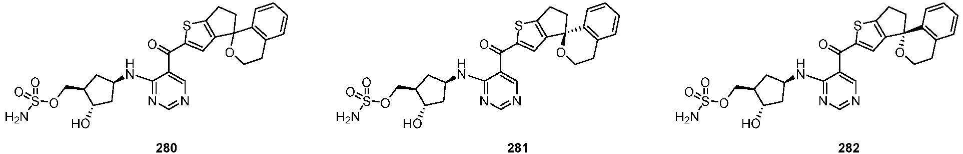

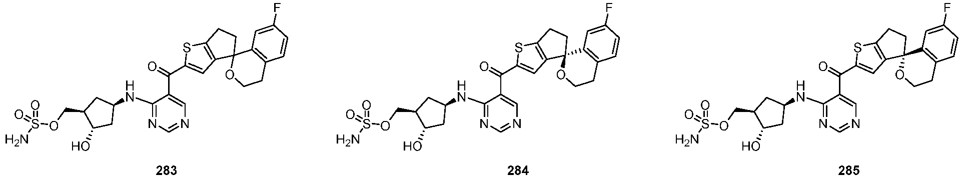

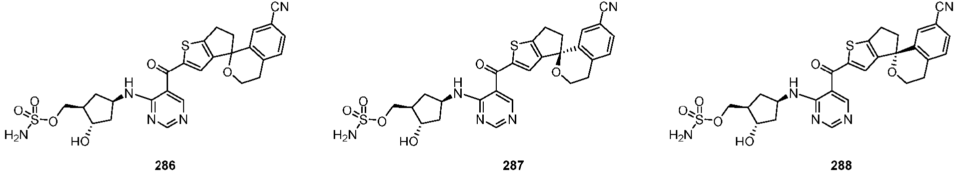

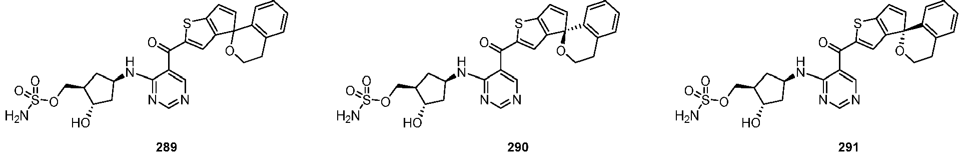

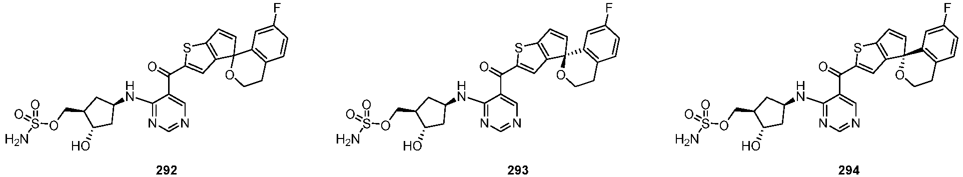

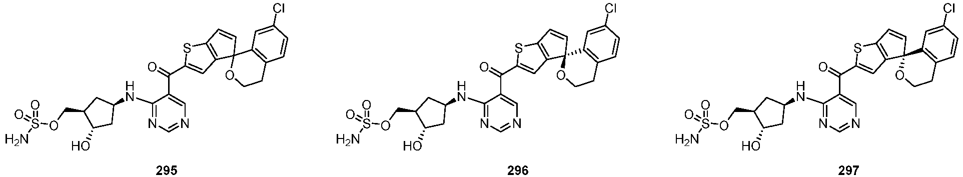

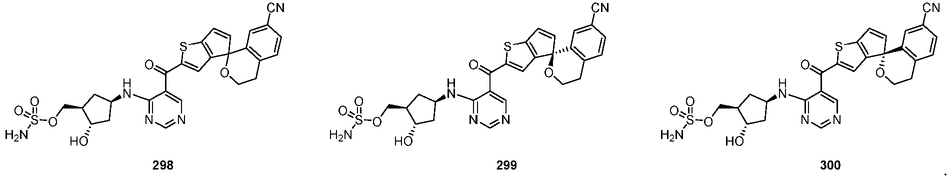

Another objective of the present disclosure is to provide a pharmaceutical composition comprising a pharmaceutically acceptable carrier, diluent, and/or excipient, as well as the compound of general formula (1) or the isomer thereof, the crystalline form thereof, the pharmaceutically acceptable salt thereof, the hydrate thereof, or the solvate thereof of the present disclosure as an active ingredient.

Still another objective of the present disclosure is to provide use of the compound of general formula (1) or the isomer thereof, the crystalline form thereof, the pharmaceutically acceptable salt thereof, the hydrate thereof, or the solvate thereof of the present disclosure, or the pharmaceutical composition described above in preparing a medicament for treating, regulating, or preventing a disease related to SAE protein, wherein the disease is preferably cancer, and the cancer is a hematologic cancer or a solid tumor.

Still another objective of the present disclosure further provides a method for treating, regulating, or preventing a disease related to SAE protein, comprising administering to a subject a therapeutically effective amount of the compound of general formula (1) or the isomer thereof, the crystalline form thereof, the pharmaceutically acceptable salt thereof, the hydrate thereof, or the solvate thereof of the present disclosure, or the pharmaceutical composition described above.

Through synthesis and careful studies of various classes of novel compounds with inhibitory effects on SAE, the inventors have discovered that the compound of general formula (1) has surprisingly strong inhibitory activity against SAE.

It should be understood that both the aforementioned general description and the following detailed description of the present disclosure are exemplary and explanatory, and are intended to provide further explanation of the present disclosure claimed.

### Synthesis of Compounds

Methods for preparing the compound of general formula (1) of the present disclosure are specifically described below, but these specific methods do not limit the present disclosure in any way.

The compound of general formula (1) described above may be synthesized using standard synthetic techniques or well-known techniques in combination with the methods described herein. In addition, the solvents, temperatures, and other reaction conditions mentioned herein may vary. Starting materials for the synthesis of the compounds may be obtained synthetically or commercially. The compounds described herein and other related compounds with different substituents may be synthesized using well-known techniques and starting materials, including the methods found in March, *ADVANCED ORGANIC CHEMISTRY,* 4^{th} Ed., (Wiley 1992); Carey and Sundberg, *ADVANCED ORGANIC CHEMISTRY,* 4^{th} Ed., Vols. A and B (Plenum 2000, 2001); and Green and Wuts, *PROTECTIVE GROUPS IN ORGANIC SYNTHESIS,* 3^{rd} Ed., (Wiley 1999). General methods for preparing the compounds may be altered by using appropriate reagents and conditions for introducing different groups into the molecular formulas provided herein.

In one aspect, the compounds described herein are prepared according to methods well known in the art. However, the conditions of the methods, such as reactants, solvents, bases, the amount of the compound used, reaction temperature, and time required for the reaction are not limited to the following explanation. The compounds of the present disclosure may also be conveniently prepared by optionally combining various synthetic methods described herein or known in the art, and such combinations may be easily determined by those skilled in the art to which the present disclosure pertains. In one aspect, the present disclosure further provides a method for preparing the compound of general formula (1), wherein the compound of general formula (1) may be prepared by the following general reaction scheme 1, general reaction scheme 2, general reaction scheme 3, general reaction scheme 4, general reaction scheme 5, general reaction scheme 6, or general reaction scheme 7:

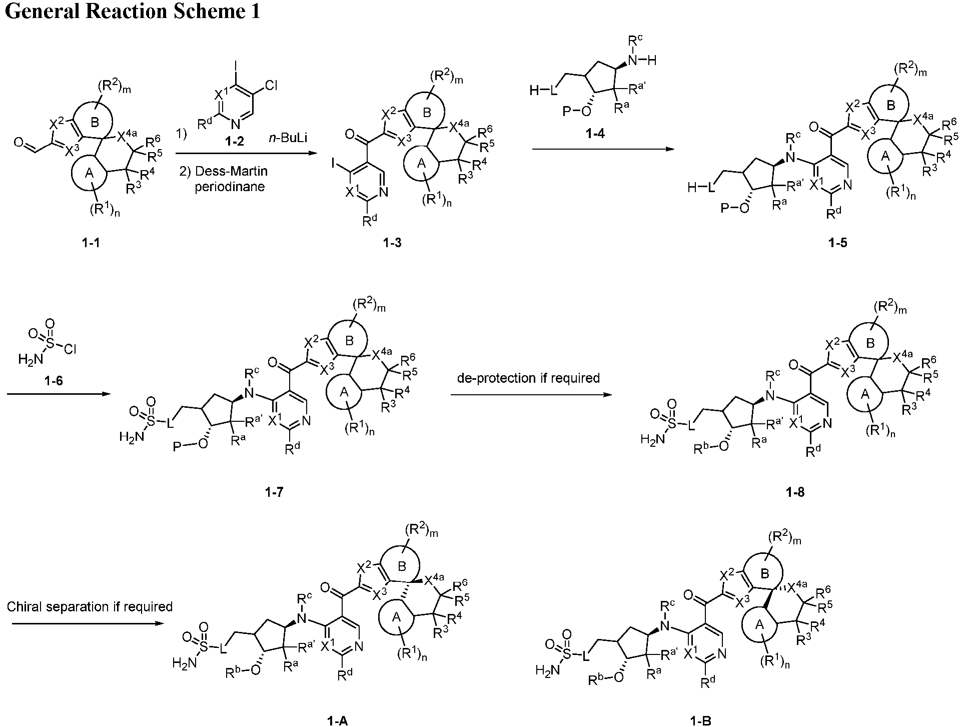

Embodiments of the compound of general formula (1) may be prepared according to general reaction scheme 1, wherein P is R^{b} or a hydroxyl-protecting group; ring A, ring B, R¹, R², R³, R⁴, R⁵, R⁶, R^{a}, R^{a'}, R^{b}, R^{c}, R^{d}, X¹, X², X³, m, and n are as defined above; H represents hydrogen, N represents nitrogen, O represents oxygen, Cl represents chlorine, X^{4a} represents O or S, and L represents O or NH. As shown in general reaction scheme 1, compound 1-1 undergoes a substitution reaction with compound 1-2 to produce an alcohol intermediate, which is further oxidized to obtain ketone compound 1-3; compound 1-3 undergoes a substitution reaction with compound 1-4 to produce compound 1-5, and compound 1-5 undergoes a substitution reaction with compound 1-6 to produce compound 1-7; in some cases, compound 1-7 undergoes hydroxyl group deprotection to produce compound 1-8, and in some cases, compound 1-8 is subjected to chiral resolution to obtain optical isomers 1-A and 1-B.

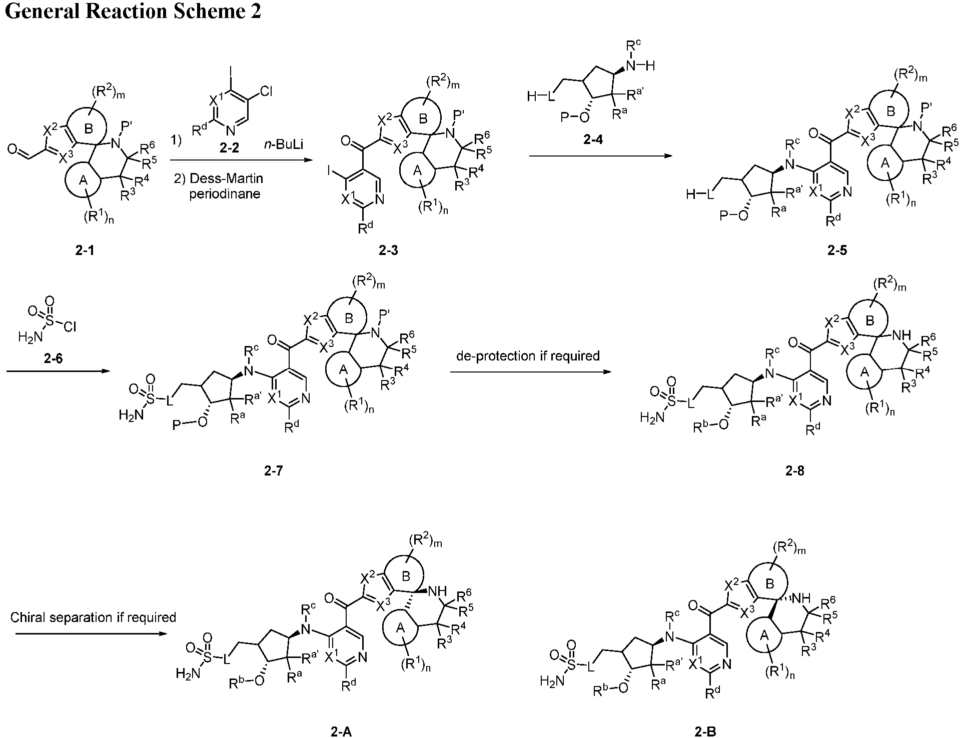

Embodiments of the compound of general formula (1) may be prepared according to general reaction scheme 2, wherein P is R^{b} or a hydroxyl-protecting group, and P' is an amino-protecting group; ring A, ring B, R¹, R², R³, R⁴, R⁵, R⁶, R^{a}, R^{a'}, R^{b}, R^{c}, R^{d}, X¹, X², X³, m, and n are as defined above; H represents hydrogen, N represents nitrogen, O represents oxygen, Cl represents chlorine, and L represents O or NH. As shown in general reaction scheme 2, compound 2-1 undergoes a substitution reaction with compound 2-2 to produce an alcohol intermediate, which is further oxidized to obtain ketone compound 2-3; compound 2-3 undergoes a substitution reaction with compound 2-4 to produce compound 2-5, and compound 2-5 undergoes a substitution reaction with compound 2-6 to produce compound 2-7; in some cases, compound 2-7 undergoes deprotection to produce compound 2-8, and in some cases, compound 2-8 is subjected to chiral resolution to obtain optical isomers 2-A and 2-B.

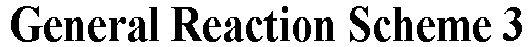

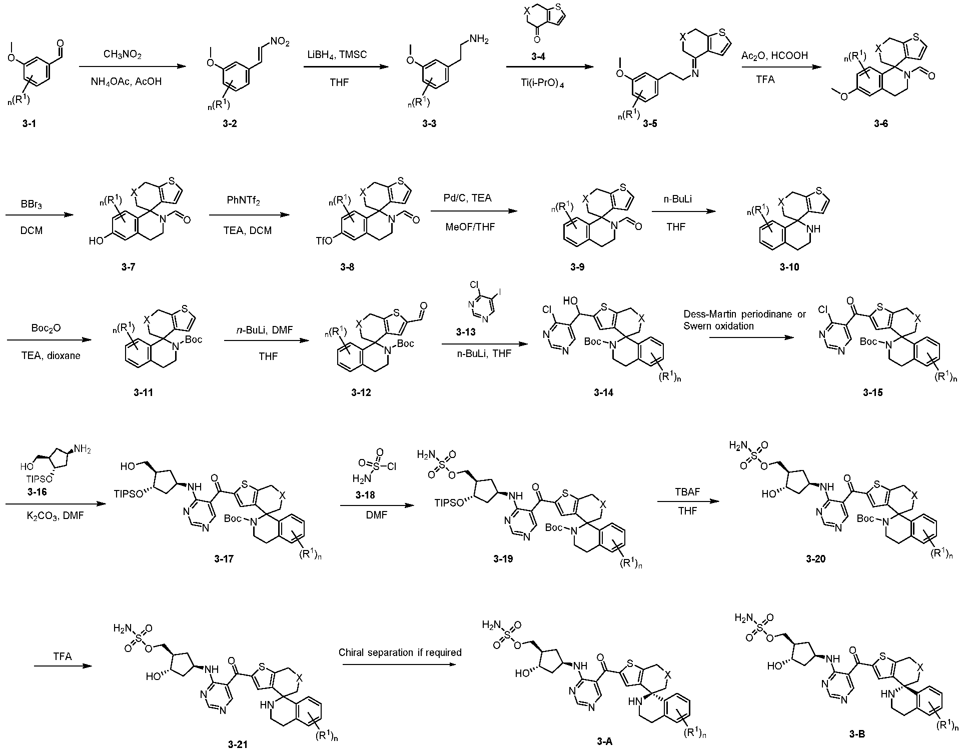

Embodiments of the compound of general formula (1) may be prepared according to general reaction scheme 3, wherein R¹ and n are as defined above, X represents O, S, or CH₂, H represents hydrogen, N represents nitrogen, O represents oxygen, and Cl represents chlorine. As shown in general reaction scheme 3, compound 3-1 undergoes a condensation reaction with nitromethane to produce compound 3-2, and compound 3-2 is reduced to obtain amine 3-3; amine 3-3 undergoes a condensation reaction with ketone 3-4 to obtain compound 3-5, and compound 3-5 undergoes a cyclization reaction under an acidic condition to produce compound 3-6; compound 3-6 is demethylated to obtain compound 3-7, and compound 3-7 reacts with PhNTf₂ to produce compound 3-8; compound 3-8 is reduced to obtain compound 3-9, and compound 3-9 undergoes formyl group removal to obtain compound 3-10; compound 3-10 is protected by (Boc)₂O to obtain compound 3-11, and compound 3-11 reacts with DMF under the condition of n-butyllithium to obtain aldehyde 3-12; aldehyde 3-12 undergoes an addition reaction with compound 3-13 under the condition of n-butyllithium to obtain compound 3-14, and compound 3-14 is subjected to Dess-Martin oxidation or Swern oxidation to obtain ketone 3-15; ketone 3-15 undergoes a substitution reaction with compound 3-16 under a alkaline condition to produce compound 3-17, and compound 3-17 reacts with compound 3-18 to produce compound 3-19; compound 3-19 undergoes deprotection by TBAF to obtain compound 3-20, and compound 3-20 undergoes amino group deprotection under an acidic condition to obtain compound 3-21; and in some cases, compound 3-21 is subjected to chiral resolution to obtain optical isomers 3-A and 3-B.

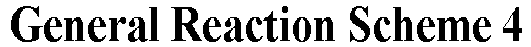

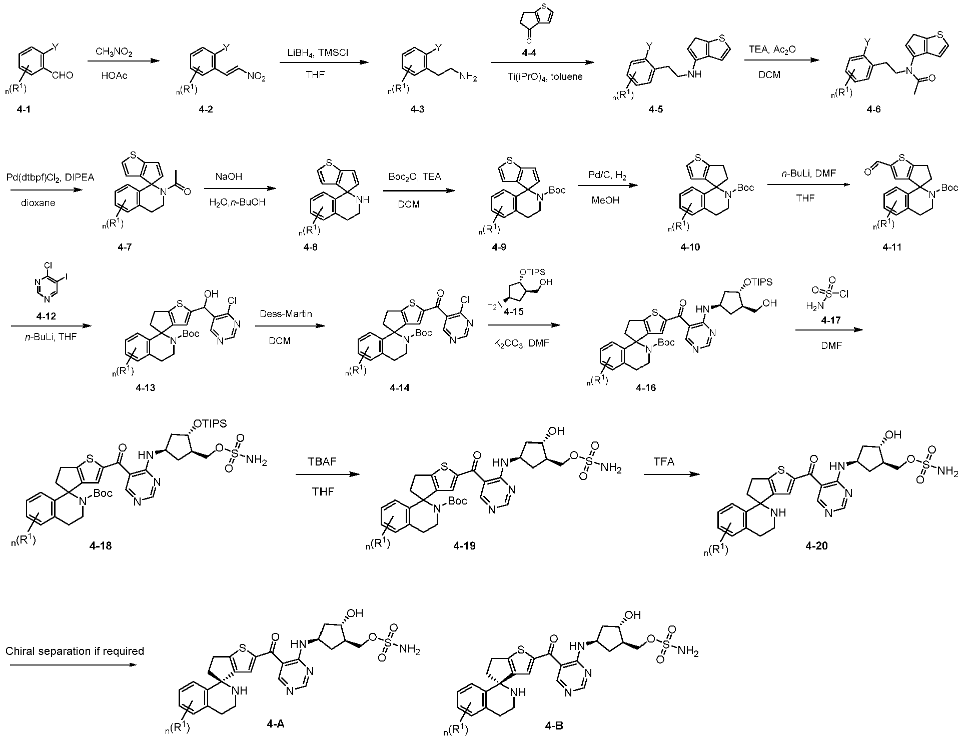

Embodiments of the compound of general formula (1) may be prepared according to general reaction scheme 4, wherein R¹ and n are as defined above, Y represents Br or I, H represents hydrogen, N represents nitrogen, O represents oxygen, and Cl represents chlorine. As shown in general reaction scheme 4, compound 4-1 undergoes a condensation reaction with nitromethane to produce compound 4-2, and compound 4-2 is reduced to obtain amine 4-3; amine 4-3 undergoes a condensation reaction with ketone 4-4 to obtain compound 4-5, and compound 4-5 reacts with acetic anhydride to produce compound 4-6; compound 4-6 undergoes a cyclization reaction under the catalysis of Pd to produce compound 4-7, and compound 4-7 undergoes acetyl group deprotection to obtain compound 4-8; compound 4-8 is protected by (Boc)₂O to obtain compound 4-9, and compound 4-9 undergoes a hydrogenation reaction to produce compound 4-10; compound 4-10 reacts with DMF under the condition of n-butyllithium to obtain aldehyde 4-11, and aldehyde 4-11 undergoes an addition reaction with compound 4-12 under the condition of n-butyllithium to obtain compound 4-13; compound 4-13 is subjected to Dess-Martin oxidation to obtain ketone 4-14, and ketone 4-14 undergoes a substitution reaction with compound 4-15 under a alkaline condition to produce compound 4-16; compound 4-16 reacts with compound 4-17 to produce compound 4-18, and compound 4-18 undergoes deprotection by TBAF to obtain compound 4-19; compound 4-19 undergoes amino group deprotection under an acidic condition to obtain compound 4-20, and in some cases, compound 4-20 is subjected to chiral resolution to obtain optical isomers 4-A and 4-B.

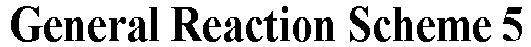

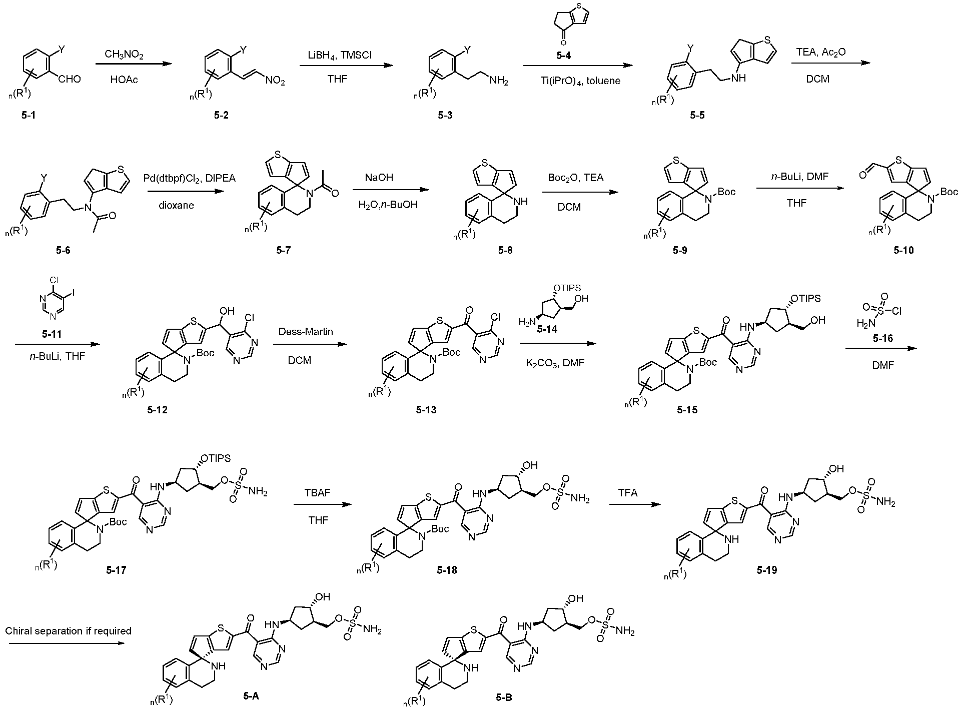

Embodiments of the compound of general formula (1) may be prepared according to general reaction scheme 5, wherein R¹ and n are as defined above, Y represents Br or I, H represents hydrogen, N represents nitrogen, O represents oxygen, and Cl represents chlorine. As shown in general reaction scheme 5, compound 5-1 undergoes a condensation reaction with nitromethane to produce compound 5-2, and compound 5-2 is reduced to obtain amine 5-3; amine 5-3 undergoes a condensation reaction with ketone 5-4 to obtain compound 5-5, and compound 5-5 reacts with acetic anhydride to produce compound 5-6; compound 5-6 undergoes a cyclization reaction under the catalysis of Pd to produce compound 5-7, and compound 5-7 undergoes acetyl group deprotection to obtain compound 5-8; compound 5-8 is protected by (Boc)₂O to obtain compound 5-9, and compound 5-9 reacts with DMF under the condition of n-butyllithium to obtain aldehyde 5-10; aldehyde 5-10 undergoes an addition reaction with compound 5-11 under the condition of n-butyllithium to obtain compound 5-12, and compound 5-12 is subjected to Dess-Martin oxidation to obtain ketone 5-13; ketone 5-13 undergoes a substitution reaction with compound 5-14 under a alkaline condition to produce compound 5-15, and compound 5-15 reacts with compound 5-16 to produce compound 5-17; compound 5-17 undergoes deprotection by TBAF to obtain compound 5-18, and compound 5-18 undergoes amino group deprotection under an acidic condition to obtain compound 5-19; and in some cases, compound 5-19 is subjected to chiral resolution to obtain optical isomers 5-A and 5-B.

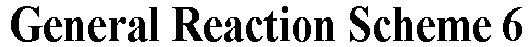

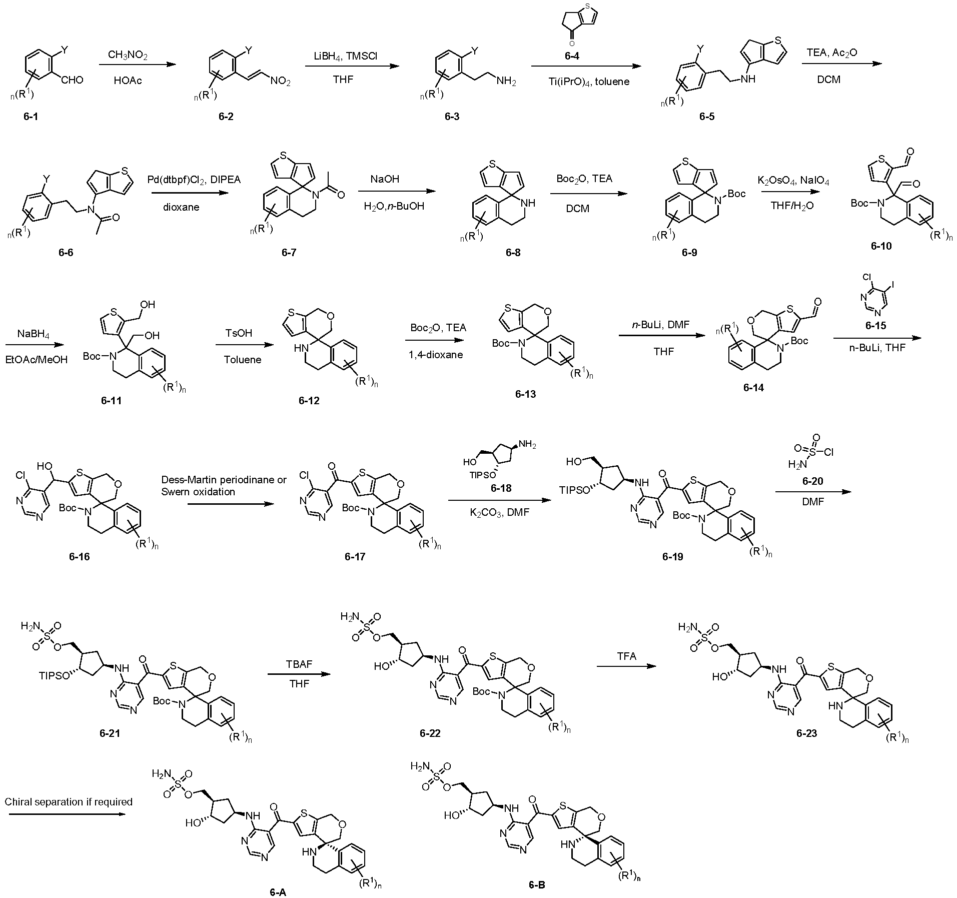

Embodiments of the compound of general formula (1) may be prepared according to general reaction scheme 6, wherein R¹ and n are as defined above, Y represents Br or I, H represents hydrogen, N represents nitrogen, O represents oxygen, and Cl represents chlorine. As shown in general reaction scheme 6, compound 6-1 undergoes a condensation reaction with nitromethane to produce compound 6-2, and compound 6-2 is reduced to obtain amine 6-3; amine 6-3 undergoes a condensation reaction with ketone 6-4 to obtain compound 6-5, and compound 6-5 reacts with acetic anhydride to produce compound 6-6; compound 6-6 undergoes a cyclization reaction under the catalysis of Pd to produce compound 6-7, and compound 6-7 undergoes acetyl group deprotection to obtain compound 6-8; compound 6-8 is protected by (Boc)₂O to obtain compound 6-9, and compound 6-9 undergoes an oxidation reaction under the condition of potassium osmate and sodium periodate to obtain dialdehyde 6-10; dialdehyde 6-10 is reduced with sodium borohydride to obtain diol 6-11, and diol 6-11 undergoes a cyclization reaction under an acidic condition to obtain compound 6-12; compound 6-12 is protected by (Boc)₂O to obtain compound 6-13, and compound 6-13 reacts with DMF under the condition of n-butyllithium to obtain aldehyde 6-14; aldehyde 6-14 undergoes an addition reaction with compound 6-15 under the condition of n-butyllithium to obtain compound 6-16, and compound 6-16 is subjected to Dess-Martin oxidation or Swern oxidation to obtain ketone 6-17; ketone 6-17 undergoes a substitution reaction with compound 6-18 under a alkaline condition to produce compound 6-19, and compound 6-19 reacts with compound 6-20 to produce compound 6-21; compound 6-21 undergoes deprotection by TBAF to obtain compound 6-22, and compound 6-22 undergoes amino group deprotection under an acidic condition to obtain compound 6-23; and in some cases, compound 6-23 is subjected to chiral resolution to obtain optical isomers 6-A and 6-B.

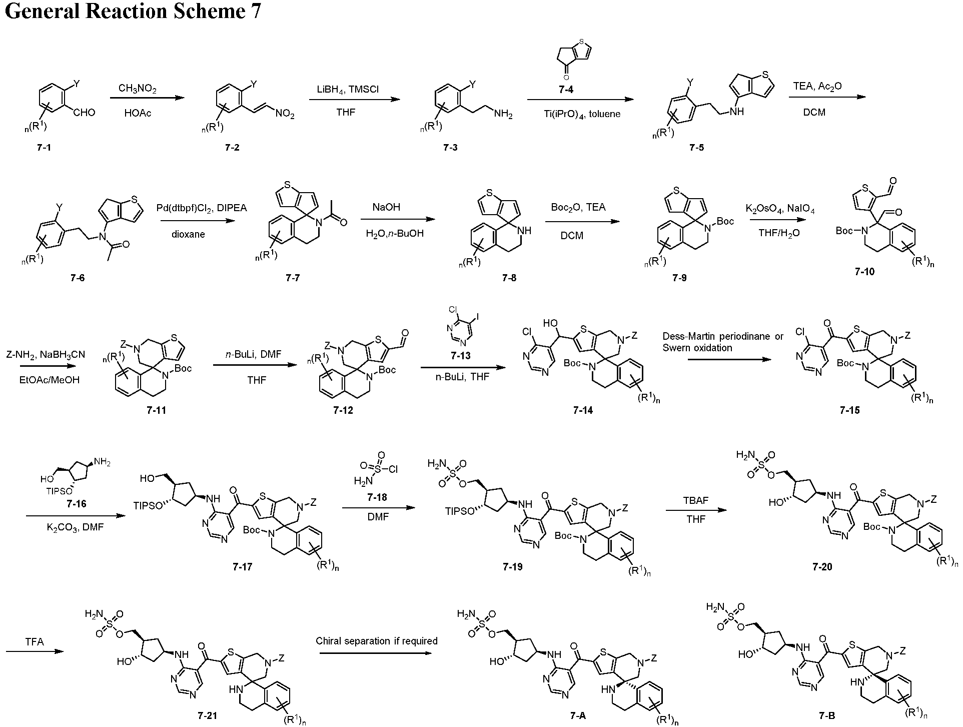

Embodiments of the compound of general formula (1) may be prepared according to general reaction scheme 7, wherein R¹ and n are as defined above, Y represents Br or I, Z represents -H, -OH, (C1-C6) alkyl, (C1-C6) haloalkyl, (C1-C6) alkoxy, (C2-C6) alkenyl, (C2-C6) alkynyl, or (C3-C8) cycloalkyl, H represents hydrogen, N represents nitrogen, O represents oxygen, and Cl represents chlorine. As shown in general reaction scheme 7, compound 7-1 undergoes a condensation reaction with nitromethane to produce compound 7-2, and compound 7-2 is reduced to obtain amine 7-3; amine 7-3 undergoes a condensation reaction with ketone 7-4 to obtain compound 7-5, and compound 7-5 reacts with acetic anhydride to produce compound 7-6; compound 7-6 undergoes a cyclization reaction under the catalysis of Pd to produce compound 7-7, and compound 7-7 undergoes acetyl group deprotection to obtain compound 7-8; compound 7-8 is protected by (Boc)₂O to obtain compound 7-9, and compound 7-9 undergoes an oxidation reaction under the condition of potassium osmate and sodium periodate to obtain dialdehyde 7-10; dialdehyde 7-10 undergoes reductive amination to obtain amine 7-11, and compound 7-11 reacts with DMF under the condition of n-butyllithium to obtain aldehyde 7-12; aldehyde 7-12 undergoes an addition reaction with compound 7-13 under the condition of n-butyllithium to obtain compound 7-14, and compound 7-14 is subjected to Dess-Martin oxidation or Swern oxidation to obtain ketone 7-15; ketone 7-15 undergoes a substitution reaction with compound 7-16 under a alkaline condition to produce compound 7-17, and compound 7-17 reacts with compound 7-18 to produce compound 7-19; compound 7-19 undergoes deprotection by TBAF to obtain compound 7-20, and compound 7-20 undergoes amino group deprotection under an acidic condition to obtain compound 7-21; and in some cases, compound 7-21 is subjected to chiral resolution to obtain optical isomers 7-A and 7-B.

### Further Forms of Compounds

"Pharmaceutically acceptable" herein refers to a substance, such as a carrier or diluent, which will not lead to loss of biological activity or properties of a compound and is relatively non-toxic. For example, when an individual is given a substance, the substance will not cause undesired biological effects or interact with any component contained therein in a deleterious manner.

The term "pharmaceutically acceptable salt" refers to a form of a compound that does not cause significant irritation to the organism receiving the administration or eliminate the biological activity and properties of the compound. In certain specific aspects, the pharmaceutically acceptable salt is obtained by subjecting the compound of the general formula to a reaction with acids or bases, wherein the acids or bases include, but are not limited to, those found in Stahl and Wermuth, *Handbook of Pharmaceutical Salts: Properties, Selection, and Use,* 1^{st} Ed., (Wiley, 2002).

It should be understood that references to pharmaceutically acceptable salts include solvent addition forms or crystalline forms, especially solvates or polymorphs. A solvate contains either stoichiometric or nonstoichiometric amount of solvent and is selectively formed during crystallization in a pharmaceutically acceptable solvent such as water and ethanol. Hydrates are formed when the solvent is water, or alcoholates are formed when the solvent is ethanol. The solvates of the compound of general formula (1) are conveniently prepared or formed according to the methods described herein. For example, hydrates of the compound of general formula (1) are conveniently prepared by recrystallization in a mixed solvent of water/organic solvent, wherein the organic solvent used includes, but is not limited to, tetrahydrofuran, acetone, ethanol or methanol. Furthermore, the compounds described herein may be present in either a non-solvated form or a solvated form. In general, the solvated forms are considered equivalent to the non-solvated forms for purposes of the compounds and methods provided herein.

In other specific examples, the compound of general formula (1) is prepared in different forms including, but not limited to, amorphous, pulverized and nanoparticle forms. In addition, the compound of general formula (1) includes crystalline forms, and may also be polymorphs. Polymorphs include different lattice arrangements of the same elements of a compound. Polymorphs generally have different X-ray diffraction spectra, infrared spectra, melting points, density, hardness, crystalline forms, optical and electrical properties, stability and solubility. Different factors such as a recrystallization solvent, crystallization rate, and storage temperature may lead to a single dominant crystalline form.

In another aspect, the compound of general formula (1) may have a chiral center and/or axial chirality, and thus may be present in the form of a racemate, a racemic mixture, a single enantiomer, a diastereomeric compound, a single diastereomer and a cis-trans isomer. Each chiral center or axial chirality will independently produce two optical isomers, and all possible optical isomers, diastereomeric mixtures, and pure or partially pure compounds are included within the scope of the present disclosure. The present disclosure is meant to include all such isomeric forms of these compounds.

The compound of the present disclosure may contain unnatural proportions of atomic isotopes at one or more of the atoms that constitute the compound. For example, the compound may be labeled with radioactive isotopes, such as tritium (³H), iodine-125 (¹²⁵I), and C-14 (¹⁴C). For another example, deuterium can be used to substitute a hydrogen atom to form a deuterated compound. The bond formed by deuterium and carbon is stronger than that formed by ordinary hydrogen and carbon. Compared with an undeuterated medicament, the deuterated medicament generally has the advantages of reduced toxic and side effects, increased pharmaceutical stability, enhanced efficacy, prolonged pharmaceutical *in vivo* half-life and the like. All isotopic variations of the compound of the present disclosure, whether radioactive or not, are contained within the scope of the present disclosure.

Any atom of the compound of the present disclosure, unless otherwise specified, refers to an isotope of the atom in stable state of the compound. Unless otherwise specified, when a site in a molecular structure is selected as "H" or "hydrogen", the site should be understood as having the natural abundance of the hydrogen isotope. Similarly, unless otherwise specified, when a site is selected as "D" or "deuterium", the site should be understood to have a deuterium isotopic abundance that is at least 3000 times its natural abundance (the natural abundance of the deuterium isotope is 0.015%).

More preferably, each deuterated site of the deuterated compounds of the present disclosure has a deuterium atom abundance that is at least 3500 times its natural abundance (52.2% deuterium atom enrichment). More preferably, the deuterium atom abundance is at least 4500 times the natural abundance (67.5% deuterium atom enrichment). More preferably, the deuterium atom abundance is at least 5000 times the natural abundance (75% deuterium atom enrichment). More preferably, the deuterium atom abundance is at least 6000 times the natural abundance (90% deuterium atom enrichment). More preferably, the deuterium atom abundance is at least 6333 times the natural abundance (95% deuterium atom enrichment). More preferably, the deuterium atom abundance is at least 6466.7 times the natural abundance (97% deuterium atom enrichment). More preferably, the deuterium atom abundance is at least 6600 times the natural abundance (99% deuterium atom enrichment). More preferably, the deuterium atom abundance is at least 6633.3 times the natural abundance (99.5% deuterium atom enrichment).

### Terminology

Unless otherwise stated, the terms used in the present application, including those in the specification and claims, are defined as follows. It must be noted that in the specification and the appended claims, the singular forms "a" and "an" include plural meanings unless clearly indicated otherwise. Unless otherwise stated, conventional methods for mass spectrometry, nuclear magnetic resonance spectroscopy, HPLC, protein chemistry, biochemistry, recombinant DNA techniques and pharmacology are used. As used herein, "or" or "and" refers to "and/or" unless otherwise stated.

Unless otherwise specified, "alkyl" refers to a saturated aliphatic hydrocarbon group, including linear and branched groups containing 1 to 6 carbon atoms. Lower alkyl groups containing 1 to 4 carbon atoms, such as methyl, ethyl, propyl, 2-propyl, n-butyl, isobutyl, or tert-butyl, are preferred. As used herein, "alkyl" includes unsubstituted and substituted alkyl, particularly alkyl substituted with one or more halogens. Preferred alkyl is selected from CH₃, CH₃CH₂, CF₃, CHF₂, CF₃CH₂, CF₃(CH₃)CH, ⁱPr, ⁿPr, ⁱBu, ⁿBu, or ^{t}Bu.

Unless otherwise specified, "alkylene" refers to a divalent alkyl as defined above. Examples of alkylene include, but are not limited to, methylene and ethylene.

Unless otherwise specified, "alkenyl" refers to an unsaturated aliphatic hydrocarbon group containing carboncarbon double bonds, including linear or branched groups containing 1 to 14 carbon atoms. Lower alkenyl groups containing 1 to 4 carbon atoms, such as vinyl, 1-propenyl, 1-butenyl, or 2-methylpropenyl, are preferred. Lower alkenyl groups containing 1 to 2 carbon atoms are further preferred.

Unless otherwise specified, "alkenylene" refers to a divalent alkenyl as defined above.

Unless otherwise specified, "alkynyl" refers to an unsaturated aliphatic hydrocarbon group containing carboncarbon triple bonds, including linear and branched groups containing 1 to 14 carbon atoms. Lower alkynyl groups containing 1 to 4 carbon atoms, such as ethynyl, 1-propynyl, or 1-butynyl, are preferred. Lower alkynyl groups containing 1 to 2 carbon atoms are further preferred.

Unless otherwise specified, "alkynylene" refers to a divalent alkynyl as defined above.

Unless otherwise specified, "cycloalkyl" refers to a non-aromatic hydrocarbon ring system (monocyclic, bicyclic, or polycyclic), and is preferably a non-aromatic hydrocarbon ring system containing 3 to 14 ring carbon atoms (C₃₋₁₄ cycloalkyl). In some embodiments, cycloalkyl has 3 to 10 ring carbon atoms (C₃₋₁₀ cycloalkyl). In some embodiments, cycloalkyl has 3 to 8 ring carbon atoms (C₃₋₈ cycloalkyl). In some embodiments, cycloalkyl has 3 to 7 ring carbon atoms (C₃₋₇ cycloalkyl). In some embodiments, cycloalkyl has 3 to 6 ring carbon atoms (C₃₋₆ cycloalkyl). In some embodiments, cycloalkyl has 4 to 6 ring carbon atoms (C₄₋₆ cycloalkyl). In some embodiments, cycloalkyl has 5 to 6 ring carbon atoms (C₅₋₆ cycloalkyl). In some embodiments, cycloalkyl has 5 to 10 ring carbon atoms (C₅₋₁₀ cycloalkyl). For cycloalkyl, partially unsaturated cycloalkyl may be referred to as "cycloalkenyl" if the carbocyclic ring contains at least one double bond, or "cycloalkynyl" if the carbocyclic ring contains at least one triple bond. Cycloalkyl may include monocyclic or polycyclic groups (e.g., having 2, 3, or 4 fused rings) and spiro rings. In some embodiments, cycloalkyl is monocyclic. In some embodiments, cycloalkyl is bicyclic. In some embodiments, cycloalkyl is monocyclic or bicyclic. In some embodiments, cycloalkyl is tricyclic. The ring carbon atoms of cycloalkyl may optionally be oxidized to form an oxo or thio group. Cycloalkyl further includes cycloalkylene. In some embodiments, cycloalkyl contains 0, 1, or 2 double bonds. In some embodiments, cycloalkyl contains 1 or 2 double bonds (partially unsaturated cycloalkyl). In some embodiments, cycloalkyl may be fused to aryl, heteroaryl, cycloalkyl, and heterocycloalkyl. In some embodiments, cycloalkyl may be fused to aryl, cycloalkyl and heterocycloalkyl. In some embodiments, cycloalkyl may be fused to aryl and heterocycloalkyl. In some embodiments, cycloalkyl may be fused to aryl and cycloalkyl. Examples of cycloalkyl include cyclopropyl, cyclobutyl, cyclopentyl, cyclohexyl, cycloheptyl, cyclopentenyl, cyclohexenyl, cyclohexadienyl, cycloheptatrienyl, norcamphanyl, norpinanyl, norcamyl, bicyclo[1.1.1]pentyl, bicyclo[2.1.1]hexyl and the like.

Unless otherwise specified, "cycloalkylene" refers to a divalent cycloalkyl as defined above.

Unless otherwise specified, "alkoxy" refers to an alkyl group that bonds to the rest of the molecule through an ether oxygen atom. Representative alkoxy groups are those having 1-6 carbon atoms, such as methoxy, ethoxy, propoxy, isopropoxy, butoxy, isobutoxy, sec-butoxy, and tert-butoxy. As used herein, "alkoxy" includes unsubstituted and substituted alkoxy, particularly alkoxy substituted with one or more halogens. Preferred alkoxy is selected from OCH₃, OCF₃, CHF₂O, CF₃CH₂O, ⁱ⁻PrO, ⁿ⁻PrO, ⁱ⁻BuO, ⁿ⁻BuO, or ^{t-}BuO.

Unless otherwise specified, "aryl" refers to an aromatic hydrocarbon group, which is monocyclic or polycyclic; for example, a monocyclic aryl ring is fused to one or more carbocyclic aromatic groups. Examples of aryl include, but are not limited to, phenyl, naphthyl, and phenanthryl.

Unless otherwise specified, "aryloxy" refers to an aryl group that bonds to the rest of the molecule through an ether oxygen atom. Examples of aryloxy include, but are not limited to, phenoxy and naphthoxy.

Unless otherwise specified, "arylene" refers to a divalent aryl as defined above. Examples of arylene include, but are not limited to, phenylene, naphthylene, and phenanthrylene.

Unless otherwise specified, "heteroaryl" refers to an aromatic group containing substitution of one or more heteroatoms or an unsubstituted aromatic group, preferably a 5- to 14-membered aromatic group containing 1 to 4 heteroatoms selected from oxygen, sulfur, and nitrogen, and more preferably a 5- to 9-membered aromatic group containing 1 to 2 heteroatoms optionally selected from oxygen, sulfur, or nitrogen. The heteroatoms are independently selected from O, N, or S, and the number of the heteroatoms is preferably 1, 2, or 3. Heteroaryl is monocyclic or polycyclic. Monocyclic heteroaryl is preferably a 5- to 6-membered aromatic group containing 1 to 3 heteroatoms optionally selected from oxygen, nitrogen, or sulfur. More preferably, monocyclic heteroaryl is a 5- to 6-membered aromatic group containing 1 to 2 heteroatoms optionally selected from oxygen, nitrogen, or sulfur. More preferably, monocyclic heteroaryl is a 5- to 6-membered aromatic group containing 1 heteroatom optionally selected from oxygen, nitrogen, or sulfur. In some embodiments, a monocyclic heteroaryl ring is fused to one or more carbocyclic aromatic groups or other monocyclic heterocycloalkyl groups. Examples of heteroaryl include, but are not limited to, pyridinyl, pyridazinyl, imidazolyl, pyrimidinyl, pyrazolyl, triazolyl, pyrazinyl, quinolinyl, isoquinolinyl, furanyl, thienyl, isoxazolyl, thiazolyl, oxazolyl, isothiazolyl, pyrrolyl, indolyl, benzimidazolyl, benzofuranyl, benzothiazolyl, benzothienyl, benzoxazolyl, benzopyridinyl, pyrrolopyrimidinyl, 1H-pyrrolo[3,2-b]pyridinyl, 1H-pyrrolo[2,3-c]pyridinyl, 1H-pyrrolo[3,2-c]pyridinyl, 1H-pyrrolo[2,3-b]pyridinyl,

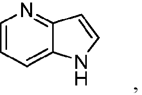

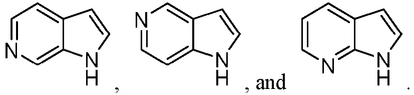

Unless otherwise specified, "heteroarylene" refers to a divalent heteroaryl as defined above.

Unless otherwise specified, "heterocycloalkyl" refers to a non-aromatic ring or a ring system, which may optionally contain one or more alkenylene groups as part of the ring structure, and has at least one heteroatom ring member independently selected from boron, phosphorus, nitrogen, sulfur, oxygen, and selenium; heterocycloalkyl is preferably a saturated or partially unsaturated ring containing 1 to 4 heteroatoms selected from oxygen, sulfur, or nitrogen, and more preferably a saturated or partially unsaturated ring containing 1 to 2 heteroatoms selected from oxygen, sulfur, or nitrogen. In some embodiments, heterocycloalkyl is a 5- to 8-membered non-aromatic ring containing ring carbon atoms and 1 to 4 ring heteroatoms, and each heteroatom is independently and optionally selected from nitrogen, oxygen, or sulfur (5- to 8-membered heterocycloalkyl). Heterocycloalkyl is a 5- to 6-membered non-aromatic ring containing ring carbon atoms and 1 to 4 ring heteroatoms, and each heteroatom is independently and optionally selected from nitrogen, oxygen, or sulfur (5- to 6-membered heterocycloalkyl). In some embodiments, 5- to 6-membered heterocycloalkyl contains 1 to 3 ring heteroatoms independently selected from nitrogen, oxygen, and sulfur. In some embodiments, 5- to 6-membered heterocycloalkyl contains 1 to 2 ring heteroatoms independently selected from nitrogen, oxygen, and sulfur. In some embodiments, 5- to 6-membered heterocycloalkyl contains 1 ring heteroatom independently selected from nitrogen, oxygen, and sulfur. Partially unsaturated heterocycloalkyl may be referred to as "heterocycloalkenyl" if heterocycloalkyl contains at least one double bond, or "heterocycloalkynyl" if the heterocycloalkyl contains at least one triple bond. Heterocycloalkyl may include monocyclic, bicyclic, spiro ring, or polycyclic (e.g., having two fused or bridged rings) systems. In some embodiments, heterocycloalkyl is a monocyclic group having 1, 2, or 3 heteroatoms independently selected from nitrogen, sulfur, and oxygen. The ring carbon atoms and heteroatoms of heterocycloalkyl may optionally be oxidized to form oxo or thio groups or other oxidized bonds (e.g., C(O), S(O), C(S) or S(O)₂, and N-oxides), or the nitrogen atoms may be quaternized. Heterocycloalkyl may be attached via a ring carbon atom or a ring heteroatom. In some embodiments, heterocycloalkyl contains 0 to 3 double bonds. In some embodiments, heterocycloalkyl contains 0 to 2 double bonds. The definition of heterocycloalkyl further includes moieties (also referred to as partially unsaturated heterocyclic rings) having one or more aromatic rings fused to (i.e., sharing a bond with) the heterocycloalkyl ring, for example, benzo-derivatives of piperidine, morpholine, azepin, and thienyl. Heterocycloalkyl containing a fused aromatic ring may be attached via any ring atom, including ring atoms of the fused aromatic ring. Examples of heterocycloalkyl include, but are not limited to, azetidinyl, azepinyl, dihydrobenzofuranyl, dihydrofuranyl, dihydropyranyl, N-morpholinyl, 3-oxa-9-azaspiro[5.5]undecyl, 1-oxa-8-azaspiro[4.5]decyl, piperidinyl, piperazinyl, oxopiperazinyl, pyranyl, pyrrolidinyl, quininyl, tetrahydrofuranyl, tetrahydropyranyl, 1,2,3,4-tetrahydroquinolinyl, tropanyl, 4,5,6,7-tetrahydrothiazolo[5,4-c]pyridinyl, 4,5,6,7-tetrahydro-1H-imidazo[4,5-c]pyridine, N-methylpiperidinyl, tetrahydroimidazolyl, pyrazolidinyl, butyrolactam, valerolactam, imidazolidinonyl, hydantoinyl, dioxolanyl, phthalimidyl, pyrimidine-2,4(1H,3H)-dione, 1,4-dioxanyl, morpholinyl, thiomorpholinyl, thiomorpholinyl-S-oxide, thiomorpholinyl-S,S-oxide, piperazinyl, pyranyl, pyridonyl, 3-pyrrolinyl, thiopyranyl, pyronyl, tetrahydrothienyl, 2-azaspiro[3.3]heptanyl, indolinyl,

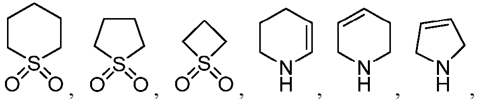

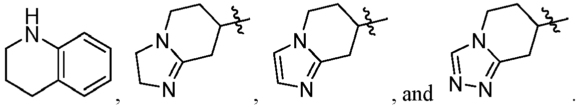

Unless otherwise specified, "heterocycloalkylene" refers to divalent heterocycloalkyl as defined above.

Unless otherwise specified, "oxo" refers to =O; for example, a group formed by substitution of carbon with one oxo is "carbonyl ( 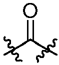 )"; a group formed by substitution of sulfur with one oxo is "sulfinyl ( 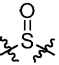 )" and a group formed by substitution of sulfur with two oxos is "sulfonyl (

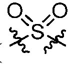

)". Unless otherwise specified, "halogen" (or halo) refers to fluorine, chlorine, bromine, or iodine. The term "halo" (or "halogenated") before a group name indicates that the group is partially or fully halogenated, that is, substituted in any combination with F, Cl, Br, or I, preferably with F or Cl.

Unless otherwise specified, the term "substituted" means that one or more hydrogen atoms on a designated atom or group are substituted with one or more substituents other than hydrogen without exceeding the normal valence of the designated atom. For example, one or more hydrogens of alkyl, alkylene, alkenyl, alkynyl, hydroxy, amino, or the like may be substituted with one or more substituents. The substituents include, but are not limited to, alkyl, alkenyl, alkynyl, alkoxy, acyl, amino, amido, amidino, aryl, azido, carbamoyl, carboxy, carboxylate, cyano, guanidino, halogen, haloalkyl, heteroalkyl, heteroaryl, heterocyclyl, hydroxy, hydrazino, imino, oxo, nitro, alkylsulfinyl, sulfonic acid, alkylsulfonyl, thiocyanate, thiol, thione, or combinations thereof. The definition of "substituted" does not include analogous indeterminate structures obtained by defining substituents having further substituents attached to infinity (e.g., substituted aryl having substituted alkyl is itself substituted with substituted aryl, which is further substituted with substituted heteroalkyl, and the like). Unless otherwise specified, the maximum number of consecutive substitutions in the compound described herein is three. For example, the consecutive substitutions of substituted aryl with two other substituted aryls are limited to ((substituted aryl)substituted aryl)substituted aryl. Similarly, the definitions described above do not include impermissible substitution patterns (e.g., methyl substituted with 5 fluorines or heteroaryl having two adjacent oxygen ring atoms). Such impermissible substitution patterns are well known to those skilled in the art. When used to modify a chemical group, "substituted" may describe other chemical groups as defined herein. For example, the term "substituted aryl" includes, but is not limited to, "alkylaryl". Unless otherwise specified, if a group is described as optionally substituted, any substituent of the group is itself unsubstituted. "Optional" or "optionally" means that the subsequently described event or circumstance may, but does not necessarily, occur, and the description includes instances where the event or circumstance occurs and instances where it does not.

Unless otherwise specified, it will be understood that the word "comprise" or variations thereof such as "comprises" or "comprising" refers to the inclusion of a stated element or integer or a group of elements or integers, but not the exclusion of any other element or integer or a group of elements or integers.

The substituent "-O-CH₂-O-" means that two oxygen atoms in the substituent are linked to two adjacent carbon atoms in the heterocycloalkyl, aryl, or heteroaryl, for example:

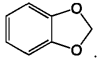

When the number of a linker group is 0, such as -(CH2)₀-, it means that the linker group is a single bond.

When one of the variables is selected from a chemical bond, it means that the two groups linked by this variable are linked directly. For example, when L in X-L-Y represents a chemical bond, it means that the structure is actually X-Y.

The term "membered ring" includes any cyclic structure. The term "membered" is intended to refer to the number of backbone atoms that form a ring. For example, cyclohexyl, pyridinyl, pyranyl and thiopyranyl are six-membered rings, and cyclopentyl, pyrrolyl, furanyl, and thienyl are five-membered rings.

The term "moiety" refers to a specific portion or functional group of a molecule. A chemical moiety is generally considered to be a chemical entity contained in or attached to a molecule.

The term "isomer" refers to any tautomer, stereoisomer, atropisomer, isotopic isomer, enantiomer, or diastereomer of any compound of the present disclosure. The compound of the present disclosure may have one or more chiral centers or double bonds, and thus exists in the form of stereoisomers, e.g., double bond isomers (i.e., E/Z geometric isomers), or diastereomers (e.g., enantiomers (i.e., (+) or (-)) or cis/trans isomers). The compound of the present disclosure therefore encompasses all corresponding stereoisomers, i.e., stereoisomerically pure (e.g., geometrically pure, enantiomerically pure, or diastereomerically pure) forms, as well as mixtures of enantiomers and stereoisomers, e.g., racemates. The mixtures of enantiomers and stereoisomers of the compound of the present disclosure may be resolved into their component enantiomers or stereoisomers by well-known methods, such as chiral gas chromatography and chiral high-performance liquid chromatography, and by crystallization of the compound in the form of chiral salt complexes or in chiral solvents. Enantiomers and stereoisomers may also be obtained from stereomerically pure or enantiomerically pure intermediates, reagents, and catalysts by well-known asymmetric synthetic methods.

The term "isotopic isomer" refers to distinct molecules that differ in structure only by their isotopic composition and are identical in the remaining structure.

The term "atropisomer" refers to a conformational stereoisomer that results when rotation about a single bond within a molecule is hindered or greatly slowed due to the steric interaction with other parts of the molecule, and the substituents at both ends of the single bond are asymmetric, i.e., the atropisomer does not require a stereocenter. In the case of sufficiently high rotational hindrance around the single bond and sufficiently slow interconversion between conformations, the separation of individual isomers may be allowed (LaPlante et al., *J. Med. Chem.* 2011, 54, 20, 7005), preferably by a chiral resolution method.

Unless otherwise stated, the absolute configuration of a stereogenic center is represented by a wedged solid bond ( 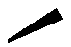 ) and a wedged dashed bond ( 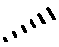 ), and the relative configuration of a stereogenic center is represented by a straight solid bond ( 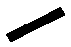 ) and a straight dashed bond ( 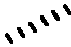 ). A wavy line ( 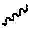 ) represents a wedged solid bond ( 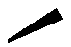 ) or a wedged dashed bond ( 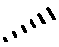 ), or a wavy line ( 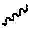 ) represents a straight solid bond ( 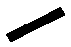 ) or a straight dashed bond ( 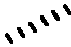 ).

Unless otherwise stated, a single bond or a double bond is represented by -̅ -̅ -̅ .

### Specific Pharmaceutical and Medical Terminology

The term "acceptable", as used herein, means that a formula component or an active ingredient does not unduly and adversely affect a general therapeutic target's health.

The terms "treatment", "treatment course", and "therapy", as used herein, include alleviating, inhibiting, or ameliorating a symptom or condition of a disease; inhibiting the development of complications; ameliorating or preventing underlying metabolic syndrome; inhibiting the development of a disease or symptom, e.g., controlling the progression of a disease or condition; alleviating a disease or symptom; leading to disease or symptom regression; and alleviating a complication caused by a disease or symptom, or preventing or treating a sign caused by a disease or symptom. As used herein, a compound or pharmaceutical composition, when administered, can ameliorate a disease, symptom, or condition, which particularly refers to ameliorating the severity, delaying the onset, slowing the progression, or reducing the duration of the disease. Fixed or temporary administration, or continuous or intermittent administration, may be attributed to or associated with the administration.

"Active ingredient" refers to the compound of general formula (1), and pharmaceutically acceptable inorganic or organic salts of the compound of general formula (1). The compound of the present disclosure may contain one or more asymmetric centers (chiral center or axial chirality) and thus occurs in the forms of a racemate, a racemic mixture, a single enantiomer, a diastereomeric compound, and a single diastereomer. Asymmetric centers that may be present depend on the nature of the various substituents on the molecule. Each of such asymmetric centers will independently produce two optical isomers, and all possible optical isomers, diastereomeric mixtures and pure or partially pure compounds are included within the scope of the present disclosure. The present disclosure is meant to include all such isomeric forms of these compounds.

The terms such as "compound", "composition", "agent", or "medicine or medicament" are used interchangeably herein and all refer to a compound or composition that, when administered to an individual (human or animal), is capable of inducing a desired pharmacological and/or physiological response by local and/or systemic action.

The term "administered, administering, or administration" refers herein to the direct administration of the compound or composition, or the administration of a prodrug, derivative, analog, or the like of the active compound.

Although the numerical ranges and parameters defining the broad scope of the present disclosure are approximations, the related numerical values set forth in the specific examples have been presented herein as precisely as possible. Any numerical value, however, inherently contains a standard deviation necessarily resulting from certain methods of testing. Herein, "about" generally means that the actual numerical value is within a particular numerical value or range ± 10%, 5%, 1%, or 0.5%. Alternatively, the term "about" indicates that the actual numerical value falls within the acceptable standard error of a mean, as considered by those skilled in the art. All ranges, quantities, numerical values, and percentages used herein (e.g., to describe an amount of a material, a length of time, a temperature, an operating condition, a quantitative ratio, and the like) are to be understood as being modified by the word "about", except in the experimental examples or where otherwise explicitly indicated. Accordingly, unless otherwise contrarily stated, the numerical parameters set forth in the specification and the appended claims are all approximations that may vary as desired. At least, these numerical parameters should be understood as the significant digits indicated or the numerical values obtained using conventional rounding rules.

Unless otherwise defined in the specification, the scientific and technical terms used herein have the same meaning as commonly understood by those skilled in the art. Furthermore, nouns in their singular forms used in the specification encompass their plural forms, unless contradicted by context; nouns in their plural forms used also encompass their singular forms.

### Therapeutic Use

The compound of general formula (1) or the pharmaceutical composition of the present disclosure is generally useful for inhibiting SAE protein, and therefore, for treating one or more disorders related to the activity of SAE protein. Therefore, in certain embodiments, the present disclosure provides a method for treating SAE protein-mediated disorders, which comprises the step of administering to a patient in need thereof the compound of general formula (1) or the pharmaceutically acceptable composition thereof of the present disclosure.

In some embodiments, a method for treating cancer is provided, the method comprising administering to an individual in need thereof an effective amount of any of the aforementioned pharmaceutical compositions comprising the compound of structural general formula (1). In some embodiments, the cancer includes but is not limited to hematologic malignancies (leukemia, lymphoma, myeloma including multiple myeloma, myelodysplastic syndrome, and myeloproliferative syndrome) and solid tumors (carcinomas such as prostate, breast, lung, colon, pancreas, kidney, ovary, and soft tissue cancers, osteosarcoma, and interstitial tumors), and the like, preferably lung cancer, cervical cancer, colorectal cancer, lymphoma, myeloma, leukemia, hepatocellular carcinoma, pancreatic cancer, kidney cancer, breast cancer, head and neck cancer, melanoma, prostate cancer, adrenal cancer, endometrial cancer, appendiceal cancer, and metastasis of these cancers.

### Route of administration

The compound and the pharmaceutically acceptable salt thereof of the present disclosure can be made into various formulations comprising a safe and effective amount of the compound or the pharmaceutically acceptable salt thereof of the present disclosure, and a pharmaceutically acceptable excipient or carrier. The "safe and effective amount" means that the amount of the compound is sufficient to significantly improve the condition without causing serious side effects. The safe and effective amount of the compound is determined according to the age, condition, course of treatment, and other specific conditions of a treated subject.

The "pharmaceutically acceptable excipient or carrier" refers to one or more compatible solid or liquid fillers or gel substances that are suitable for human use and must be of sufficient purity and sufficiently low toxicity. "Compatible" herein means that the components of the composition are capable of intermixing with the compound of the present disclosure and with each other, without significantly diminishing the pharmaceutical efficacy of the compound. Examples of pharmaceutically acceptable excipients or carriers include cellulose and derivatives thereof (e.g., sodium carboxymethylcellulose, sodium ethylcellulose, or cellulose acetate), gelatin, talc, solid lubricants (e.g., stearic acid or magnesium stearate), calcium sulfate, vegetable oil (e.g., soybean oil, sesame oil, peanut oil, or olive oil), polyols (e.g., propylene glycol, glycerol, mannitol, or sorbitol), emulsifiers (e.g., Tween^{®}), wetting agents (e.g., sodium lauryl sulfate), colorants, flavoring agents, stabilizers, antioxidants, preservatives, pyrogen-free water, etc.

When the compound of the present disclosure is administered, it may be administered orally, rectally, parenterally (intravenously, intramuscularly, or subcutaneously), or topically.

Solid dosage forms for oral administration include capsules, tablets, pills, pulvises, and granules. In these solid dosage forms, the active compound is mixed with at least one conventional inert excipient (or carrier), such as sodium citrate or dicalcium phosphate, or with the following ingredients: (a) fillers or extenders, such as starch, lactose, sucrose, glucose, mannitol, and silicic acid; (b) binders, such as hydroxymethyl cellulose, alginate, gelatin, polyvinylpyrrolidone, sucrose, and acacia; (c) humectants, such as glycerol; (d) disintegrants, such as agar, calcium carbonate, potato or tapioca starch, alginic acid, certain complex silicates, and sodium carbonate; (e) solution retarders, such as paraffin; (f) absorption accelerators, such as quaternary ammonium compounds; (g) wetting agents, such as cetyl alcohol and glycerol monostearate; (h) adsorbents, such as kaolin; and (i) lubricants, such as talc, calcium stearate, magnesium stearate, solid polyethylene glycol and sodium lauryl sulfate, or mixtures thereof. In the case of capsules, tablets, and pills, the dosage forms may further include buffers.

Solid dosage forms such as tablets, dragees, capsules, pills and granules can be prepared using coatings and shells such as enteric coatings and other materials well known in the art. They may include opacifying agents, and the active compound or compound in such a composition may be released in a certain part of the digestive tract in a delayed manner. Examples of embedding components that can be used are polymeric substances and wax-based substances. If necessary, the active compound can also be in microcapsule form with one or more of the excipients described above.

Liquid dosage forms for oral administration include pharmaceutically acceptable emulsions, solutions, suspensions, syrups, and elixirs. In addition to the active compound, the liquid dosage form may include inert diluents commonly used in the art, such as water or other solvents, solubilizers and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil and sesame oil, or mixtures of these substances.

Besides such inert diluents, the composition may further include adjuvants, such as wetting agents, emulsifiers, suspending agents, sweeteners, flavoring agents, and perfuming agents.

In addition to the active compound, suspensions may include suspending agents, such as ethoxylated isostearyl alcohols, polyoxyethylene sorbitol and sorbitan esters, microcrystalline cellulose, aluminum methylate and agar, or mixtures of these substances.

Compositions for parenteral injection may include physiologically acceptable sterile aqueous or anhydrous solutions, dispersions, suspensions or emulsions, and sterile powders for redissolving into sterile injectable solutions or dispersions. Suitable aqueous and non-aqueous carriers, diluents, solvents, or excipients include water, ethanol, polyols, and suitable mixtures thereof.

Dosage forms for topical administration of the compound of the present disclosure include ointments, pulvises, patches, sprays, and inhalants. The active ingredient is mixed under sterile conditions with a physiologically acceptable carrier and any preservatives, buffers or propellants that may be required if necessary.

The compound of the present disclosure may be administered alone or in combination with other pharmaceutically acceptable compounds. When the pharmaceutical composition is used, a safe and effective amount of the compound of the present disclosure is administered to a mammal (such as a human) to be treated, wherein the dose of administration is a pharmaceutically effective dose. For a human of 60 kg, the daily dose of administration is usually 1-2000 mg, preferably 50-1000 mg. In determining a specific dose, such factors as the route of administration, the health condition of the patient and the like will also be considered, which are well-known to skilled physicians.

The above features mentioned in the present disclosure or those mentioned in the examples may be combined arbitrarily. All the features disclosed in this specification may be used with any composition form and the various features disclosed in this specification may be replaced with any alternative features that provide the same, equivalent, or similar purpose. Thus, unless otherwise specified, the features disclosed herein are merely general examples of equivalent or similar features.

### DETAILED DESCRIPTION

Various specific aspects, features, and advantages of the compounds, methods, and pharmaceutical compositions described above will be set forth in detail in the following description, which will make the content of the present disclosure very clear. It should be understood that the detailed description and examples below describe specific examples for reference only. After reading the description of the present disclosure, those skilled in the art can make various changes or modifications to the present disclosure, and such equivalents also fall within the scope of the present application defined herein.

In all the examples, ¹H-NMR spectra were recorded with a Varian Mercury 400 nuclear magnetic resonance spectrometer, and chemical shifts are represented by δ (ppm); silica gel for separation was 200-300 mesh silica gel if not specified, and the ratio of the eluents was a volume ratio.

The following abbreviations are used in the present disclosure: (Boc)₂O for di-tert-butyl dicarbonate; CDCl₃ for deuterated chloroform; (COCl)₂ for oxalyl chloride; Cs₂CO₃ for cesium carbonate; EtOAc for ethyl acetate; Hexane for n-hexane; HPLC for high-performance liquid chromatography; MeCN for acetonitrile; DCM for dichloromethane; DIPEA for diisopropylethylamine; Dioxane for 1,4-dioxane; DME for glycol dimethyl ether; DMF for N,N-dimethylformamide; DMAP for 4-(dimethylamino)pyridine; DMSO for dimethyl sulfoxide; EtOH for ethanol; h for hour; IPA for isopropanol; ISCO^{®} for a Biotage Isolera Prime flash preparative liquid chromatograph; min for minute; K₂CO₃ for potassium carbonate; KOAc for potassium acetate; KOH for potassium hydroxide; K₃PO₄ for potassium phosphate; LiBH₄ for lithium borohydride; LiHMDS for lithium bis(trimethylsilyl)amide; min for minute; MeOH for methanol; MeONa for sodium methoxide; MS for mass spectrometry; NaBH(OAc)₃ for sodium triacetoxyborohydride; NaH for sodium hydrogen; n-BuLi for n-butyllithium; NMR for nuclear magnetic resonance; NIS for iodosuccinimide; PBST for a phosphate-buffered saline solution containing Tween; Pd/C for palladium on carbon; Pd(PPh₃)₄ for tetrakis(triphenylphosphine)palladium; Pd(OAc)₂ for palladium acetate; Pd(dppf)Cl₂ for [1,1'-bis(diphenylphosphino)ferrocene]dichloropalladium(II); Pd(dtbpf)Cl₂ for Dichloro(1,1'-bis(di-tert-butylphosphino)ferrocene)palladium(II); PE for petroleum ether; PFA for paraformaldehyde; PhNTf₂ for N-Phenylbis(trifluoromethanesulfonimide); PPh₃ for triphenylphosphine; TEA for triethylamine; TFA for trifluoroacetic acid; TMSCl for trimethylchlorosilane; TsOH for p-toluenesulfonic acid; XantPhos for 4,5-bis(diphenylphosphino)-9,9-dimethylxanthene; TfOH for trifluoromethanesulfonic acid; TLC for thin-layer chromatography; and XPhos for 2-dicyclohexylphosphonium-2',4',6'-triisopropylbiphenyl.

### Example 1. Synthesis of Compound 1

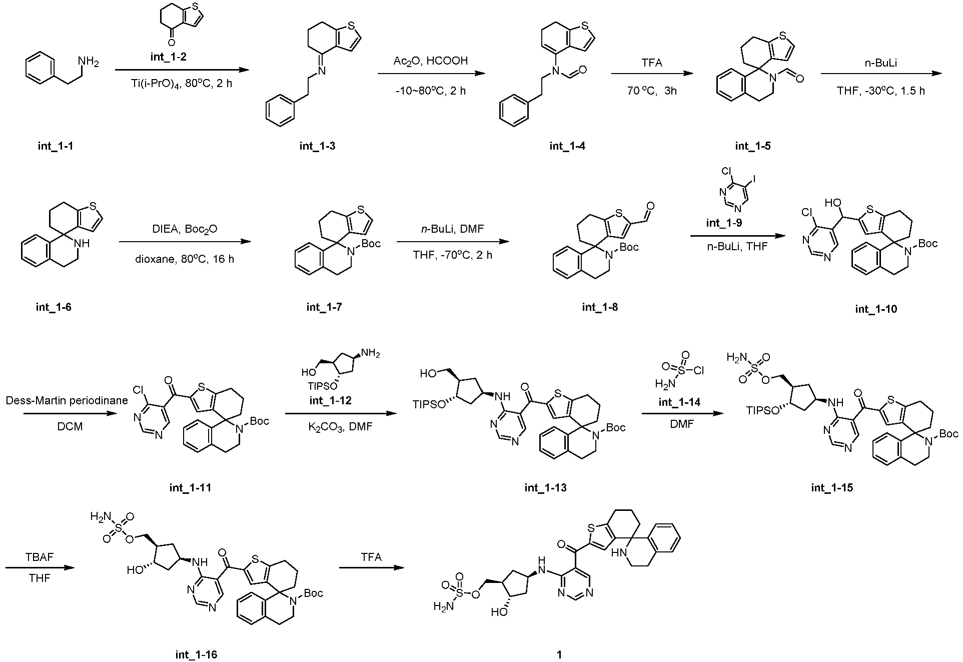

### Step 1: Synthesis of compound int_1-3:

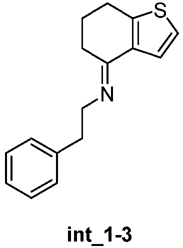

**Int_1-1** (2.8 g, 22.99 mmol) and **int_1-2** (3.5 g, 22.99 mmol, 454.55 µL) were dissolved in Ti(i-PrO)₄ (20 mL). The mixed solution was purged with nitrogen three times, heated to 80 °C, and stirred for 2 h. LC-MS monitoring showed the reaction was completed. The reaction solution was cooled to room temperature, which was directly used in the next step.

ESI-MS m/z: 256 [M+H]⁺.

### Step 2: Synthesis of compound int_1-4:

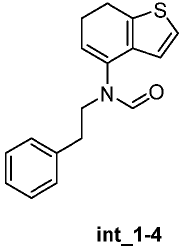

HCOOH (78 mL) was slowly and dropwise added to Ac₂O (194 mL) at 0 °C, and after the addition was completed, the reaction solution was allowed to react at 20 °C for 0.5 h. Then, the **int_1-3** solution obtained in step 1 was cooled to -10 °C, and the mixed solution of HOOCH and Ac₂O described above was slowly and dropwise added to the **int_1-3** solution. The temperature was kept at -10 °C during the dropwise addition. After the dropwise addition was completed, the reaction solution was warmed to 80 °C and allowed to react for 2 h. LC-MS monitoring showed the reaction was completed. The reaction solution was directly used in the next step.

ESI-MS m/z: 284 [M+H]⁺.

### Step 3: Synthesis of compound int_1-5:

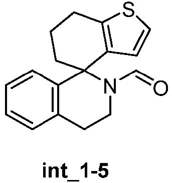

TFA (316 mL) was slowly added to the **int_1-4** solution obtained in step 2 at 70 °C within 1 h. The mixed solution was cooled, stirred at 70 °C, and allowed to react for 3 h. LC-MS monitoring showed the reaction was completed. After the reaction solution was cooled to room temperature, the reaction solution was concentrated under reduced pressure to give a crude product, and the crude product was adjusted to a weakly basic pH with an aqueous sodium bicarbonate solution (400 mL). The aqueous phase was extracted with ethyl acetate (300 mL × 3), and the organic phase was dried over anhydrous sodium sulfate. The organic phase was concentrated under reduced pressure to give a crude product. The crude product was purified by preparative column chromatography (SiO₂, PE/EtOAc = 4/1) to give a solid (3.5 g, yield: 48.6%).

ESI-MS m/z: 284 [M+H]⁺.

### Step 4: Synthesis of compound int_1-6:

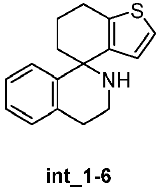

**Int_1-5** (1.2 g, 4.23 mmol) was dissolved in THF (20 mL), and the mixture was cooled to -30 °C. n-BuLi (2.5 M, 3.38 mL) was slowly and dropwise added to the reaction solution under nitrogen atmosphere, and the reaction solution was allowed to react at -30 °C for 2 h. LC-MS monitoring showed the reaction was completed. After the reaction solution was warmed to room temperature, a saturated aqueous ammonium chloride solution (20 mL) was added to the reaction solution. The aqueous phase was extracted with ethyl acetate (50 mL × 3), and the organic phase was dried over anhydrous sodium sulfate. The organic phase was concentrated under reduced pressure to give a crude product, which was directly used in the next step.

ESI-MS m/z: 256 [M+H]⁺.

### Step 5: Synthesis of compound int_1-7:

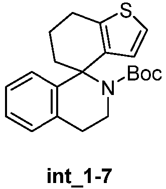

**Int_1-6** (952 mg, 3.73 mmol) and (Boc)₂O (1.22 g, 5.59 mmol, 1.28 mL) were dissolved in dioxane (20 mL), and TEA (1.13 g, 11.18 mmol, 1.56 mL) was added to the reaction solution at room temperature. The reaction solution was warmed to 90 °C and allowed to react for 16 h. LC-MS monitoring showed the reaction was completed. After the reaction solution was cooled to room temperature, the reaction solution was concentrated under reduced pressure to give a crude product. The crude product was purified by preparative column chromatography (SiO₂, PE/EtOAc = 4/1) to give a solid (1.2 g, yield: 83.4%).

ESI-MS m/z: 356 [M+H]⁺.

### Step 6: Synthesis of compound int_1-8:

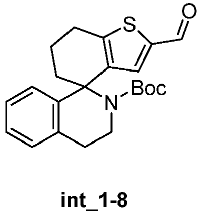

**Int_1-7** (1.1 g, 3.11 mmol) was dissolved in THF (25 mL), and the mixture was cooled to -70 °C. n-BuLi (2.5 M, 3.74 mL) was slowly and dropwise added to the reaction solution under nitrogen atmosphere, and the reaction solution was allowed to react at -70 °C for 0.5 h. Then, DMF (682.55 mg, 9.34 mmol, 718.48 µL) was dropwise added to the reaction solution at -70 °C, and after the dropwise addition was completed, the reaction solution was allowed to react at -60 °C for 1.5 h. LC-MS monitoring showed the reaction was completed. A saturated ammonium chloride solution (20 mL) was slowly added to the reaction solution. The aqueous phase was extracted with ethyl acetate (30 mL × 3), and the organic phase was dried over anhydrous sodium sulfate. The organic phase was concentrated under reduced pressure to give a crude product. The crude product was purified by preparative column chromatography (SiO₂, PE/EtOAc = 3/1) to give a solid (1.08 g, yield: 83.9%).

MS (ESI): 384 [M+H]⁺.

### Step 7: Synthesis of compound int_1-10:

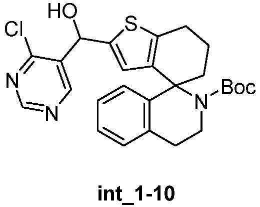

**Int_1-9** (1.50 g, 6.26 mmol) was dissolved in THF (30 mL), and the mixture was cooled to -70 °C. n-BuLi (2.5 M, 5.01 mL) was slowly and dropwise added to the reaction solution under nitrogen atmosphere, and the reaction solution was allowed to react at -70 °C for 1 h. Then, a solution of **int_1-8** (800 mg, 2.09 mmol) in THF (15 mL) was dropwise added to the reaction solution at -70 °C, and after the dropwise addition was completed, the reaction solution was allowed to react at -70 °C for 1 h, and was then warmed to room temperature and allowed to react for 16 h. LC-MS monitoring showed the reaction was completed. A saturated ammonium chloride solution (50 mL) was slowly added to the reaction solution. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography (SiO₂, PE/EtOAc = 2/1) to give a solid (600 mg, yield: 57.7%).
¹H NMR (400MHz, CHLOROFORM-d) δ = 9.07 - 9.02 (m, 1H), 8.96 - 8.91 (m, 1H), 7.19 (d, *J* = 4.4 Hz, 2H), 7.12 - 7.03 (m, 1H), 6.60 - 6.50 (m, 2H), 6.24 - 6.15 (m, 1H), 4.47 - 4.36 (m, 1H), 3.21 (dt, *J* = 2.6, 12.2 Hz, 1H), 3.10 - 2.99 (m, 1H), 2.93 - 2.85 (m, 1H), 2.84 - 2.66 (m, 4H), 1.85 - 1.59 (m, 6H), 1.27 - 1.17 (m, 9H)_{∘}

MS (ESI): 498 [M+H]⁺.

### Step 8: Synthesis of compound int_1-11:

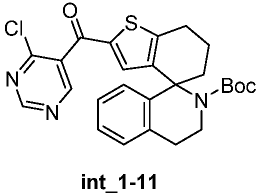

**Int_1-10** (600 mg, 1.20 mmol) was dissolved in DCM (6 mL), and a Dess-Martin oxidant (766.46 mg, 1.81 mmol) was added to the reaction solution at room temperature. The reaction solution was allowed to react at room temperature for 1 h. LC-MS monitoring showed the reaction was completed. A saturated aqueous sodium bicarbonate solution was slowly added to the reaction solution to adjust the pH to about 8. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography (SiO₂, PE/EtOAc = 3/1) to give a solid (550 mg, yield: 92%). MS (ESI): 496 [M+H]⁺.

### Step 9: Synthesis of compound int_1-13:

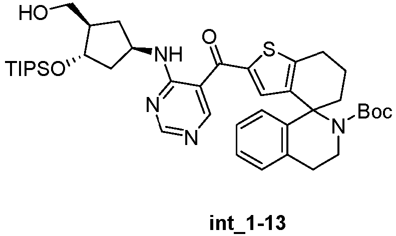

**Int_1-11** (550 mg, 1.11 mmol) and **int_1-12** (638.2 mg, 2.22 mmol) were dissolved in DMF (20 mL), and K₂CO₃ (766.25 mg, 5.54 mmol) was added to the reaction solution at room temperature. The reaction solution was allowed to react at room temperature for 3 h. LC-MS monitoring showed the reaction was completed. Ice water (20 mL) was added to the reaction solution. The aqueous phase was extracted with ethyl acetate (30 mL × 3), and the organic phase was dried over anhydrous sodium sulfate. The organic phase was concentrated under reduced pressure to give a crude product. The crude product was purified by preparative column chromatography (SiO₂, PE/EtOAc = 2/1) to give a product (700 mg, yield: 84.5%).

MS (ESI): 747 [M+H]⁺.

### Step 10: Synthesis of compound int_1-15:

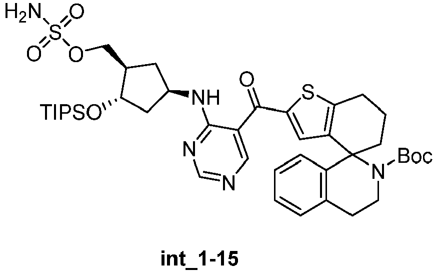

**Int_1-13** (700 mg, 936.99 µmol) was dissolved in DMF (15 mL), and **int_1-14** (216.52 mg, 1.87 mmol) was added to the reaction solution at room temperature. The reaction solution was allowed to react at room temperature for 1 h. LC-MS monitoring showed the reaction was completed. Ice water (20 mL) was added to the reaction solution. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography (SiO₂, PE/EtOAc = 3/1) to give a solid (750 mg, yield: 96.9%).

MS (ESI): 826 [M+H]⁺.

### Step 11: Synthesis of compound int_1-16:

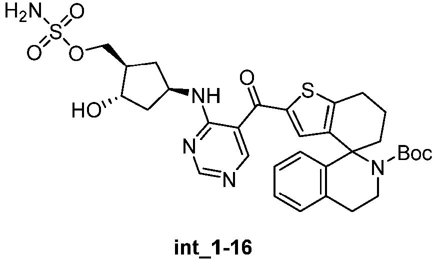

**Int_1-15** (750 mg, 907.83 µmol) was dissolved in THF (20 mL), and TBAF (1 M, 1.82 mL) was added to the reaction solution at room temperature. The reaction solution was allowed to react at room temperature for 4 h. LC-MS monitoring showed the reaction was completed. Water (15 mL) was added to the reaction solution. The aqueous phase was extracted with ethyl acetate (30 mL × 3), and the organic phase was dried over anhydrous sodium sulfate. The organic phase was concentrated under reduced pressure to give a crude product. The crude product was purified by preparative column chromatography (SiO₂, PE/EtOAc = 1/1) to give a solid (500 mg, yield: 82.2%).

¹H NMR (400MHz, CHLOROFORM-d) δ = 8.63 - 8.49 (m, 3H), 7.14 - 7.08 (m, 2H), 7.04 (d, *J* = 2.1 Hz, 1H), 7.03 - 6.97 (m, 1H), 6.46 (d, *J =* 7.6 Hz, 1H), 5.44 (br s, 2H), 4.74 - 4.60 (m, 1H), 4.37 - 4.12 (m, 4H), 3.22 - 3.14 (m, 1H), 3.01 - 2.88 (m, 2H), 2.87 - 2.77 (m, 2H), 2.75 - 2.64 (m, 1H), 2.49 - 2.39 (m, 1H), 2.23 (br d, *J* = 5.1 Hz, 1H), 2.09 - 1.99 (m, 2H), 1.90 (td, *J* = 6.6, 13.2 Hz, 2H), 1.63 (br d, *J* = 13.9 Hz, 1H), 1.44 - 1.32 (m, 1H), 1.27 - 1.19 (m, 9H).

MS (ESI): 670 [M+H]⁺.

### Step 12: Synthesis of compound 1

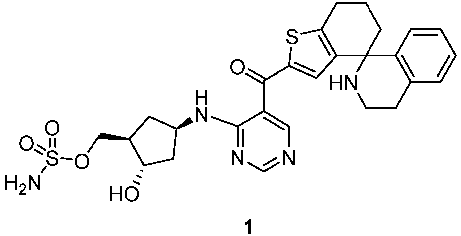

**Int_1-16** (300 mg, 447.89 µmol) was added to TFA (5.11 g, 44.79 mmol, 3.32 mL) at room temperature, and the reaction solution was allowed to react at room temperature for 5 min. LC-MS monitoring showed the reaction was completed. A saturated aqueous sodium bicarbonate solution was added to the reaction solution to adjust the pH to 8, and then the reaction solution was purified by preparative HPLC (column: Boston Prime C18 150 × 30 mm × 5 µm; mobile phase: [water (ammonia hydroxide v/v)-ACN]; B%: 30%-50%,9 min) to give a solid (90 mg, yield: 35.2%).

¹H NMR (400MHz, CHLOROFORM-d) δ = 8.61 (d, *J =* 3.8 Hz, 1H), 8.58 (s, 1H), 8.50 (br t, *J =* 6.3 Hz, 1H), 7.18 - 7.11 (m, 2H), 7.09 (br d, *J* = 7.4 Hz, 1H), 7.01 (s, 1H), 6.78 (br d, *J* = 7.9 Hz, 1H), 4.70 (br d, *J* = 6.4 Hz, 1H), 4.36 (br s, 1H), 4.32 - 4.19 (m, 2H), 3.30 - 3.11 (m, 2H), 3.11 - 3.00 (m, 2H), 3.00 - 2.89 (m, 1H), 2.80 (br d, *J* = 16.5 Hz, 1H), 2.52 (br s, 1H), 2.41 - 2.25 (m, 3H), 2.15 - 1.90 (m, 5H), 1.51 - 1.39 (m, 1H).

MS (ESI): 570 [M+H]⁺.

### Example 2. Synthesis of Compound 2 and Compound 3

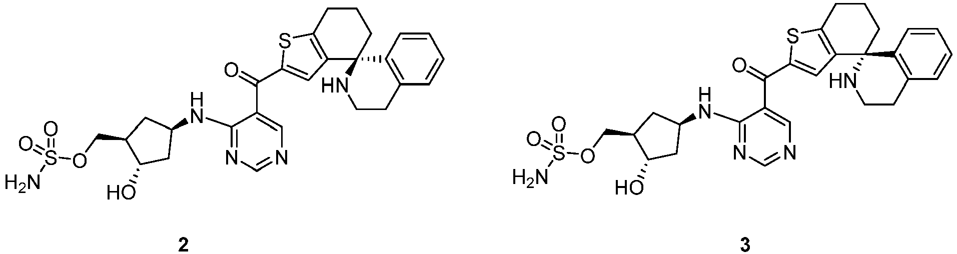

**Compound 1** (0.09 g, 157 µmol) was subjected to SFC chiral resolution (column: DAICEL CHIRALPAK IG (250 mm × 30 mm, 10 µm); mobile phase: [CO₂-EtOH (0.1 %NH₃H₂O)]; B%: 60%, isocratic elution mode) to give **compound 2** (30 mg) and **compound 3** (30 mg).

**Compound 2:** ¹H NMR (400MHz, METHANOL-d4) δ = 8.53 (s, 1H), 8.50 (s, 1H), 7.15 (d, *J* = 4.0 Hz, 2H), 7.09 (dt, *J* = 3.7, 8.0 Hz, 1H), 7.03 (s, 1H), 6.83 (d, *J* = 7.9 Hz, 1H), 4.80 - 4.73 (m, 1H), 4.25 - 4.13 (m, 3H), 3.29 - 3.20 (m, 1H), 3.16 - 2.98 (m, 4H), 2.88 - 2.81 (m, 1H), 2.55 - 2.44 (m, 1H), 2.41 - 2.32 (m, 1H), 2.30 - 2.21 (m, 1H), 2.20 - 2.09 (m, 2H), 2.06 - 1.97 (m, 2H), 1.92 - 1.79 (m, 1H), 1.40 (td, *J* = 9.0, 13.2 Hz, 1H).; MS (ESI): 570 [M+H]⁺.

SFC retention time analysis: 1.580 min (Instrument: Waters UPCC with PDA Detector; Column: Chiralpak IG-3 50x4.6mm I.D., 3um; Mobile phase: A: CO₂, B: Ethanol (0.05% DEA); Isocratic: 40% B; Flow rate: 4mL/min; Column temp.: 35° C; ABPR: 1500psi).

**Compound 3:** ¹HNMR (400MHz, CHLOROFORM-d) δ = 8.61 (s, 1H), 8.57 (s, 1H), 8.50 (br d, *J* = 7.3 Hz, 1H), 7.18 - 7.11 (m, 2H), 7.11 - 7.05 (m, 1H), 7.02 (s, 1H), 6.79 (d, *J* = 7.7 Hz, 1H), 4.78 - 4.65 (m, 1H), 4.36 (dd, *J* = 4.0, 10.1 Hz, 1H), 4.33 - 4.22 (m, 2H), 3.31 - 3.13 (m, 2H), 3.12 - 2.90 (m, 3H), 2.80 (br d, *J* = 16.5 Hz, 1H), 2.57 - 2.44 (m, 1H), 2.40 - 2.26 (m, 2H), 2.16 - 1.92 (m, 7H), 1.48 - 1.41 (m, 1H).; MS (ESI): 570 [M+H]⁺.

SFC retention time analysis: 0.927 min (Instrument: Waters UPCC with PDA Detector; Column: Chiralpak IG-3 50x4.6mm I.D., 3um; Mobile phase: A: CO₂, B: Ethanol (0.05% DEA); Isocratic: 40% B; Flow rate: 4mL/min; Column temp.: 35°C; ABPR: 1500psi).

### Example 3. Synthesis of Compound 4

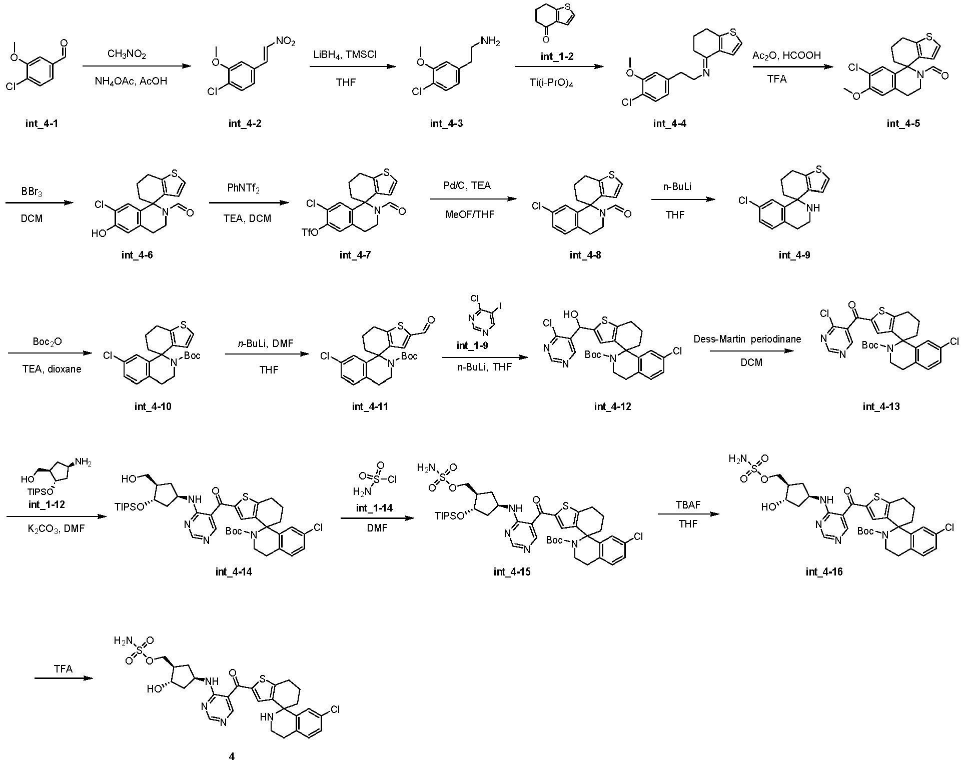

### Step 1: Synthesis of compound int_4-2:

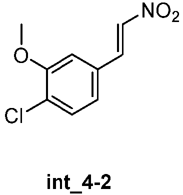

**Int_4-1** (50.00 g, 293.10 mmol) and NH₄OAc (51.96 g, 674.12 mmol) were dissolved in acetic acid (500 mL), and the mixed solution was purged with nitrogen three times. CH₃NO₂ (46.52 g, 762.05 mmol, 41.16 mL) was added to the reaction solution under nitrogen atmosphere, and the reaction solution was heated to 100 °C and stirred for 16 h. LC-MS monitoring showed the reaction was completed. The reaction solution was cooled to room temperature and then poured slowly into 1000 mL of ice water. The aqueous phase was extracted with ethyl acetate (800 mL × 3), and the organic phase was dried over anhydrous sodium sulfate. The organic phase was concentrated under reduced pressure to give a crude product (60 g, yield: 95.8%), which was directly used in the next step.

¹H NMR (400MHz, DMSO-d6) δ = 8.36 (d, *J* = 13.6 Hz, 1H), 8.12 (d, *J* = 13.4 Hz, 1H), 7.67 (d, *J* = 1.5 Hz, 1H), 7.55 (d, *J* = 8.1 Hz, 1H), 7.48 - 7.42 (m, 1H), 3.92 (s, 3H).

### Step 2: Synthesis of compound int_4-3:

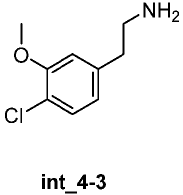

LiBH₄ (20.39 g, 936.25 mmol) was dissolved in tetrahydrofuran (200 mL), and TMSCl (203.43 g, 1.87 mol, 237.66 mL) was added to the reaction solution at 0 °C under nitrogen atmosphere. The reaction solution was stirred at 0 °C for 30 min, and a solution of **int_4-2** (20.00 g, 93.63 mmol) in tetrahydrofuran (200 mL) was slowly and dropwise added to the reaction solution within 15 min. The reaction solution was warmed to 75 °C and allowed to react for another 2 h. LC-MS monitoring showed the reaction was completed. The reaction solution was cooled to 0 °C, and 100 mL of methanol was slowly added to quench the reaction. The organic phase was concentrated under reduced pressure to give a crude product, and the crude product was adjusted to pH>7 with ammonia water. The aqueous phase was extracted with ethyl acetate (300 mL × 3), and the organic phase was dried over anhydrous sodium sulfate. The organic phase was concentrated under reduced pressure to give a crude product (15 g, yield: 86.3%), which was directly used in the next step.

¹H NMR (400MHz, DMSO-d6) δ = 7.31 (d, *J* = 7.9 Hz, 1H), 7.01 (d, *J* = 1.3 Hz, 1H), 6.80 (dd, *J* = 1.7, 8.0 Hz, 1H), 3.85 (s, 3H), 2.92 - 2.80 (m, 2H), 2.78 - 2.64 (m, 2H).

### Step 3: Synthesis of compound int_4-4:

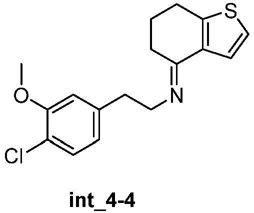

**Int_4-3** (9.00 g, 48.48 mmol) and **int_1-2** (8.12 g, 53.33 mmol) were dissolved in Ti(i-PrO)₄ (170 mL). The mixed solution was purged with nitrogen three times, heated to 80 °C, and stirred for 2 h. LC-MS monitoring showed the reaction was completed. The reaction solution was cooled to room temperature, which was directly used in the next step.

ESI-MS m/z: 320 [M+H]⁺.

### Step 4: Synthesis of compound int_4-5:

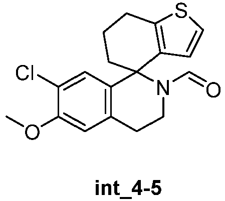

HCOOH (200 mL) was slowly and dropwise added to Ac₂O (500 mL) at -10°C, and after the addition was completed, the reaction solution was allowed to react at 20 °C for 0.5 h. Then, the **int_4-4** solution obtained in step 3 was cooled to -10 °C, and the mixed solution of HOOCH and Ac₂O described above was slowly and dropwise added to the **int_4-4** solution. The temperature was kept at -10 °C during the dropwise addition. After the dropwise addition was completed, the reaction solution was warmed to 70 °C and allowed to react for 3 h. LC-MS monitoring showed the reaction was completed. The reaction solution was cooled to room temperature. The organic phase was concentrated under reduced pressure to give a crude product, and the crude product was adjusted to pH>7 with a saturated aqueous sodium bicarbonate solution. The aqueous phase was extracted with ethyl acetate (500 mL × 3), and the organic phase was dried over anhydrous sodium sulfate. The organic phase was concentrated under reduced pressure to give a crude product. The crude product was purified by preparative column chromatography (SiO₂, PE/EtOAc = 1/1) to give a solid (2 g, yield: 10.6%).

¹H NMR (400MHz, DMSO-d6) δ = 7.57 (s, 1H), 7.32 (d, *J* = 5.1 Hz, 1H), 6.98 (s, 1H), 6.72 (s, 1H), 6.24 (d, *J* = 5.3 Hz, 1H), 4.40 (td, *J* = 3.9, 12.9 Hz, 1H), 3.85 (s, 3H), 3.24 - 3.12 (m, 1H), 2.93 (br dd, *J* = 3.7, 7.9 Hz, 2H), 2.89 - 2.79 (m, 2H), 2.35 (br dd, *J* = 2.0*,* 11.9 Hz, 1H), 2.01 - 1.81 (m, 2H), 1.70 - 1.53 (m, 1H). ESI-MS m/z: 348 [M+H]⁺.

### Step 5: Synthesis of compound int_4-6:

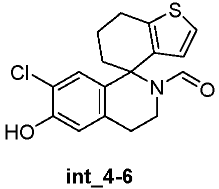

**Int_4-5** (3.30 g, 9.49 mmol) was dissolved in dichloromethane (30 mL), and the mixed solution was purged with nitrogen three times. The reaction solution was cooled to 0 °C, and BBr₃ (11.88 g, 47.43 mmol, 4.57 mL) was slowly and dropwise added to the reaction solution. The reaction solution was warmed to room temperature and allowed to react for 1 h. LC-MS monitoring showed the reaction was completed. The reaction solution was poured slowly into 300 mL of ice water. The aqueous phase was extracted with ethyl acetate (300 mL × 3), and the organic phase was dried over anhydrous sodium sulfate. The organic phase was concentrated under reduced pressure to give a crude product (2.8 g, yield: 88.4%), which was directly used in the next step. ¹H NMR (400MHz, DMSO-d6) δ = 10.36 (s, 1H), 7.72 (s, 1H), 7.48 (d, *J* = 5.1 Hz, 1H), 6.91 (s, 1H), 6.79 (s, 1H), 6.41 (d, *J* = 5.3 Hz, 1H), 4.57 - 4.45 (m, 1H), 3.38 - 3.28 (m, 1H), 3.15 - 3.04 (m, 2H), 2.98 - 2.85 (m, 2H), 2.55 - 2.44 (m, 1H), 2.14 - 1.97 (m, 2H), 1.86 - 1.66 (m, 1H).

ESI-MS m/z: 334 [M+H]⁺.

### Step 6: Synthesis of compound int_4-7:

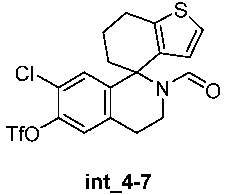

**Int_4-6** (2.80 g, 8.39 mmol), PhNTf₂ (5.39 g, 15.10 mmol), and TEA (2.12 g, 20.97 mmol, 2.92 mL) were dissolved in dichloromethane (30 mL). The reaction solution was purged with nitrogen three times, and allowed to react at room temperature for 16 h. LC-MS monitoring showed the reaction was completed. The reaction solution was poured slowly into 300 mL of ice water. The aqueous phase was extracted with ethyl acetate (300 mL × 3), and the organic phase was dried over anhydrous sodium sulfate. The organic phase was concentrated under reduced pressure to give a crude product. The crude product was purified by preparative column chromatography (SiO₂, PE/EtOAc = 1/1) to give a solid (2.3 g, yield: 58.8%).

¹H NMR (400MHz, DMSO-d6) δ = 7.45 - 7.39 (m, 1H), 7.36 (d, *J* = 5.3 Hz, 1H), 7.31 - 7.24 (m, 1H), 7.12 (s, 1H), 6.28 (d, *J* = 5.3 Hz, 1H), 4.51 - 4.38 (m, 1H), 3.02 - 2.85 (m, 4H), 2.42 (td, *J* = 2.0, 12.0 Hz, 2H), 2.06 - 1.94 (m, 1H), 1.90 (br d, *J* = 13.8 Hz, 1H), 1.94 - 1.83 (m, 1H), 1.68 - 1.50 (m, 1H) .

ESI-MS m/z: 466 [M+H]⁺.

### Step 7: Synthesis of compound int_4-8:

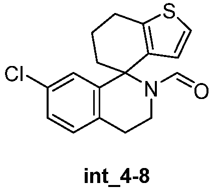

**Int_4-7** (2.30 g, 4.94 mmol), Pd/C (1.05 g, 10% purity), and TEA (2.00 g, 19.75 mmol, 2.75 mL) were dissolved in a mixed solvent of methanol (21 mL) and tetrahydrofuran (7 mL). The reaction solution was purged with hydrogen three times, and allowed to react at room temperature for 16 h. LC-MS monitoring showed the reaction was completed. The reaction solution was filtered to give a filtrate, and the filtrate was concentrated under reduced pressure to give a crude product. The crude product was purified by preparative column chromatography (SiO₂, PE/EtOAc = 1/1) to give a solid (0.64 g, yield: 40.7%).

¹H NMR (400MHz, DMSO-d6) δ = 7.57 (s, 1H), 7.34 (d, *J* = 5.3 Hz, 1H), 7.25 (d, *J* = 0.9 Hz, 2H), 6.76 (s, 1H), 6.24 (d, *J* = 5.3 Hz, 1H), 4.41 (td, *J* = 4.1, 13.1 Hz, 1H), 3.25 - 3.13 (m, 1H), 2.95 (dd, *J* = 3.9, 7.8 Hz, 2H), 2.86 (dd, *J* = 4.5, 8.3 Hz, 2H), 2.45 - 2.35 (m, 1H), 2.03 - 1.95 (m, 1H), 1.95 - 1.83 (m, 1H), 1.69 - 1.51 (m, 1H).

ESI-MS m/z: 318 [M+H]⁺.

### Step 8: Synthesis of compound int_4-9:

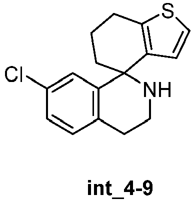

**Int_4-8** (0.64 g, 2.01 mmol) was dissolved in THF (10 mL), and the mixture was cooled to -30 °C. n-BuLi (2.5 M, 2.42 mL) was slowly and dropwise added to the reaction solution under nitrogen atmosphere, and the reaction solution was allowed to react at -30 °C for 2 h. Subsequently, the reaction solution was warmed to room temperature and allowed to react for 1 h. LC-MS monitoring showed the reaction was completed. A saturated aqueous ammonium chloride solution (10 mL) was added to the reaction solution. The aqueous phase was extracted with ethyl acetate (30 mL × 3), and the organic phase was dried over anhydrous sodium sulfate. The organic phase was concentrated under reduced pressure to give a crude product (0.5 g, yield: 85.6%), which was directly used in the next step.

ESI-MS m/z: 290 [M+H]⁺.

### Step 9: Synthesis of compound int_4-10:

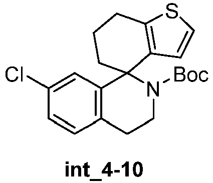

**Int_4-9** (0.50 g, 1.73 mmol) and (Boc)₂O (828.34 mg, 3.80 mmol, 871.93 µL) were dissolved in 1,4-dioxane (10 mL), and TEA (523.71 mg, 5.18 mmol, 720.37 µL) was added to the reaction solution at room temperature. The reaction solution was warmed to 80 °C and allowed to react for 16 h. LC-MS monitoring showed the reaction was completed. After the reaction solution was cooled to room temperature, the reaction solution was concentrated under reduced pressure to give a crude product. The crude product was purified by preparative column chromatography (SiO₂, PE/EtOAc = 10/1) to give a solid (0.11 g, yield: 16.3%).

¹H NMR (400MHz, DMSO-d6) δ = 7.33 - 7.23 (m, 3H), 6.53 (d, *J* = 5.1 Hz, 1H), 6.40 (d, *J* = 2.0 Hz, 1H), 4.27 (td, *J* = 3.7, 12.0 Hz, 1H), 3.11 (ddd, *J* = 5.1, 9.8, 12.2 Hz, 1H), 2.97 - 2.87 (m, 3H), 2.75 - 2.63 (m, 2H), 1.77 (br d*, J* = 13.2 Hz, 1H), 1.65 - 1.49 (m, 2H), 1.15 (s, 9H).

### Step 10: Synthesis of compound int_4-11:

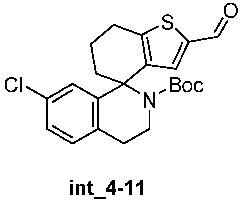

**Int_4-10** (1.44 g, 3.693 mmol) was dissolved in THF (20 mL), and the mixture was cooled to -75 °C. n-BuLi (2.5 M, 4.43 mL) was slowly and dropwise added to the reaction solution under nitrogen atmosphere, and the reaction solution was allowed to react at -75 °C for 1 h. Then, DMF (809 mg, 11.079 mmol) was dropwise added to the reaction solution at -75 °C, and after the dropwise addition was completed, the reaction solution was allowed to react at -75 °C for 1 h. LC-MS monitoring showed the reaction was completed. A saturated ammonium chloride solution (100 mL) was slowly added to the reaction solution. The aqueous phase was extracted with ethyl acetate (100 mL × 3), and the organic phase was dried over anhydrous sodium sulfate. The organic phase was concentrated under reduced pressure to give a crude product. The crude product was purified by preparative column chromatography to give a solid (1.38 g, yield: 89.9%).

MS (ESI): 418 [M+H]⁺.

### Step 11: Synthesis of compound int_4-12:

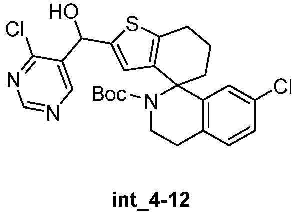

**Int_1-9** (4.79 g, 19.911 mmol) was dissolved in THF (50 mL), and the mixture was cooled to -75 °C. n-BuLi (2.5 M, 16 mL) was slowly and dropwise added to the reaction solution under nitrogen atmosphere, and the reaction solution was allowed to react at -75 °C for 1 h. Then, a solution of **int_4-11** (1.387 g, 3.319 mmol) in THF (25 mL) was dropwise added to the reaction solution at -75 °C, and after the dropwise addition was completed, the reaction solution was allowed to react at -75 °C for 1 h, and was then warmed to room temperature and allowed to react for 16 h. LC-MS monitoring showed the reaction was completed. A saturated ammonium chloride solution (200 mL) was slowly added to the reaction solution. The aqueous phase was extracted with ethyl acetate (200 mL × 3), and the organic phase was dried over anhydrous sodium sulfate. The organic phase was concentrated under reduced pressure to give a crude product. The crude product was purified by preparative column chromatography to give a solid (1.427 g, yield: 80%).

MS (ESI): 532 [M+H]⁺.

### Step 12: Synthesis of compound int_4-13:

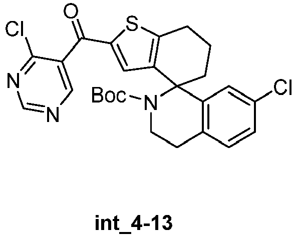

**Int_4-12** (1.427 g, 2.68 mmol) was dissolved in DCM (50 mL), and a Dess-Martin oxidant (1.36 g, 3.22 mmol) was added to the reaction solution at room temperature. The reaction solution was allowed to react at room temperature for 1 h. LC-MS monitoring showed the reaction was completed. A saturated aqueous sodium bicarbonate solution was slowly added to the reaction solution to adjust the pH to about 8. The aqueous phase was extracted with ethyl acetate (100 mL × 3), and the organic phase was dried over anhydrous sodium sulfate. The organic phase was concentrated under reduced pressure to give a crude product. The crude product was purified by preparative column chromatography to give a solid (1.324 g, yield: 93%).

MS (ESI): 530 [M+H]⁺.

### Step 13: Synthesis of compound int_4-14:

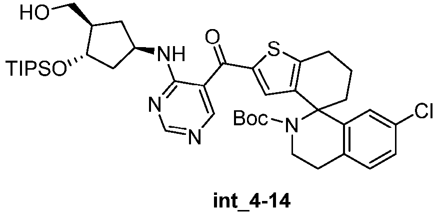

**Int_4-13** (1.053 g, 1.985 mmol) and **int_1-12** (628 mg, 2.184 mmol) were dissolved in DMF (10 mL), and K₂CO₃ (824 mg, 5.962 mmol) was added to the reaction solution at room temperature. The reaction solution was allowed to react at room temperature for 3 h. LC-MS monitoring showed the reaction was completed. Ice water (100 mL) was added to the reaction solution. The aqueous phase was extracted with ethyl acetate (100 mL × 3), and the organic phase was dried over anhydrous sodium sulfate. The organic phase was concentrated under reduced pressure to give a crude product. The crude product was purified by preparative column chromatography to give a product (1.425 g, yield: 91%).

MS (ESI): 781 [M+H]⁺.

### Step 14: Synthesis of compound int_4-15:

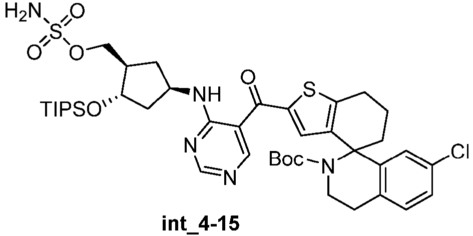

**Int_4-14** (1.425 g, 1.823 mmol) was dissolved in DMF (30 mL), and **int_1-14** (421 mg, 3.647 mmol) was added to the reaction solution at room temperature. The reaction solution was allowed to react at room temperature for 1 h. LC-MS monitoring showed the reaction was completed. Ice water (50 mL) was added to the reaction solution. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography to give a solid (1.5 g, yield: 96.1%).

MS (ESI): 860 [M+H]⁺.

### Step 15: Synthesis of compound int_4-16:

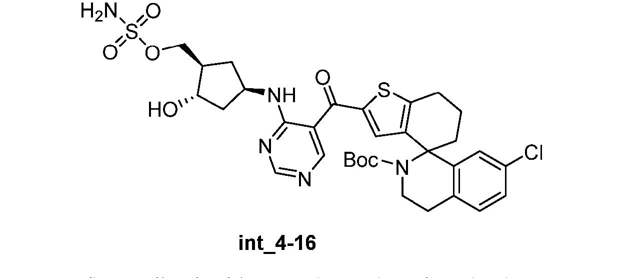

**Int_4-15** (1.5 g, 1.742 mmol) was dissolved in THF (20 mL), and TBAF (1 M, 3.6 mL) was added to the reaction solution at room temperature. The reaction solution was allowed to react at room temperature for 4 h. LC-MS monitoring showed the reaction was completed. Water (50 mL) was added to the reaction solution. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography to give a solid (1.1 g, yield: 90.1%). MS (ESI): 704 [M+H]⁺.

### Step 16: Synthesis of compound 4

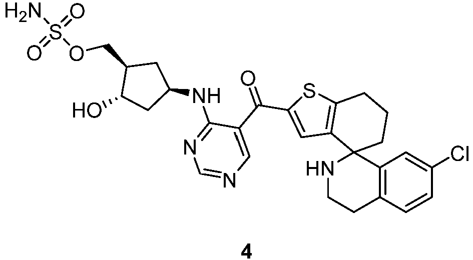

**Int_4-16** (1.28 g, 1.823 mmol) was dissolved in DCM (7 mL) at room temperature, and the mixture was added into TFA (7.8 mL). The reaction solution was allowed to react at room temperature for 5 min. LC-MS monitoring showed the reaction was completed. The reaction solution was cooled to 0 °C. A saturated aqueous sodium bicarbonate solution was added to the reaction solution to adjust the pH to 8. The aqueous phase was extracted with ethyl acetate (50 mL × 3), and the organic phase was dried over anhydrous sodium sulfate. The organic phase was concentrated under reduced pressure to give a crude product. The crude product was purified by preparative HPLC to give a solid (897 mg, yield: 81.5%).

MS (ESI): 604 [M+H]⁺.

### Example 4. Synthesis of Compound 5 and Compound 6

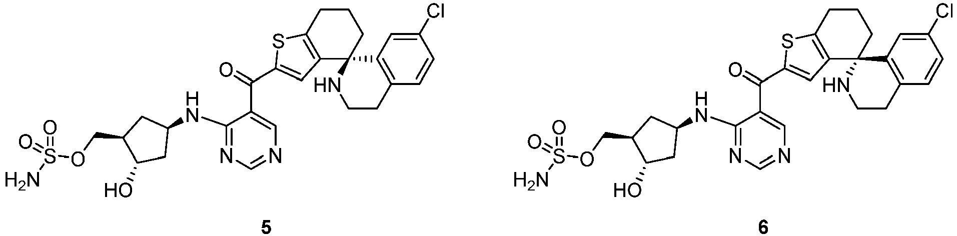

**Compound 4** (100 mg, 0.165 mmol) was subjected to SFC chiral resolution (column: DAICEL CHIRALPAK IG (250 mm × 30 mm, 10 µm); mobile phase: [CO₂-EtOH (0.1% NH₃H₂O)]; B%: 60%, isocratic elution mode) to give **compound 5** (45 mg) and **compound 6** (40 mg).

**Compound 5:** ¹H NMR (400 MHz, DMSO-d6) δ 8.55 (s, 1H), 8.50 (s, 1H), 8.24 (d, J = 7.4 Hz, 1H), 7.40 (s, 2H), 7.14 (d, J = 2.6 Hz, 2H), 6.95 (s, 1H), 6.76 (d, J = 1.9 Hz, 1H), 4.85 (d, J = 4.6 Hz, 1H), 4.64 (h, J = 7.9 Hz, 1H), 4.06 (dd, J = 9.8, 6.0 Hz, 1H), 3.92 (dt, J = 8.8, 5.8 Hz, 2H), 3.10 - 2.80 (m, 5H), 2.67 (d, J = 19.0 Hz, 1H), 2.35 - 2.14 (m, 2H), 2.07 (d, J = 7.6 Hz, 2H), 1.93 (ddd, J = 12.4, 7.7, 3.7 Hz, 1H), 1.82 (s, 2H), 1.72 (ddd, J = 13.1, 8.6, 6.6 Hz, 1H), 1.22 (dt, J = 13.0, 9.2 Hz, 1H).; MS (ESI): 604 [M+H]⁺.

SFC retention time analysis: 1.659 min (Instrument: Waters UPCC with PDA Detector; Column: Chiralpak IG-3 50x4.6mm I.D., 3um; Mobile phase: A: CO₂, B: Ethanol (0.05% DEA); Isocratic: 40% B; Flow rate: 4mL/min; Column temp.: 35°C; ABPR: 1500psi) .

**Compound 6:** ¹H NMR (400 MHz, DMSO-d6) δ 8.54 (s, 1H), 8.49 (s, 1H), 8.22 (d, *J* = 7.5 Hz, 1H), 7.41 (s, 2H), 7.13 (d, *J* = 2.0 Hz, 2H), 6.93 (s, 1H), 6.75 (d, *J* = 1.8 Hz, 1H), 4.84 (d, *J* = 4.5 Hz, 1H), 4.64 (h, *J* = 8.2 Hz, 1H), 4.07 (dd, *J* = 9.7, 6.0 Hz, 1H), 4.00 - 3.80 (m, 2H), 3.07 - 2.78 (m, 5H), 2.64 (d, *J* = 16.5 Hz, 1H), 2.28 (dt, *J* = 12.5, 7.6 Hz, 1H), 2.22 - 2.13 (m, 1H), 2.08 (d, *J* = 9.7 Hz, 2H), 1.90 (ddd, *J* = 11.9, 7.5, 3.8 Hz, 1H), 1.85 - 1.63 (m, 3H), 1.23 (dt, *J* = 12.8, 9.2 Hz, 1H).; MS (ESI): 604 [M+H]⁺.

SFC retention time analysis: 0.925 min (Instrument: Waters UPCC with PDA Detector; Column: Chiralpak IG-3 50x4.6mm I.D., 3um; Mobile phase: A: CO₂, B: Ethanol (0.05% DEA); Isocratic: 40% B; Flow rate: 4mL/min; Column temp.: 35°C; ABPR: 1500psi) .

### Example 5. Synthesis of Compound 7

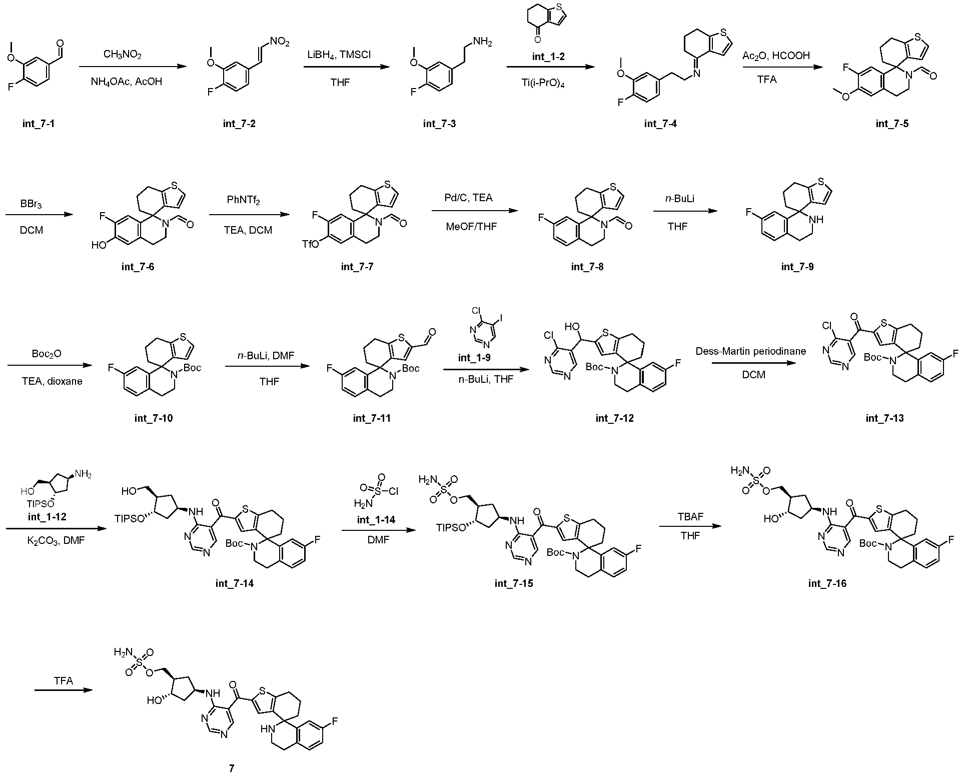

### Step 1: Synthesis of compound int_7-2:

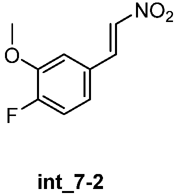

**Int_7-1** (25.0 g, 162 mmol) and NH₄OAc (28.8 g, 373 mmol) were dissolved in acetic acid (250 mL), and the mixed solution was purged with nitrogen three times. CH₃NO₂ (25.7 g, 422 mmol, 22.8 mL) was added to the reaction solution under nitrogen atmosphere, and the reaction solution was heated to 100 °C and stirred for 16 h. LC-MS monitoring showed the reaction was completed. The reaction solution was cooled to room temperature and then poured slowly into 1000 mL of ice water. The aqueous phase was extracted with ethyl acetate (800 mL × 3), and the organic phase was dried over anhydrous sodium sulfate. The organic phase was concentrated under reduced pressure to give a crude product (50 g, yield: 78.2%), which was directly used in the next step.

¹HNMR (400 MHz, DMSO-d6) δ = 3.89 (s, 3 H), 7.33 (dd, *J* = 11.25, 8.44 Hz, 1 H), 7.46 (ddd, *J* = 8.25, 4.59, 1.96 Hz, 1 H), 7.72 (dd, *J* = 8.44, 1.96 Hz, 1 H), 8.10 (d, *J* = 13.57 Hz, 1 H), 8.29 (d, *J* = 13.57 Hz, 1 H).

### Step 2: Synthesis of compound int_7-3:

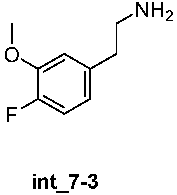

LiBH₄ (27.6 g, 1.27 mol) was dissolved in tetrahydrofuran (300 mL), and TMSCl (276 g, 2.54 mol, 322 mL) was added to the reaction solution at 0 °C under nitrogen atmosphere. The reaction solution was stirred at 0 °C for 30 min, and a solution of **int_7-2** (25.0 g, 127 mmol) in tetrahydrofuran (250 mL) was slowly and dropwise added to the reaction solution within 30 min. The reaction solution was warmed to 75 °C and allowed to react for another 2 h. LC-MS monitoring showed the reaction was completed. The reaction solution was cooled to 0 °C, and 600 mL of methanol was slowly added to quench the reaction. The organic phase was concentrated under reduced pressure to give a crude product, and the crude product was adjusted to pH>7 with ammonia water. The aqueous phase was extracted with ethyl acetate (500 mL × 3), and the organic phase was dried over anhydrous sodium sulfate. The organic phase was concentrated under reduced pressure to give a crude product (25 g), which was directly used in the next step.

ESI-MS m/z: 170 [M+H]⁺.

### Step 3: Synthesis of compound int_7-4:

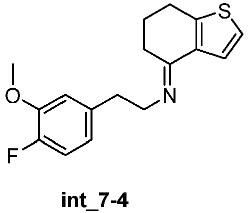

**Int_7-3** (12.5 g, 73.9 mmol) and **int_1-2** (11.8 g, 77.6 mmol) were dissolved in Ti(i-PrO)₄ (1130 mL). The mixed solution was purged with nitrogen three times, heated to 80 °C, and stirred for 2 h. LC-MS monitoring showed the reaction was completed. The reaction solution was cooled to room temperature, which was directly used in the next step.

ESI-MS m/z: 304 [M+H]⁺.

### Step 4: Synthesis of compound int_7-5:

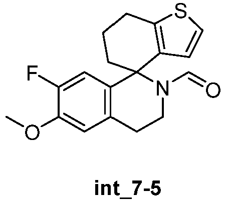

HCOOH (400 mL) was slowly and dropwise added to Ac₂O (1000 mL) at -10 °C, and after the addition was completed, the reaction solution was allowed to react at 20 °C for 0.5 h. Then, the **int_7-4** solution obtained in step 3 was cooled to -10 °C, and the mixed solution of HOOCH and Ac₂O described above was slowly and dropwise added to the **int_7-4** solution. The temperature was kept at -10 °C during the dropwise addition. After the dropwise addition was completed, the reaction solution was warmed to 80 °C and allowed to react for 16 h. LC-MS monitoring showed the reaction was completed. The reaction solution was cooled to room temperature. The organic phase was concentrated under reduced pressure to give a crude product, and the crude product was adjusted to pH>7 with a saturated aqueous sodium bicarbonate solution. The aqueous phase was extracted with ethyl acetate (1000 mL × 3), and the organic phase was dried over anhydrous sodium sulfate. The organic phase was concentrated under reduced pressure to give a crude product. The crude product was purified by preparative column chromatography (SiO₂, PE/EtOAc = 3/2) to give a solid (1.5 g, yield: 5.05%).

¹HNMR (400 MHz, DMSO-d6) δ = 1.52 - 1.66 (m, 1H), 1.82 - 1.94 (m, 2H), 2.28 - 2.38 (m, 1H), 2.78 - 2.86 (m, 2H), 2.89 - 2.97 (m, 2H), 3.10 - 3.22 (m, 1H), 3.82 (s, 3H), 4.40 (dt, *J =* 12.93, 3.99 Hz, 1H), 6.22 (d, *J =* 5.28 Hz, 1H), 6.52 (d, *J =* 12.98 Hz, 1H), 6.97 (d, *J =* 9.02 Hz, 1H), 7.30 (d, *J =* 5.28 Hz, 1H), 7.57 (s, 1H) . ESI-MS m/z: 332 [M+H]⁺ .

### Step 5: Synthesis of compound int_7-6:

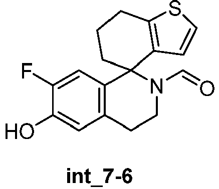

**Int_7-5** (4.00 g, 12.1 mmol) was dissolved in dichloromethane (40 mL), and the mixed solution was purged with nitrogen three times. The reaction solution was cooled to 0 °C, and BBr₃ (15.1 g, 60.4 mmol, 5.81 mL) was slowly and dropwise added to the reaction solution. The reaction solution was warmed to room temperature and allowed to react for 1 h. LC-MS monitoring showed the reaction was completed. The reaction solution was poured slowly into 200 mL of ice water. The aqueous phase was extracted with ethyl acetate (200 mL × 3), and the organic phase was dried over anhydrous sodium sulfate. The organic phase was concentrated under reduced pressure to give a crude product (4.5 g, yield: 99.3%), which was directly used in the next step. ESI-MS m/z: 318 [M+H]⁺.

### Step 6: Synthesis of compound int_7-7:

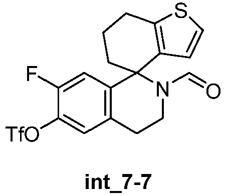

**Int_7-6** (4.50 g, 14.2 mmol), PhNTf₂ (9.12 g, 25.5 mmol), and TEA (3.59 g, 35.5 mmol, 4.93 mL) were dissolved in dichloromethane (50 mL) at 0 °C. The reaction solution was purged with nitrogen three times, and allowed to react at room temperature for 16 h. LC-MS monitoring showed the reaction was completed. The reaction solution was poured slowly into 200 mL of ice water. The aqueous phase was extracted with ethyl acetate (200 mL × 3), and the organic phase was dried over anhydrous sodium sulfate. The organic phase was concentrated under reduced pressure to give a crude product. The crude product was purified by preparative column chromatography (SiO₂, PE/EtOAc = 2/1) to give a solid (4 g, yield: 62.8%).

¹HNMR (400 MHz, DMSO-d6) δ = 1.52 - 1.67 (m, 1H), 1.86 - 1.94 (m, 1H), 1.99 (s, 1H), 2.41 (dt, *J =* 11.83, 2.01 Hz, 1H), 2.91 (br s, 2H), 2.94 (br dd, *J =* 8.03, 3.85 Hz, 2H), 3.18 (ddd, *J =* 13.20, 10.45, 4.95 Hz, 1H), 4.40 - 4.49 (m, 1H), 6.26 (d, *J =* 5.28 Hz, 1H), 6.98 (d, *J =* 11.66 Hz, 1H), 7.35 (d, *J =* 5.28 Hz, 1H), 7.56 (s, 1H), 7.60 (d, *J =* 7.70 Hz, 1H) .

ESI-MS m/z: 450 [M+H]⁺ .

### Step 7: Synthesis of compound int_7-8:

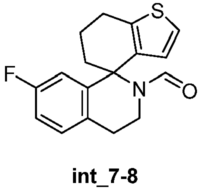

**Int_7-7** (4.00 g, 8.90 mmol), Pd/C (1.00 g, 10.0% purity), and TEA (3.60 g, 35.6 mmol, 4.96 mL) were dissolved in methanol (80 mL). The reaction solution was purged with hydrogen three times, and allowed to react at room temperature for 16 h under hydrogen atmosphere (20.0 Psi). LC-MS monitoring showed the reaction was completed. The reaction solution was filtered to give a filtrate, and the filtrate was concentrated under reduced pressure to give a crude product. The crude product was purified by preparative column chromatography (SiO₂, PE/EtOAc = 1/1) to give a solid (2.2 g, yield: 81.2%).

¹HNMR (400 MHz, DMSO-d6) δ = 1.53 - 1.67 (m, 1H), 1.84 - 1.93 (m, 1H), 1.98 (td, *J =* 13.45, 2.69 Hz, 1H), 2.35 - 2.43 (m, 1H), 2.84 (br dd, *J =* 7.27, 3.85 Hz, 2H), 2.94 (dd, *J =* 8.01, 3.97 Hz, 2H), 3.19 (dt, *J =* 12.93, 7.78 Hz, 1H), 4.40 (dt, *J =* 13.02, 4.07 Hz, 1H), 6.22 (d, *J =* 5.26 Hz, 1H), 6.53 (dd, *J =* 10.51, 2.69 Hz, 1H), 7.04 (td, *J =* 8.50, 2.69 Hz, 1H), 7.25 (dd, *J =* 8.50, 6.05 Hz, 1H), 7.32 (d, *J =* 5.26 Hz, 1H), 7.59 (s, 1H) .

ESI-MS m/z: 302 [M+H]⁺.

### Step 8: Synthesis of compound int_7-9:

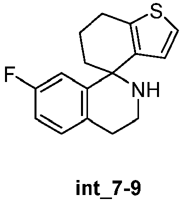

**Int_7-8** (2.20 g, 7.30 mmol) was dissolved in THF (25 mL), and the mixture was cooled to -78 °C. n-BuLi (2.50 M, 8.76 mL) was slowly and dropwise added to the reaction solution under nitrogen atmosphere, and the reaction solution was allowed to react at -30 °C for 2 h. Subsequently, the reaction solution was warmed to room temperature and allowed to react for 0.5 h. LC-MS monitoring showed the reaction was completed. A saturated aqueous ammonium chloride solution (50 mL) was added to the reaction solution. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography (SiO₂, DCM/MeOH = 95/5) to give a solid (1.4 g, yield: 56.1%).

¹HNMR (400 MHz, DMSO-d6) δ = 1.72 - 1.84 (m, 2H), 1.96 - 2.08 (m, 1H), 2.10 - 2.22 (m, 1H), 2.67 (br s, 1H), 2.75 - 2.90 (m, 4H), 2.95 - 3.00 (m, 2H), 6.34 (d, *J =* 5.28 Hz, 1H), 6.45 (dd, *J =* 10.56, 2.64 Hz, 1H), 6.92 (td, *J =* 8.53, 2.75 Hz, 1H), 7.10 (br s, 1H), 7.11 - 7.15 (m, 1H) .

ESI-MS m/z: 274 [M+H]⁺ .

### Step 9: Synthesis of compound int_7-10:

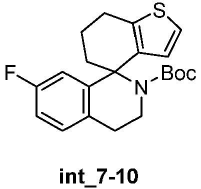

**Int_7-9** (1.40 g, 5.12 mmol) and (Boc)₂O (2.46 g, 11.3 mmol, 2.59 mL) were dissolved in 1,4-dioxane (15 mL), and TEA (1.55 g, 15.4 mmol, 2.14 mL) was added to the reaction solution at room temperature. The reaction solution was warmed to 80 °C and allowed to react for 16 h. LC-MS monitoring showed the reaction was completed. After the reaction solution was cooled to room temperature, the reaction solution was concentrated under reduced pressure to give a crude product. The crude product was purified by preparative column chromatography (SiO₂, PE/EtOAc = 4/1) to give a solid (870 mg, yield: 45.2%).

ESI-MS m/z: 374 [M+H]⁺.

### Step 10: Synthesis of compound int_7-11:

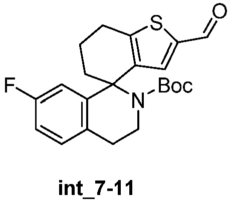

**Int_7-10** (900 mg, 2.41 mmol) was dissolved in THF (10 mL), and the mixture was cooled to -70 °C. n-BuLi (2.50 M, 2.89 mL) was slowly and dropwise added to the reaction solution under nitrogen atmosphere, and the reaction solution was allowed to react at -70 °C for 0.5 h. Then, DMF (528 mg, 7.23 mmol, 556 µL) was dropwise added to the reaction solution at -70 °C, and after the dropwise addition was completed, the reaction solution was allowed to react at -75 °C for 1 h. LC-MS monitoring showed the reaction was completed. A saturated ammonium chloride solution (25 mL) was slowly added to the reaction solution. The aqueous phase was extracted with ethyl acetate (30 mL × 3), and the organic phase was dried over anhydrous sodium sulfate. The organic phase was concentrated under reduced pressure to give a crude product. The crude product was purified by preparative column chromatography (SiO₂, PE/EtOAc = 4/1) to give a solid (970 mg, yield: 95.9%).

¹HNMR (400 MHz, CHLOROFORM-d) δ = 1.23 (s, 9H), 1.66 - 1.80 (m, 2H), 1.82 - 1.91 (m, 1H), 2.73 - 2.94 (m, 3H), 2.95 - 3.06 (m, 2H), 3.25 (td, *J =* 12.07, 3.12 Hz, 1H), 4.40 - 4.49 (m, 1H), 6.25 (dd, *J=* 10.45, 2.63 Hz, 1H), 6.90 (td, *J =* 8.25, 2.57 Hz, 1H), 7.17 (dd, *J =* 8.07, 5.87 Hz, 1H), 7.27 - 7.28 (m, 1H), 9.75 (s, 1H) . MS (ESI): 402 [M+H]⁺ .

### Step 11: Synthesis of compound int_7-12:

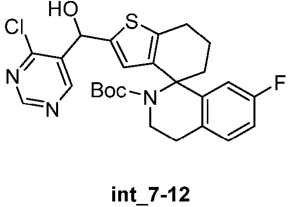

**Int_1-9** (1.62 g, 6.72 mmol) was dissolved in THF (15 mL), and the mixture was cooled to -75 °C. n-BuLi (2.50 M, 5.38 mL) was slowly and dropwise added to the reaction solution under nitrogen atmosphere, and the reaction solution was allowed to react at -75 °C for 1 h. Then, a solution of int_7-11 (900 mg, 2.24 mmol) in THF (25 mL) was dropwise added to the reaction solution at -75 °C, and after the dropwise addition was completed, the reaction solution was allowed to react at -75 °C for 1 h, and was then warmed to room temperature and allowed to react for 16 h. LC-MS monitoring showed the reaction was completed. A saturated ammonium chloride solution (100 mL) was slowly added to the reaction solution. The aqueous phase was extracted with ethyl acetate (100 mL × 3), and the organic phase was dried over anhydrous sodium sulfate.

The organic phase was concentrated under reduced pressure to give a crude product. The crude product was purified by preparative column chromatography (SiO₂, PE/EtOAc = 7/3) to give a solid (690 mg, yield: 59.7%).

¹HNMR (400 MHz, DMSO-d6) δ = 0.94 - 1.18 (m, 9H), 1.44 - 1.80 (m, 4H), 2.55 - 2.71 (m, 2H), 2.77 - 2.94 (m, 3H), 3.01 - 3.12 (m, 1H), 4.18 - 4.28 (m, 1H), 5.96 - 6.12 (m, 1H), 6.16 - 6.27 (m, 1H), 6.40 - 6.45 (m, 1H), 6.57 - 6.72 (m, 1H), 6.99 - 7.07 (m, 1H), 7.28 (br dd, *J =* 8.47, 6.05 Hz, 1H), 8.93 - 8.97 (m, 1H) .

MS (ESI): 516 [M+H]⁺ .

### Step 12: Synthesis of compound int_7-13:

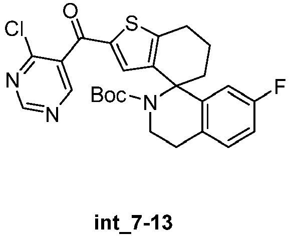

**Int_7-12** (640 mg, 1.24 mmol) was dissolved in DCM (10 mL), and a Dess-Martin oxidant (1.05 g, 2.48 mmol) was added to the reaction solution at room temperature. The reaction solution was allowed to react at room temperature for 1 h. LC-MS monitoring showed the reaction was completed. A saturated aqueous sodium bicarbonate solution was slowly added to the reaction solution to adjust the pH to about 8. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography (SiO₂, PE/EtOAc = 10/1) to give a solid (630 mg, yield: 98.8%).

¹HNMR (400 MHz, DMSO-d6) δ = 1.19 (s, 9H), 1.54 - 1.66 (m, 2H), 1.75 - 1.85 (m, 1H), 2.59 - 2.81 (m, 2H), 2.85 - 2.91 (m, 2H), 3.03 - 3.18 (m, 2H), 4.17 - 4.26 (m, 1H), 6.22 (dd, *J =* 10.78, 2.64 Hz, 1H), 7.05 (td, *J =* 8.47, 2.64 Hz, 1H), 7.25 - 7.34 (m, 2H), 8.86 (s, 1H), 9.15 (s, 1H).

MS (ESI): 514 [M+H]⁺ .

### Step 13: Synthesis of compound int_7-14:

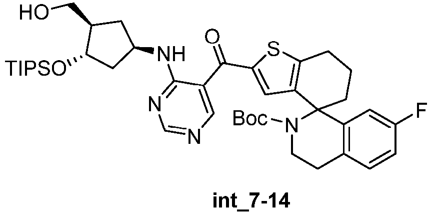

**Int_7-13** (390 mg, 759 µmol) and **int_1-12** (327 mg, 1.14 mmol) were dissolved in DMF (10 mL), and K₂ CO ₃ (524 mg, 3.79 mmol) was added to the reaction solution at room temperature. The reaction solution was allowed to react at room temperature for 16 h. LC-MS monitoring showed the reaction was completed. Ice water (50 mL) was added to the reaction solution. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography (SiO₂, PE/EtOAc = 7/3) to give a product (350 mg, yield: 60.3%).

MS (ESI): 765 [M+H]⁺.

### Step 14: Synthesis of compound int_7-15:

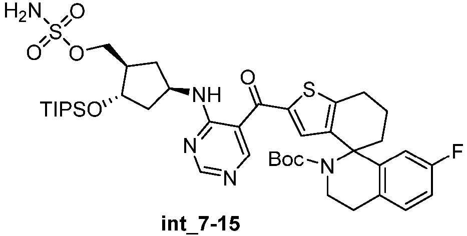

**Int_7-14** (550 mg, 719 µmol) was dissolved in DMF (10 mL), and **int_1-14** (249 mg, 2.16 mmol) was added to the reaction solution at room temperature. The reaction solution was allowed to react at room temperature for 0.5 h. LC-MS monitoring showed the reaction was completed. Ice water (20 mL) was added to the reaction solution. The aqueous phase was extracted with ethyl acetate (20 mL × 3), and the organic phase was dried over anhydrous sodium sulfate. The organic phase was concentrated under reduced pressure to give a crude product. The crude product was purified by preparative column chromatography to give a solid (600 mg, yield: 98.9%).

MS (ESI): 844 [M+H]⁺.

### Step 15: Synthesis of compound int_7-16:

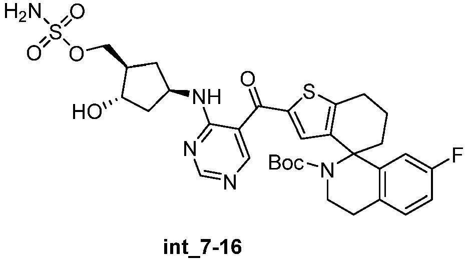

**Int_7-15** (550 mg, 652 µmol) was dissolved in THF (10 mL), and TBAF (1.00 M, 652 µL) was added to the reaction solution at room temperature. The reaction solution was allowed to react at room temperature for 16 h. LC-MS monitoring showed the reaction was completed. Water (20 mL) was added to the reaction solution. The aqueous phase was extracted with ethyl acetate (20 mL × 3), and the organic phase was dried over anhydrous sodium sulfate. The organic phase was concentrated under reduced pressure to give a crude product. The crude product was purified by preparative column chromatography (SiO₂, PE/EtOAc = 4/1) to give a solid (320 mg, yield: 58.6%).

¹HNMR (400 MHz, CHLOROFORM-d) δ = 1.23 (s, 9H), 1.51 - 1.96 (m, 6H), 2.03 - 2.16 (m, 1H), 2.16 - 2.37 (m, 1H), 2.63 - 2.71 (m, 1H), 2.79 (br s, 1H), 2.91 (br s, 2H), 3.05 (br dd, *J =* 17.45, 2.69 Hz, 1H), 3.89 - 3.98 (m, 2H), 4.06 - 4.11 (m, 1H), 4.20 - 4.28 (m, 1H), 4.62 - 4.72 (m, 1H), 4.84 - 4.90 (m, 1H), 6.23 (dd, *J =* 10.88, 2.13 Hz, 1H), 7.05 (td, *J =* 8.32, 2.25 Hz, 1H), 7.22 (s, 1H), 7.31 (dd, *J =* 8.07, 6.19 Hz, 1H), 7.43 (d, *J =* 5.13 Hz, 2H), 8.26 (br d, *J* = 6.75 Hz, 1H), 8.41 (s, 1H), 8.57 (s, 1H) .

MS (ESI): 688 [M+H]⁺ .

### Step 16: Synthesis of compound 7

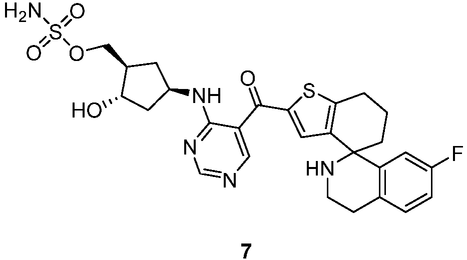

**Int_7-16** (200 mg, 291 µmol) was dissolved in DCM (5 mL) at room temperature, and the mixture was added into TFA (2 mL). The reaction solution was allowed to react at room temperature for 5 min. LC-MS monitoring showed the reaction was completed. The reaction solution was cooled to 0 °C. A saturated aqueous sodium bicarbonate solution was added to the reaction solution to adjust the pH to 8. The aqueous phase was extracted with ethyl acetate (20 mL × 3), and the organic phase was dried over anhydrous sodium sulfate. The organic phase was concentrated under reduced pressure to give a crude product, and the crude product was purified by preparative HPLC (column: Phenomenex C18 75 × 30 mm × 3 µm; mobile phase: [water (ammonia hydroxide)-ACN]; gradient: 38%-58% B over 9 min) to give a solid (50 mg, yield: 29.3%).

¹HNMR (400 MHz, DMSO-d6) δ = 1.24 (dt, *J =* 8.71, 4.51 Hz, 1H), 1.71 - 1.78 (m, 1H), 1.78 - 1.86 (m, 2H), 1.87 - 1.98 (m, 1H), 2.08 (br d, *J =* 8.56 Hz, 2H), 2.17 (br dd, *J =* 11.68, 2.51 Hz, 1H), 2.24 - 2.35 (m, 1H), 2.61 - 2.68 (m, 1H), 2.86 (br dd, *J =* 15.65, 7.58 Hz, 1H), 2.96 (dt, *J =* 8.56, 4.28 Hz, 4H), 3.30 (s, 1H), 3.94 (br dd, *J* = 9.72, 6.91 Hz, 2H), 4.07 (ddd, *J* = 9.32, 6.14, 2.75 Hz, 1H), 4.58 - 4.71 (m, 1H), 4.87 (dd, *J =* 4.46, 3.00 Hz, 1H), 6.54 (br d, *J* = 9.41 Hz, 1H), 6.90 - 6.97 (m, 2H), 7.13 (dd, *J =* 8.38, 6.17 Hz, 1H), 7.43 (d, *J =* 2.57 Hz, 2H), 8.22 (dd, *J =* 7.40, 2.51 Hz, 1H), 8.50 (s, 1 H), 8.55 (s, 1H) .

MS (ESI): 588 [M+H]⁺ .

### Example 6. Synthesis of Compound 8 and Compound 9

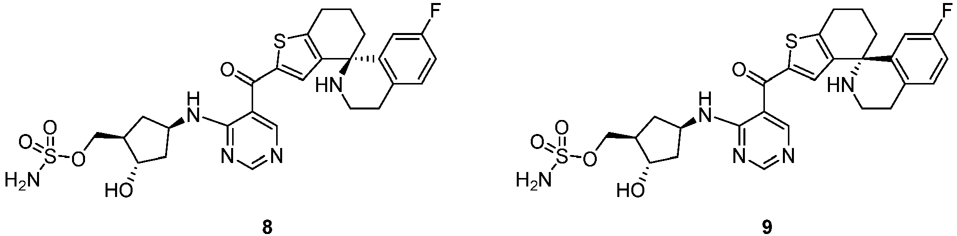

**Compound 7** (50 mg, 0.085 mmol) was subjected to SFC chiral resolution (column: DAICEL CHIRALPAK AD (250 mm × 30 mm, 10 µm); mobile phase: [CO₂-EtOH (0.1% NH₃H₂O)]; B%: 50%, isocratic elution mode) to give **compound 8** (22 mg) and **compound 9** (14 mg).

**Compound 8:** ¹HNMR (400 MHz, DMSO-d6) δ = 1.24 (dt, *J =* 12.65, 9.19 Hz, 1H), 1.68 - 1.85 (m, 3H), 1.85 - 1.96 (m, 1H), 2.02 - 2.14 (m, 2H), 2.14 - 2.34 (m, 2H), 2.64 (br d, *J =* 15.85 Hz, 1H), 2.70 - 3.08 (m, 5H), 3.30 (s, 1H), 3.89 - 3.98 (m, 2H), 4.08 (dd, *J =* 9.68, 5.94 Hz, 1H), 4.60 - 4.70 (m, 1H), 4.88 (d, *J =* 4.62 Hz, 1H), 6.53 (dd, *J =* 10.34, 2.64 Hz, 1H), 6.90 - 6.96 (m, 2H), 7.13 (dd, *J =* 8.36, 6.16 Hz, 1H), 7.44 (s, 2H), 8.22 (d, *J =* 7.48 Hz, 1H), 8.50 (s, 1H), 8.55 (s, 1H).; MS (ESI): 588 [M+H]⁺ .

SFC retention time analysis: 0.820 min (Instrument: Waters UPCC with PDA Detector; Column: Chiralpak AD-3 50x4.6mm I.D., 3um; Mobile phase: A: CO₂, B: Ethanol (0.05% DEA); Isocratic: 40% B; Flow rate: 4mL/min; Column temp.: 35°C; ABPR: 1500psi) .

**Compound 9:** ¹HNMR (400 MHz, DMSO-d6) δ = 1.23 (dt, *J* = 12.82, 9.33 Hz, 1H), 1.69 - 1.86 (m, 3H), 1.88 - 1.98 (m, 1H), 2.08 (br d, *J =* 6.38 Hz, 2H), 2.16 - 2.33 (m, 2H), 2.62 - 2.70 (m, 1H), 2.77 - 3.08 (m, 5H), 3.30 (s, 1H), 3.90 - 3.98 (m, 2H), 4.07 (dd, *J =* 9.57, 6.05 Hz, 1H), 4.60 - 4.70 (m, 1H), 4.88 (d, *J* = 4.62 Hz, 1H), 6.54 (dd, *J =* 10.23, 2.53 Hz, 1H), 6.91 - 6.97 (m, 2H), 7.14 (t, *J* = 6.69 Hz, 1H), 7.43 (s, 2H), 8.23 (d, *J =* 7.48 Hz, 1H), 8.50 (s, 1H), 8.56 (s, 1H).; MS (ESI): 588 [M+H]⁺ .

SFC retention time analysis: 0.636 min (Instrument: Waters UPCC with PDA Detector; Column: Chiralpak AD-3 50x4.6mm I.D., 3um; Mobile phase: A: CO₂, B: Ethanol (0.05% DEA); Isocratic: 40% B; Flow rate: 4mL/min; Column temp.: 35°C; ABPR: 1500psi) .

### Example 7. Synthesis of Compound 19

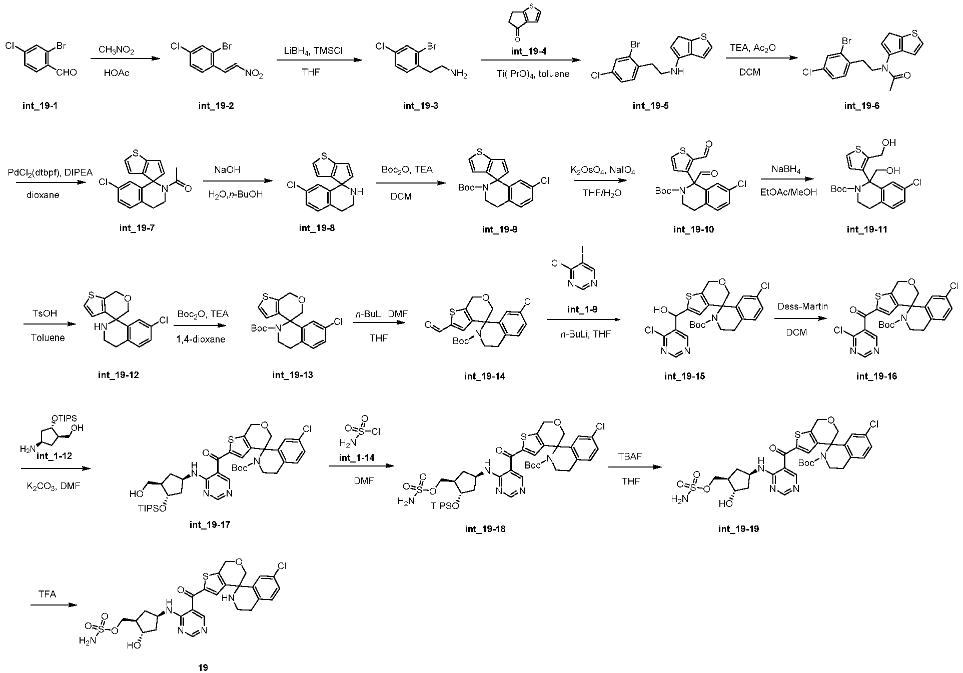

### Step 1: Synthesis of compound int_19-2:

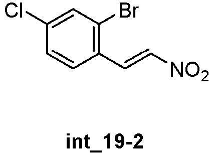

**Int_19-1** (50.0 g, 228 mmol) and NH₄OAc (40.4 g, 524 mmol) were dissolved in acetic acid (500 mL), and the mixed solution was purged with nitrogen three times. CH₃NO₂ (36.2 g, 593 mmol, 32.0 mL) was added to the reaction solution under nitrogen atmosphere, and the reaction solution was heated to 100 °C and stirred for 16 h. LC-MS monitoring showed the reaction was completed. The reaction solution was cooled to room temperature and then poured slowly into 1000 mL of ice water. The aqueous phase was extracted with ethyl acetate (800 mL × 3), and the organic phase was dried over anhydrous sodium sulfate. The organic phase was concentrated under reduced pressure to give a crude product (50 g, yield: 83.6%), which was directly used in the next step.

¹H NMR (400MHz, CHLOROFORM-d) δ = 8.35 (d, *J =* 13.6 Hz, 1H), 7.73 (d, *J =* 2.0 Hz, 1H), 7.58 - 7.51 (m, 2H), 7.40 (dd, *J =* 2.0, 8.4 Hz, 1H).

### Step 2: Synthesis of compound int_19-3:

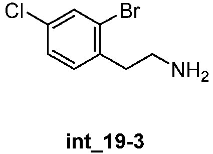

LiBH₄ (20.8 g, 952 mmol) was dissolved in tetrahydrofuran (300 mL), and TMSCl (207 g, 1.90 mol, 242 mL) was added to the reaction solution at 0 °C under nitrogen atmosphere. The reaction solution was stirred at 0 °C for 30 min, and a solution of **int_19-2** (25.0 g, 95.2 mmol) in tetrahydrofuran (250 mL) was slowly and dropwise added to the reaction solution within 30 min. The reaction solution was warmed to 70 °C and allowed to react for another 2 h. LC-MS monitoring showed the reaction was completed. The reaction solution was cooled to 0 °C, and 200 mL of methanol was slowly added to quench the reaction. The organic phase was concentrated under reduced pressure to give a crude product, and the crude product was adjusted to pH>7 with ammonia water. The aqueous phase was extracted with ethyl acetate (500 mL × 3), and the organic phase was dried over anhydrous sodium sulfate. The organic phase was concentrated under reduced pressure to give a crude product (20 g), which was directly used in the next step.

ESI-MS m/z: 234 [M+H]⁺.

### Step 3: Synthesis of compound int_19-5:

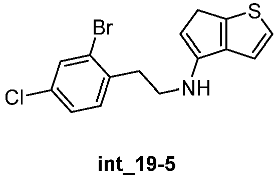

**Int_19-3** (20.0 g, 85.3 mmol), **int_19-4** (9.07 g, 65.6 mmol), and Ti(i-PrO)₄ (37.3 g, 131 mmol) were dissolved in toluene (200 mL). The mixed solution was purged with nitrogen three times, heated to 90 °C, and stirred for 2 h. LC-MS monitoring showed the reaction was completed. The reaction solution was cooled to room temperature and concentrated under reduced pressure to give a crude product (23 g), which was directly used in the next step.

ESI-MS m/z: 354 [M+H]⁺.

### Step 4: Synthesis of compound int_19-6:

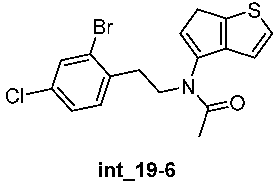

**Int_19-5** (23.0 g, 64.9 mmol), TEA (65.6 g, 648 mmol, 90.3 mL), and Ac₂O (33.1 g, 324 mmol, 30.5 mL) were dissolved in dichloromethane (400 mL), and the reaction solution was allowed to react at 20 °C for 2 h under nitrogen atmosphere. LC-MS monitoring showed the reaction was completed. The reaction solution was cooled to room temperature. The organic phase was concentrated under reduced pressure to give a crude product, and the crude product was adjusted to pH>7 with a saturated aqueous sodium bicarbonate solution. The aqueous phase was extracted with dichloromethane (500 mL × 3), and the organic phase was dried over anhydrous sodium sulfate. The organic phase was concentrated under reduced pressure to give a crude product. The crude product was purified by preparative column chromatography (SiO₂, PE/EtOAc = 3/1) to give a solid (20 g, yield: 77.7%).

¹H NMR (400 MHz, DMSO-d6) δ =1.09 - 1.17 (m, 1H), 1.88 (br d, *J =* 7.00 Hz, 3H), 2.82 - 2.96 (m, 2H), 3.41 - 3.48 (m, 2H), 3.80 (br t, *J =* 7.13 Hz, 2H), 3.99 (q, *J =* 7.13 Hz, 1H), 7.18 - 7.38 (m, 2H), 7.43 - 7.54 (m, 1H), 7.63 (d, *J* = 2.13 Hz, 1H).

### Step 5: Synthesis of compound int_19-7:

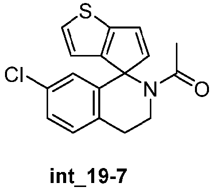

**Int_19-6** (20.0 g, 50.4 mmol), DIPEA (13.0 g, 101 mmol, 17.6 mL), and ditert-butyl(cyclopentyl)phosphane;dichloropalladium;iron (3.29 g, 5.04 mmol) were dissolved in 1,4-dioxane (300 mL). The reaction solution was purged with nitrogen three times, warmed to 110 °C, and allowed to react for 7 h. LC-MS monitoring showed the reaction was completed. The reaction solution was cooled to room temperature and then poured slowly into 300 mL of ice water. The aqueous phase was extracted with ethyl acetate (300 mL × 3), and the organic phase was dried over anhydrous sodium sulfate. The organic phase was concentrated under reduced pressure to give a crude product. The crude product was purified by preparative column chromatography (SiO₂, PE/EtOAc = 3/1) to give a solid (9.4 g, yield: 59%).

¹H NMR (400 MHz, DMSO-d6) δ = 1.69 - 1.96 (m, 3H), 2.91 - 3.01 (m, 2H), 3.35 (s, 11H), 3.73 - 3.86 (m, 1H), 3.89 - 3.99 (m, 1H), 6.40 - 6.47 (m, 1H), 6.49 - 6.60 (m, 1H), 6.77 - 6.85 (m, 1H), 6.87 - 6.94 (m, 1H), 7.15 - 7.21 (m, 1H), 7.23 - 7.31 (m, 1H), 7.39 - 7.49 (m, 1H) .

ESI-MS m/z: 316 [M+H]⁺ .

### Step 6: Synthesis of compound int_19-8:

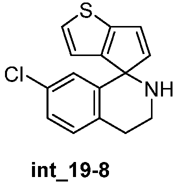

**Int_19-7** (9.40 g, 29.8 mmol) was dissolved in a mixed solvent of n-butanol (15 mL) and water (5 mL), and sodium hydroxide (23.8 g, 595 mmol) was added to the reaction solution. The reaction solution was warmed to 100 °C and allowed to react for 16 h. Subsequently, the reaction solution was warmed to room temperature and allowed to react for 1 h. LC-MS monitoring showed the reaction was completed. Water (200 mL) was added to the reaction solution. The aqueous phase was extracted with ethyl acetate (200 mL × 3), and the organic phase was dried over anhydrous sodium sulfate. The organic phase was concentrated under reduced pressure to give a crude product. The crude product was purified by preparative column chromatography (SiO₂, PE/EtOAc = 1/4) to give a solid (8 g, yield: 98.2%).

¹H NMR (500 MHz, DMSO-d6) δ = 1.99 (s, 1H), 2.94 - 3.00 (m, 1H), 3.08 - 3.17 (m, 1H), 3.19 - 3.27 (m, 1H), 3.36 - 3.43 (m, 1H), 6.44 (d, *J* = 2.14 Hz, 1H), 6.64 (dd, *J =* 5.57, 1.45 Hz, 1H), 6.80 (d, *J* = 5.49 Hz, 1H), 6.94 (d, *J* = 4.88 Hz, 1H), 7.12 - 7.20 (m, 2H), 7.38 (dd, *J =* 4.81, 1.45 Hz, 1H) .

ESI-MS m/z: 274 [M+H]⁺ .

### Step 7: Synthesis of compound int_19-9:

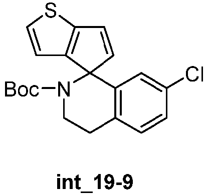

**Int_19-8** (8.00 g, 29.2 mmol) and (Boc)₂O (9.57 g, 43.8 mmol, 10.1 mL) were dissolved in dichloromethane (90 mL), and TEA (8.87 g, 87.7 mmol, 12.2 mL) was added to the reaction solution at room temperature. The reaction solution was allowed to react at room temperature for 16 h under nitrogen atmosphere. LC-MS monitoring showed the reaction was completed. The reaction solution was poured slowly into 100 mL of ice water. The aqueous phase was extracted with dichloromethane (100 mL × 3), and the organic phase was dried over anhydrous sodium sulfate. The organic phase was concentrated under reduced pressure to give a crude product. The crude product was purified by preparative column chromatography (SiO₂, PE/EtOAc = 3/1) to give a solid (8.9 g, yield: 81.5%).

¹H NMR (400 MHz, DMSO-d6) δ = 1.13 (s, 9H), 2.88 - 3.06 (m, 2H), 3.72 - 3.91 (m, 2H), 6.47 - 6.63 (m, 2H), 6.79 (d, *J* = 5.50 Hz, 1H), 6.85 - 6.95 (m, 1H), 7.16 - 7.29 (m, 2H), 7.41 - 7.49 (m, 1H).

### Step 8: Synthesis of compound int_19-10:

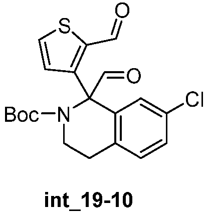

**Int_19-9** (2.5 g, 6.686 mmol), sodium periodate (7.2 g, 33.431 mmol) and K₂OsO₄·2H₂O (250 mg, 0.678 mmol) were dissolved in a mixed solvent of tetrahydrofuran (250 mL) and water (125 mL). The reaction solution was purged with nitrogen three times, and allowed to react at room temperature for 2 h. LC-MS monitoring showed the reaction was completed. The reaction solution was poured slowly into 100 mL of ice water. The aqueous phase was extracted with dichloromethane (100 mL × 3), and the organic phase was dried over anhydrous sodium sulfate. The organic phase was concentrated under reduced pressure to give a crude product. The crude product was purified by preparative column chromatography to give a solid (2.6 g, yield: 96.2%).

ESI-MS m/z: 406 [M+H]⁺.

### Step 9: Synthesis of compound int_19-11:

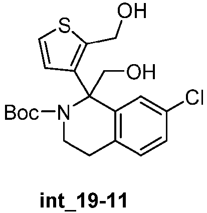

**Int_19-10** (2.7 g, 6.686 mmol) was dissolved in a mixed solvent of ethyl acetate (60 mL) and methanol (15 mL), and sodium borohydride (1.0 g, 26.744 mmol) was added to the reaction solution. The reaction solution was allowed to react at room temperature for 2 h. LC-MS monitoring showed the reaction was completed. The reaction solution was poured slowly into 100 mL of ice water. The aqueous phase was extracted with ethyl acetate (100 mL × 3), and the organic phase was dried over anhydrous sodium sulfate. The organic phase was concentrated under reduced pressure to give a crude product. The crude product was purified by preparative column chromatography (SiO₂, PE/EtOAc = 2/1) to give a solid (1.8 g, yield: 65.6%).

ESI-MS m/z: 410 [M+H]⁺.

### Step 10: Synthesis of compound int_19-12:

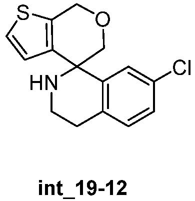

**Int_19-11** (1.2 g, 2.93 mmol) was dissolved in toluene (50 mL), and p-toluenesulfonic acid monohydrate (5.6 g, 29.3 mmol) was added to the reaction solution. The reaction solution was warmed to 110 °C and allowed to react for 1 h. LC-MS monitoring showed the reaction was completed. The reaction solution was cooled to room temperature and then poured slowly into 50 mL of ice water. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography to give a solid (310 mg, yield: 38.7%).

ESI-MS m/z: 292 [M+H]⁺.

### Step 11: Synthesis of compound int_19-13:

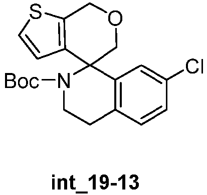

**Int_19-12** (1.87 g, 6.426 mmol) and (Boc)₂O (2.1 g, 9.639 mmol) were dissolved in 1,4-dioxane (16 mL), and TEA (973 mg, 9.639 mmol) was added to the reaction solution at room temperature. The reaction solution was warmed to 80 °C and allowed to react for 16 h under nitrogen atmosphere. LC-MS monitoring showed the reaction was completed. The reaction solution was cooled to room temperature and adjusted to a weakly acidic pH with 2 M aqueous hydrochloric acid solution. The aqueous phase was extracted with ethyl acetate (100 mL × 3), and the organic phase was dried over anhydrous sodium sulfate. The organic phase was concentrated under reduced pressure to give a crude product. The crude product was purified by preparative column chromatography (SiO₂, PE/EtOAc = 2/1) to give a solid (1.6 g, yield: 63.5%).

ESI-MS m/z: 392 [M+H]⁺.

### Step 12: Synthesis of compound int_19-14:

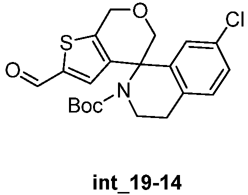

**Int_19-13** (265 mg, 0.676 mmol) was dissolved in THF (10 mL), and the mixture was cooled to -70 °C. n-BuLi (2.5 M, 0.81 mL) was slowly and dropwise added to the reaction solution under nitrogen atmosphere, and the reaction solution was allowed to react at -70 °C for 1 h. Then, DMF (148 mg, 2.028 mmol) was dropwise added to the reaction solution at -60 °C, and after the dropwise addition was completed, the reaction solution was allowed to react at 70°C for 1 h. LC-MS monitoring showed the reaction was completed. A saturated ammonium chloride solution (20 mL) was slowly added to the reaction solution. The aqueous phase was extracted with ethyl acetate (20 mL × 3), and the organic phase was dried over anhydrous sodium sulfate. The organic phase was concentrated under reduced pressure to give a crude product. The crude product was purified by preparative column chromatography (SiO₂, PE/EtOAc = 5/1) to give a solid (170 mg, yield: 60.1%).

MS (ESI): 420 [M+H]⁺.

### Step 13: Synthesis of compound int_19-15:

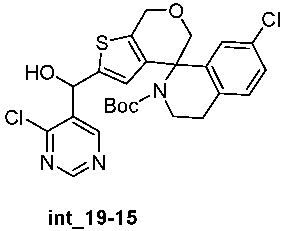

**Int_1-9** (592 mg, 2.463 mmol) was dissolved in THF (10 mL), and the mixture was cooled to -70 °C. n-BuLi (2.5 M, 2 mL, 4.926 mmol) was slowly and dropwise added to the reaction solution under nitrogen atmosphere, and the reaction solution was allowed to react at -70 °C for 1 h. Then, a solution of **int_19-14** (170 mg, 0.411 mmol) in THF (1 mL) was dropwise added to the reaction solution at -75 °C, and after the dropwise addition was completed, the reaction solution was allowed to react at -75 °C for 1 h, and was then warmed to room temperature and allowed to react for 16 h. LC-MS monitoring showed the reaction was completed. A saturated ammonium chloride solution (20 mL) was slowly added to the reaction solution. The aqueous phase was extracted with ethyl acetate (20 mL × 3), and the organic phase was dried over anhydrous sodium sulfate. The organic phase was concentrated under reduced pressure to give a crude product. The crude product was purified by preparative column chromatography to give a solid (180 mg, yield: 81.2%).

MS (ESI): 534 [M+H]⁺.

### Step 14: Synthesis of compound int_19-16:

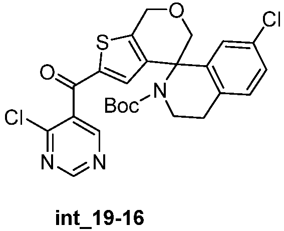

**Int_19-15** (180 mg, 0.337 mmol) was dissolved in DCM (15 mL), and a Dess-Martin oxidant (172 mg, 0.404 mmol) was added to the reaction solution at room temperature. The reaction solution was allowed to react at room temperature for 1 h. LC-MS monitoring showed the reaction was completed. A saturated aqueous sodium bicarbonate solution was slowly added to the reaction solution to adjust the pH to about 8. The aqueous phase was extracted with ethyl acetate (20 mL × 3), and the organic phase was dried over anhydrous sodium sulfate. The organic phase was concentrated under reduced pressure to give a crude product. The crude product was purified by preparative column chromatography (SiO₂, PE/EtOAc = 2/1) to give a solid (156 mg, yield: 87.1%).

MS (ESI): 532 [M+H]⁺.

### Step 15: Synthesis of compound int_19-17:

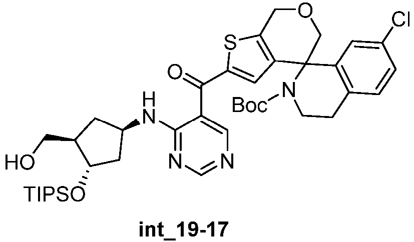

**Int_19-16** (174 mg, 0.328 mmol) and **int_1-12** (141 mg, 0.491 mmol) were dissolved in DMF (10 mL), and K₂CO₃ (138 mg, 1 mmol) was added to the reaction solution at room temperature. The reaction solution was allowed to react at room temperature for 16 h. LC-MS monitoring showed the reaction was completed. Ice water (30 mL) was added to the reaction solution. The aqueous phase was extracted with ethyl acetate (30 mL × 3), and the organic phase was dried over anhydrous sodium sulfate. The organic phase was concentrated under reduced pressure to give a crude product. The crude product was purified by preparative column chromatography (SiO₂, PE/EtOAc = 2/1) to give a product (230 mg, yield: 89.8%).

MS (ESI): 783 [M+H]⁺.

### Step 16: Synthesis of compound int_19-18:

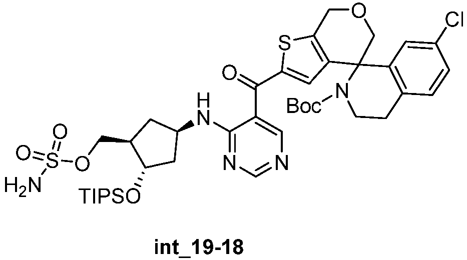

**Int_19-17** (230 mg, 0.294 mmol) was dissolved in DMF (4 mL), and int_1-14 (67.8 mg, 0.587 mmol) was added to the reaction solution at room temperature. The reaction solution was allowed to react at room temperature for 1 h. LC-MS monitoring showed the reaction was completed. Ice water (30 mL) was added to the reaction solution. The aqueous phase was extracted with ethyl acetate (30 mL × 3), and the organic phase was dried over anhydrous sodium sulfate. The organic phase was concentrated under reduced pressure to give a crude product. The crude product was purified by preparative column chromatography to give a solid (248 mg, yield: 98%).

MS (ESI): 862 [M+H]⁺.

### Step 17: Synthesis of compound int_19-19:

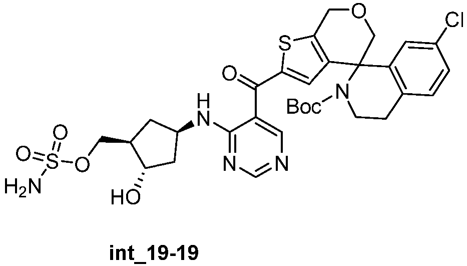

**Int_19-18** (248 mg, 0.288 mmol) was dissolved in THF (4 mL), and TBAF (1 M, 0.58 mL, 0.575 mmol) was added to the reaction solution at room temperature. The reaction solution was allowed to react at room temperature for 16 h. LC-MS monitoring showed the reaction was completed. Water (30 mL) was added to the reaction solution. The aqueous phase was extracted with ethyl acetate (30 mL × 3), and the organic phase was dried over anhydrous sodium sulfate. The organic phase was concentrated under reduced pressure to give a crude product. The crude product was purified by preparative column chromatography to give a solid (183 mg, yield: 90.1%).

MS (ESI): 706 [M+H]⁺.

### Step 18: Synthesis of compound 19:

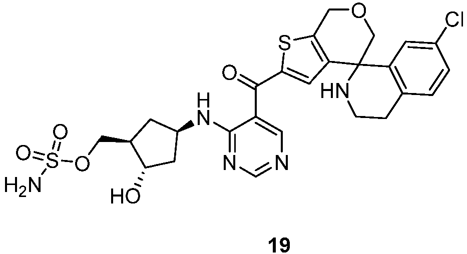

**Int_19-19** (172 mg, 0.244 mmol) was dissolved in dichloromethane (6 mL), and TFA (1.5 mL) was added to the reaction solution at room temperature. The reaction solution was allowed to react at room temperature for 10 min. LC-MS monitoring showed the reaction was completed. Ammonia water (1 mL) and water (10 mL) were added to the reaction solution. The aqueous phase was extracted with dichloromethane (10 mL × 3), and the organic phase was dried over anhydrous sodium sulfate. The organic phase was concentrated under reduced pressure to give a crude product. The crude product was purified by preparative HPLC to give a solid (65 mg, yield: 44.2%).

¹H NMR (400 MHz, DMSO-d6) δ 8.56 (s, 1H), 8.49 (s, 1H), 8.22 (dd, *J=* 7.5, 4.3 Hz, 1H), 7.42 (d, *J* = 3.7 Hz, 2H), 7.26 - 7.17 (m, 2H), 7.16 (s, 1H), 6.90 (s, 1H), 5.09 - 4.91 (m, 2H), 4.87 (t, *J =* 4.4 Hz, 1H), 4.65 (q, *J=* 8.2 Hz, 1H), 4.06 (ddd, *J=* 9.5, 5.4, 2.2 Hz, 2H), 3.93 (dd, *J=* 10.0, 6.7 Hz, 2H), 3.84 *(d, J=* 11.6 Hz, 1H), 3.17 - 2.84 (m, 4H), 2.70 (d, *J =* 16.2 Hz, 1H), 2.28 (d, *J =* 12.4 Hz, 1H), 2.07 (s, 1H), 1.89 (s, 1H), 1.73 (dt, *J=* 13.7, 7.6 Hz, 1H), 1.30 - 1.19 (m, 1H), 1.17 (t, *J=* 7.2 Hz, 1H) .

MS (ESI): 606 [M+H]⁺ .

### Example 8. Synthesis of Compound 20 and Compound 21

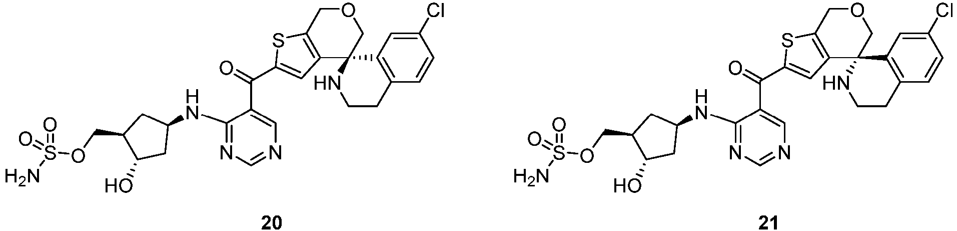

**Compound 19** (50 mg, 0.082 mmol) was subjected to SFC chiral resolution (column: DAICEL CHIRALPAK IG (250 mm × 30 mm, 10 µm); mobile phase: [CO₂-EtOH (0.1% NH₃H₂O)]; B%: 60%, isocratic elution mode) to give **compound 20** (21 mg) and **compound 21** (15 mg).

**Compound 20:** ¹H NMR (400 MHz, DMSO-d6) δ 8.56 (s, 1H), 8.49 (s, 1H), 8.22 (dd, *J=* 7.5, 4.3 Hz, 1H), 7.42 (d, *J=* 3.7 Hz, 2H), 7.26 - 7.17 (m, 2H), 7.16 (s, 1H), 6.90 (s, 1H), 5.09 - 4.91 (m, 2H), 4.87 (t, *J* = 4.4 Hz, 1H), 4.65 (q, *J =* 8.2 Hz, 1H), 4.06 (ddd, *J* = 9.5, 5.4, 2.2 Hz, 2H), 3.93 (dd, *J=* 10.0, 6.7 Hz, 2H), 3.84 (d, *J=* 11.6 Hz, 1H), 3.17 - 2.84 (m, 4H), 2.70 (d, *J =* 16.2 Hz, 1H), 2.28 (d, *J =* 12.4 Hz, 1H), 2.07 (s, 1H), 1.89 (s, 1H), 1.73 (dt, *J =* 13.7, 7.6 Hz, 1H), 1.30 - 1.19 (m, 1H), 1.17 (t, *J =* 7.2 Hz, 1H); MS (ESI): 606 [M+H]⁺.

SFC retention time analysis: 1.910 min (Instrument: Waters UPCC with PDA Detector; Column: Chiralpak IG-3 50x4.6mm I.D., 3um; Mobile phase: A: CO₂, B: Ethanol (0.05% DEA); Isocratic: 40% B; Flow rate: 4mL/min; Column temp.: 35°C; ABPR: 1500psi) .

**Compound 21:** ¹H NMR (400 MHz, DMSO-d6) δ 8.58 (s, 1H), 8.50 (s, 1H), 8.21 (d, *J* = 7.5 Hz, 1H), 7.44 (s, 2H), 7.24 - 7.18 (m, 2H), 7.17 (s, 1H), 6.90 (d, *J=* 1.9 Hz, 1H), 5.07 - 4.94 (m, 2H), 4.88 (d, *J=* 4.5 Hz, 1H), 4.67 (d, *J=* 7.5 Hz, 1H), 4.09 (dd, *J=* 9.6, 6.0 Hz, 1H), 4.02 (d, *J =* 11.3 Hz, 1H), 3.99 - 3.89 (m, 2H), 3.84 (d, J = 11.4 Hz, 1H), 3.03 (m, 2H), 2.88 (m, 2H), 2.68 (m, 1H), 2.36 - 2.23 (m, 2H), 2.10 (m, 1H), 1.90 (m, 1H), 1.75 (m, 1H), 1.26 (m, 2H).; MS (ESI): 606 [M+H]⁺.

SFC retention time analysis: 1.038 min (Instrument: Waters UPCC with PDA Detector; Column: Chiralpak IG-3 50x4.6mm I.D., 3um; Mobile phase: A: CO₂, B: Ethanol (0.05% DEA); Isocratic: 40% B; Flow rate: 4mL/min; Column temp.: 35°C; ABPR: 1500psi) .

### Example 9. Synthesis of Compound 46

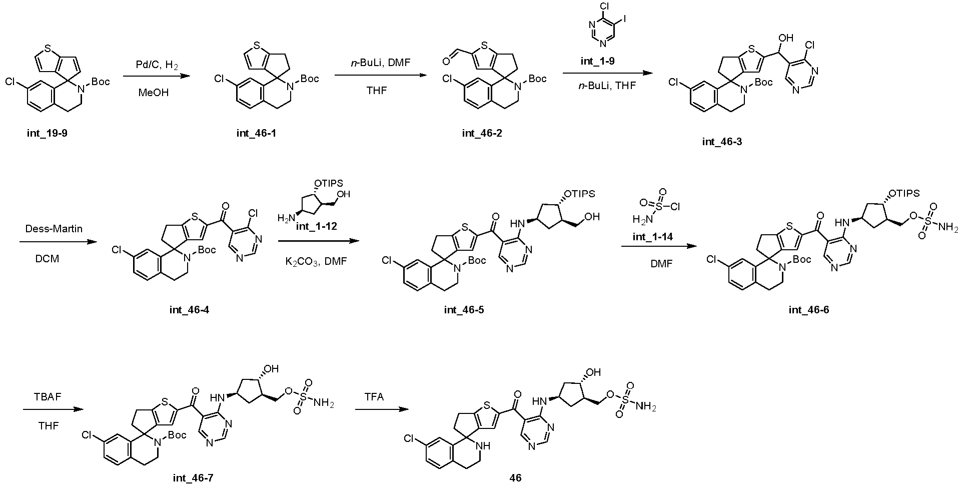

### Step 1: Synthesis of compound int_46-1:

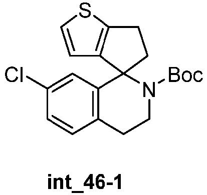

**Int_19-9** (1.50 g, 4.01 mmol) and Pd/C (1.50 g, 10% purity) were dissolved in methanol (20 mL). The reaction solution was purged with hydrogen three times, and allowed to react at room temperature for 16 h under hydrogen atmosphere (25 Psi). LC-MS monitoring showed the reaction was completed. The reaction solution was filtered to give a filtrate, and the filtrate was concentrated under reduced pressure to give a crude product. The crude product was purified by preparative column chromatography (SiO₂, PE/EtOAc = 0/1) to give a solid (1.3 g, yield: 86.2%).

ESI-MS m/z: 376 [M+H]⁺.

### Step 2: Synthesis of compound int_46-2:

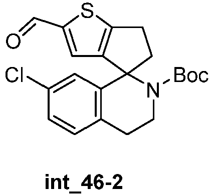

**Int_46-1** (0.30 g, 798 µmol) was dissolved in THF (10 mL), and the mixture was cooled to -60 °C. n-BuLi (2.50 M, 479 µL) was slowly and dropwise added to the reaction solution under nitrogen atmosphere, and the reaction solution was allowed to react at -60 °C for 1 h. Then, DMF (175 mg, 2.39 mmol, 184 µL) was dropwise added to the reaction solution at -60 °C, and after the dropwise addition was completed, the reaction solution was allowed to react at -60 °C for 3 h. LC-MS monitoring showed the reaction was completed. A saturated ammonium chloride solution (100 mL) was slowly added to the reaction solution. The aqueous phase was extracted with ethyl acetate (100 mL × 3), and the organic phase was dried over anhydrous sodium sulfate. The organic phase was concentrated under reduced pressure to give a crude product. The crude product was purified by preparative column chromatography (SiO₂, PE/EtOAc = 3/1) to give a solid (150 mg, yield: 46.5%).

MS (ESI): 404 [M+H]⁺.

### Step 3: Synthesis of compound int_46-3:

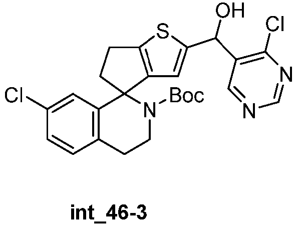

**Int_1-9** (4.79 g, 19.9 mmol) was dissolved in THF (50 mL), and the mixture was cooled to -75 °C. n-BuLi (2.5 M, 16 mL) was slowly and dropwise added to the reaction solution under nitrogen atmosphere, and the reaction solution was allowed to react at -75 °C for 1 h. Then, a solution of int_46-2 (1.3 g, 3.2 mmol) in THF (25 mL) was dropwise added to the reaction solution at -75 °C, and after the dropwise addition was completed, the reaction solution was allowed to react at -75 °C for 1 h, and was then warmed to room temperature and allowed to react for 16 h. LC-MS monitoring showed the reaction was completed. A saturated ammonium chloride solution (200 mL) was slowly added to the reaction solution. The aqueous phase was extracted with ethyl acetate (200 mL × 3), and the organic phase was dried over anhydrous sodium sulfate. The organic phase was concentrated under reduced pressure to give a crude product. The crude product was purified by preparative column chromatography to give a solid (1.15 g, yield: 69.6%).

MS (ESI): 518 [M+H]⁺.

### Step 4: Synthesis of compound int_46-4:

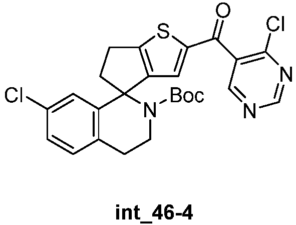

**Int_46-3** (0.200 g, 386 µmol) was dissolved in DCM (10 mL), and a Dess-Martin oxidant (327 mg, 772 µmol, 239 µL) was added to the reaction solution at room temperature. The reaction solution was allowed to react at room temperature for 1 h. LC-MS monitoring showed the reaction was completed. A saturated aqueous sodium bicarbonate solution was slowly added to the reaction solution to adjust the pH to about 8. The aqueous phase was extracted with ethyl acetate (30 mL × 3), and the organic phase was dried over anhydrous sodium sulfate. The organic phase was concentrated under reduced pressure to give a crude product. The crude product was purified by preparative column chromatography (SiO₂, PE/EtOAc = 3/1) to give a solid (170 mg, yield: 85.3%).

¹H NMR (400MHz, CHLOROFORM-d) δ = 9.10 (s, 1H), 8.71 (s, 1H), 7.12 (q, *J=* 8.0 Hz, 2H), 6.86 (s, 1H), 6.58 - 6.50 (m, 1H), 3.94 - 3.84 (m, 1H), 3.75 - 3.63 (m, 1H), 3.34 - 3.11 (m, 3H), 2.92 - 2.84 (m, 2H), 2.77 - 2.68 (m, 1H), 1.24 - 1.11 (m, 3H), 0.96 - 0.81 (m, 2H) .

MS (ESI): 516 [M+H]⁺ .

### Step 5: Synthesis of compound int_46-5:

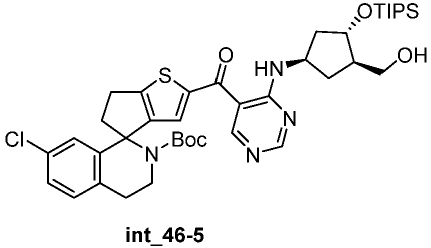

**Int_46-4** (403 mg, 782 µmol) and **int_1-12** (336 mg, 1.17 mmol) were dissolved in DMF (10 mL), and K₂ CO ₃ (540 mg, 3.91 mmol) was added to the reaction solution at room temperature. The reaction solution was allowed to react at room temperature for 16 h. LC-MS monitoring showed the reaction was completed. Ice water (50 mL) was added to the reaction solution. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography (ISCO^{®}; Silica Flash Column, Eluent of 0-20% Ethyl acetate/Petroleum ether gradient) to give a product (0.5 g, yield: 83.3%).

MS (ESI): 767 [M+H]⁺.

### Step 6: Synthesis of compound int_46-6:

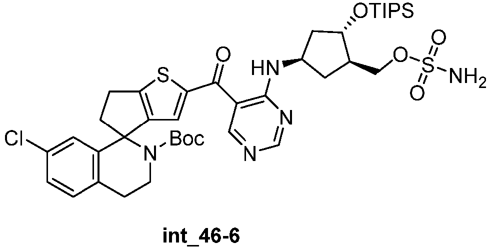

**Int_46-5** (0.500 g, 651 µmol) was dissolved in DMF (4 mL), and **int_1-14** (150 mg, 1.30 mmol) was added to the reaction solution at room temperature. The reaction solution was allowed to react at room temperature for 1 h. LC-MS monitoring showed the reaction was completed. Ice water (50 mL) was added to the reaction solution. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography to give a solid (400 mg, yield: 72.5%).

MS (ESI): 846 [M+H]⁺.

### Step 7: Synthesis of compound int_46-7:

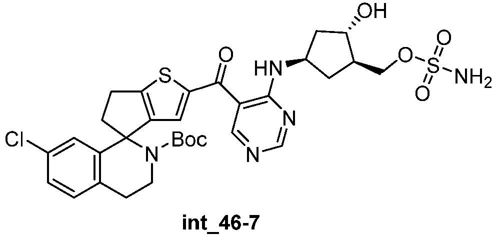

**Int_46-6** (0.400 g, 473 µmol) was dissolved in THF (4 mL), and TBAF (1 M, 473 µL) was added to the reaction solution at room temperature. The reaction solution was allowed to react at room temperature for 16 h. LC-MS monitoring showed the reaction was completed. Water (30 mL) was added to the reaction solution. The aqueous phase was extracted with ethyl acetate (30 mL × 3), and the organic phase was dried over anhydrous sodium sulfate. The organic phase was concentrated under reduced pressure to give a crude product. The crude product was purified by preparative column chromatography (ISCO^{®}; Silica Flash Column, Eluent of 0-100% Ethyl acetate/Petroleum ether gradient) to give a solid (170 mg, yield: 52.1%).

MS (ESI): 690 [M+H]⁺.

### Step 8: Synthesis of compound 46:

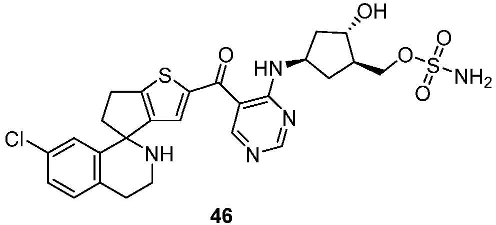

**Int_46-7** (0.17 g, 246 µmol) was added to TFA (0.500 mL) at room temperature, and the reaction solution was allowed to react at room temperature for 10 min. LC-MS monitoring showed the reaction was completed. Ammonia water (1 mL) and water (10 mL) were added to the reaction solution. The aqueous phase was extracted with dichloromethane (10 mL × 3), and the organic phase was dried over anhydrous sodium sulfate. The organic phase was concentrated under reduced pressure to give a crude product, and the crude product was purified by preparative HPLC (column: Boston Prime C18 150 × 30 mm × 5 µm; mobile phase: [water (ammonia hydroxide)-ACN]; gradient: 33%-53% B over 16 min) to give a solid (35 mg, yield: 24.1%).

MS (ESI): 590 [M+H]⁺.

### Example 10. Synthesis of Compound 47 and Compound 48

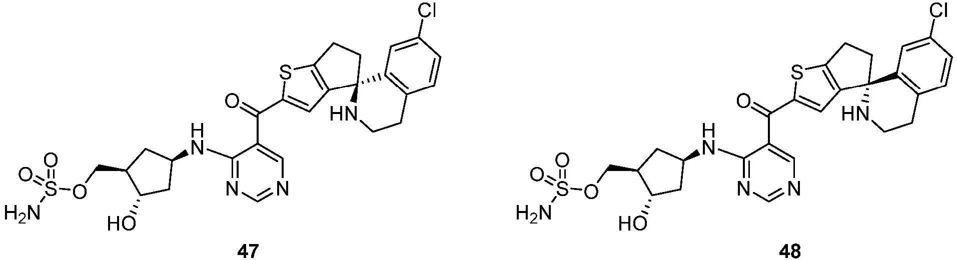

**Compound** 46 (50 mg, 84.7 µmol) was subjected to SFC chiral resolution (column: DAICEL CHIRALPAK AD (250 mm × 30 mm, 10 µm); mobile phase: [CO₂-EtOH (0.1% NH₃H₂O]; B%: 45%, isocratic elution mode) to give **compound 47** (15 mg) and **compound 48** (16 mg).

**Compound 47:** ¹H NMR (400MHz, DMSO-d6) δ = 8.57 (d, *J =* 10.8 Hz, 2H), 8.18 (d, *J=* 7.5 Hz, 1H), 7.45 (s, 2H), 7.23 (s, 1H), 7.18 - 7.12 (m, 2H), 6.77 (d, J = 1.8 Hz, 1H), 4.88 (d, *J* = 4.5 Hz, 1H), 4.76 - 4.55 (m, 1H), 4.09 (dd, *J =* 6.0, 9.7 Hz, 1H), 3.96 (br dd, *J =* 7.0, 9.8 Hz, 2H), 3.16 - 3.03 (m, 3H), 3.01 - 2.90 (m, 1H), 2.88 - 2.79 (m, 1H), 2.72 - 2.71 (m, 2H), 2.65 - 2.63 (m, 1H), 2.31 - 2.21 (m, 1H), 2.10 (br s, 1H), 1.98 - 1.85 (m, 1H), 1.78 - 1.64 (m, 1H), 1.34 - 1.21 (m, 2H).; MS (ESI): 590 [M+H]⁺.

SFC retention time analysis: 0.758 min (Instrument: Waters UPCC with PDA Detector; Column: Chiralpak AD-3 50x4.6mm I.D., 3um; Mobile phase: A: CO₂, B: Ethanol (0.05% DEA); Isocratic: 40% B; Flow rate: 4mL/min; Column temp.: 35°C; ABPR: 1500psi) .

**Compound 48:** ¹H NMR (400MHz, DMSO-d6) δ = 8.57 (d, *J=* 10.8 Hz, 2H), 8.18 (d, *J=* 7.5 Hz, 1H), 7.45 (s, 2H), 7.23 (s, 1H), 7.18 - 7.12 (m, 2H), 6.77 (d, J = 1.8 Hz, 1H), 4.88 (d, *J* = 4.5 Hz, 1H), 4.76 - 4.55 (m, 1H), 4.09 (dd, *J=* 6.0, 9.7 Hz, 1H), 3.96 (br dd, *J =* 7.0, 9.8 Hz, 2H), 3.16 - 3.03 (m, 3H), 3.01 - 2.90 (m, 1H), 2.88 - 2.79 (m, 1H), 2.70 - 2.58 (m, 3H), 2.31 - 2.21 (m, 1H), 2.10 (br s, 1H), 1.98 - 1.85 (m, 1H), 1.78 - 1.64 (m, 1H), 1.34 - 1.21 (m, 2H).; MS (ESI): 590 [M+H]⁺.

SFC retention time analysis: 0.545 min (Instrument: Waters UPCC with PDA Detector; Column: Chiralpak AD-3 50x4.6mm I.D., 3um; Mobile phase: A: CO₂, B: Ethanol (0.05% DEA); Isocratic: 40% B; Flow rate: 4mL/min; Column temp.: 35°C; ABPR: 1500psi) .

### Example 11. Synthesis of Compound 49

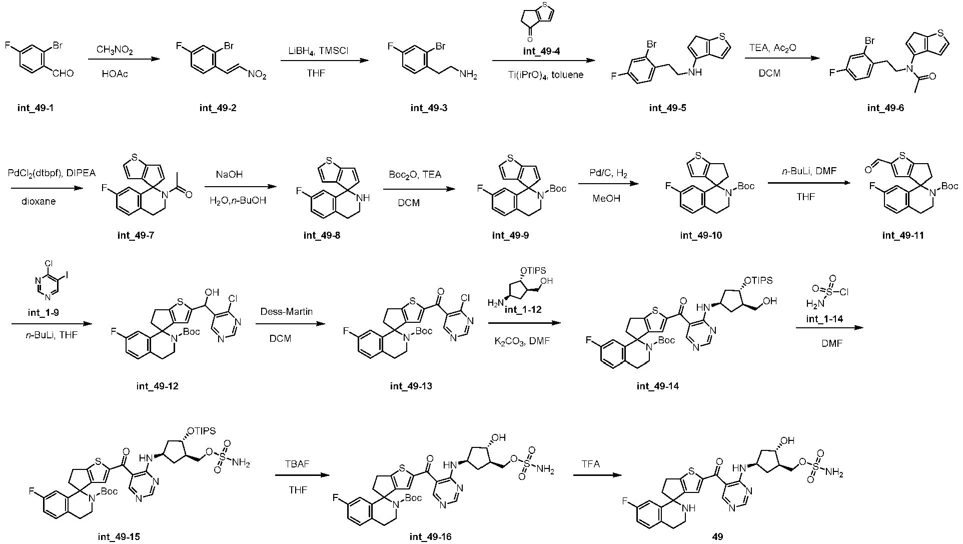

### Step 1: Synthesis of compound int_49-2:

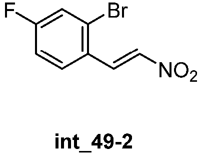

**Int_49-1** (59.0 g, 291 mmol) and NH₄OAc (51.52 g, 668.45 mmol) were dissolved in acetic acid (600 mL), and the mixed solution was purged with nitrogen three times. CH₃NO₂ (46.1 g, 756 mmol, 40.9 mL) was added to the reaction solution under nitrogen atmosphere, and the reaction solution was heated to 100 °C and stirred for 16 h. LC-MS monitoring showed the reaction was completed. The reaction solution was cooled to room temperature and then poured slowly into 1000 mL of ice water. The aqueous phase was extracted with ethyl acetate (1000 mL × 3), and the organic phase was dried over anhydrous sodium sulfate. The organic phase was concentrated under reduced pressure to give a crude product (60 g, yield: 83.9%), which was directly used in the next step.

### Step 2: Synthesis of compound int_49-3:

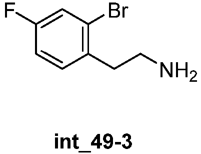

LiBH₄ (21.3 g, 975 mmol) was dissolved in tetrahydrofuran (300 mL), and TMSCl (132.47 g, 1.22 mol, 154.75 mL) was added to the reaction solution at 0 °C under nitrogen atmosphere. The reaction solution was stirred at 0 °C for 30 min, and a solution of int_49-2 (30 g, 122 mmol) in tetrahydrofuran (250 mL) was slowly and dropwise added to the reaction solution within 30 min. The reaction solution was warmed to 70 °C and allowed to react for another 2 h. LC-MS monitoring showed the reaction was completed. The reaction solution was cooled to 0 °C, and 200 mL of methanol was slowly added to quench the reaction. The organic phase was concentrated under reduced pressure to give a crude product, and the crude product was adjusted to pH>7 with ammonia water. The aqueous phase was extracted with ethyl acetate (500 mL × 3), and the organic phase was dried over anhydrous sodium sulfate. The organic phase was concentrated under reduced pressure to give a crude product (16 g, yield: 60.2%), which was directly used in the next step.

ESI-MS m/z: 218 [M+H]⁺.

### Step 3: Synthesis of compound int_49-5:

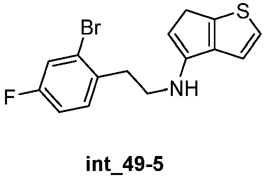

**Int_49-3** (17.0 g, 78.0 mmol), int_49-4 (8.00 g, 57.9 mmol), and Ti(i-PrO)₄ (34.1 g, 120 mmol, 35.4 mL) were dissolved in toluene (200 mL). The mixed solution was purged with nitrogen three times, heated to 90 °C, and stirred for 2 h. LC-MS monitoring showed the reaction was completed. The reaction solution was cooled to room temperature and concentrated under reduced pressure to give a crude product (19 g, yield: 93.7%), which was directly used in the next step.

ESI-MS m/z: 338 [M+H]⁺.

### Step 4: Synthesis of compound int_49-6:

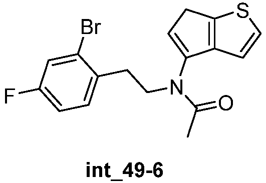

**Int_49-5** (19.0 g, 56.2 mmol), TEA (56.8 g, 562 mmol, 78.2 mL), and Ac₂O (28.7 g, 281 mmol, 26.4 mL) were dissolved in dichloromethane (400 mL), and the reaction solution was allowed to react at 20 °C for 2 h under nitrogen atmosphere. LC-MS monitoring showed the reaction was completed. The reaction solution was cooled to room temperature. The organic phase was concentrated under reduced pressure to give a crude product, and the crude product was adjusted to pH>7 with a saturated aqueous sodium bicarbonate solution. The aqueous phase was extracted with dichloromethane (500 mL × 3), and the organic phase was dried over anhydrous sodium sulfate. The organic phase was concentrated under reduced pressure to give a crude product. The crude product was purified by preparative column chromatography (SiO₂, PE/EtOAc = 3/1) to give a solid (9.2 g, yield: 43.1%).

### Step 5: Synthesis of compound int_49-7:

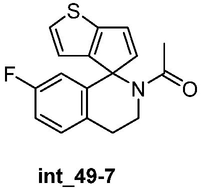

**Int_49-6** (7.10 g, 18.7 mmol), DIPEA (4.83 g, 37.3 mmol, 6.50 mL), and ditert-butyl(cyclopentyl)phosphane;dichloropalladium;iron (1.22 g, 1.87 mmol) were dissolved in 1,4-dioxane (100 mL). The reaction solution was purged with nitrogen three times, warmed to 110 °C, and allowed to react for 16 h. LC-MS monitoring showed the reaction was completed. The reaction solution was cooled to room temperature and then poured slowly into 200 mL of ice water. The aqueous phase was extracted with ethyl acetate (200 mL × 3), and the organic phase was dried over anhydrous sodium sulfate. The organic phase was concentrated under reduced pressure to give a crude product. The crude product was purified by preparative column chromatography (SiO₂, PE/EtOAc = 2/1) to give a solid (4 g, yield: 71.6%).

ESI-MS m/z: 300 [M+H]⁺.

### Step 6: Synthesis of compound int_49-8:

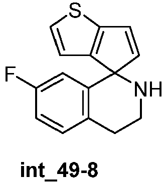

**Int_49-7** (4.70 g, 15.7 mmol) was dissolved in a mixed solvent of n-butanol (75 mL) and water (25 mL), and sodium hydroxide (12.6 g, 314 mmol) was added to the reaction solution. The reaction solution was warmed to 100 °C and allowed to react for 16 h. Subsequently, the reaction solution was warmed to room temperature and allowed to react for 1 h. LC-MS monitoring showed the reaction was completed. Water (100 mL) was added to the reaction solution. The aqueous phase was extracted with ethyl acetate (100 mL × 3), and the organic phase was dried over anhydrous sodium sulfate. The organic phase was concentrated under reduced pressure to give a crude product. The crude product was purified by preparative column chromatography (SiO₂, PE/EtOAc = 1/4) to give a solid (4 g, yield: 99%).

ESI-MS m/z: 258 [M+H]⁺.

### Step 7: Synthesis of compound int_49-9:

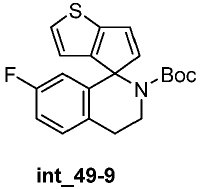

**Int_49-8** (4.00 g, 15.5 mmol) and (Boc)₂O (5.09 g, 23.3 mmol, 5.36 mL) were dissolved in dichloromethane (45 mL), and TEA (4.72 g, 46.6 mmol, 6.49 mL) was added to the reaction solution at room temperature. The reaction solution was allowed to react at room temperature for 16 h under nitrogen atmosphere. LC-MS monitoring showed the reaction was completed. The reaction solution was poured slowly into 100 mL of ice water. The aqueous phase was extracted with dichloromethane (100 mL × 3), and the organic phase was dried over anhydrous sodium sulfate. The organic phase was concentrated under reduced pressure to give a crude product. The crude product was purified by preparative column chromatography (SiO₂, PE/EtOAc = 3/1) to give a solid (4 g, yield: 72%).

¹H NMR (400 MHz, DMSO-d6) δ = 1.05 (s, 9H), 2.80 - 2.98 (m, 2H), 3.65 - 3.85 (m, 2H), 6.14 - 6.26 (m, 1H), 6.41 - 6.55 (m, 1H), 6.71 (d, *J =* 5.50 Hz, 1H), 6.78 - 6.86 (m, 1H), 6.88 - 7.00 (m, 1H), 7.19 (br dd, *J*= 7.81, 6.49 Hz, 1H), 7.37 (br d, *J=* 4.62 Hz, 1H).

### Step 8: Synthesis of compound int_49-10:

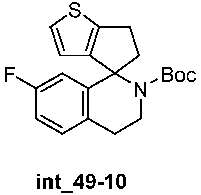

**Int_46-9** (2.00 g, 5.60 mmol) and Pd/C (2.00 g, 10% purity) were dissolved in methanol (20 mL). The reaction solution was purged with hydrogen three times, and allowed to react at room temperature for 16 h under hydrogen atmosphere (25 Psi). LC-MS monitoring showed the reaction was completed. The reaction solution was filtered to give a filtrate, and the filtrate was concentrated under reduced pressure to give a crude product. The crude product was purified by preparative column chromatography (SiO₂, PE/EtOAc = 1/0 to 0/1) to give a solid (1.7 g, yield: 85%).

¹H NMR (400 MHz, DMSO-d6) δ = 1.04 - 1.24 (m, 9H), 2.54 - 2.66 (m, 1H), 2.76 - 2.94 (m, 2H), 2.99 - 3.08 (m, 2H), 3.11 - 3.22 (m, 1H), 3.59 - 3.71 (m, 1H), 3.72 - 3.80 (m, 1H), 6.26 (dd, *J =* 10.94, 2.63 Hz, 1H), 6.57 *(d, J= 5.01* Hz, 1H), 6.93 - 7.02 (m, 1H), 7.19 - 7.28 (m, 1H), 7.35 (d, *J* = 5.01 Hz, 1H) .

ESI-MS m/z: 360 [M+H]⁺ .

### Step 9: Synthesis of compound int_49-11:

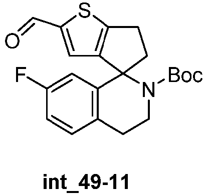

**Int_49-10** (1.70 g, 4.73 mmol) was dissolved in THF (30 mL), and the mixture was cooled to -60 °C. n-BuLi (2.50 M, 2.84 mL) was slowly and dropwise added to the reaction solution under nitrogen atmosphere, and the reaction solution was allowed to react at -60 °C for 1 h. Then, DMF (1.04 g, 14.2 mmol, 1.09 mL) was dropwise added to the reaction solution at -60 °C, and after the dropwise addition was completed, the reaction solution was allowed to react at -60 °C for 3 h. LC-MS monitoring showed the reaction was completed. A saturated ammonium chloride solution (100 mL) was slowly added to the reaction solution. The aqueous phase was extracted with dichloromethane (100 mL × 3), and the organic phase was dried over anhydrous sodium sulfate. The organic phase was concentrated under reduced pressure to give a crude product. The crude product was purified by preparative column chromatography (SiO₂, PE/EtOAc = 3/1) to give a solid (1.8 g, yield: 98.2%). ¹H NMR (400 MHz, DMSO-d6) δ =1.15 - 1.20 (m, 9H), 2.65 - 2.76 (m, 1H), 2.79 - 2.99 (m, 2H), 3.05 - 3.16 (m, 2H), 3.16 - 3.26 (m, 1H), 3.59 - 3.71 (m, 1H), 3.72 - 3.80 (m, 1H), 6.30 - 6.42 (m, 1H), 6.94 - 7.09 (m, 1H), 7.21 - 7.35 (m, 1H), 7.47 - 7.64 (m, 1H), 9.68 - 9.74 (m, 1H) .

MS (ESI): 388 [M+H]⁺ .

### Step 10: Synthesis of compound int_49-12:

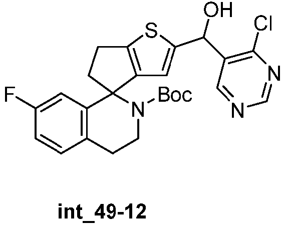

**Int_1-9** (2.79 g, 11.6 mmol) was dissolved in THF (40 mL), and the mixture was cooled to -70 °C. n-BuLi (2.50 M, 9.29 mL) was slowly and dropwise added to the reaction solution under nitrogen atmosphere, and the reaction solution was allowed to react at -70 °C for 1 h. Then, a solution of **int_49-11** (1.50 g, 3.87 mmol) in THF (20 mL) was dropwise added to the reaction solution at -70°C, and after the dropwise addition was completed, the reaction solution was allowed to react at -70 °C for 1 h, and was then warmed to room temperature and allowed to react for 16 h. LC-MS monitoring showed the reaction was completed. A saturated ammonium chloride solution (200 mL) was slowly added to the reaction solution. The aqueous phase was extracted with ethyl acetate (200 mL × 3), and the organic phase was dried over anhydrous sodium sulfate. The organic phase was concentrated under reduced pressure to give a crude product. The crude product was purified by preparative column chromatography to give a solid (1.35 g, yield: 69.5%).

MS (ESI): 502 [M+H]⁺.

### Step 11: Synthesis of compound int_49-13:

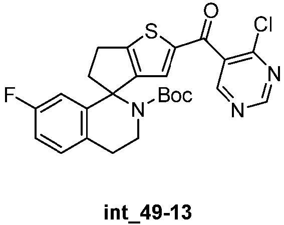

**Int_49-12** (1.35 g, 2.69 mmol) was dissolved in DCM (25 mL), and a Dess-Martin oxidant (2.28 g, 5.38 mmol) was added to the reaction solution at room temperature. The reaction solution was allowed to react at room temperature for 1 h. LC-MS monitoring showed the reaction was completed. A saturated aqueous sodium bicarbonate solution was slowly added to the reaction solution to adjust the pH to about 8. The aqueous phase was extracted with ethyl acetate (100 mL × 3), and the organic phase was dried over anhydrous sodium sulfate. The organic phase was concentrated under reduced pressure to give a crude product. The crude product was purified by preparative column chromatography (SiO₂, PE/EtOAc = 3/1) to give a solid (1 g, yield: 74.4%).

MS (ESI): 500 [M+H]⁺.

### Step 12: Synthesis of compound int_49-14:

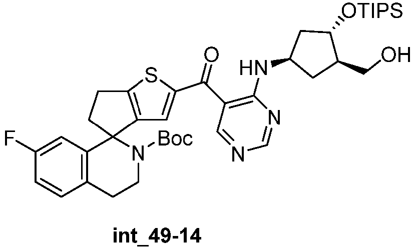

**Int_49-13** (0.60 g, 1.20 mmol) and **int_1-12** (1.30 g, 4.52 mmol) were dissolved in DMF (20 mL), and K₂CO₃ (829 mg, 6.00 mmol) was added to the reaction solution at room temperature. The reaction solution was allowed to react at room temperature for 16 h. LC-MS monitoring showed the reaction was completed. Ice water (50 mL) was added to the reaction solution. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography (ISCO^{®}; Silica Flash Column, Eluent of 0-80% Ethyl acetate/Petroleum ether gradient) to give a product (610 mg, yield: 67.7%).

MS (ESI): 751 [M+H]⁺.

### Step 13: Synthesis of compound int_49-15:

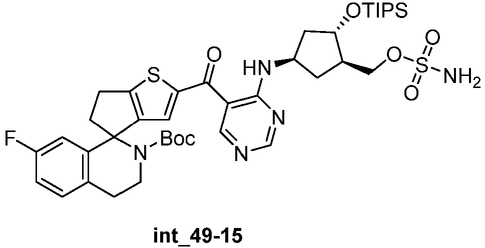

**Int_49-14** (610 mg, 812 µmol) was dissolved in DMF (20 mL), and **int_1-14** (375 mg, 3.25 mmol) was added to the reaction solution at room temperature. The reaction solution was allowed to react at room temperature for 1 h. LC-MS monitoring showed the reaction was completed. Ice water (50 mL) was added to the reaction solution. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography to give a solid (670 mg, yield: 99.4%).

MS (ESI): 830 [M+H]⁺.

### Step 14: Synthesis of compound int_49-16:

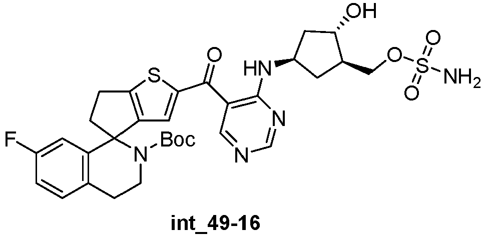

**Int_49-15** (670 mg, 807 µmol) was dissolved in THF (20 mL), and TBAF (1.00 M, 807 µL) was added to the reaction solution at room temperature. The reaction solution was allowed to react at room temperature for 16 h. LC-MS monitoring showed the reaction was completed. Water (50 mL) was added to the reaction solution.

The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography (ISCO^{®}; Silica Flash Column, Eluent of 0-80% Ethyl acetate/Petroleum ether gradient) to give a solid (300 mg, yield: 55.2%).

¹H NMR (400 MHz, DMSO-d6) δ = 1.23 - 1.26 (m, 9H), 1.69 - 1.84 (m, 1H), 1.88 - 1.96 (m, 1H), 2.05 - 2.17 (m, 1H), 2.20 - 2.37 (m, 1H), 2.62 - 2.71 (m, 1H), 2.77 - 2.88 (m, 1H), 2.91 (s, 1H), 3.07 - 3.17 (m, 1H), 3.18 - 3.26 (m, 2H), 3.64 - 3.83 (m, 2H), 3.90 - 4.01 (m, 2H), 4.01 - 4.06 (m, 1H), 4.06 - 4.13 (m, 1H), 4.63 - 4.73 (m, 1H), 4.82 - 4.94 (m, 1H), 6.30 - 6.42 (m, 1H), 6.94 - 7.06 (m, 1H), 7.22 - 7.29 (m, 1H), 7.36 - 7.40 (m, 1H), 7.42 - 7.48 (m, 2H), 8.16 - 8.28 (m, 1H), 8.44 - 8.49 (m, 1H), 8.57 - 8.62 (m, 1H).

MS (ESI): 674 [M+H]⁺ .

### Step 15: Synthesis of compound 49:

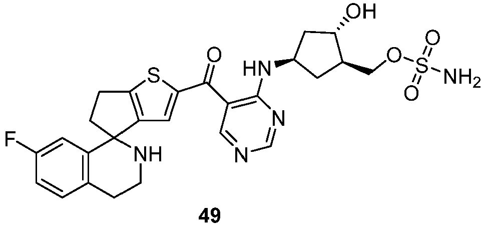

**Int_49-16** (200 mg, 297 µmol) was added to TFA (0.5 mL) at room temperature, and the reaction solution was allowed to react at room temperature for 10 min. LC-MS monitoring showed the reaction was completed. Ammonia water (1 mL) and water (30 mL) were added to the reaction solution. The aqueous phase was extracted with dichloromethane (30 mL × 3), and the organic phase was dried over anhydrous sodium sulfate. The organic phase was concentrated under reduced pressure to give a crude product, and the crude product was purified by preparative HPLC (column: Boston Prime C18 150 × 30 mm × 5 µm; mobile phase: [water (ammonia hydroxide)-ACN]; gradient: 33%-53% B over 10 min) to give a solid (60 mg, yield: 35.2%).

MS (ESI): 574 [M+H]⁺.

### Example 12. Synthesis of Compound 50 and Compound 51

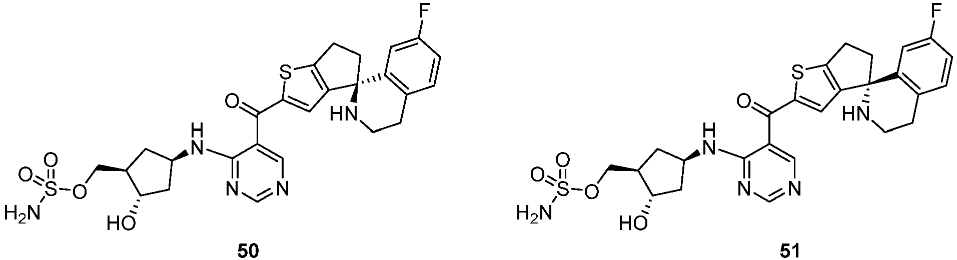

**Compound 49** (60 mg, 104 µmol) was subjected to SFC chiral resolution (column: DAICEL CHIRALPAK IE (250 mm × 30 mm, 10 µm); mobile phase: [Heptane-EtOH (0.1% NH₃H₂O]; B%: 55%, isocratic elution mode) to give **compound 50** (25 mg) and **compound 51** (26 mg).

**Compound 50:** ¹H NMR (400 MHz, DMSO-d6) δ = 1.19 - 1.32 (m, 2H), 1.69 - 1.81 (m, 1H), 1.87 - 2.00 (m, 1H), 2.03 - 2.16 (m, 1H), 2.23 - 2.35 (m, 1H), 2.35 - 2.91 (m, 4H), 2.92 - 3.03 (m, 1H), 3.04 - 3.18 (m, 3H), 3.90 - 4.00 (m, 2H), 4.05 - 4.12 (m, 1H), 4.63 - 4.72 (m, 1H), 4.85 - 4.91 (m, 1H), 6.50 - 6.58 (m, 1H), 6.92 - 7.00 (m, 1H), 7.11 - 7.18 (m, 1H), 7.22 - 7.25 (m, 1H), 7.41 - 7.47 (m, 2H), 8.14 - 8.20 (m, 1H), 8.57 (s, 1H), 8.58 - 8.61 (m, 1H).; MS (ESI): 574 [M+H]⁺.

SFC retention time analysis: 4.478 min (Instrument: Shimadzu LC-20AD with PDA detector; Column: Chiralpak IE 100x4.6mm I.D., 3um; Mobile phase: A: Hexane(0.1%DEA), B: Ethanol (0.05% DEA); Isocratic: 40% B; Flow rate: 1mL/min; Column temp.: 35°C) ..

**Compound 51:** ¹H NMR (400 MHz, DMSO-d6) δ = 1.19 - 1.32 (m, 2H), 1.69 - 1.81 (m, 1H), 1.87 - 2.00 (m, 1H), 2.03 - 2.16 (m, 1H), 2.23 - 2.35 (m, 1H), 2.35 - 2.91 (m, 4H), 2.92 - 3.03 (m, 1H), 3.04 - 3.18 (m, 3H), 3.90 - 4.00 (m, 2H), 4.05 - 4.12 (m, 1H), 4.63 - 4.72 (m, 1H), 4.85 - 4.91 (m, 1H), 6.50 - 6.58 (m, 1H), 6.92 - 7.00 (m, 1H), 7.11 - 7.18 (m, 1H), 7.22 - 7.25 (m, 1H), 7.41 - 7.47 (m, 2H), 8.14 - 8.20 (m, 1H), 8.57 (s, 1H), 8.58 - 8.61 (m, 1H).; MS (ESI): 574 [M+H]⁺.

SFC retention time analysis: 3.339 min (Instrument: Shimadzu LC-20AD with PDA detector; Column: Chiralpak IE 100x4.6mm I.D., 3um; Mobile phase: A: Hexane(0.1%DEA), B: Ethanol (0.05% DEA); Isocratic: 40% B; Flow rate: 1mL/min; Column temp.: 35°C) .

### Example 13. Synthesis of Compound 78

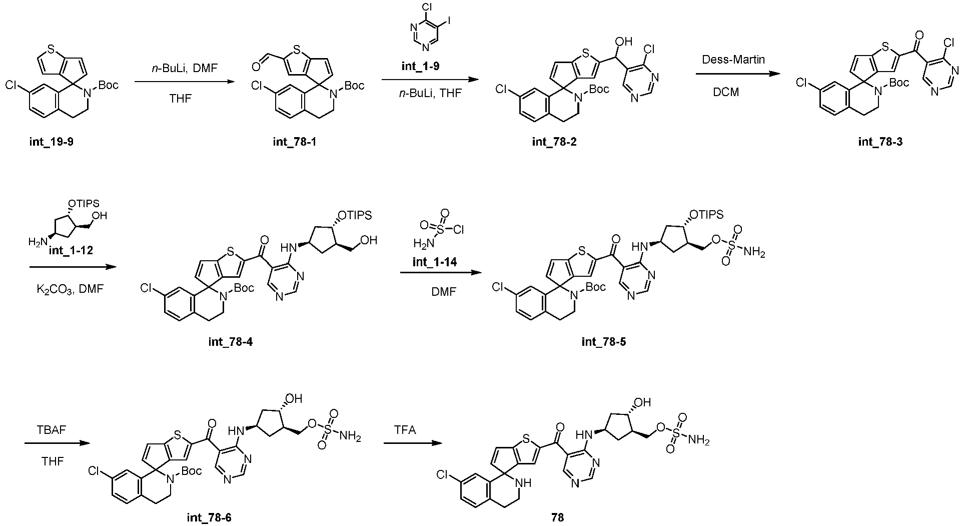

### Step 1: Synthesis of compound int_78-1:

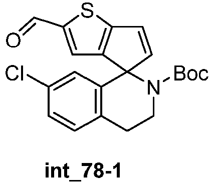

**Int_78-1** (0.30 g, 798 µmol) was dissolved in THF (10 mL), and the mixture was cooled to -60 °C. n-BuLi (2.50 M, 479 µL) was slowly and dropwise added to the reaction solution under nitrogen atmosphere, and the reaction solution was allowed to react at -60 °C for 1 h. Then, DMF (175 mg, 2.39 mmol, 184 µL) was dropwise added to the reaction solution at -60 °C, and after the dropwise addition was completed, the reaction solution was allowed to react at -60 °C for 3 h. LC-MS monitoring showed the reaction was completed. A saturated ammonium chloride solution (100 mL) was slowly added to the reaction solution. The aqueous phase was extracted with ethyl acetate (100 mL × 3), and the organic phase was dried over anhydrous sodium sulfate. The organic phase was concentrated under reduced pressure to give a crude product. The crude product was purified by preparative column chromatography (SiO₂, PE/EtOAc = 2/1) to give a solid (140 mg, yield: 43.4%).

¹H NMR (400 MHz, DMSO-d6) δ = 1.12 (s, 9H), 2.88 - 3.08 (m, 2H), 3.66 - 3.93 (m, 2H), 6.53 - 6.59 (m, 1H), 6.90 (d, J=5.62 Hz, 1H), 6.94 - 7.00 (m, 1H), 7.19 - 7.26 (m, 1H), 7.27 - 7.33 (m, 1H), 7.74 - 7.85 (m, 1H), 9.75 (s, 1H) .

MS (ESI): 402 [M+H]⁺ .

### Step 2: Synthesis of compound int_78-2:

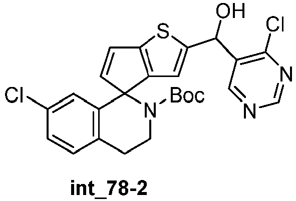

**Int_1-9** (4.79 g, 19.9 mmol) was dissolved in THF (50 mL), and the mixture was cooled to -75 °C. n-BuLi (2.5 M, 16 mL) was slowly and dropwise added to the reaction solution under nitrogen atmosphere, and the reaction solution was allowed to react at -75 °C for 1 h. Then, a solution of **int_78-1** (1.3 g, 3.2 mmol) in THF (25 mL) was dropwise added to the reaction solution at -75 °C, and after the dropwise addition was completed, the reaction solution was allowed to react at -75 °C for 1 h, and was then warmed to room temperature and allowed to react for 16 h. LC-MS monitoring showed the reaction was completed. A saturated ammonium chloride solution (200 mL) was slowly added to the reaction solution. The aqueous phase was extracted with ethyl acetate (200 mL × 3), and the organic phase was dried over anhydrous sodium sulfate. The organic phase was concentrated under reduced pressure to give a crude product. The crude product was purified by preparative column chromatography to give a solid (1.3 g, yield: 78.7%).

¹H NMR (400 MHz, DMSO-d6) δ = 1.14 (s, 9H), 2.82 - 3.02 (m, 2H), 3.67 - 3.82 (m, 2H), 5.74 - 5.78 (m, 1H), 6.53 - 6.59 (m, 1H), 6.90 - 6.94 (m, 1H), 6.98 - 7.03 (m, 1H), 7.19 - 7.29 (m, 2H), 7.57 - 7.62 (m, 1H), 8.97 - 9.01 (m, 1H), 9.19 (s, 1H) .

MS (ESI): 516 [M+H]⁺ .

### Step 3: Synthesis of compound int_78-3:

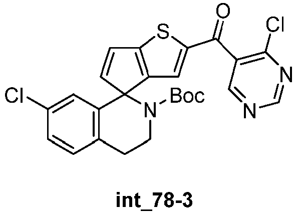

**Int_78-2** (1.60 g, 3.10 mmol) was dissolved in DCM (100 mL), and a Dess-Martin oxidant (2.63 g, 6.20 mmol) was added to the reaction solution at room temperature. The reaction solution was allowed to react at room temperature for 1 h. LC-MS monitoring showed the reaction was completed. A saturated aqueous sodium bicarbonate solution was slowly added to the reaction solution to adjust the pH to about 8. The aqueous phase was extracted with ethyl acetate (100 mL × 3), and the organic phase was dried over anhydrous sodium sulfate. The organic phase was concentrated under reduced pressure to give a crude product. The crude product was purified by preparative column chromatography (SiO₂, PE/EtOAc = 2/1) to give a solid (0.9 g, yield: 56.5%). MS (ESI): 514 [M+H]⁺.

### Step 4: Synthesis of compound int_78-4:

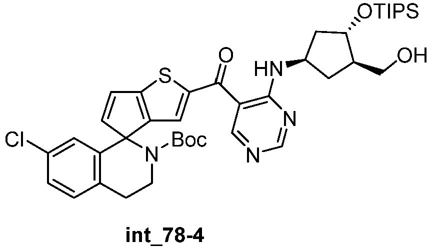

**Int_78-3** (0.90 g, 1.75 mmol) and **int_1-12** (1.50 g, 5.22 mmol) were dissolved in DMF (20 mL), and K₂CO₃ (1.21 g, 8.75 mmol) was added to the reaction solution at room temperature. The reaction solution was allowed to react at room temperature for 16 h. LC-MS monitoring showed the reaction was completed. Ice water (100 mL) was added to the reaction solution. The aqueous phase was extracted with ethyl acetate (100 mL × 3), and the organic phase was dried over anhydrous sodium sulfate. The organic phase was concentrated under reduced pressure to give a crude product. The crude product was purified by preparative column chromatography (ISCO^{®}; Silica Flash Column, Eluent of 0-30% Ethyl acetate/Petroleum ether gradient) to give a product (0.7 g, yield: 52.3%).

¹H NMR (400 MHz, CHLOROFORM-d) δ = 1.00 - 1.01 (m, 18H), 1.12 - 1.29 (m, 9H), 1.65 - 1.83 (m, 3H), 2.05 - 2.17 (m, 2H), 2.34 - 2.48 (m, 1H), 2.80 - 2.97 (m, 3H), 3.55 - 3.66 (m, 2H), 3.74 - 3.91 (m, 2H), 4.19 - 4.27 (m, 1H), 4.60 - 4.78 (m, 1H), 4.70 (sxt, *J =* 7.68 Hz, 1H), 6.57 - 6.61 (m, 2H), 6.69 (d, *J =* 5.50 Hz, 1H), 7.02 (d, *J=* 1.13 Hz, 2H), 7.15 (s, 1H), 7.86 - 8.06 (m, 1H), 8.45 (br dd, *J =* 7.07, 3.94 Hz, 1H), 8.54 (s, 1H), 8.59 (s, 1H).

MS (ESI): 765 [M+H]⁺ .

### Step 5: Synthesis of compound int_78-5:

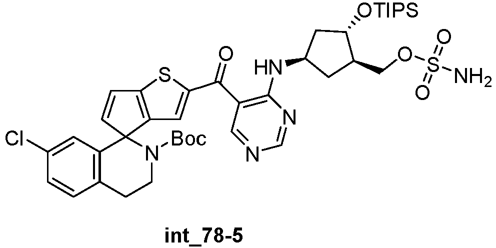

**Int_78-4** (0.70 g, 914 µmol) was dissolved in DMF (20 mL), and **int_1-14** (423 mg, 3.66 mmol) was added to the reaction solution at room temperature. The reaction solution was allowed to react at room temperature for 1 h. LC-MS monitoring showed the reaction was completed. Ice water (50 mL) was added to the reaction solution. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography to give a solid (0.77 g, yield: 99.7%).

MS (ESI): 844 [M+H]⁺.

### Step 6: Synthesis of compound int_78-6:

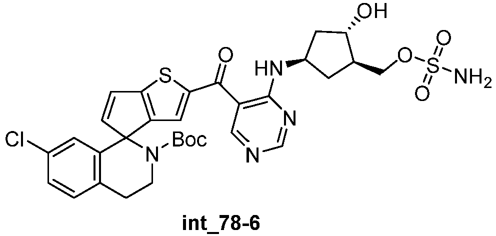

**Int_78-5** (0.77 g, 912 µmol) was dissolved in THF (4 mL), and TBAF (1 M, 912 µL) was added to the reaction solution at room temperature. The reaction solution was allowed to react at room temperature for 16 h. LC-MS monitoring showed the reaction was completed. Water (50 mL) was added to the reaction solution. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography (ISCO^{®}; Silica Flash Column, Eluent of 0-30% Ethyl acetate/Petroleum ether gradient) to give a solid (400 mg, yield: 63.8%).

MS (ESI): 688 [M+H]⁺.

### Step 7: Synthesis of compound 78:

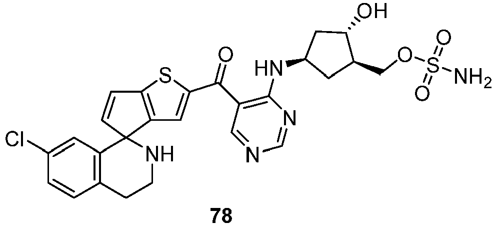

**Int_78-6** (200 mg, 291 µmol) was added to TFA (0.500 mL) at room temperature, and the reaction solution was allowed to react at room temperature for 10 min. LC-MS monitoring showed the reaction was completed. Ammonia water (1 mL) and water (10 mL) were added to the reaction solution. The aqueous phase was extracted with dichloromethane (10 mL × 3), and the organic phase was dried over anhydrous sodium sulfate. The organic phase was concentrated under reduced pressure to give a crude product, and the crude product was purified by preparative HPLC (column: Boston Prime C18 150 × 30 mm × 5 µm; mobile phase: [water (ammonia hydroxide)-ACN]; gradient: 33%-55% B over 10 min) to give a solid (90 mg, yield: 52.7%).

¹H NMR (400 MHz, DMSO-d6) δ =1.11 - 1.25 (m, 1H), 1.66 - 1.73 (m, 1H), 1.86 - 1.96 (m, 1H), 1.99 - 2.10 (m, 1H), 2.15 - 2.27 (m, 1H), 2.70 - 2.90 (m, 2H), 3.03 - 3.07 (m, 1H), 3.15 - 3.19 (m, 2H), 3.81 - 3.95 (m, 2H), 3.96 - 4.06 (m, 1H), 4.51 - 4.70 (m, 1H), 4.75 - 4.91 (m, 1H), 6.36 - 6.43 (m, 1H), 6.90 - 6.97 (m, 2H), 7.08 - 7.19 (m, 2H), 7.32 - 7.45 (m, 2H), 7.48 - 7.60 (m, 1H), 8.01 - 8.06 (m, 1H), 8.45 - 8.50 (m, 1H), 8.53 (br d, *J=* 12.38 Hz, 2 H) .

MS (ESI): 588 [M+H]⁺ .

### Example 14. Synthesis of Compound 175 and Compound 176

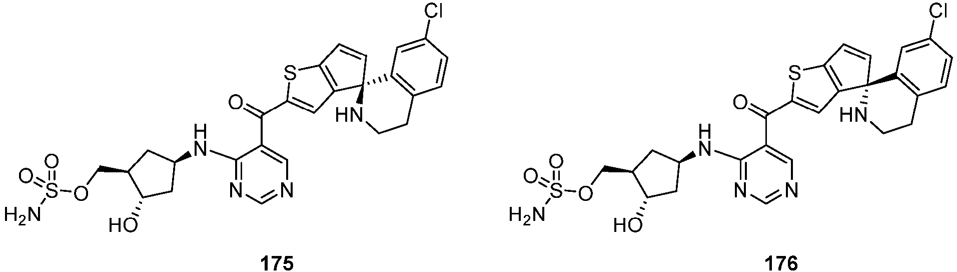

**Compound** 78 (100 mg, 170 µmol) was subjected to SFC chiral resolution (column: DAICEL CHIRALPAK AD (250 mm × 30 mm, 10 µm); mobile phase: [CO₂-EtOH (0.1% NH₃H₂O]; B%: 50%, isocratic elution mode) to give **compound 175** (15 mg) and **compound 176** (16 mg).

**Compound 175:** ¹H NMR (400 MHz, DMSO-d6) δ =1.11 - 1.25 (m, 1H), 1.66 - 1.73 (m, 1H), 1.86 - 1.96 (m, 1H), 1.99 - 2.10 (m, 1H), 2.15 - 2.27 (m, 1H), 2.70 - 2.90 (m, 2H), 3.03 - 3.07 (m, 1H), 3.15 - 3.19 (m, 2H), 3.81 - 3.95 (m, 2H), 3.96 - 4.06 (m, 1H), 4.51 - 4.70 (m, 1H), 4.75 - 4.91 (m, 1H), 6.36 - 6.43 (m, 1H), 6.90 - 6.97 (m, 2H), 7.08 - 7.19 (m, 2H), 7.32 - 7.45 (m, 2H), 7.48 - 7.60 (m, 1H), 8.01 - 8.06 (m, 1H), 8.45 - 8.50 (m, 1H), 8.53 (br d, *J=* 12.38 Hz, 2H).; MS (ESI): 588 [M+H]⁺.

SFC retention time analysis: 2.629 min (Instrument: Waters UPCC with PDA Detector; Column: Chiralpak AD-3 50x4.6mm I.D., 3um; Mobile phase: A: CO₂, B: Ethanol (0.05% DEA); Gradient: from 5% to 40% of B in 2 min and hold 40% for 1.2 min, then 5% of B for 0.8 min; Flow rate: 4mL/min; Column temp.: 35°C; ABPR: 1500psi) .

**Compound 176:** ¹H NMR (400 MHz, DMSO-d6) δ =1.11 - 1.25 (m, 1H), 1.66 - 1.73 (m, 1H), 1.86 - 1.96 (m, 1H), 1.99 - 2.10 (m, 1H), 2.15 - 2.27 (m, 1H), 2.70 - 2.90 (m, 2H), 3.03 - 3.07 (m, 1H), 3.15 - 3.19 (m, 2H), 3.81 - 3.95 (m, 2H), 3.96 - 4.06 (m, 1H), 4.51 - 4.70 (m, 1H), 4.75 - 4.91 (m, 1H), 6.36 - 6.43 (m, 1H), 6.90 - 6.97 (m, 2H), 7.08 - 7.19 (m, 2H), 7.32 - 7.45 (m, 2H), 7.48 - 7.60 (m, 1H), 8.01 - 8.06 (m, 1H), 8.45 - 8.50 (m, 1H), 8.53 (br d, *J=* 12.38 Hz, 2H).; MS (ESI): 588 [M+H]⁺.

SFC retention time analysis: 2.205 min (Instrument: Waters UPCC with PDA Detector; Column: Chiralpak AD-3 50x4.6mm I.D., 3um; Mobile phase: A: CO₂, B: Ethanol (0.05% DEA); Gradient: from 5% to 40% of B in 2 min and hold 40% for 1.2 min, then 5% of B for 0.8 min; Flow rate: 4mL/min; Column temp.: 35°C; ABPR: 1500psi) .

### Example 15. Synthesis of Compound 91

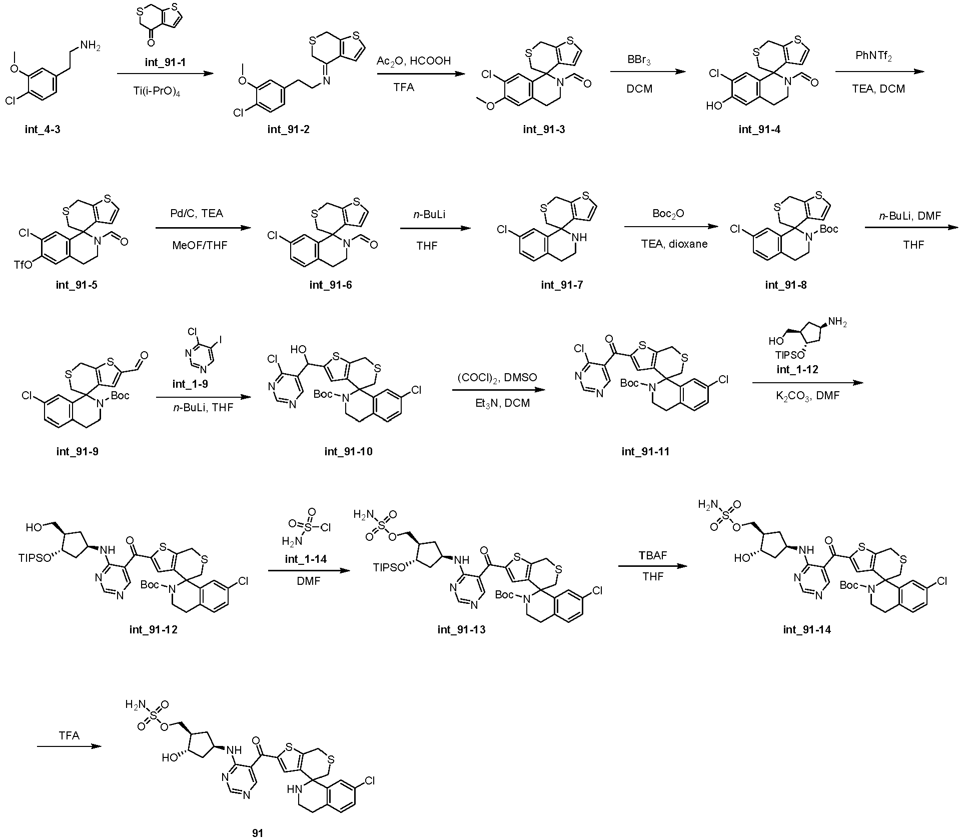

### Step 1: Synthesis of compound int_91-2:

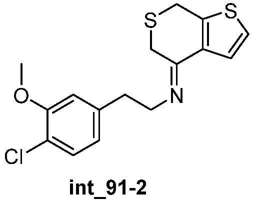

**Int_4-3** (9.00 g, 48.48 mmol) and **int_91-1** (9.07 g, 53.33 mmol) were dissolved in Ti(i-PrO)₄ (170 mL). The mixed solution was purged with nitrogen three times, heated to 80 °C, and stirred for 2 h. LC-MS monitoring showed the reaction was completed. The reaction solution was cooled to room temperature, which was directly used in the next step.

ESI-MS m/z: 338 [M+H]⁺.

### Step 2: Synthesis of compound int_91-3:

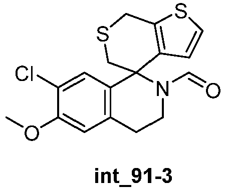

HCOOH (200 mL) was slowly and dropwise added to Ac₂O (500 mL) at -10°C, and after the addition was completed, the reaction solution was allowed to react at 20 °C for 0.5 h. Then, the int_91-2 solution obtained in step 3 was cooled to -10°C, and the mixed solution of HOOCH and Ac₂O described above was slowly and dropwise added to the **int_91-2** solution. The temperature was kept at -10 °C during the dropwise addition. After the dropwise addition was completed, the reaction solution was warmed to 70 °C and allowed to react for 3 h. LC-MS monitoring showed the reaction was completed. The reaction solution was cooled to room temperature. The organic phase was concentrated under reduced pressure to give a crude product, and the crude product was adjusted to pH>7 with a saturated aqueous sodium bicarbonate solution. The aqueous phase was extracted with ethyl acetate (500 mL × 3), and the organic phase was dried over anhydrous sodium sulfate. The organic phase was concentrated under reduced pressure to give a crude product. The crude product was purified by preparative column chromatography (SiO₂, PE/EtOAc = 1/1) to give a solid (2.3 g, yield: 11.7%). ¹H NMR (400 MHz, CHLOROFORM-d) δ 7.91 (s, 1H), 7.06 (d, *J =* 5.3 Hz, 1H), 6.73 (s, 1H), 6.70 (s, 1H), 6.25 (d, *J=* 5.3 Hz, 1H), 4.80 - 4.69 (m, 1H), 4.18 (d, *J=* 16.3 Hz, 1H), 3.90 (s, 3H), 3.71 (dd, *J=* 16.3, 1.5 Hz, 1H), 3.27 - 3.11 (m, 2H), 3.06 - 2.97 (m, 2H), 2.87 - 2.75 (m, 1H).

ESI-MS m/z: 366 [M+H]⁺ .

### Step 3: Synthesis of compound int_91-4:

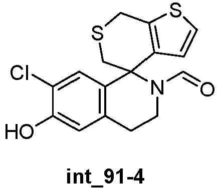

**Int_91-3** (3.50 g, 9.5 mmol) was dissolved in dichloromethane (30 mL), and the mixed solution was purged with nitrogen three times. The reaction solution was cooled to 0 °C, and BBr₃ (11.88 g, 47.43 mmol) was slowly and dropwise added to the reaction solution. The reaction solution was warmed to room temperature and allowed to react for 1 h. LC-MS monitoring showed the reaction was completed. The reaction solution was poured slowly into 300 mL of ice water. The aqueous phase was extracted with ethyl acetate (300 mL × 3), and the organic phase was dried over anhydrous sodium sulfate. The organic phase was concentrated under reduced pressure to give a crude product (3 g, yield: 90.9%), which was directly used in the next step.

ESI-MS m/z: 352 [M+H]⁺.

### Step 4: Synthesis of compound int_91-5:

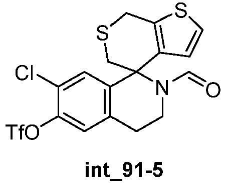

**Int_91-4** (3 g, 8.5 mmol), PhNTf₂ (5.46 g, 15.3 mmol), and TEA (2.15 g, 21.25 mmol, 2.92 mL) were dissolved in dichloromethane (30 mL). The reaction solution was purged with nitrogen three times, and allowed to react at room temperature for 16 h. LC-MS monitoring showed the reaction was completed. The reaction solution was poured slowly into 300 mL of ice water. The aqueous phase was extracted with ethyl acetate (300 mL × 3), and the organic phase was dried over anhydrous sodium sulfate. The organic phase was concentrated under reduced pressure to give a crude product. The crude product was purified by preparative column chromatography (SiO₂, PE/EtOAc = 1/1) to give a solid (2.9 g, yield: 70.7%).

ESI-MS m/z: 484 [M+H]⁺.

### Step 5: Synthesis of compound int_91-6:

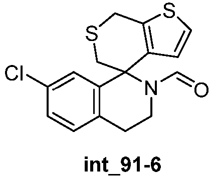

**Int_91-5** (2.9 g, 5.99 mmol), Pd/C (1.2 g, 10% purity), and TEA (2.42 g, 23.96 mmol, 3.33 mL) were dissolved in a mixed solvent of methanol (25 mL) and tetrahydrofuran (10 mL). The reaction solution was purged with hydrogen three times, and allowed to react at room temperature for 16 h. LC-MS monitoring showed the reaction was completed. The reaction solution was filtered to give a filtrate, and the filtrate was concentrated under reduced pressure to give a crude product. The crude product was purified by preparative column chromatography (SiO₂, PE/EtOAc = 1/1) to give a solid (0.85 g, yield: 42.5%).

ESI-MS m/z: 336 [M+H]⁺.

### Step 6: Synthesis of compound int_91-7:

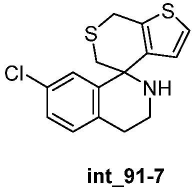

**Int_91-6** (0.85 g, 2.53 mmol) was dissolved in THF (10 mL), and the mixture was cooled to -30 °C. n-BuLi (2.5 M, 3.04 mL) was slowly and dropwise added to the reaction solution under nitrogen atmosphere, and the reaction solution was allowed to react at -30 °C for 2 h. Subsequently, the reaction solution was warmed to room temperature and allowed to react for 1 h. LC-MS monitoring showed the reaction was completed. A saturated aqueous ammonium chloride solution (10 mL) was added to the reaction solution. The aqueous phase was extracted with ethyl acetate (30 mL × 3), and the organic phase was dried over anhydrous sodium sulfate. The organic phase was concentrated under reduced pressure to give a crude product (0.71 g, yield: 91.2%), which was directly used in the next step.

¹H NMR (400 MHz, DMSO-d6) δ 7.16 (d, *J=* 1.4 Hz, 2H), 7.14 (d, *J* = 5.3 Hz, 1H), 6.85 *(t, J=* 1.3 Hz, 1H), 6.35 (d, *J=* 5.3 Hz, 1H), 4.16 (d, *J=* 16.1 Hz, 1H), 3.69 (dd, *J=* 16.0, 1.3 Hz, 1H), 3.25 - 3.11 (m, 2H), 2.98 (m, 2H), 2.88 (m, 1H), 2.74 (br, 1H), 2.68 (dt, *J=* 16.0, 3.5 Hz, 1H) .

ESI-MS m/z: 308 [M+H]⁺ .

### Step 7: Synthesis of compound int_91-8:

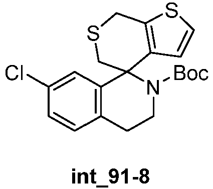

**Int_91-7** (0.71 g, 2.30 mmol) and (Boc)₂O (1 g, 4.60 mmol) were dissolved in 1,4-dioxane (10 mL), and TEA (581.8 mg, 5.75 mmol, 801.4 µL) was added to the reaction solution at room temperature. The reaction solution was warmed to 80 °C and allowed to react for 16 h. LC-MS monitoring showed the reaction was completed. After the reaction solution was cooled to room temperature, the reaction solution was concentrated under reduced pressure to give a crude product. The crude product was purified by preparative column chromatography (SiO₂, PE/EtOAc = 10/1) to give a solid (0.65 g, yield: 69.2%).

¹H NMR (400 MHz, DMSO-d6) δ 7.29 (d, *J* = 5.3 Hz, 1H), 7.24 (d, *J =* 1.4 Hz, 2H), 6.73 (t, *J =* 1.3 Hz, 1H), 6.53 (d, *J =* 5.3 Hz, 1H), 4.23 (dt, *J =* 12.1, 3.7 Hz, 1H), 3.96 (d, *J* = 16.1 Hz, 1H), 3.90 (d, *J* = 13.5 Hz, 1H), 3.81 (dd, *J =* 16.1, 1.5 Hz, 1H), 3.15 (td, *J =* 12.0, 3.2 Hz, 1H), 2.98 (ddd, *J =* 15.8, 11.7, 4.1 Hz, 1H), 2.89 (dt, *J =* 15.5, 3.3 Hz, 1H), 2.52 (d, *J =* 1.6 Hz, 1H), 1.15 (s, 9H) .

ESI-MS m/z: 408 [M+H]⁺ .

### Step 8: Synthesis of compound int_91-9:

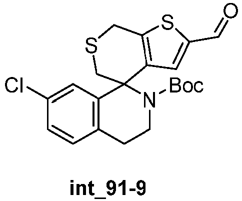

**Int_91-8** (1.5 g, 3.67 mmol) was dissolved in THF (20 mL), and the mixture was cooled to -75 °C. n-BuLi (2.5 M, 4.43 mL) was slowly and dropwise added to the reaction solution under nitrogen atmosphere, and the reaction solution was allowed to react at -75 °C for 1 h. Then, DMF (809 mg, 11.08 mmol) was dropwise added to the reaction solution at -75 °C, and after the dropwise addition was completed, the reaction solution was allowed to react at -75°C for 1 h. LC-MS monitoring showed the reaction was completed. A saturated ammonium chloride solution (100 mL) was slowly added to the reaction solution. The aqueous phase was extracted with ethyl acetate (100 mL × 3), and the organic phase was dried over anhydrous sodium sulfate. The organic phase was concentrated under reduced pressure to give a crude product. The crude product was purified by preparative column chromatography to give a solid (1.25 g, yield: 78.1%).

MS (ESI): 436 [M+H]⁺.

### Step 9: Synthesis of compound int_91-10:

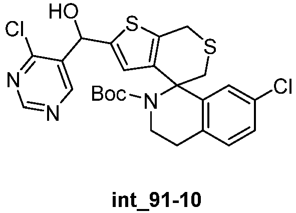

**Int_1-9** (4.8 g, 19.92 mmol) was dissolved in THF (50 mL), and the mixture was cooled to -75 °C. n-BuLi (2.5 M, 16 mL) was slowly and dropwise added to the reaction solution under nitrogen atmosphere, and the reaction solution was allowed to react at -75 °C for 1 h. Then, a solution of **int_91-9** (1.45 g, 3.33 mmol) in THF (25 mL) was dropwise added to the reaction solution at -75 °C, and after the dropwise addition was completed, the reaction solution was allowed to react at -75 °C for 1 h, and was then warmed to room temperature and allowed to react for 16 h. LC-MS monitoring showed the reaction was completed. A saturated ammonium chloride solution (200 mL) was slowly added to the reaction solution. The aqueous phase was extracted with ethyl acetate (200 mL × 3), and the organic phase was dried over anhydrous sodium sulfate. The organic phase was concentrated under reduced pressure to give a crude product. The crude product was purified by preparative column chromatography to give a solid (1.51 g, yield: 82.5%).

MS (ESI): 550 [M+H]⁺.

### Step 10: Synthesis of compound int_91-11:

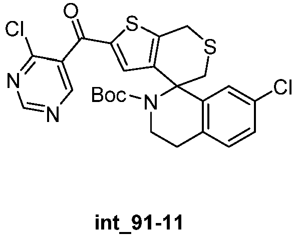

Oxalyl chloride (413.7 mg, 3.26 mmol, 279 µL) was dissolved in dichloromethane (20 mL), and dimethyl sulfoxide (254.7 mg, 3.26 mmol, 231 µL) was slowly and dropwise added to the reaction solution at -78 °C. The reaction solution was allowed to react at -78 °C for 0.5 h. A solution of **int_91-10** (1.5 g, 2.72 mmol) in DCM (10 mL) was dropwise added to the reaction solution, and the reaction solution was allowed to react at -78 °C for another 0.5 h. Triethylamine (1.65 g, 16.32 mmol) was dropwise added to the reaction solution, and the reaction solution was allowed to react at -78 °C for another 0.5 h and then slowly warmed to room temperature. LC-MS monitoring showed the reaction was completed. A saturated aqueous sodium bicarbonate solution was slowly added to the reaction solution to adjust the pH to about 8. The aqueous phase was extracted with ethyl acetate (100 mL × 3), and the organic phase was dried over anhydrous sodium sulfate. The organic phase was concentrated under reduced pressure to give a crude product. The crude product was purified by preparative column chromatography to give a solid (1.38 g, yield: 92.6%).

MS (ESI): 548 [M+H]⁺.

### Step 11: Synthesis of compound int_91-12:

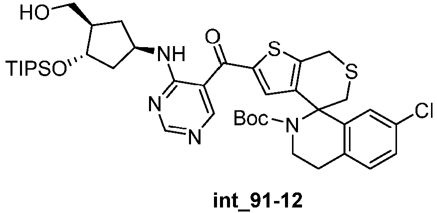

**Int_91-11** (1.097 g, 2.0 mmol) and **int_1-12** (632.5 mg, 2.2 mmol) were dissolved in DMF (10 mL), and K₂CO₃ (829.2 mg, 6.0 mmol) was added to the reaction solution at room temperature. The reaction solution was allowed to react at room temperature for 3 h. LC-MS monitoring showed the reaction was completed. Ice water (100 mL) was added to the reaction solution. The aqueous phase was extracted with ethyl acetate (100 mL × 3), and the organic phase was dried over anhydrous sodium sulfate. The organic phase was concentrated under reduced pressure to give a crude product. The crude product was purified by preparative column chromatography to give a product (1.37 g, yield: 86.1%).

MS (ESI): 799 [M+H]⁺.

### Step 12: Synthesis of compound int_91-13:

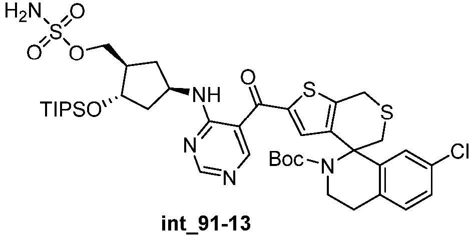

**Int_91-12** (1.37 g, 1.71 mmol) was dissolved in DMF (30 mL), and **int_1-14** (395.9 mg, 3.42 mmol) was added to the reaction solution at room temperature. The reaction solution was allowed to react at room temperature for 1 h. LC-MS monitoring showed the reaction was completed. Ice water (50 mL) was added to the reaction solution. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography to give a solid (1.3 g, yield: 86.6%).

MS (ESI): 878 [M+H]⁺.

### Step 13: Synthesis of compound int_91-14:

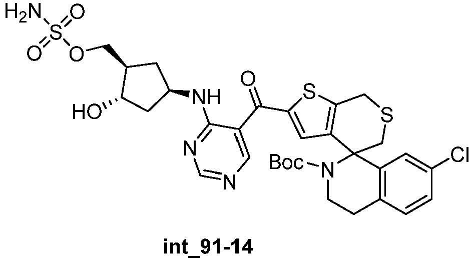

**Int_91-13** (1.58 g, 1.8 mmol) was dissolved in THF (20 mL), and TBAF (1 M, 3.6 mL) was added to the reaction solution at room temperature. The reaction solution was allowed to react at room temperature for 4 h. LC-MS monitoring showed the reaction was completed. Water (50 mL) was added to the reaction solution. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography to give a solid (1.05 g, yield: 80.7%). MS (ESI): 722 [M+H]⁺.

### Step 14: Synthesis of compound 91

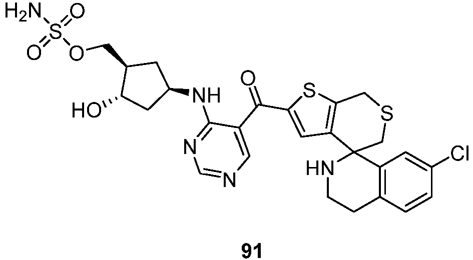

**Int_91-14** (1.05 g, 1.45 mmol) was dissolved in DCM (7 mL) at room temperature, and the mixture was added into TFA (7.8 mL). The reaction solution was allowed to react at room temperature for 5 min. LC-MS monitoring showed the reaction was completed. The reaction solution was cooled to 0 °C. A saturated aqueous sodium bicarbonate solution was added to the reaction solution to adjust the pH to 8. The aqueous phase was extracted with ethyl acetate (50 mL × 3), and the organic phase was dried over anhydrous sodium sulfate. The organic phase was concentrated under reduced pressure to give a crude product. The crude product was purified by preparative HPLC to give a solid (862 mg, yield: 95.3%).

¹H NMR (400 MHz, DMSO-d6) δ 8.54 (s, 1H), 8.38 (d, *J =* 1.2 Hz, 1H), 8.20 (d, *J =* 7.4 Hz, 1H), 7.41 (d, *J* = 2.2 Hz, 2H), 7.21 - 7.09 (m, 2H), 7.00 - 6.92 (m, 2H), 4.88 (dd, *J =* 4.6, 3.0 Hz, 1H), 4.63 (h, *J =* 8.0 Hz, 1H), 4.27 (d, *J =* 16.8 Hz, 1H), 4.09 - 4.03 (m, 1H), 3.97 - 3.81 (m, 3H), 3.31 - 3.16 (m, 3H), 3.01 (m, 2H), 2.89 (m, 1H), 2.67 (d, *J* = 15.9 Hz, 1H), 2.25 (m, 1H), 2.07 (m, 1H), 1.93 - 1.83 (m, 1H), 1.72 (m, 1H), 1.32 - 1.18 (m, 1H) .

MS (ESI): 622 [M+H]⁺ .

### Example 16. Synthesis of Compound 92 and Compound 93

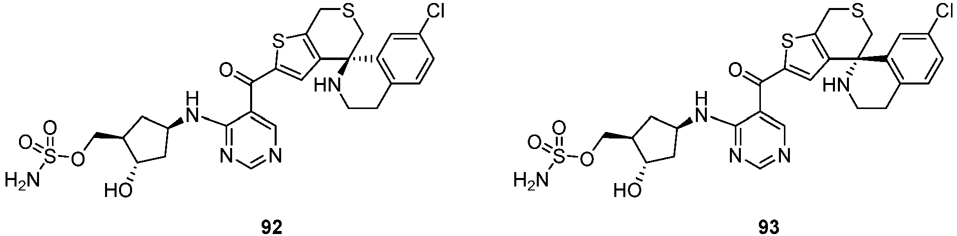

**Compound** 91 (85 mg, 0.136 mmol) was subjected to SFC chiral resolution to give compound 92 (27 mg) and **compound 93** (29 mg).

**Compound 92:** ¹H NMR (400 MHz, DMSO-d6) δ 8.54 (s, 1H), 8.38 (d, *J=* 1.2 Hz, 1H), 8.20 (d, *J =* 7.4 Hz, 1H), 7.41 (d, *J* = 2.2 Hz, 2H), 7.21 - 7.09 (m, 2H), 7.00 - 6.92 (m, 2H), 4.88 (dd, *J =* 4.6, 3.0 Hz, 1H), 4.63 (h, *J=* 8.0 Hz, 1H), 4.27 (d, *J=* 16.8 Hz, 1H), 4.09 - 4.03 (m, 1H), 3.97 - 3.81 (m, 3H), 3.31 - 3.16 (m, 3H), 3.01 (d, *J=* 9.6 Hz, 2H), 2.89 (dt, *J =* 16.0, 7.1 Hz, 1H), 2.67 (d, *J=* 15.9 Hz, 1H), 2.25 (dt, *J =* 15.4, 7.7 Hz, 1H), 2.07 (d, *J=* 7.4 Hz, 1H), 1.93 - 1.83 (m, 1H), 1.72 (dt, *J=* 15.2, 6.6 Hz, 1H), 1.32 - 1.18 (m, 1H).; MS (ESI): 622 [M+H]⁺ .

**Compound 93:** ¹H NMR (400 MHz, DMSO-d6) δ 8.55 (s, 1H), 8.38 (d, *J=* 1.2 Hz, 1H), 8.21 (d, *J=* 7.4 Hz, 1H), 7.42 (d, *J* = 2.2 Hz, 2H), 7.21 - 7.09 (m, 2H), 7.01 - 6.92 (m, 2H), 4.89 (dd, *J* = 4.6, 3.0 Hz, 1H), 4.64 (h, *J=* 8.0 Hz, 1H), 4.28 (d, *J=* 16.8 Hz, 1H), 4.10 - 4.03 (m, 1H), 3.97 - 3.82 (m, 3H), 3.32 - 3.16 (m, 3H), 3.01 (d, *J=* 9.6 Hz, 2H), 2.90 (dt, *J =* 16.0, 7.1 Hz, 1H), 2.67 (d, *J=* 15.9 Hz, 1H), 2.26 (dt, *J =* 15.4, 7.7 Hz, 1H), 2.07 (d, *J=* 7.4 Hz, 1H), 1.94 - 1.83 (m, 1H), 1.73 (dt, *J=* 15.2, 6.6 Hz, 1H), 1.32 - 1.19 (m, 1H).; MS (ESI): 622 [M+H]⁺ .

### Example 17. Synthesis of Compound 112

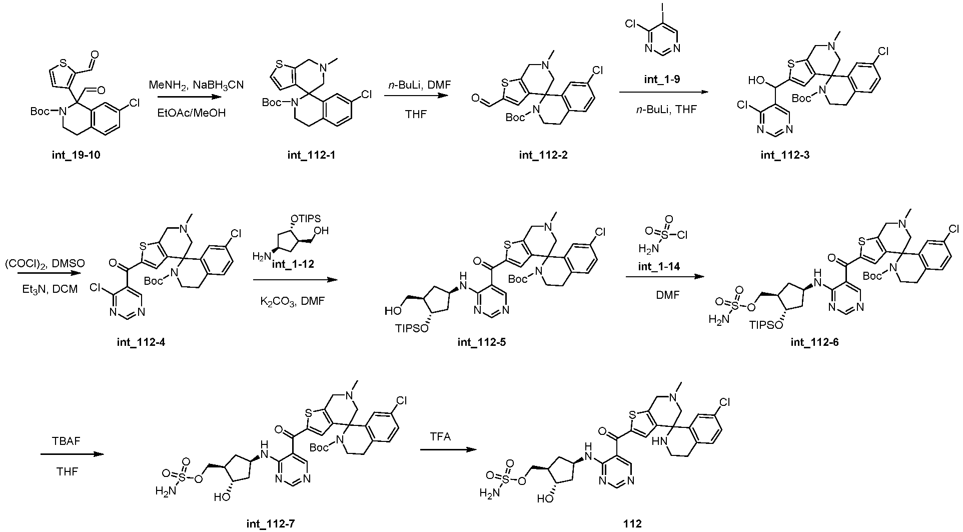

### Step 1: Synthesis of compound int_112-1:

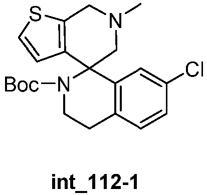

**Int_19-10** (2.5 g, 6.15 mmol) was dissolved in a mixed solvent of ethyl acetate (60 mL) and methanol (15 mL), and methylamine (573 mg, 18.45 mmol, 1.88 mL, 40% in MeOH) and sodium cyanoborohydride (1.53 g, 24.63 mmol) were added to the reaction solution. The reaction solution was allowed to react at room temperature for 16 h. LC-MS monitoring showed the reaction was completed. The reaction solution was poured slowly into 100 mL of ice water. The aqueous phase was extracted with ethyl acetate (100 mL × 3), and the organic phase was dried over anhydrous sodium sulfate. The organic phase was concentrated under reduced pressure to give a crude product. The crude product was purified by preparative column chromatography to give a solid (2.1 g, yield: 84.3%).

ESI-MS m/z: 405 [M+H]⁺.

### Step 2: Synthesis of compound int_112-2:

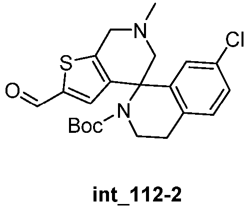

**Int_112-1** (283.4 mg, 0.7 mmol) was dissolved in THF (10 mL), and the mixture was cooled to -70 °C. n-BuLi (2.5 M, 0.83 mL) was slowly and dropwise added to the reaction solution under nitrogen atmosphere, and the reaction solution was allowed to react at -70 °C for 1 h. Then, DMF (153 mg, 2.1 mmol) was dropwise added to the reaction solution at -60 °C, and after the dropwise addition was completed, the reaction solution was allowed to react at 70 °C for 1 h. LC-MS monitoring showed the reaction was completed. A saturated ammonium chloride solution (20 mL) was slowly added to the reaction solution. The aqueous phase was extracted with ethyl acetate (20 mL × 3), and the organic phase was dried over anhydrous sodium sulfate. The organic phase was concentrated under reduced pressure to give a crude product. The crude product was purified by preparative column chromatography to give a solid (169 mg, yield: 55.7%).

MS (ESI): 433 [M+H]⁺.

### Step 3: Synthesis of compound int_112-3:

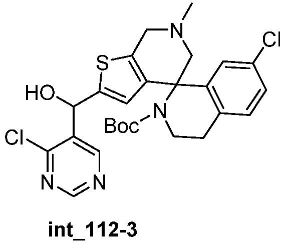

**Int_1-9** (577 mg, 2.4 mmol) was dissolved in THF (10 mL), and the mixture was cooled to -70 °C. n-BuLi (2.5 M, 1.9 mL, 4.8 mmol) was slowly and dropwise added to the reaction solution under nitrogen atmosphere, and the reaction solution was allowed to react at -70 °C for 1 h. Then, a solution of **int_112-2** (173.1 mg, 0.4 mmol) in THF (1 mL) was dropwise added to the reaction solution at -75 °C, and after the dropwise addition was completed, the reaction solution was allowed to react at -75 °C for 1 h, and was then warmed to room temperature and allowed to react for 16 h. LC-MS monitoring showed the reaction was completed. A saturated ammonium chloride solution (20 mL) was slowly added to the reaction solution. The aqueous phase was extracted with ethyl acetate (20 mL × 3), and the organic phase was dried over anhydrous sodium sulfate. The organic phase was concentrated under reduced pressure to give a crude product. The crude product was purified by preparative column chromatography to give a solid (162 mg, yield: 73.9%).

MS (ESI): 547 [M+H]⁺.

### Step 4: Synthesis of compound int_112-4:

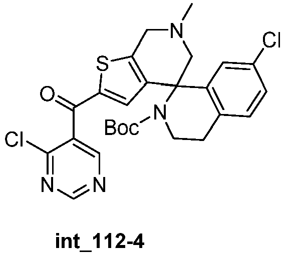

Oxalyl chloride (415 mg, 3.27 mmol, 280 µL) was dissolved in dichloromethane (20 mL), and dimethyl sulfoxide (254.75 mg, 3.27 mmol, 232 µL) was slowly and dropwise added to the reaction solution at -78 °C. The reaction solution was allowed to react at -78 °C for 0.5 h. A solution of **int_112-3** (1.5 g, 2.73 mmol) in DCM (10 mL) was dropwise added to the reaction solution, and the reaction solution was allowed to react at -78 °C for another 0.5 h. Triethylamine (1.65 g, 16.32 mmol) was dropwise added to the reaction solution, and the reaction solution was allowed to react at -78 °C for another 0.5 h and then slowly warmed to room temperature. LC-MS monitoring showed the reaction was completed. A saturated aqueous sodium bicarbonate solution was slowly added to the reaction solution to adjust the pH to about 8. The aqueous phase was extracted with ethyl acetate (100 mL × 3), and the organic phase was dried over anhydrous sodium sulfate. The organic phase was concentrated under reduced pressure to give a crude product. The crude product was purified by preparative column chromatography to give a solid (0.56 g, yield: 37.8%).

MS (ESI): 545 [M+H]⁺.

### Step 5: Synthesis of compound int_112-5:

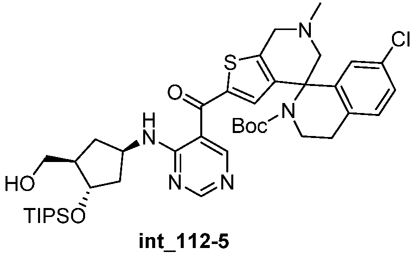

**Int_112-4** (272.7 mg, 0.5 mmol) and **int_1-12** (172.5 mg, 0.6 mmol) were dissolved in DMF (10 mL), and K₂CO₃ (207.3 mg, 1.5 mmol) was added to the reaction solution at room temperature. The reaction solution was allowed to react at room temperature for 16 h. LC-MS monitoring showed the reaction was completed. Ice water (30 mL) was added to the reaction solution. The aqueous phase was extracted with ethyl acetate (30 mL × 3), and the organic phase was dried over anhydrous sodium sulfate. The organic phase was concentrated under reduced pressure to give a crude product. The crude product was purified by preparative column chromatography to give a product (237 mg, yield: 59.5%).

MS (ESI): 796 [M+H]⁺.

### Step 6: Synthesis of compound int_112-6:

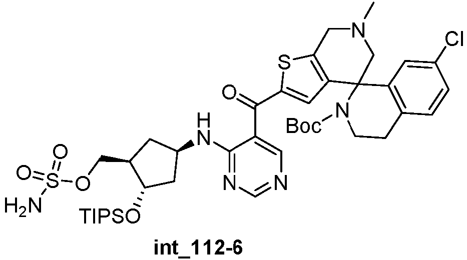

**Int_112-5** (280 mg, 0.35 mmol) was dissolved in DMF (4 mL), and **int_1-14** (80.8 mg, 0.7 mmol) was added to the reaction solution at room temperature. The reaction solution was allowed to react at room temperature for 1 h. LC-MS monitoring showed the reaction was completed. Ice water (30 mL) was added to the reaction solution. The aqueous phase was extracted with ethyl acetate (30 mL × 3), and the organic phase was dried over anhydrous sodium sulfate. The organic phase was concentrated under reduced pressure to give a crude product. The crude product was purified by preparative column chromatography to give a solid (255 mg, yield: 83.3%).

MS (ESI): 875 [M+H]⁺.

### Step 7: Synthesis of compound int_112-7:

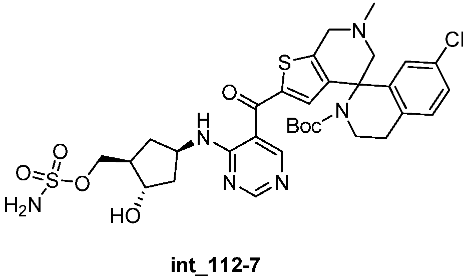

**Int_112-6** (255 mg, 0.29 mmol) was dissolved in THF (4 mL), and TBAF (1 M, 0.58 mL, 0.58 mmol) was added to the reaction solution at room temperature. The reaction solution was allowed to react at room temperature for 16 h. LC-MS monitoring showed the reaction was completed. Water (30 mL) was added to the reaction solution. The aqueous phase was extracted with ethyl acetate (30 mL × 3), and the organic phase was dried over anhydrous sodium sulfate. The organic phase was concentrated under reduced pressure to give a crude product. The crude product was purified by preparative column chromatography to give a solid (170 mg, yield: 81.3%).

MS (ESI): 719 [M+H]⁺.

### Step 8: Synthesis of compound 112:

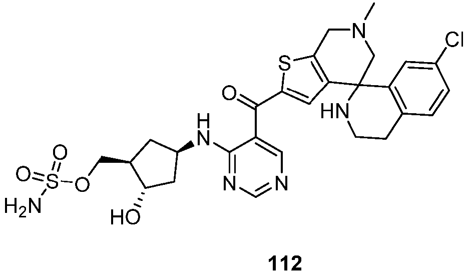

**Int_112-7** (170 mg, 0.236 mmol) was dissolved in dichloromethane (6 mL), and TFA (1.5 mL) was added to the reaction solution at room temperature. The reaction solution was allowed to react at room temperature for 10 min. LC-MS monitoring showed the reaction was completed. Ammonia water (1 mL) and water (10 mL) were added to the reaction solution. The aqueous phase was extracted with dichloromethane (10 mL × 3), and the organic phase was dried over anhydrous sodium sulfate. The organic phase was concentrated under reduced pressure to give a crude product. The crude product was purified by preparative HPLC to give a solid (87 mg, yield: 59.5%).

MS (ESI): 619 [M+H]⁺.

### Example 18. Synthesis of Compound 113 and Compound 114

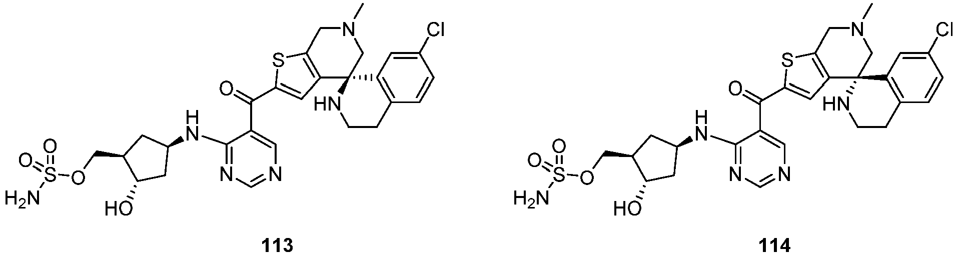

**Compound 112** (80 mg, 0.129 mmol) was subjected to SFC chiral resolution (column: Phenomenex-Cellulose-2 (250 mm × 30 mm, 10 µm)); mobile phase: [CO₂-MeOH (0.1% NH₃H₂O)]; B%: 45%, isocratic elution mode) to give **compound 113** (21 mg) and **compound 114** (25 mg).

**Compound 113:** ¹H NMR (400 MHz, DMSO-d6) δ 8.57 (s, 1H), 8.45 (s, 1H), 8.20 (d, *J =* 7.5 Hz, 1H), 7.44 (s, 2H), 7.20 (d, *J =* 3.4 Hz, 2H), 7.08 (s, 1H), 6.92 (d, *J* = 2.0 Hz, 1H), 4.90 (s, 1H), 4.66 (q, *J =* 8.2 Hz, 1H), 4.08 (dd, *J =* 9.7, 6.0 Hz, 1H), 4.04 - 3.81 (m, 3H), 3.72 (d, *J =* 16.2 Hz, 1H), 3.17 - 2.87 (m, 4H), 2.74 (d, *J* = 12.2 Hz, 2H), 2.43 (s, 3H), 2.27 (dt, *J =* 12.7, 7.8 Hz, 1H), 2.09 (d, *J* = 7.6 Hz, 1H), 1.94 (ddd, *J =* 11.9, 7.4, 3.7 Hz, 1H), 1.83 - 1.67 (m, 1H), 1.33 - 1.16 (m, 1H).; MS (ESI): 619 [M+H]⁺.

SFC retention time analysis: 4.624 min (Instrument: Waters UPCC with PDA Detector and QDa Detector; Column: Cellulose-2 100×4.6mm, I.D., 3um; Mobile phase: A: CO₂, B: Methanol (0.05% DEA); Isocratic: 50% B; Flow rate: 2.8mL/min; Column temp.: 35°C; ABPR: 1500psi) .

**Compound 114:** ¹H NMR (400 MHz, DMSO-d6) δ 8.57 (s, 1H), 8.44 (s, 1H), 8.18 (d, *J =* 7.5 Hz, 1H), 7.45 (s, 2H), 7.18 (d, *J* = 2.8 Hz, 2H), 7.08 (s, 1H), 6.91 (s, 1H), 4.89 (d, *J* = 4.3 Hz, 1H), 4.66 (q, *J* = 8.0 Hz, 1H), 4.08 (dd, *J* = 9.7, 5.9 Hz, 1H), 3.95 (dd, *J* = 9.9, 6.9 Hz, 2H), 3.84 (d, *J =* 16.1 Hz, 1H), 3.72 (d, *J =* 16.1 Hz, 1H), 2.96 (dd, *J* = 36.7, 25.1 Hz, 4H), 2.71 (dd, *J* = 13.5, 7.5 Hz, 2H), 2.41 (s, 3H), 2.29 (dt, *J* = 14.2, 7.7 Hz, 1H), 2.10 (s, 1H), 1.97 - 1.84 (m, 1H), 1.72 (dt, *J* = 13.3, 7.4 Hz, 1H), 1.26 (dd, *J* = 15.7, 6.7 Hz, 1H).; MS (ESI): 619 [M+H]⁺.

SFC retention time analysis: 3.796 min (Instrument: Waters UPCC with PDA Detector and QDa Detector; Column: Cellulose-2 100×4.6mm, I.D., 3um; Mobile phase: A: CO₂, B: Methanol (0.05% DEA); Isocratic: 50% B; Flow rate: 2.8mL/min; Column temp.: 35°C; ABPR: 1500psi) .

### Example 19. Synthesis of Compound 172

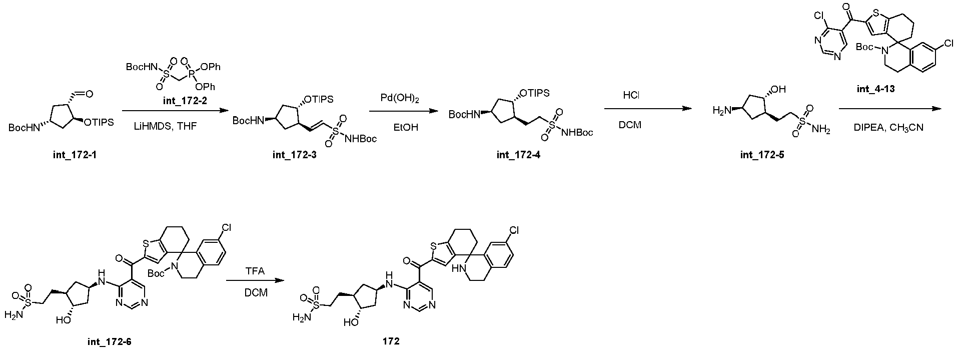

### Step 1: Synthesis of compound int_172-3:

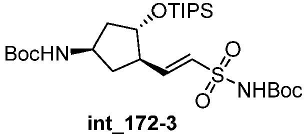

**Int_172-2** (2.22 g, 5.19 mmol) was dissolved in anhydrous THF (20 mL), and the mixture was purged with nitrogen three times and then cooled to -10 °C. LiHMDS (10.4 mL, 1 M) was dropwise added to the reaction solution, and after the dropwise addition was completed, the reaction solution was allowed to react at room temperature for 1 h. The reaction solution was cooled to -10 °C, and a solution of **int_172-1** (1 g, 2.59 mmol) in anhydrous THF (10 mL) was dropwise added to the reaction solution. The reaction solution was warmed slowly to room temperature and allowed to react for 1 h. LC-MS monitoring showed the reaction was completed. The reaction solution was cooled to room temperature and then poured slowly into 50 mL of saturated aqueous ammonium chloride solution. The aqueous phase was extracted with ethyl acetate (50 mL × 3), and the organic phase was dried over anhydrous sodium sulfate. The organic phase was concentrated under reduced pressure to give a crude product. The crude product was purified by preparative column chromatography (SiO₂, **PE/EtOAc** = 3/1) to give a solid (298 mg, yield: 20%).

ESI-MS m/z: 563 [M+H]⁺.

### Step 2: Synthesis of compound int_172-4:

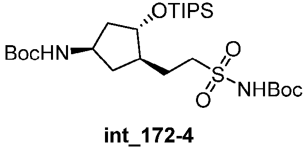

**Int_172-3** (298 mg, 0.529 mmol) and Pd(OH)₂ (30 mg) were dissolved in ethanol (30 mL), and the reaction solution was purged with hydrogen three times, and allowed to react at room temperature for 16 h under hydrogen atmosphere. LC-MS monitoring showed the reaction was completed. The reaction solution was filtered to give a filtrate, and the filtrate was concentrated under reduced pressure to give a crude product. The crude product was purified by preparative column chromatography to give a solid (290 mg, yield: 97.3%).

ESI-MS m/z: 565 [M+H]⁺.

### Step 3: Synthesis of compound int_172-5:

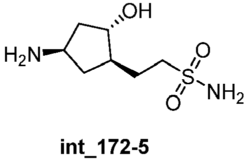

**Int_172-4** (310 mg, 0.529 mmol) was dissolved in dichloromethane (10 mL), and HCl/Diox (4 mL, 15.87 mmol) was added. The reaction solution was stirred at room temperature for 2 h. LC-MS monitoring showed the reaction was completed. The reaction solution was cooled to room temperature and concentrated under reduced pressure to give a crude product, which was directly used in the next step.

ESI-MS m/z: 209 [M+H]⁺.

### Step 4: Synthesis of compound int_172-6:

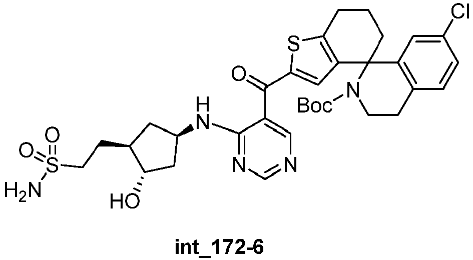

**Int_172-5** (110 mg, 0.529 mmol), DIPEA (136 mg, 1.056 mmol), and **int_4-13** (100 mg, 0.176 mmol) were dissolved in acetonitrile (10 mL), and the reaction solution was warmed to 60 °C and allowed to react for 4 h. LC-MS monitoring showed the reaction was completed. The reaction solution was concentrated under reduced pressure to give a crude product, and the crude product was purified by preparative column chromatography to give a product (59 mg, yield: 44%).

MS (ESI): 702 [M+H]⁺.

### Step 5: Synthesis of compound 172

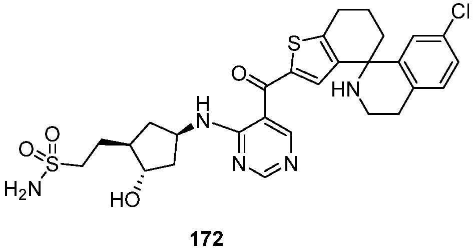

**Int_172-6** (59 mg, 0.084 mmol) was dissolved in DCM (1 mL) at room temperature, and the mixture was added into TFA (0.35 mL). The reaction solution was allowed to react at room temperature for 30 min. LC-MS monitoring showed the reaction was completed. The reaction solution was cooled to 0 °C and adjusted to pH=8 by adding triethylamine. The organic phase was concentrated under reduced pressure to give a crude product. The crude product was purified by preparative HPLC to give a solid (26 mg, yield: 51%).

¹H NMR (400 MHz, DMSO-d6) δ 8.53 (s, 1H), 8.48 (d, *J =* 1.6 Hz, 1H), 8.21 (d, *J =* 7.5 Hz, 1H), 7.12 (d, *J =* 2.0 Hz, 2H), 6.91 (s, 1H), 6.74 (s, 1H), 6.70 (s, 2H), 4.78 (t, *J* = 4.6 Hz, 1H), 4.55 (h, *J* = 7.8 Hz, 1H), 3.75 (p, *J* = 6.*3* Hz, 1H), 3.06 - 2.87 (m, 6H), 2.87 - 2.79 (m, 1H), 2.63 (dt*, J =* 16.2, 3.3 Hz, 1H), 2.25 (dt, *J* = 12.9*,* 7.3 Hz, 1H), 2.20 - 2.12 (m, 1H), 2.11 - 1.99 (m, 1H), 1.95 - 1.58 (m, 7H), 1.27 - 1.19 (m, 1H), 1.10 (dtd, *J* = 14.1, 9.5, 4.8 Hz, 1H).

MS (ESI): 602 [M+H]⁺ .

### Example 20. Synthesis of Compound 173 and Compound 174

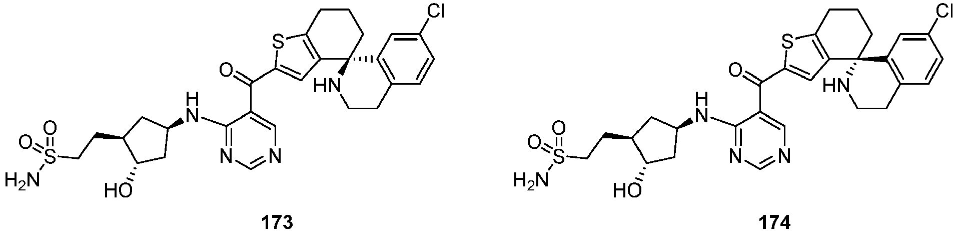

**Compound 172** (100 mg, 0.166 mmol) was subjected to SFC chiral resolution to give **compound 173** (47 mg) and **compound 174** (43 mg).

**Compound 173:** ¹H NMR (400 MHz, DMSO-d6) δ 8.53 (s, 1H), 8.48 (d, *J =* 1.6 Hz, 1H), 8.21 (d, *J =* 7.5 Hz, 1H), 7.12 (d, *J =* 2.0 Hz, 2H), 6.91 (s, 1H), 6.74 (s, 1H), 6.70 (s, 2H), 4.78 (t, *J =* 4.6 Hz, 1H), 4.55 (h, *J =* 7.8 Hz, 1H), 3.75 (p, *J =* 6.3 Hz, 1H), 3.06 - 2.87 (m, 6H), 2.87 - 2.79 (m, 1H), 2.63 (dt, *J =* 16.2, 3.3 Hz, 1H), 2.25 (dt, *J =* 12.9, 7.3 Hz, 1H), 2.20 - 2.12 (m, 1H), 2.11 - 1.99 (m, 1H), 1.95 - 1.58 (m, 7H), 1.27 - 1.19 (m, 1H), 1.10 (dtd, *J* = 14.1, 9.5, 4.8 Hz, 1H).; MS (ESI): 602 [M+H]⁺ .

**Compound 174:** ¹H NMR (400 MHz, DMSO-d6) δ 8.53 (s, 1H), 8.48 (d, *J =* 1.6 Hz, 1H), 8.21 (d, *J =* 7.5 Hz, 1H), 7.12 (d, *J* = 2.0 Hz, 2H), 6.91 (s, 1H), 6.74 (s, 1H), 6.70 (s, 2H), 4.78 (t, *J* = 4.6 Hz, 1H), 4.55 (h, *J* = 7.8 Hz, 1H), 3.75 (p, *J* = 6.3 Hz, 1H), 3.06 - 2.87 (m, 6H), 2.87 - 2.79 (m, 1H), 2.63 (dt, *J* = 16.2, 3.3 Hz, 1H), 2.25 (dt, *J =* 12.9, 7.3 Hz, 1H), 2.20 - 2.12 (m, 1H), 2.11 - 1.99 (m, 1H), 1.95 - 1.58 (m, 7H), 1.27 - 1.19 (m, 1H), 1.10 (dtd, *J* = 14.1, 9.5, 4.8 Hz, 1H).; MS (ESI): 602 [M+H]⁺ .

The target **compounds 10-18, 22-45, 52-77, 79-90, 94-111, 115-171, and 177-300** in Table 1 were obtained using the synthetic methods described above with different starting materials.

**Table 1**

| **Compoun d** | **Compound structure** | **MS (M+H) +** | **Compoun d** | **Compound structure** | **MS (M+H) +** |
|---|---|---|---|---|---|
| **4** | 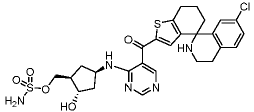 | 604 | **5** | 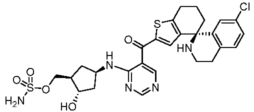 | 604 |
| **6** | 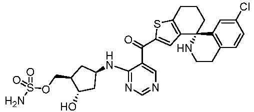 | 604 | **7** | 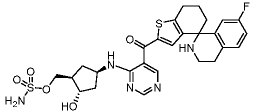 | 588 |
| **8** | 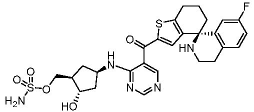 | 588 | **9** | 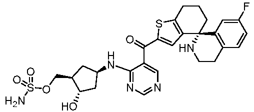 | 588 |
| **10** | 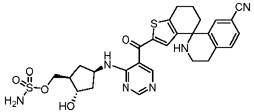 | 595 | **11** | 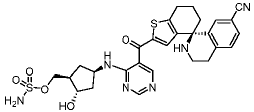 | 595 |
| **12** | 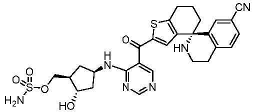 | 595 | **13** | 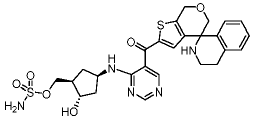 | 572 |
| **14** | 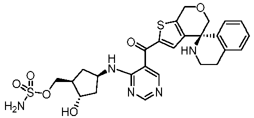 | 572 | **15** | 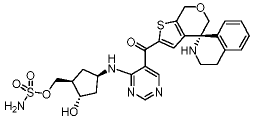 | 572 |
| **16** | 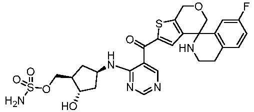 | 590 | **17** | 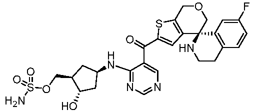 | 590 |
| **18** | 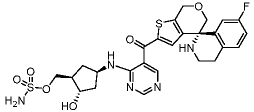 | 590 | **19** | 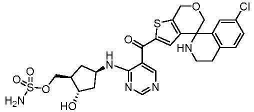 | 606 |
| **20** | 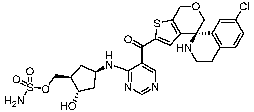 | 606 | **21** | 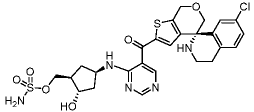 | 606 |
| **22** | 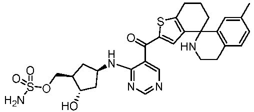 | 584 | **23** | 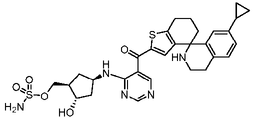 | 610 |
| **24** | 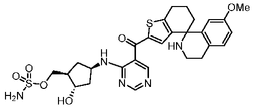 | 600 | **25** | 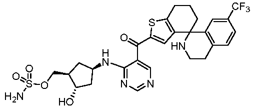 | 638 |
| **26** | 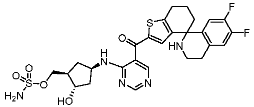 | 606 | **27** | 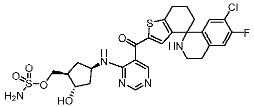 | 622 |
| **28** | 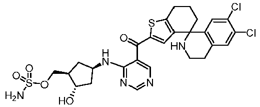 | 638 | **29** | 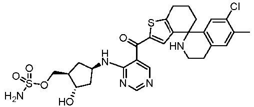 | 618 |
| **30** | 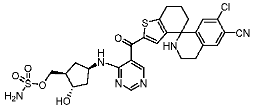 | 629 | **31** | 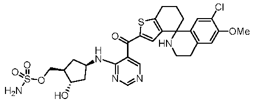 | 634 |
| **32** | 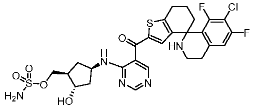 | 640 | **33** | 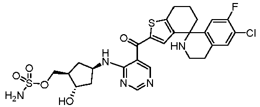 | 622 |
| **34** | 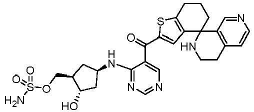 | 571 | **35** | 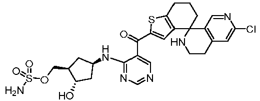 | 605 |
| **36** | 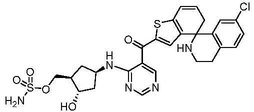 | 602 | **37** | 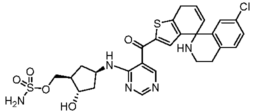 | 602 |
| **38** | 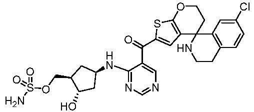 | 606 | **39** | 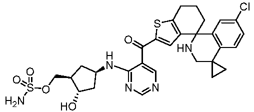 | 630 |
| **40** | 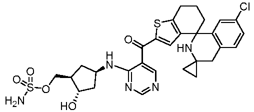 | 630 | **41** | 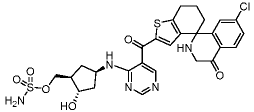 | 618 |
| **42** | 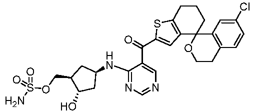 | 605 | **43** | 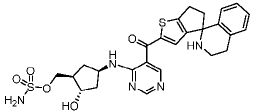 | 556 |
| **44** | 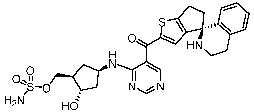 | 556 | **45** | 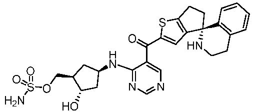 | 556 |
| **46** | 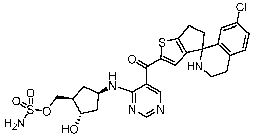 | 590 | **47** | 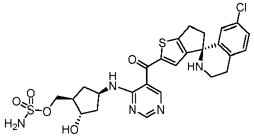 | 590 |
| **48** | 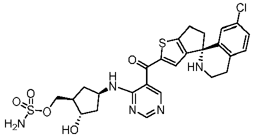 | 590 | **49** | 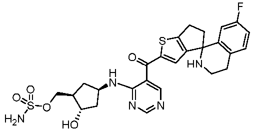 | 574 |
| **50** | 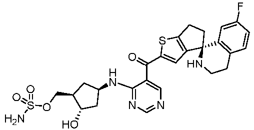 | 574 | **51** | 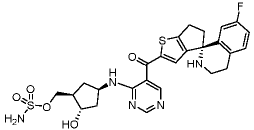 | 574 |
| **52** | 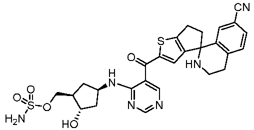 | 581 | **53** | 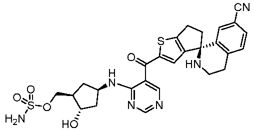 | 581 |
| **54** | 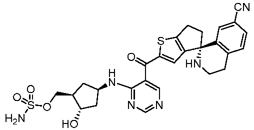 | 581 | **55** | 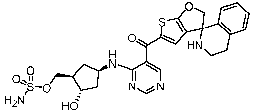 | 558 |
| **56** | 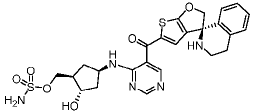 | 558 | **57** | 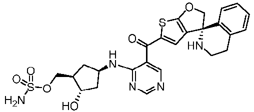 | 558 |
| **58** | 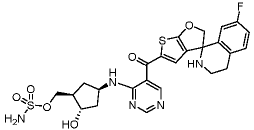 | 576 | **59** | 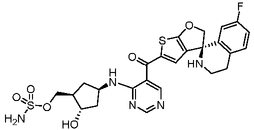 | 576 |
| **60** | 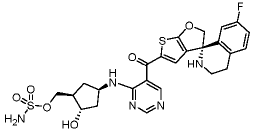 | 576 | **61** | 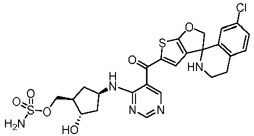 | 592 |
| **62** | 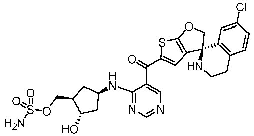 | 592 | **63** | 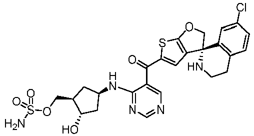 | 592 |
| **64** | 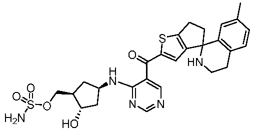 | 570 | **65** | 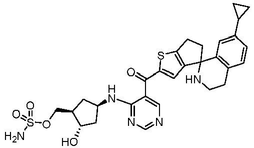 | 596 |
| **66** | 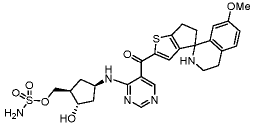 | 586 | **67** | 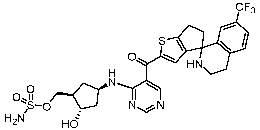 | 624 |
| **68** | 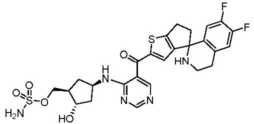 | 592 | **69** | 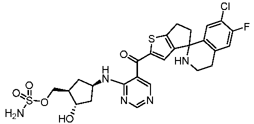 | 608 |
| **70** | 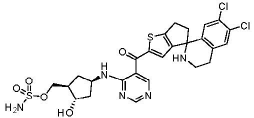 | 624 | **71** | 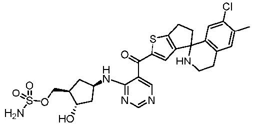 | 604 |
| **72** | 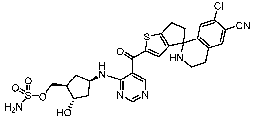 | 615 | **73** | 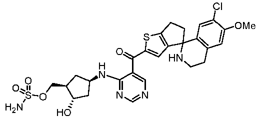 | 620 |
| **74** | 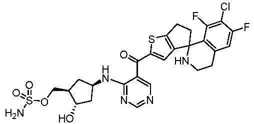 | 626 | **75** | 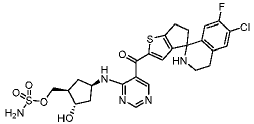 | 608 |
| **76** | 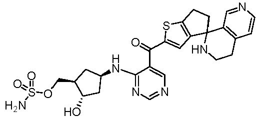 | 557 | **77** | 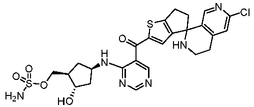 | 591 |
| **78** | 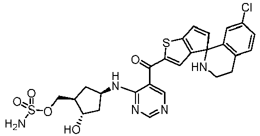 | 588 | **79** | 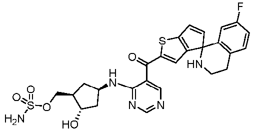 | 572 |
| **80** | 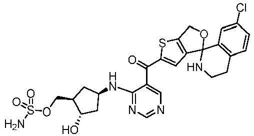 | 592 | **81** | 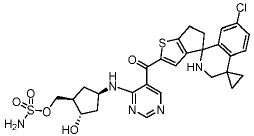 | 616 |
| **82** | 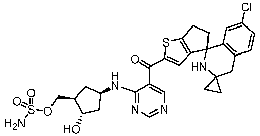 | 616 | **83** | 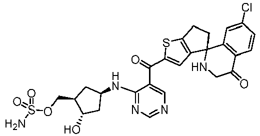 | 604 |
| **84** | 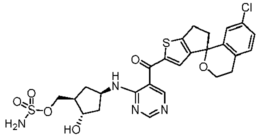 | 591 | **85** | 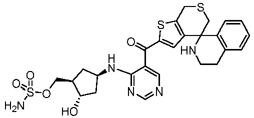 | 588 |
| **86** | 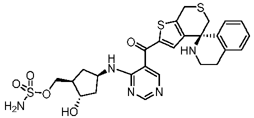 | 588 | **87** | 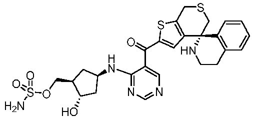 | 588 |
| **88** | 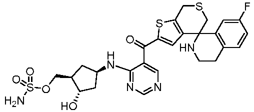 | 606 | **89** | 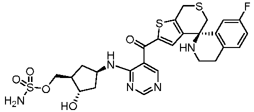 | 606 |
| **90** | 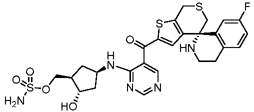 | 606 | **91** | 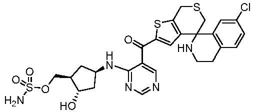 | 622 |
| **92** | 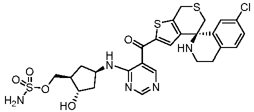 | 622 | **93** | 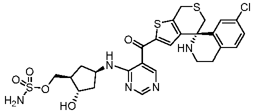 | 622 |
| **94** | 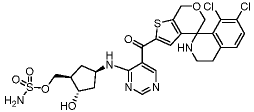 | 640 | **95** | 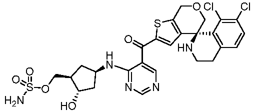 | 640 |
| **96** | 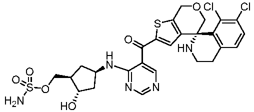 | 640 | **97** | 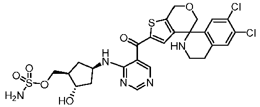 | 640 |
| **98** | 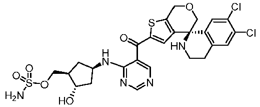 | 640 | **99** | 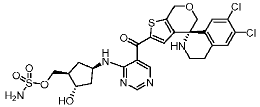 | 640 |
| **100** | 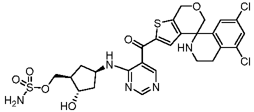 | 640 | **101** | 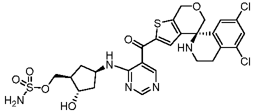 | 640 |
| **102** | 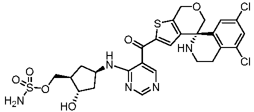 | 640 | **103** | 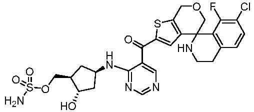 | 624 |
| **104** | 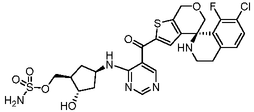 | 624 | **105** | 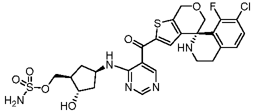 | 624 |
| **106** | 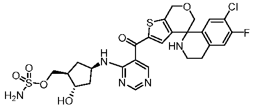 | 624 | **107** | 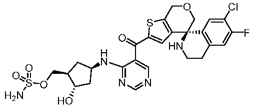 | 624 |
| **108** | 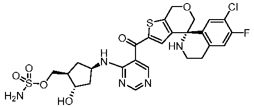 | 624 | **109** | 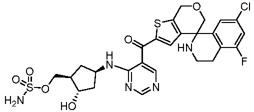 | 624 |
| **110** | 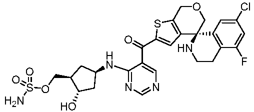 | 624 | **111** | 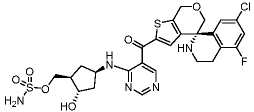 | 624 |
| **112** | 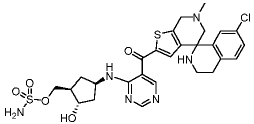 | 619 | **113** | 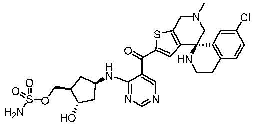 | 619 |
| **114** | 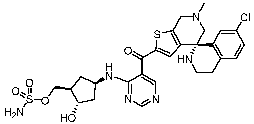 | 619 | **115** | 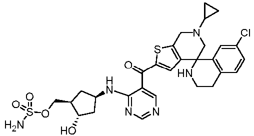 | 645 |
| **116** | 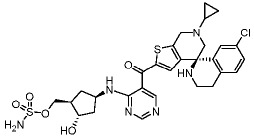 | 645 | **117** | 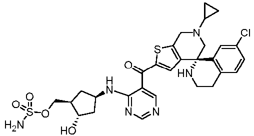 | 645 |
| **118** | 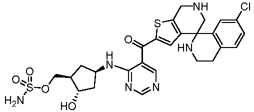 | 605 | **119** | 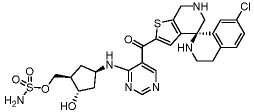 | 605 |
| **120** | 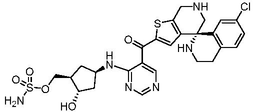 | 605 | **121** | 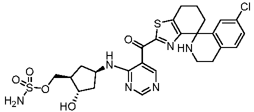 | 605 |
| **122** | 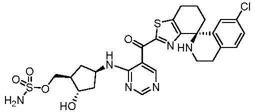 | 605 | **123** | 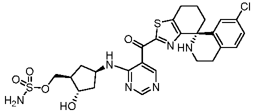 | 605 |
| **124** | 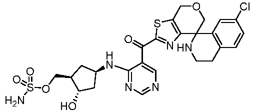 | 607 | **125** | 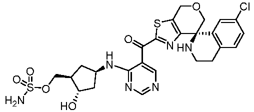 | 607 |
| **126** | 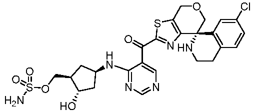 | 607 | **127** | 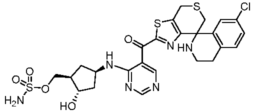 | 623 |
| **128** | 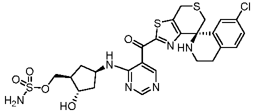 | 623 | **129** | 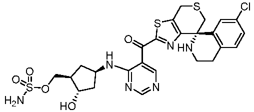 | 623 |
| **130** | 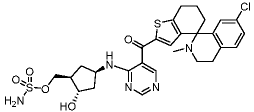 | 618 | **131** | 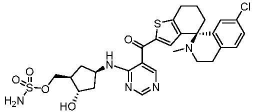 | 618 |
| **132** | 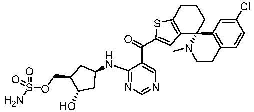 | 618 | **133** | 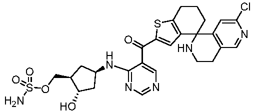 | 605 |
| **134** | 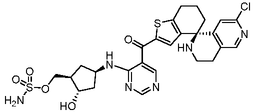 | 605 | **135** | 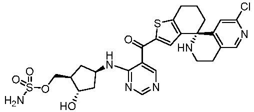 | 605 |
| **136** | 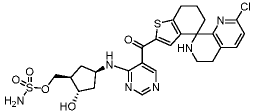 | 605 | **137** | 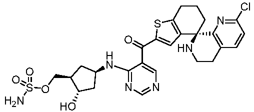 | 605 |
| **138** | 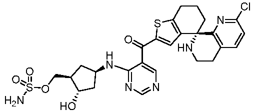 | 605 | **139** | 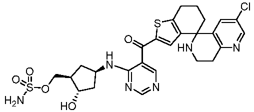 | 605 |
| **140** | 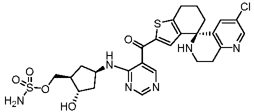 | 605 | **141** | 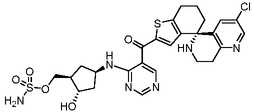 | 605 |
| **142** | 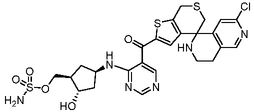 | 623 | **143** | 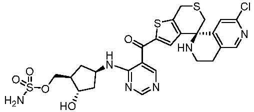 | 623 |
| **144** | 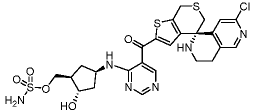 | 623 | **145** | 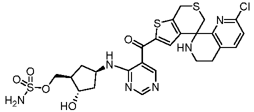 | 623 |
| **146** | 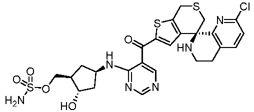 | 623 | **147** | 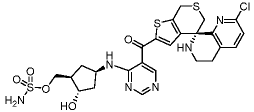 | 623 |
| **148** | 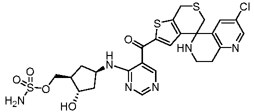 | 623 | **149** | 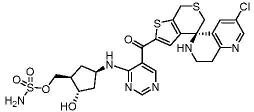 | 623 |
| **150** | 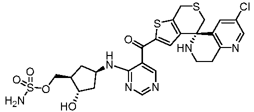 | 623 | **151** | 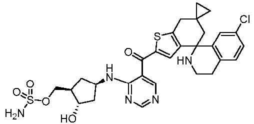 | 630 |
| **152** | 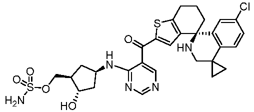 | 630 | **153** | 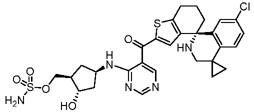 | 630 |
| **154** | 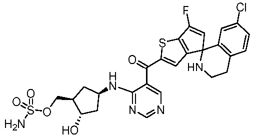 | 606 | **155** | 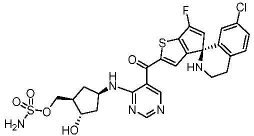 | 606 |
| **156** | 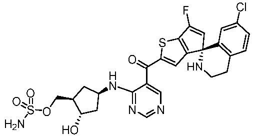 | 606 | **157** | 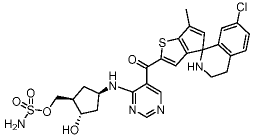 | 602 |
| **158** | 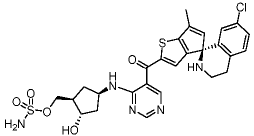 | 602 | **159** | 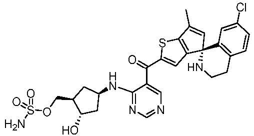 | 602 |
| **160** | 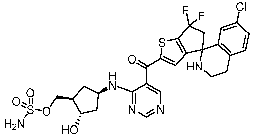 | 626 | **161** | 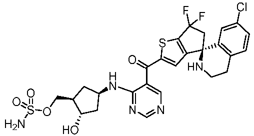 | 626 |
| **162** | 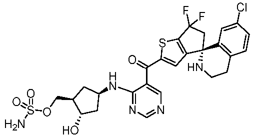 | 626 | **163** | 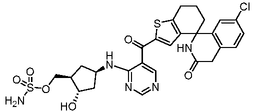 | 618 |
| **164** | 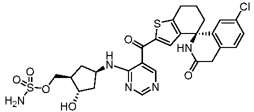 | 618 | **165** | 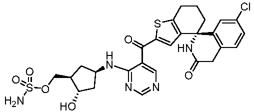 | 618 |
| | | | | | |
| **166** | 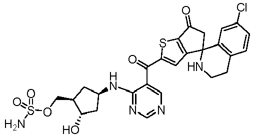 | 604 | **167** | 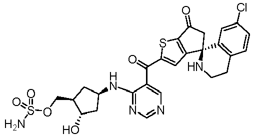 | 604 |
| **168** | 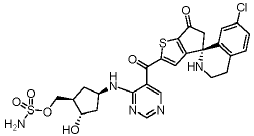 | 604 | **169** | 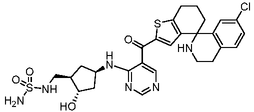 | 603 |
| **170** | 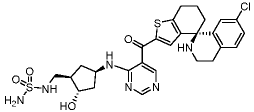 | 603 | **171** | 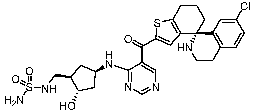 | 603 |
| **172** | 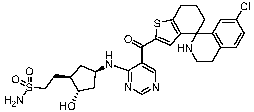 | 602 | **173** | 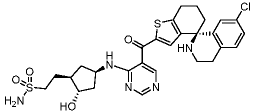 | 602 |
| **174** | 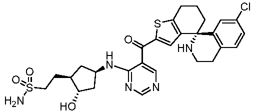 | 602 | **175** | 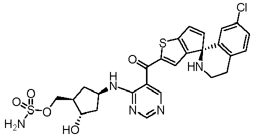 | 588 |
| **176** | 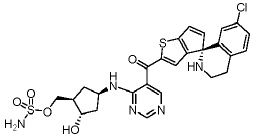 | 588 | **177** | 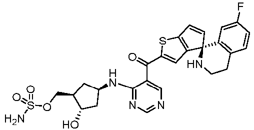 | 572 |
| **178** | 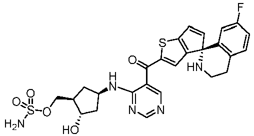 | 572 | **179** | 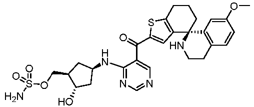 | 600 |
| **180** | 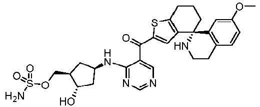 | 600 | **181** | 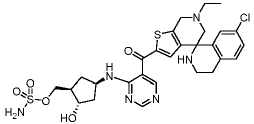 | 633 |
| **182** | 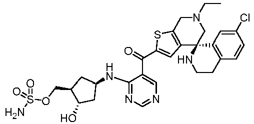 | 633 | **183** | 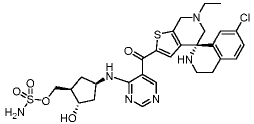 | 633 |
| **184** | 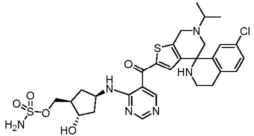 | 647 | **185** | 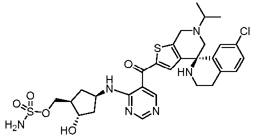 | 647 |
| **186** | 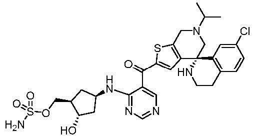 | 647 | **187** | 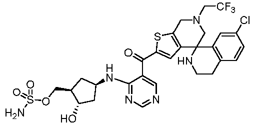 | 687 |
| **188** | 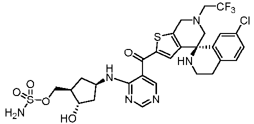 | 687 | **189** | 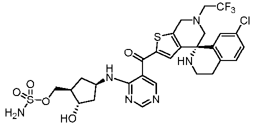 | 687 |
| **190** | 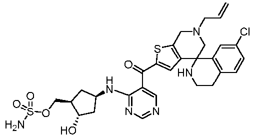 | 645 | **191** | 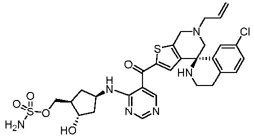 | 645 |
| **192** | 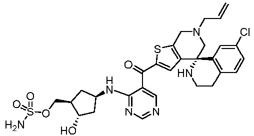 | 645 | **193** | 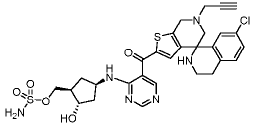 | 643 |
| **194** | 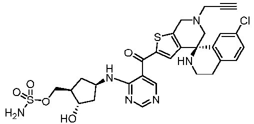 | 643 | **195** | 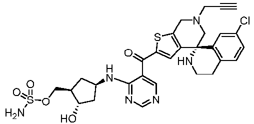 | 643 |
| **196** | 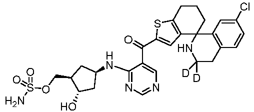 | 606 | **197** | 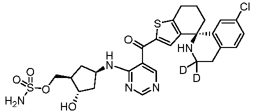 | 606 |
| **198** | 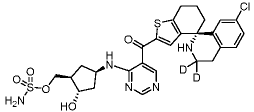 | 606 | **199** | 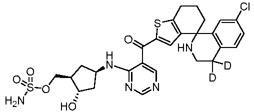 | 606 |
| **200** | 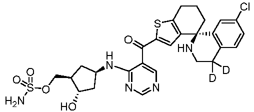 | 606 | **201** | 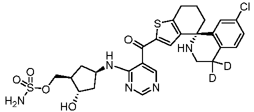 | 606 |
| **202** | 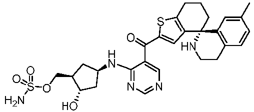 | 584 | **203** | 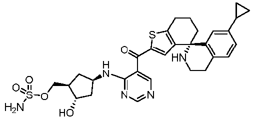 | 610 |
| **204** | 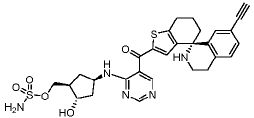 | 594 | **205** | 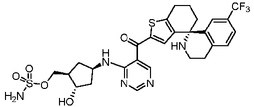 | 638 |
| **206** | 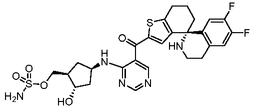 | 606 | **207** | 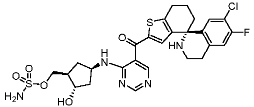 | 622 |
| **208** | 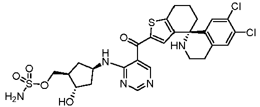 | 638 | **209** | 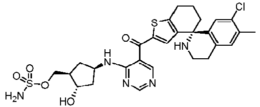 | 618 |
| **210** | 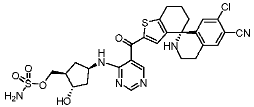 | 629 | **211** | 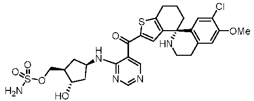 | 634 |
| **212** | 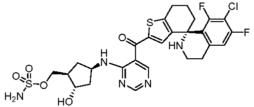 | 640 | **213** | 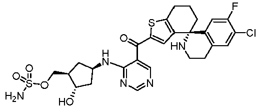 | 622 |
| **214** | 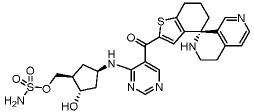 | 571 | **215** | 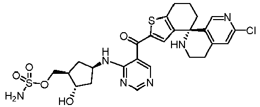 | 605 |
| **216** | 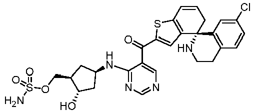 | 602 | **217** | 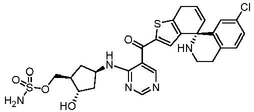 | 602 |
| **218** | 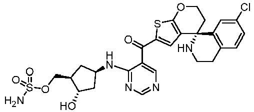 | 606 | **219** | 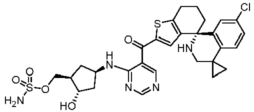 | 630 |
| **220** | 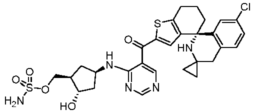 | 630 | **221** | 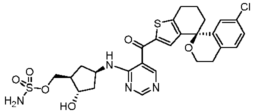 | 605 |
| **222** | 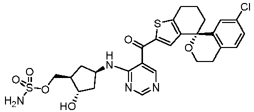 | 605 | **223** | 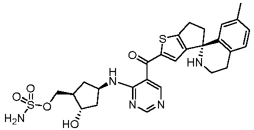 | 570 |
| **224** | 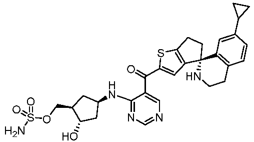 | 596 | **225** | 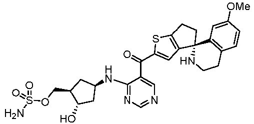 | 586 |
| **226** | 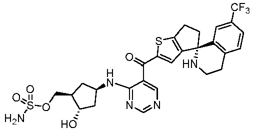 | 624 | **227** | 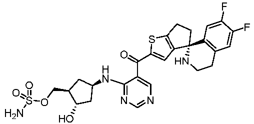 | 592 |
| **228** | 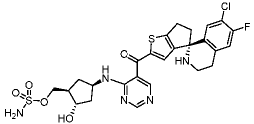 | 608 | **229** | 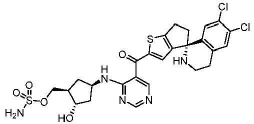 | 624 |
| **230** | 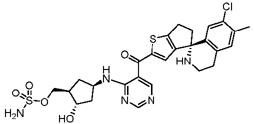 | 604 | **231** | 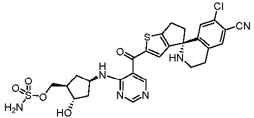 | 615 |
| **232** | 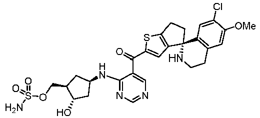 | 620 | **233** | 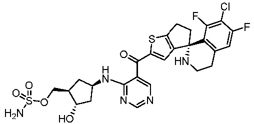 | 626 |
| | | | | | |
| **234** | 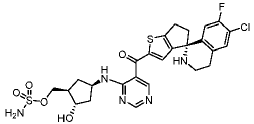 | 608 | **235** | 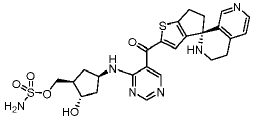 | 557 |
| **236** | 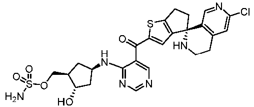 | 591 | **237** | 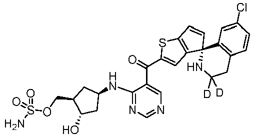 | 590 |
| **238** | 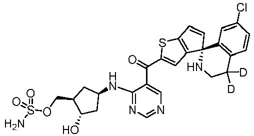 | 590 | **239** | 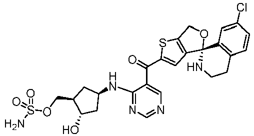 | 592 |
| **240** | 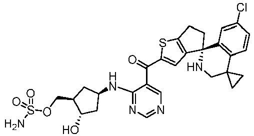 | 616 | **241** | 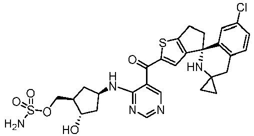 | 616 |
| **242** | 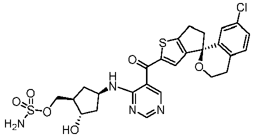 | 591 | **243** | 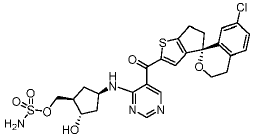 | 591 |
| **244** | 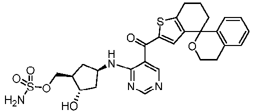 | 571 | **245** | 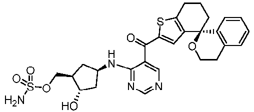 | 571 |
| **246** | 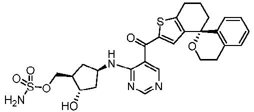 | 571 | **247** | 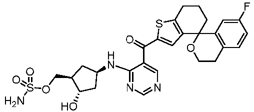 | 589 |
| **248** | 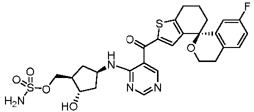 | 589 | **249** | 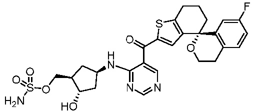 | 589 |
| **250** | 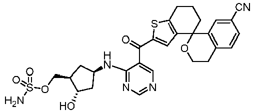 | 596 | **251** | 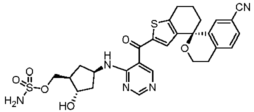 | 596 |
| **252** | 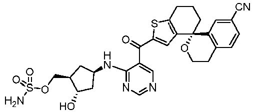 | 596 | **253** | 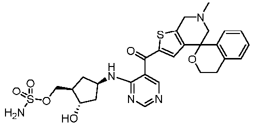 | 586 |
| **254** | 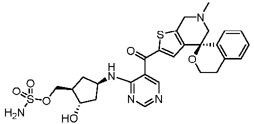 | 586 | **255** | 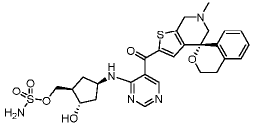 | 586 |
| **256** | 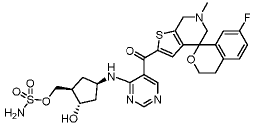 | 604 | **257** | 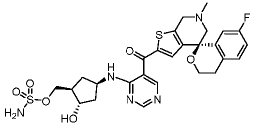 | 604 |
| **258** | 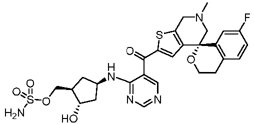 | 604 | **259** | 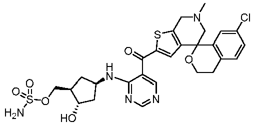 | 620 |
| **260** | 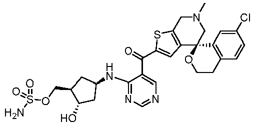 | 620 | **261** | 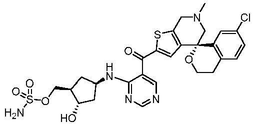 | 620 |
| **262** | 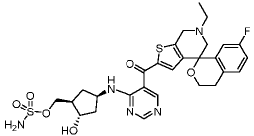 | 618 | **263** | 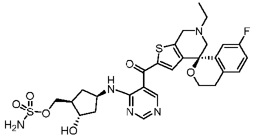 | 618 |
| **264** | 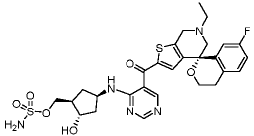 | 618 | **265** | 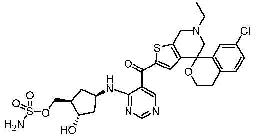 | 634 |
| | | | | | |
| **266** | 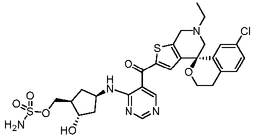 | 634 | **267** | 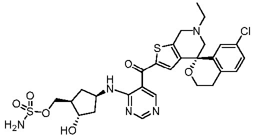 | 634 |
| **268** | 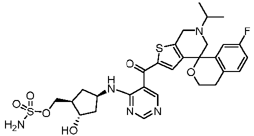 | 632 | **269** | 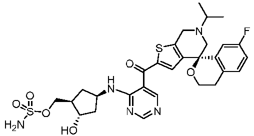 | 632 |
| **270** | 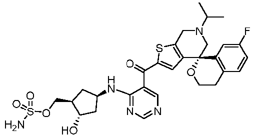 | 632 | **271** | 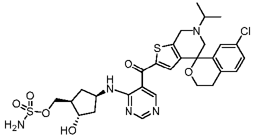 | 648 |
| **272** | 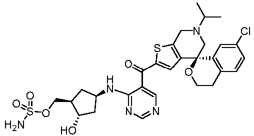 | 648 | **273** | 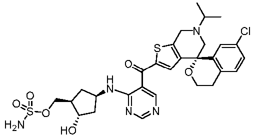 | 648 |
| **274** | 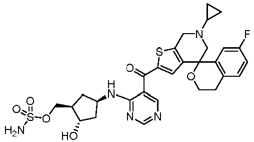 | 630 | **275** | 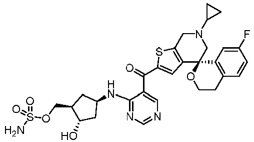 | 630 |
| **276** | 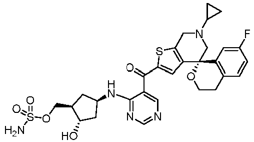 | 630 | **277** | 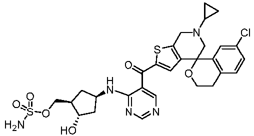 | 646 |
| **278** | 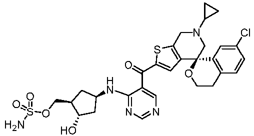 | 646 | **279** | 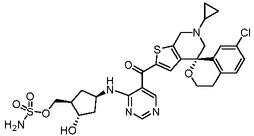 | 646 |
| **280** | 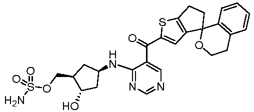 | 557 | **281** | 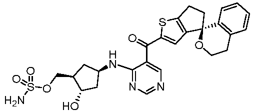 | 557 |
| **282** | 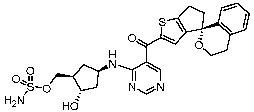 | 557 | **283** | 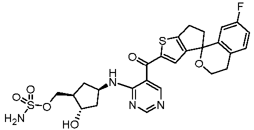 | 575 |
| **284** | 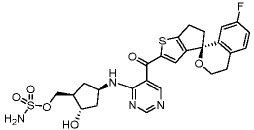 | 575 | **285** | 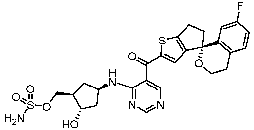 | 575 |
| **286** | 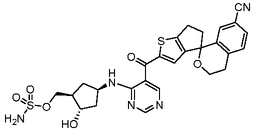 | 582 | **287** | 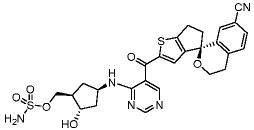 | 582 |
| **288** | 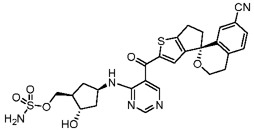 | 582 | **289** | 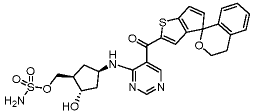 | 555 |
| **290** | 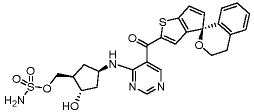 | 555 | **291** | 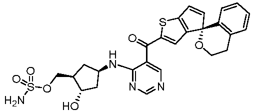 | 555 |
| **292** | 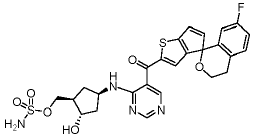 | 573 | **293** | 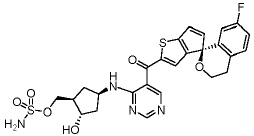 | 573 |
| **294** | 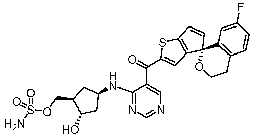 | 573 | **295** | 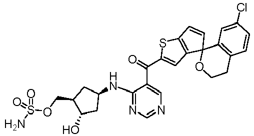 | 589 |
| **296** | 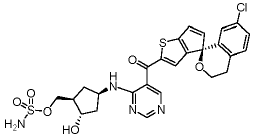 | 589 | **297** | 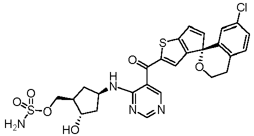 | 589 |
| **298** | 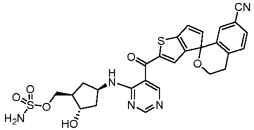 | 580 | **299** | 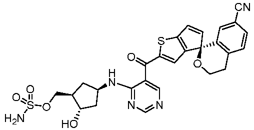 | 580 |
| **300** | 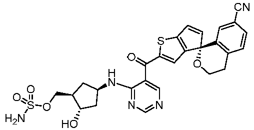 | 580 | | | |

### Biological Example 1. In Vitro Inhibitory Activity Assay of Compounds of the Present Disclosure Against SAE

HCT116 cells were seeded into a 96-well black transparent bottom plate at about 20000/well with 90 µL per well. The cells were incubated at 37 °C overnight for 24 h. The test compounds were 10-fold diluted to give a final concentration, and 10 µL of the diluted compounds was added to each well of the cell culture plate. The mixture was incubated at 37 °C for 6 h. The cells were then washed gently, the culture medium was discarded, and the cells were washed once with 200 µL of 0.1% PBST. 50 µL of 4% PFA was added to each well, and the cells were immobilized at room temperature for 20 min, and then washed with PBS 2-3 times. 50 µL of 0.2% Triton X-100 was added to each well, and the cells were left to stand at room temperature for 15 min and washed with 0.1% PBST 3 times. 100 uL of 3% BSA (0.6 g BSA + 20 mL PBS) was added to each well, and the mixture was blocked at 37 °C for 30 min. After the blocking was completed, the liquid was removed and a primary antibody (SUMO-2/3 (18H8) Rabbit mAb, 1:400 dilution) was prepared with 1% BSA. 30 µL of the diluted primary antibody was added to each well, and the mixture was incubated at 4 °C overnight. The cells were washed 3 times with 200 µL of 0.1% PBST. A FITC secondary antibody (Fluorescein (FITC)-conjugated Affinipure Goat Anti-Rabbit IgG (H+L), 1:300 dilution) was further prepared with 1% BSA, and 40 µL of the diluted secondary antibody was added to each well. The mixture was incubated at room temperature in the dark for 2 h, and then the cells were washed 4 times with 0.1% PBST. The fluorescence signal of the sample was detected and compared to the DMSO group, and the inhibition rate and IC₅₀ were calculated. The results are shown in Table 2 below.

**Table 2. Inhibitory activity of the compounds of the present disclosure against SAE (IC₅₀, nM)**

| **Compound** | **SUMO IF IC₅₀** | **Compound** | **SUMO IF IC₅₀** | **Compound** | **SUMO IF IC₅₀** |
|---|---|---|---|---|---|
| **1** | ++ | **2** | + | **3** | +++ |

**Table 3. Inhibitory activity of the compounds of the present disclosure against SAE (IC₅₀, nM)**

| **Compound** | **SUMO IF IC₅₀** | **Compound** | **SUMO IF IC₅₀** | **Compound** | **SUMO IF IC₅₀** |
|---|---|---|---|---|---|
| **4** | +++ | **5** | >1000 | **6** | 27.8 |
| **7** | ++ | **8** | >1000 | **9** | 36.0 |
| **10** | ++ | **11** | + | **12** | +++ |
| **13** | + | **14** | + | **15** | ++ |
| **16** | ++ | **17** | >1000 | **18** | 103.9 |
| **19** | ++ | **20** | >1000 | **21** | 87.5 |
| **22** | ++ | **23** | ++ | **24** | + |
| **25** | ++ | **26** | ++ | **27** | +++ |
| **28** | ++ | **29** | ++ | **30** | ++ |
| **31** | ++ | **32** | ++ | **33** | ++ |
| **34** | ++ | **35** | ++ | **36** | +++ |
| **37** | +++ | **38** | ++ | **39** | ++ |
| **40** | ++ | **41** | ++ | **42** | +++ |
| **43** | + | **44** | >1000 | **45** | 125.0 |
| **46** | ++ | **47** | >1000 | **48** | 41.0 |
| **49** | ++ | **50** | >1000 | **51** | 50.7 |
| **52** | ++ | **53** | + | **54** | ++ |
| **55** | + | **56** | + | **57** | ++ |
| **58** | + | **59** | + | **60** | ++ |
| **61** | ++ | **62** | + | **63** | ++ |
| **64** | ++ | **65** | ++ | **66** | + |
| **67** | ++ | **68** | ++ | **69** | +++ |
| **70** | ++ | **71** | ++ | **72** | ++ |
| **73** | ++ | **74** | ++ | **75** | ++ |
| **76** | ++ | **77** | ++ | **78** | +++ |
| **79** | ++ | **80** | ++ | **81** | ++ |
| **82** | ++ | **83** | ++ | **84** | +++ |
| **85** | + | **86** | + | **87** | ++ |
| **88** | +++ | **89** | + | **90** | +++ |
| **91** | +++ | **92** | >1000 | **93** | 28.9 |
| **94** | ++ | **95** | + | **96** | ++ |
| **97** | ++ | **98** | + | **99** | ++ |
| **100** | ++ | **101** | + | **102** | ++ |
| **103** | ++ | **104** | + | **105** | ++ |
| **106** | ++ | **107** | + | **108** | ++ |
| **109** | ++ | **110** | + | **111** | ++ |
| **112** | +++ | **113** | >1000 | **114** | 228 |
| **115** | +++ | **116** | >1000 | **117** | 19.1 |
| **118** | ++ | **119** | + | **120** | ++ |
| **121** | + | **122** | + | **123** | ++ |
| **124** | + | **125** | + | **126** | + |
| **127** | + | **128** | + | **129** | ++ |
| **130** | ++ | **131** | + | **132** | ++ |
| **133** | ++ | **134** | + | **135** | +++ |
| **136** | +++ | **137** | + | **138** | +++ |
| **139** | ++ | **140** | + | **141** | +++ |
| **142** | ++ | **143** | + | **144** | +++ |
| **145** | ++ | **146** | + | **147** | +++ |
| **148** | ++ | **149** | + | **150** | +++ |
| **151** | +++ | **152** | + | **153** | +++ |
| **154** | +++ | **155** | + | **156** | +++ |
| **157** | ++ | **158** | + | **159** | +++ |
| **160** | ++ | **161** | + | **162** | ++ |
| **163** | + | **164** | + | **165** | + |
| **166** | ++ | **167** | + | **168** | ++ |
| **175** | >1000 | **176** | 232 | **177** | + |
| **178** | +++ | **179** | + | **180** | + |
| **181** | +++ | **182** | >1000 | **183** | 187 |
| **184** | +++ | **185** | >1000 | **186** | 163 |
| **187** | +++ | **188** | >1000 | **189** | 245 |
| **190** | +++ | **191** | >1000 | **192** | 25.1 |
| **193** | +++ | **194** | >1000 | **195** | 21.3 |
| **196** | ++ | **197** | >1000 | **198** | 28.8 |
| **199** | +++ | **200** | >1000 | **201** | 265 |
| **202** | ++ | **203** | ++ | **204** | ++ |
| **205** | ++ | **206** | +++ | **207** | +++ |
| **208** | +++ | **209** | +++ | **210** | +++ |
| **211** | ++ | **212** | +++ | **213** | +++ |
| **214** | ++ | **215** | ++ | **216** | +++ |
| **217** | +++ | **218** | ++ | **219** | +++ |
| **220** | ++ | **221** | + | **222** | +++ |
| **223** | ++ | **224** | ++ | **225** | ++ |
| **226** | ++ | **227** | +++ | **228** | +++ |
| **229** | +++ | **230** | +++ | **231** | +++ |
| **232** | ++ | **233** | +++ | **234** | +++ |
| **235** | ++ | **236** | ++ | **237** | +++ |
| **238** | +++ | **239** | ++ | **240** | ++ |
| **241** | ++ | **242** | + | **243** | +++ |
| **244** | + | **245** | + | **246** | ++ |
| **247** | ++ | **248** | + | **249** | +++ |
| **250** | ++ | **251** | + | **252** | +++ |
| **253** | ++ | **254** | + | **255** | ++ |
| **256** | ++ | **257** | + | **258** | +++ |
| **259** | +++ | **260** | + | **261** | +++ |
| **262** | ++ | **263** | + | **264** | +++ |
| **265** | +++ | **266** | + | **267** | +++ |
| **268** | ++ | **269** | + | **270** | +++ |
| **271** | ++ | **272** | + | **273** | +++ |
| **274** | ++ | **275** | + | **276** | +++ |
| **277** | ++ | **278** | + | **279** | +++ |
| **280** | + | **281** | + | **282** | ++ |
| **283** | ++ | **284** | + | **285** | ++ |
| **286** | ++ | **287** | + | **288** | +++ |
| **289** | ++ | **290** | + | **291** | ++ |
| **292** | ++ | **293** | + | **294** | +++ |
| **295** | +++ | **296** | + | **297** | +++ |
| **298** | ++ | **299** | + | **300** | +++ |
| **TAK-981** | 53.2 | | | | |

| | | | | | |
|---|---|---|---|---|---|
| +++ means that IC₅₀ is less than or equal to 50 nM ++ means that IC₅₀ is 50 nM to 200 nM + means that IC₅₀ is greater than 200 nM | | | | | |

TAK-981 is compound I-263a in WO2016004136A1, and has the following chemical structure:

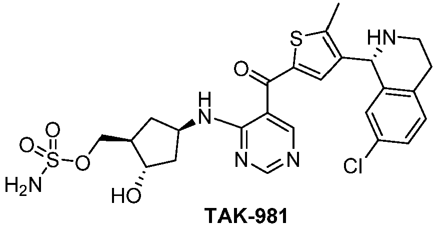

As can be seen from the data in Tables 2 and 3, compared to TAK-981, most of the compounds of the present disclosure have a stronger inhibitory activity against SAE in the *in vitro* inhibitory activity assay against SAE.

### Biological Example 2. Assay for Compounds of the Present Disclosure in Promoting in Vitro Killing Activity of NK Cells on Tumor Cell OVCAR3

OVCAR3 cells and NK92MI cells were seeded separately at about 8000/well and at about 80000/well into a 96-well black transparent cell culture plate with 90 µL per well. The cells were incubated at 37 °C overnight for 24 h. The test compounds were 10-fold diluted to give a final concentration, and 10 µL of the diluted compounds was added to each well of the cell culture plate. The cells were incubated at 37 °C for 48 h. The OVCAR3 cell culture medium was discarded. The OVCAR3 cells were stained with 1 µM Calcein AM for 50 min, washed once with PBS, and then 100 µL of the culture solution was added. The NK92MI cells were mixed well and then gently added to the OVCAR3 cells. After about 4 h of co-incubation, the DMSO control group and the high concentration drug group were photographed using PICO, and the killing of OVCAR3 cells by NK92MI cells was observed. When a significant difference in killing between the two groups was observed, the co-incubation was stopped. The culture medium was discarded, and the cells were gently washed once with 100 µL of PBS. 100 µL of 4% PFA was added to each well, and the cells were immobilized at room temperature for 20 min, and then gently washed once with PBS. The fluorescence signal of the sample was detected through the FITC channel of a microplate reader. The fluorescence signal was compared to the DMSO group, and the inhibition rate and IC₅₀ were calculated.

### Biological Example 3. In Vivo Pharmacokinetic Experiment of Compound of the Present Disclosure

CD-1 female mice aged 6 to 8 weeks were intravenously administered at a dose of 2 mg/kg. The mice were fasted for at least 12 h before the administration and given food after the administration, and they were given *ad libitum* access to water during the experiment. On the day of the experiment, animals in the intravenous group were administered the corresponding compound by single injection via the tail vein at an administration volume of 0.2 mL/animal. The sample collection time was 0.083 h, 0.167 h, 0.5 h, 1 h, 2 h, 4 h, 8 h, and 24 h. About 150 µL of whole blood was collected through the submaxillary venous plexus at each time point (30 µL of whole blood was diluted with ultrapure water at a ratio of 1:3, and 120 µL of whole blood was centrifuged to obtain plasma) and used for concentration determination by high-performance liquid chromatography-tandem mass spectrometry (LC-MS/MS). All animals were sacrificed after the PK samples were collected at the last time point. The plasma concentration was processed using a non-compartmental model of Phoenix WinNonlin^{™} version 8.3 (Certara) pharmacokinetic software, and the pharmacokinetic parameters were calculated using a linear-log trapezoidal method. The *in vivo* pharmacokinetic results are shown in Tables 4 and 5 below.

**Table 4. Results of in vivo pharmacokinetic evaluation of compound of the present disclosure**

| **Compound** | **Administration dose** | **T_{1/2} (h)** | **Cmax (ng/mL)** | **C0 (ng/mL)** | **AUClast (h*ng/mL)** | **Vdss (L/kg)** | **CL (mL/min/kg)** |
|---|---|---|---|---|---|---|---|
| **Compound 6** | 2 mpk | 1.45 | 798 | 1079 | 881 | 3.5 | 36.9 |
| **Compound 9** | 2 mpk | 1.31 | 597 | 730 | 580 | 3.91 | 56.9 |
| **Compound 21** | 2mpk | 2.17 | 1154 | 1610 | 1479 | 3.03 | 21.1 |
| **Compound 48** | 2 mpk | 1.32 | 719 | 839 | 749 | 3.43 | 44 |

**Table 5. Plasma drug concentration and whole blood drug concentration of compound of the present disclosure**

| | **Compound 6** | **Compound 9** | **Compound 21** |
|---|---|---|---|
| **Administration dose** | 2 mpk | 2 mpk | 2 mpk |
| **Plasma Cmax (ng/mL)** | 798 | 597 | 1154 |
| **Whole blood Cmax (ng/mL)** | 15800 | 14300 | 20233 |
| **Plasma AUClast (h × ng/mL)** | 881 | 580 | 1479 |
| **Whole blood AUClast (h × ng/mL)** | 34423 | 19020 | 67937 |
| **Whole blood Cmax/plasma Cmax ratio** | 19.7 | 23.9 | 17.5 |
| **Whole blood AUClast/plasma AUClast ratio** | 39.0 | 32.7 | 45.9 |

### Biological Example 4. In Vivo Pharmacodynamic Study - Mouse MC38 Subcutaneous Xenograft Tumor Model

Each C57BL/6 mouse was inoculated subcutaneously with 1 × 10⁶ MC38 cells, and when the tumors grew to 100-200 mm³, the vehicle was administered alone (intravenous injection, twice a week), the compound was administered alone (intravenous injection, twice a week), the anti-PD-1 antibody was administered alone (intravenous injection, once a week), the anti-VEGF antibody was administered alone (intraperitoneal injection, once a week), the anti-PD-1 antibody (intravenous injection, once a week) and the anti-VEGF antibody (intraperitoneal injection, once a week) were administered in combination, the compound (intravenous injection, twice a week) and the anti-PD-1 antibody (intravenous injection, once a week) were administered in combination, the compound (intravenous injection, twice a week) and the anti-VEGF antibody (intraperitoneal injection, once a week) were administered in combination, or the compound (intravenous injection, twice a week), the anti-PD-1 antibody (intravenous injection, once a week), and the anti-VEGF antibody (intraperitoneal injection, once a week) were administered in combination. The tumor volume was measured twice a week and at the end of treatment. Tumor growth inhibition rate of the compound was calculated according to the following equation: tumor growth inhibition (TGI) = 1 - (tumor volume on day 20 in treatment group - tumor volume on day 1 in treatment group)/(tumor volume on day 20 in vehicle control group - tumor volume on day 1 in treatment group).

### Biological Example 5. In Vivo Pharmacodynamic Study - Mouse MC38 Subcutaneous Xenograft Tumor Model

Each C57BL/6 mouse was inoculated subcutaneously with 1 × 10⁶ MC38 cells, and when the tumors grew to 50-80 mm³, the vehicle was administered alone (intravenous injection, twice a week), the compound was administered alone (intravenous injection, twice a week), the anti-PD-1 antibody was administered alone (intravenous injection, once a week), the anti-VEGF antibody was administered alone (intraperitoneal injection, once a week), the anti-PD-1 antibody (intravenous injection, once a week) and the anti-VEGF antibody (intraperitoneal injection, once a week) were administered in combination, the compound (intravenous injection, twice a week) and the anti-PD-1 antibody (intravenous injection, once a week) were administered in combination, the compound (intravenous injection, twice a week) and the anti-VEGF antibody (intraperitoneal injection, once a week) were administered in combination, or the compound (intravenous injection, twice a week), the anti-PD-1 antibody (intravenous injection, once a week), and the anti-VEGF antibody (intraperitoneal injection, once a week) were administered in combination. The tumor volume was measured twice a week and at the end of treatment. Tumor growth inhibition rate of the compound was calculated according to the following equation: tumor growth inhibition (TGI) = 1 - (tumor volume on day 20 in treatment group - tumor volume on day 1 in treatment group)/(tumor volume on day 20 in vehicle control group - tumor volume on day 1 in treatment group).

### Biological Example 6. In Vivo Pharmacodynamic Study - Mouse MC38 Subcutaneous Xenograft Tumor Model

Each C57BL/6 mouse was inoculated subcutaneously with 1 × 10⁶ MC38 cells, and the treatment was administered immediately after the cell inoculation. The vehicle was administered alone (intravenous injection, twice a week), the compound was administered alone (intravenous injection, twice a week), the anti-PD-1 antibody was administered alone (intravenous injection, once a week), the anti-VEGF antibody was administered alone (intraperitoneal injection, once a week), the anti-PD-1 antibody (intravenous injection, once a week) and the anti-VEGF antibody (intraperitoneal injection, once a week) were administered in combination, the compound (intravenous injection, twice a week) and the anti-PD-1 antibody (intravenous injection, once a week) were administered in combination, the compound (intravenous injection, twice a week) and the anti-VEGF antibody (intraperitoneal injection, once a week) were administered in combination, or the compound (intravenous injection, twice a week), the anti-PD-1 antibody (intravenous injection, once a week), and the anti-VEGF antibody (intraperitoneal injection, once a week) were administered in combination. The tumor volume was measured twice a week and at the end of treatment. Tumor growth inhibition rate of the compound was calculated according to the following equation: tumor growth inhibition (TGI) = 1 - (tumor volume on day 20 in treatment group - tumor volume on day 1 in treatment group)/(tumor volume on day 20 in vehicle control group - tumor volume on day 1 in treatment group).

### Biological Example 7. In Vivo Pharmacodynamic Study - Mouse CT26 Subcutaneous Xenograft Tumor Model

Each BALB/c mouse was inoculated subcutaneously with 2 × 10⁵ CT26 cells, and when the tumors grew to 100-200 mm³, the vehicle was administered alone (intravenous injection, twice a week), the compound was administered alone (intravenous injection, twice a week), the anti-PD-1 antibody was administered alone (intravenous injection, once a week), the anti-VEGF antibody was administered alone (intraperitoneal injection, once a week), the anti-PD-1 antibody (intravenous injection, once a week) and the anti-VEGF antibody (intraperitoneal injection, once a week) were administered in combination, the compound (intravenous injection, twice a week) and the anti-PD-1 antibody (intravenous injection, once a week) were administered in combination, the compound (intravenous injection, twice a week) and the anti-VEGF antibody (intraperitoneal injection, once a week) were administered in combination, or the compound (intravenous injection, twice a week), the anti-PD-1 antibody (intravenous injection, once a week), and the anti-VEGF antibody (intraperitoneal injection, once a week) were administered in combination. The tumor volume was measured twice a week and at the end of treatment. Tumor growth inhibition rate of the compound was calculated according to the following equation: tumor growth inhibition (TGI) = 1 - (tumor volume on day 20 in treatment group - tumor volume on day 1 in treatment group)/(tumor volume on day 20 in vehicle control group - tumor volume on day 1 in treatment group).

### Biological Example 8. In Vivo Pharmacodynamic Study - Mouse CT26 Subcutaneous Xenograft Tumor Model

Each BALB/c mouse was inoculated subcutaneously with 2 × 10⁵ CT26 cells, and when the tumors grew to 50-80 mm³, the vehicle was administered alone (intravenous injection, twice a week), the compound was administered alone (intravenous injection, twice a week), the anti-PD-1 antibody was administered alone (intravenous injection, once a week), the anti-VEGF antibody was administered alone (intraperitoneal injection, once a week), the anti-PD-1 antibody (intravenous injection, once a week) and the anti-VEGF antibody (intraperitoneal injection, once a week) were administered in combination, the compound (intravenous injection, twice a week) and the anti-PD-1 antibody (intravenous injection, once a week) were administered in combination, the compound (intravenous injection, twice a week) and the anti-VEGF antibody (intraperitoneal injection, once a week) were administered in combination, or the compound (intravenous injection, twice a week), the anti-PD-1 antibody (intravenous injection, once a week), and the anti-VEGF antibody (intraperitoneal injection, once a week) were administered in combination. The tumor volume was measured twice a week and at the end of treatment. Tumor growth inhibition rate of the compound was calculated according to the following equation: tumor growth inhibition (TGI) = 1 - (tumor volume on day 20 in treatment group - tumor volume on day 1 in treatment group)/(tumor volume on day 20 in vehicle control group - tumor volume on day 1 in treatment group).

### Biological Example 9. In Vivo Pharmacodynamic Study - Mouse CT26 Subcutaneous Xenograft Tumor Model

Each BALB/c mouse was inoculated subcutaneously with 2 × 10⁵ CT26 cells, and the treatment was administered immediately after the cell inoculation. The vehicle was administered alone (intravenous injection, twice a week), the compound was administered alone (intravenous injection, twice a week), the anti-PD-1 antibody was administered alone (intravenous injection, once a week), the anti-VEGF antibody was administered alone (intraperitoneal injection, once a week), the anti-PD-1 antibody (intravenous injection, once a week) and the anti-VEGF antibody (intraperitoneal injection, once a week) were administered in combination, the compound (intravenous injection, twice a week) and the anti-PD-1 antibody (intravenous injection, once a week) were administered in combination, the compound (intravenous injection, twice a week) and the anti-VEGF antibody (intraperitoneal injection, once a week) were administered in combination, or the compound (intravenous injection, twice a week), the anti-PD-1 antibody (intravenous injection, once a week), and the anti-VEGF antibody (intraperitoneal injection, once a week) were administered in combination. The tumor volume was measured twice a week and at the end of treatment. Tumor growth inhibition rate of the compound was calculated according to the following equation: tumor growth inhibition (TGI) = 1 - (tumor volume on day 20 in treatment group - tumor volume on day 1 in treatment group)/(tumor volume on day 20 in vehicle control group - tumor volume on day 1 in treatment group).

### Biological Example 10. In Vivo Pharmacodynamic Study - Mouse A20 Subcutaneous Xenograft Tumor Model

Each BALB/c mouse was inoculated subcutaneously with 2 × 10⁶ A20 cells, and when the tumors grew to 100-200 mm³, the vehicle was administered alone (intravenous injection, twice a week), the compound was administered alone (intravenous injection, twice a week), the anti-PD-1 antibody was administered alone (intravenous injection, once a week), the anti-VEGF antibody was administered alone (intraperitoneal injection, once a week), the anti-PD-1 antibody (intravenous injection, once a week) and the anti-VEGF antibody (intraperitoneal injection, once a week) were administered in combination, the compound (intravenous injection, twice a week) and the anti-PD-1 antibody (intravenous injection, once a week) were administered in combination, the compound (intravenous injection, twice a week) and the anti-VEGF antibody (intraperitoneal injection, once a week) were administered in combination, or the compound (intravenous injection, twice a week), the anti-PD-1 antibody (intravenous injection, once a week), and the anti-VEGF antibody (intraperitoneal injection, once a week) were administered in combination. The tumor volume was measured twice a week and at the end of treatment. Tumor growth inhibition rate of the compound was calculated according to the following equation: tumor growth inhibition (TGI) = 1 - (tumor volume on day 20 in treatment group - tumor volume on day 1 in treatment group)/(tumor volume on day 20 in vehicle control group - tumor volume on day 1 in treatment group).

### Biological Example 11. In Vivo Pharmacodynamic Study - Mouse A20 Subcutaneous Xenograft Tumor Model

Each BALB/c mouse was inoculated subcutaneously with 2 × 10⁶ A20 cells, and when the tumors grew to 50-80 mm³, the vehicle was administered alone (intravenous injection, twice a week), the compound was administered alone (intravenous injection, twice a week), the anti-PD-1 antibody was administered alone (intravenous injection, once a week), the anti-VEGF antibody was administered alone (intraperitoneal injection, once a week), the anti-PD-1 antibody (intravenous injection, once a week) and the anti-VEGF antibody (intraperitoneal injection, once a week) were administered in combination, the compound (intravenous injection, twice a week) and the anti-PD-1 antibody (intravenous injection, once a week) were administered in combination, the compound (intravenous injection, twice a week) and the anti-VEGF antibody (intraperitoneal injection, once a week) were administered in combination, or the compound (intravenous injection, twice a week), the anti-PD-1 antibody (intravenous injection, once a week), and the anti-VEGF antibody (intraperitoneal injection, once a week) were administered in combination. The tumor volume was measured twice a week and at the end of treatment. Tumor growth inhibition rate of the compound was calculated according to the following equation: tumor growth inhibition (TGI) = 1 - (tumor volume on day 20 in treatment group - tumor volume on day 1 in treatment group)/(tumor volume on day 20 in vehicle control group - tumor volume on day 1 in treatment group).

### Biological Example 12. In Vivo Pharmacodynamic Study - Mouse A20 Subcutaneous Xenograft Tumor Model

Each BALB/c mouse was inoculated subcutaneously with 2 × 10⁶ A20 cells, and the treatment was administered immediately after the cell inoculation. The vehicle was administered alone (intravenous injection, twice a week), the compound was administered alone (intravenous injection, twice a week), the anti-PD-1 antibody was administered alone (intravenous injection, once a week), the anti-VEGF antibody was administered alone (intraperitoneal injection, once a week), the anti-PD-1 antibody (intravenous injection, once a week) and the anti-VEGF antibody (intraperitoneal injection, once a week) were administered in combination, the compound (intravenous injection, twice a week) and the anti-PD-1 antibody (intravenous injection, once a week) were administered in combination, the compound (intravenous injection, twice a week) and the anti-VEGF antibody (intraperitoneal injection, once a week) were administered in combination, or the compound (intravenous injection, twice a week), the anti-PD-1 antibody (intravenous injection, once a week), and the anti-VEGF antibody (intraperitoneal injection, once a week) were administered in combination. The tumor volume was measured twice a week and at the end of treatment. Tumor growth inhibition rate of the compound was calculated according to the following equation: tumor growth inhibition (TGI) = 1 - (tumor volume on day 20 in treatment group - tumor volume on day 1 in treatment group)/(tumor volume on day 20 in vehicle control group - tumor volume on day 1 in treatment group).

### Biological Example 13. In Vivo Pharmacodynamic Study - Mouse B16F10-OVA Subcutaneous Xenograft Tumor Model

On day 14, day 11, day 7, and day 4 prior to inoculation with B16F10-OVA cells, each C57BL/6 mouse was administered vehicle alone (intravenous injection), the compound alone (intravenous injection), Ovalbumin alone (intravenous injection), the anti-PD-1 antibody alone (intravenous injection), the anti-VEGF antibody alone (intraperitoneal injection), a combination of the compound (intravenous injection) and Ovalbumin (intravenous injection), a combination of the anti-PD-1 antibody (intravenous injection) and the anti-VEGF antibody (intraperitoneal injection), a combination of the compound (intravenous injection) and the anti-PD-1 antibody (intravenous injection), a combination of the compound (intravenous injection) and the anti-VEGF antibody (intraperitoneal injection), a combination of the compound (intravenous injection), the anti-PD-1 antibody (intravenous injection), and Ovalbumin (intravenous injection), a combination of the compound (intravenous injection), the anti-VEGF antibody (intraperitoneal injection), and Ovalbumin (intravenous injection), a combination of the compound (intravenous injection), the anti-PD-1 antibody (intravenous injection), and the anti-VEGF antibody (intraperitoneal injection), or a combination of the compound (intravenous injection), the anti-PD-1 antibody (intravenous injection), the anti-VEGF antibody (intraperitoneal injection), and Ovalbumin (intravenous injection). Each C57BL/6 mouse was inoculated subcutaneously with 0.3 × 10⁶ B16F10-OVA cells, and the tumor volume was measured twice weekly and at the end of treatment. Tumor growth inhibition rate of the compound was calculated according to the following equation: tumor growth inhibition (TGI) = 1 - (tumor volume on day 20 in treatment group - tumor volume on day 1 in treatment group)/(tumor volume on day 20 in vehicle control group - tumor volume on day 1 in treatment group).

Although specific embodiments of the present disclosure have been described above, it will be appreciated by those skilled in the art that these embodiments are merely illustrative and that many changes or modifications can be made to these embodiments without departing from the principles and spirit of the present disclosure. The protection scope of the present disclosure is therefore defined by the appended claims.

## Claims

1. A compound of general formula (1) or an isomer thereof, a crystalline form thereof, a pharmaceutically acceptable salt thereof, a hydrate thereof, or a solvate thereof: 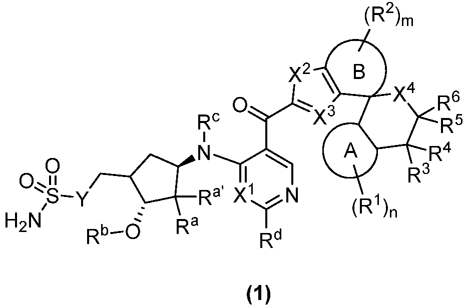 wherein in general formula (1):
Y is -O-, -CH₂-, or -N(H)-;
R^{a} is -H, -F, -NH₂, or -OH;
R^{a}' is -H or -F, and when R^{a} is -NH₂ or -OH, R^{a}' is -H;
R^{b} is -H or (C1-C4) alkyl;
R^{c} is -H or (C1-C4) alkyl;
R^{d} is -H, halogen, -CF₃, or (C1-C4) alkyl;
X¹ is C(H), C(F), or N;
X² is S or O;
X³ is C(R^{x3}) or N;
R^{x3} is -H, halogen, or -CH₃;
X⁴ is S, O, C(R^{x41})(R^{X41}'), or N(^{x42});
R^{x42} is -H, (C1-C4) alkyl or (C3-C5) cycloalkyl;
R^{x41} and R^{x41}' are each independently and optionally -H, halogen, -OH, -OR^{x411}, -N(R^{x411})(R^{x412}), -CN, (Cl-C6) alkyl, (C1-C6) haloalkyl, (C3-C9) cycloalkyl, or (C1-C6) alkoxy;
R^{x411} and R^{x412} are each independently and optionally -H, (C1-C4) alkyl, or (C3-C5) cycloalkyl, or R^{x411} and R^{x412} on a same nitrogen atom, together with the N atom to which they are attached, can form (3- to 6-membered) heterocycloalkyl, wherein the (3- to 6-membered) heterocycloalkyl may be optionally substituted with 1, 2, 3, or 4 of the following groups: -H or halogen;
R³ and R⁴ are each independently and optionally -H, -D, -OH, -NH₂, -CN, (C1-C6) alkyl, (C1-C6) haloalkyl, (C2-C6) alkenyl, (C2-C6) alkynyl, (C3-C9) cycloalkyl, (C1-C6) alkoxy, (C6-C14) aryl, (3- to 11-membered) heterocycloalkyl, or (5- to 11-membered) heteroaryl, wherein the (C1-C6) alkyl, (C1-C6) haloalkyl, (C2-C6) alkenyl, (C2-C6) alkynyl, (C3-C9) cycloalkyl, (C1-C6) alkoxy, (C6-C14) aryl, (3- to 11-membered) heterocycloalkyl, or (5- to 11-membered) heteroaryl may be each independently and optionally substituted with 1, 2, 3, or 4 of the following groups: -H, halogen, -OH, -(CH₂)ᵣOR³¹, -(CH₂)ᵣNR³¹R³², -OR³¹, -NR³¹R³², -CN, -C(0)NR³¹R³², -NR³²C(O)R³¹, -NR³²S(O)₂R³¹, -S(O)ₚR³¹, and -S(O)₂NR³¹R³²; or R³ and R⁴, together with the carbon atom to which they are attached, can form a (4- to 7-membered) heterocycloalkyl or (C3-C6) cycloalkyl, wherein the (4- to 7-membered) heterocycloalkyl or (C3-C6) cycloalkyl may be optionally substituted with 1, 2, 3, or 4 of the following groups: -H, halogen, (C1-C6) alkyl, or (C1-C6) alkoxy; or R³ and the adjacent R⁵, together with the atoms to which they are attached, can form a (C3-C9) cycloalkyl or (3-to 11-membered) heterocycloalkyl, wherein the (C3-C9) cycloalkyl or (3- to 11-membered) heterocycloalkyl may be each independently and optionally substituted with 1, 2, 3, or 4 of the following groups: -H, halogen, (C1-C6) alkyl, or (C1-C6) alkoxy; or when R³ and the adjacent R⁵ are both absent, an endocyclic double bond is formed; or R³ and R⁴ together form an oxo;
R⁵ and R⁶ are each independently and optionally -H, -D, -OH, -NH₂, -CN, (C1-C6) alkyl, (C1-C6) haloalkyl, (C2-C6) alkenyl, (C2-C6) alkynyl, (C3-C9) cycloalkyl, (C1-C6) alkoxy, (C6-C14) aryl, (3- to 11-membered) heterocycloalkyl, or (5- to 11-membered) heteroaryl, wherein the (C1-C6) alkyl, (C1-C6) haloalkyl, (C2-C6) alkenyl, (C2-C6) alkynyl, (C3-C9) cycloalkyl, (C1-C6) alkoxy, (C6-C14) aryl, (3- to 11-membered) heterocycloalkyl, or (5- to 11-membered) heteroaryl may be each independently and optionally substituted with 1, 2, 3, or 4 of the following groups: -H, halogen, -OH, -(CH₂)ᵣOR³¹, -(CH₂)ᵣNR³¹R³², -OR³¹, -NR³¹R³², -CN, -C(O)NR³¹R³², -NR³²C(O)R³¹, -NR³²S(O)₂R³¹, -S(O)ₚR³¹, and -S(O)₂NR³¹R³²; or R⁵ and R⁶, together with the carbon atom to which they are attached, can form a (4- to 7-membered) heterocycloalkyl or (C3-C6) cycloalkyl, wherein the (4- to 7-membered) heterocycloalkyl or (C3-C6) cycloalkyl may be optionally substituted with 1, 2, 3, or 4 of the following groups: -H, halogen, (C1-C6) alkyl, and (C1-C6) alkoxy; or R⁵ and R⁶ together form an oxo;
ring A is (C6-C10) aryl or (5- to 10-membered) heteroaryl;
each R¹ is independently and optionally: -H, halogen, -OH, -NO₂, -NR³¹R³², -(CH₂)ᵣOR³¹, -(CH₂)ᵣNR³¹R³², - CN, (C1-C6) alkyl, (C1-C6) haloalkyl, (C1-C6) alkoxy, (C2-C6) alkenyl, (C2-C6) alkynyl, (C3-C8) cycloalkyl, -C(O)NR³¹R³², -NR³²C(O)R³¹, -NR³²S(O)₂R³¹, -S(O)ₚR³¹, or -S(O)₂NR³¹R³², wherein the (C1-C6) alkyl, (C1-C6) haloalkyl, (C1-C6) alkoxy, (C2-C6) alkenyl, (C2-C6) alkynyl, or (C3-C8) cycloalkyl may be each independently and optionally substituted with 1, 2, 3, or 4 of the following groups: -H, halogen, -OH, - (CH₂)ᵣOR³¹, -(CH₂)ᵣNR³¹R³², -OR³¹, -NR³¹R³², -CN, and (C1-C6) alkyl;
ring B is (C5-C7) cycloalkyl or (5- to 7-membered) heterocycloalkyl;
each R² is independently and optionally: -H, halogen, -OH, -NR³¹R³², -CN, (C1-C6) alkyl, (C1-C6) haloalkyl, (C1-C6) alkoxy, (C2-C6) alkenyl, (C2-C6) alkynyl, or (C3-C8) cycloalkyl; or two R² on a same carbon atom, together with the carbon atom to which they are attached, can form (4- to 6-membered) heterocycloalkyl or (C3-C6) cycloalkyl, wherein the (4- to 6-membered) heterocycloalkyl or (C3-C6) cycloalkyl may be optionally substituted with 1, 2, 3, or 4 of the following groups: -H, halogen, (C1-C6) alkyl, and (C1-C6) alkoxy; or two R² on a same carbon atom together form an oxo;
R³¹ and R³² are each independently and optionally -H, (C1-C4) alkyl, or (C3-C5) cycloalkyl, or R³¹ and R³² on a same nitrogen atom, together with the N atom to which they are attached, can form (3- to 6-membered) heterocycloalkyl, wherein the (3- to 6-membered) heterocycloalkyl may be optionally substituted with 1, 2, 3, or 4 of the following groups: -H and halogen; and
n is an integer selected from 0, 1, 2, 3, or 4, m is an integer selected from 0, 1, 2, 3, or 4, r is an integer selected from 0, 1, or 2, and p is an integer selected from 0, 1, or 2.

2. The compound or the isomer thereof, the crystalline form thereof, the pharmaceutically acceptable salt thereof, the hydrate thereof, or the solvate thereof according to claim 1, wherein in the general formula (1), R^{d} is -H, -F, -CF₃, or -CH₃.

3. The compound or the isomer thereof, the crystalline form thereof, the pharmaceutically acceptable salt thereof, the hydrate thereof, or the solvate thereof according to claim 1 or 2, wherein in the general formula (1), R^{x42} is -H, (C1-C3) alkyl, or (C3-C5) cycloalkyl.

4. The compound or the isomer thereof, the crystalline form thereof, the pharmaceutically acceptable salt thereof, the hydrate thereof, or the solvate thereof according to claim 3, wherein in the general formula (1), R^{x42} is -H,

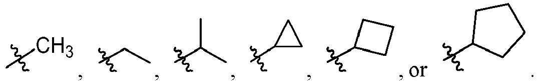

5. The compound or the isomer thereof, the crystalline form thereof, the pharmaceutically acceptable salt thereof, the hydrate thereof, or the solvate thereof according to any one of claims 1-4, wherein in the general formula (1), R^{x41} and R^{x41}' are each independently and optionally -H, -F, -OH, -OCH₃, -N(CH₃)₂, -NH₂, - CN, 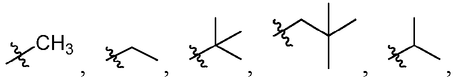 -CF₃, -CH₂CF₃,

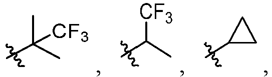

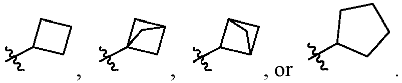

6. The compound or the isomer thereof, the crystalline form thereof, the pharmaceutically acceptable salt thereof, the hydrate thereof, or the solvate thereof according to any one of claims 1-5, wherein in the general formula (1), R³ and R⁴ are each independently and optionally -H, -D, -OH, -NH₂, -CN, (C1-C3) alkyl, (C1-C3) haloalkyl, (C2-C4) alkenyl, (C2-C4) alkynyl, (C3-C5) cycloalkyl, (C1-C3) alkoxy, phenyl, (4- to 6-membered) heterocycloalkyl, or (5- to 6-membered) heteroaryl, wherein the (C1-C3) alkyl, (C1-C3) haloalkyl, (C2-C4) alkenyl, (C2-C4) alkynyl, (C3-C5) cycloalkyl, (C1-C3) alkoxy, phenyl, (4- to 6-membered) heterocycloalkyl, or (5- to 6-membered) heteroaryl may be each independently and optionally substituted with 1, 2, 3, or 4 of the following groups: -H, -F, -OH, -CH₂OCH₃, -CH₂N(CH₃)₂, -OCH₃, -N(CH₃)₂, -CN, - C(O)N(CH₃)₂, -NCH₃C(O)CH₃, -NHC(O)CH₃, -NCH₃S(O)₂CH₃, -NHS(O)₂CH₃, -SCH₃, -S(O)₂CH₃, - S(O)₂NH₂, and -S(O)₂N(CH₃)₂; or R³ and R⁴, together with the carbon atom to which they are attached, can form (4- to 6-membered) heterocycloalkyl or (C3-C4) cycloalkyl, wherein the (4- to 6-membered) heterocycloalkyl or (C3-C4) cycloalkyl may be optionally substituted with 1, 2, 3, or 4 of the following groups: -H, -F, 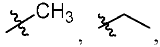 or -OCH₃; or R³ and the adjacent R⁵, together with the atoms to which they are attached, can form a (C3-C6) cycloalkyl or (3- to 6-membered) heterocycloalkyl, wherein the (C3-C6) cycloalkyl or (3- to 6-membered) heterocycloalkyl may be each independently and optionally substituted with 1, 2, 3, or 4 of the following groups: -H, -F, 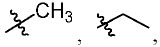 or -OCH₃; or when R³ and the adjacent R⁵ are both absent, an endocyclic double bond is formed; or R³ and R⁴ together form an oxo.

7. The compound or the isomer thereof, the crystalline form thereof, the pharmaceutically acceptable salt thereof, the hydrate thereof, or the solvate thereof according to any one of claims 1-6, wherein in the general formula (1), R⁵ and R⁶ are each independently and optionally -H, -D, -OH, -NH₂, -CN, (C1-C3) alkyl, (C1-C3) haloalkyl, (C2-C4) alkenyl, (C2-C4) alkynyl, (C3-C5) cycloalkyl, (C1-C3) alkoxy, phenyl, (4- to 6-membered) heterocycloalkyl, or (5- to 6-membered) heteroaryl, wherein the (C1-C3) alkyl, (C1-C3) haloalkyl, (C2-C4) alkenyl, (C2-C4) alkynyl, (C3-C5) cycloalkyl, (C1-C3) alkoxy, phenyl, (4- to 6-membered) heterocycloalkyl, or (5- to 6-membered) heteroaryl may be each independently and optionally substituted with 1, 2, 3, or 4 of the following groups: -H, -F, -OH, -CH₂OCH₃, -CH₂N(CH₃)₂, -OCH₃, -N(CH₃)₂, -NH₂, -CN, - C(O)N(CH₃)₂, -NCH₃C(O)CH₃, -NHC(O)CH₃, -NCH₃S(O)₂CH₃, -NHS(O)₂CH₃, -SCH₃, -S(O)₂CH₃, - S(O)₂NH₂, and -S(O)₂N(CH₃)₂; or R⁵ and R⁶, together with the carbon atom to which they are attached, can form a (4- to 6-membered) heterocycloalkyl or (C3-C4) cycloalkyl, wherein the (4- to 6-membered) heterocycloalkyl or (C3-C4) cycloalkyl may be optionally substituted with 1, 2, 3, or 4 of the following groups: -H, -F, 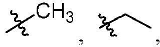 or -OCH₃; or R⁵ and R⁶ together form an oxo.

8. The compound or the isomer thereof, the crystalline form thereof, the pharmaceutically acceptable salt thereof, the hydrate thereof, or the solvate thereof according to any one of claims 1-7, wherein in the general formula (1), ring A is phenyl or (5- to 6-membered) heteroaryl.

9. The compound or the isomer thereof, the crystalline form thereof, the pharmaceutically acceptable salt thereof, the hydrate thereof, or the solvate thereof according to claim 8, wherein in the general formula (1), ring A is:

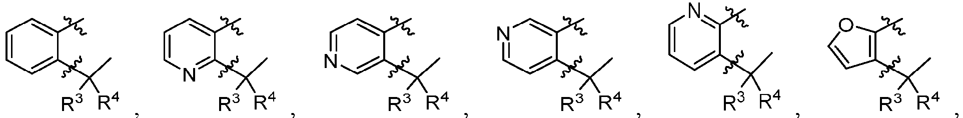

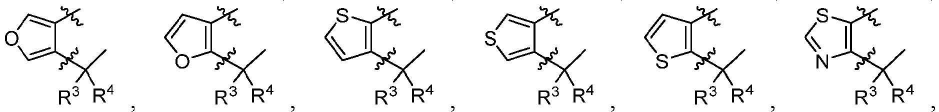

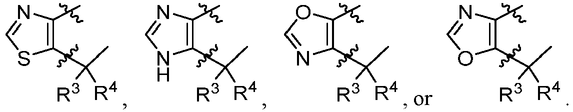

10. The compound or the isomer thereof, the crystalline form thereof, the pharmaceutically acceptable salt thereof, the hydrate thereof, or the solvate thereof according to any one of claims 1-9, wherein in the general formula (1), each R¹ is independently and optionally: -H, -F, -Cl, -Br, -I, -OH, -NO₂, -N(CH₃)₂, -NH₂, - CH₂OCH₃, -CH₂N(CH₃)₂, -CN, (C1-C3) alkyl, (C1-C3) haloalkyl, (C1-C3) alkoxy, (C2-C4) alkenyl, (C2-C4) alkynyl, (C3-C6) cycloalkyl, -C(O)N(CH₃)₂, -NCH₃C(O)CH₃, -NHC(O)CH₃, -NCH₃S(O)₂CH₃, - NHS(O)₂CH₃, -SCH₃, -S(O)₂CH₃, -S(O)₂NH₂, and -S(O)₂N(CH₃)₂, wherein the (C1-C3) alkyl, (C1-C3) haloalkyl, (C1-C3) alkoxy, (C2-C4) alkenyl, (C2-C4) alkynyl, or (C3-C6) cycloalkyl may be each independently and optionally substituted with 1, 2, 3, or 4 of the following groups: -H, -F, -OH, -CH₂OCH₃, - CH₂N(CH₃)₂, -OCH₃, -N(CH₃)₂, -NH₂, -CN,

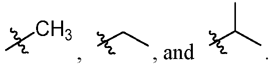

11. The compound or the isomer thereof, the crystalline form thereof, the pharmaceutically acceptable salt thereof, the hydrate thereof, or the solvate thereof according to claim 10, wherein in the general formula (1), each R¹ is independently: -H, -F, -Cl, -Br, -I, -OH, -NO₂, -N(CH₃)₂, -NH₂, -CH₂OCH₃, -CH₂N(CH₃)₂, -CN, -C(O)N(CH₃)₂, -NCH₃C(O)CH₃, -NHC(O)CH₃, -NCH₃S(O)₂CH₃, -NHS(O)₂CH₃, -SCH₃, -S(O)₂CH₃, -S(O)₂NH₂, -S(O)₂N(CH₃)₂, 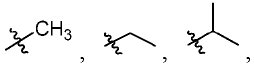 -CF₃, -CH₂CF₃, 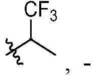 OCH₃, -OCH₂CH₃, -OCH(CH₃)₂,

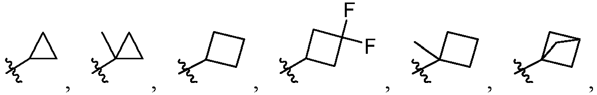

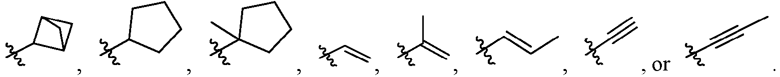

12. The compound or the isomer thereof, the crystalline form thereof, the pharmaceutically acceptable salt thereof, the hydrate thereof, or the solvate thereof according to any one of claims 1-11, wherein in the general formula (1), ring B is (C5-C6) cycloalkyl or (5- to 6-membered) heterocycloalkyl.

13. The compound or the isomer thereof, the crystalline form thereof, the pharmaceutically acceptable salt thereof, the hydrate thereof, or the solvate thereof according to claim 12, wherein in the general formula (1), the structural unit 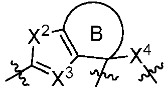 is:

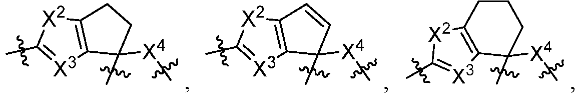

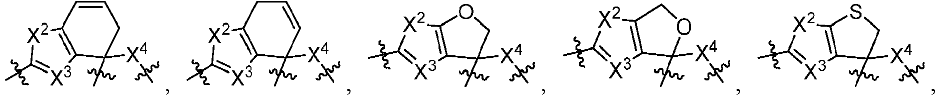

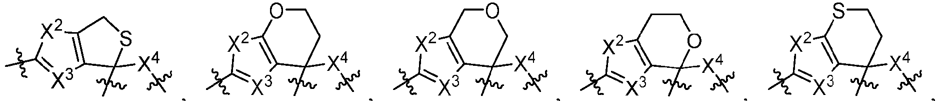

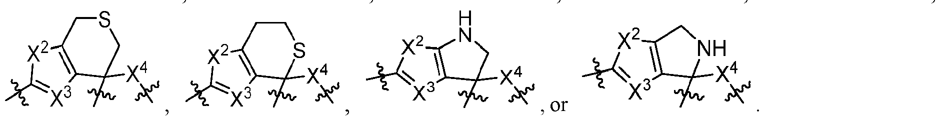

14. The compound or the isomer thereof, the crystalline form thereof, the pharmaceutically acceptable salt thereof, the hydrate thereof, or the solvate thereof according to claim 12, wherein in the general formula (1), the structural unit 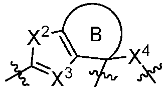 is:

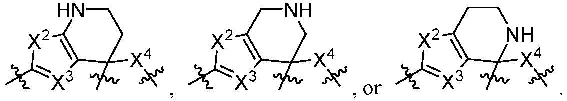

15. The compound or the isomer thereof, the crystalline form thereof, the pharmaceutically acceptable salt thereof, the hydrate thereof, or the solvate thereof according to any one of claims 1-14, wherein in the general formula (1), each R² is independently and optionally: -H, -F, -Cl, -Br, -I, -OH, -N(CH₃)₂, -NH₂, -CN, (C1-C3) alkyl, (C1-C3) haloalkyl, (C1-C3) alkoxy, (C2-C4) alkenyl, (C2-C4) alkynyl, or (C3-C5) cycloalkyl; or two R² on a same carbon atom, together the carbon atom to which they are attached, can form (4- to 5-membered) heterocycloalkyl or (C3-C5) cycloalkyl, wherein the (4- to 5-membered) heterocycloalkyl or (C3-C5) cycloalkyl may be optionally substituted with 1, 2, 3, or 4 of the following groups: -H, -F, 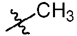 , and - OCH₃; or two R² on a same carbon atom together form an oxo.

16. The compound or the isomer thereof, the crystalline form thereof, the pharmaceutically acceptable salt thereof, the hydrate thereof, or the solvate thereof according to claim 15, wherein in the general formula (1), each R² is independently and optionally: -H, -F, -Cl, -Br, -I, -OH, -N(CH₃)₂, -NH₂, -CN, 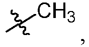 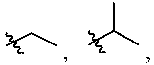 -CF₃, -CH₂CF₃, 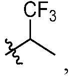 -OCH₃, -OCH₂CH₃, -OCH(CH₃)₂,

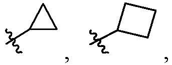

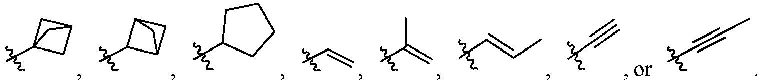

17. The compound or the isomer thereof, the crystalline form thereof, the pharmaceutically acceptable salt thereof, the hydrate thereof, or the solvate thereof according to any one of claims 1-16, wherein in the general formula (1), the structural unit 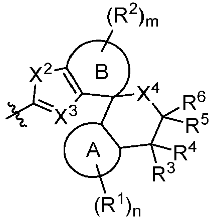 is: 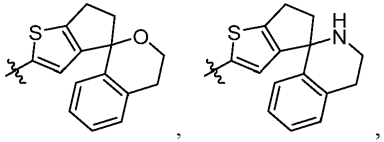 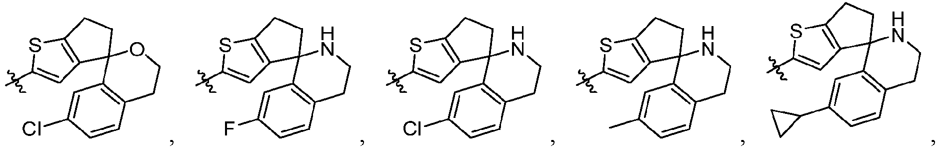 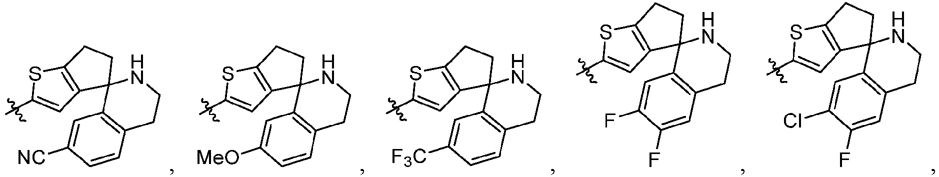 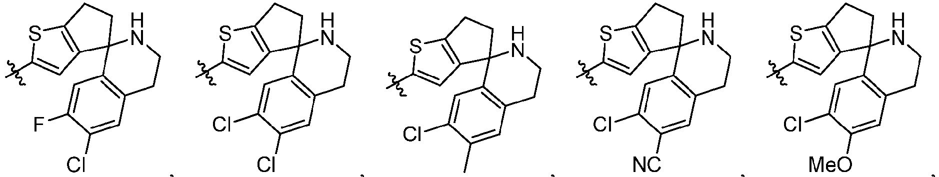 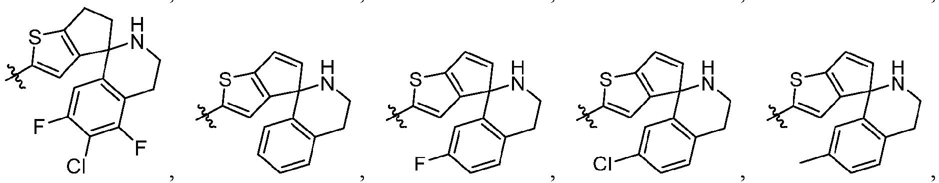 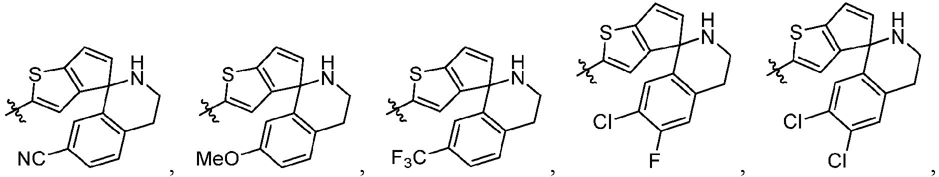 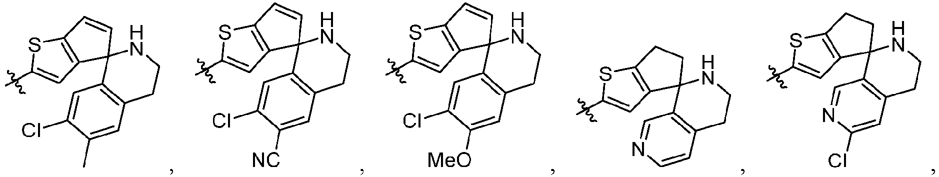 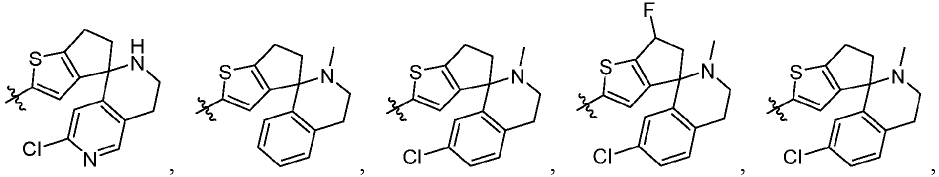 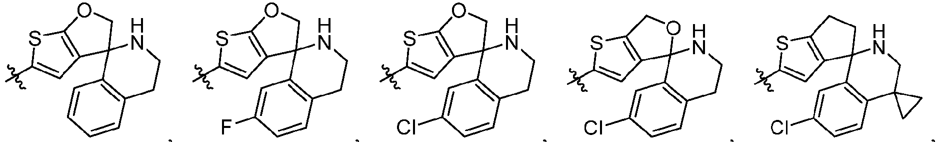 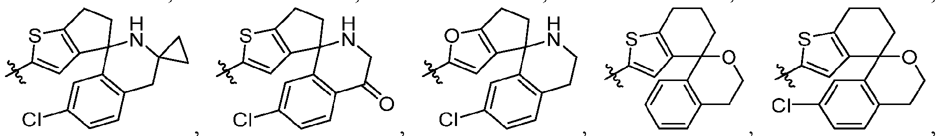 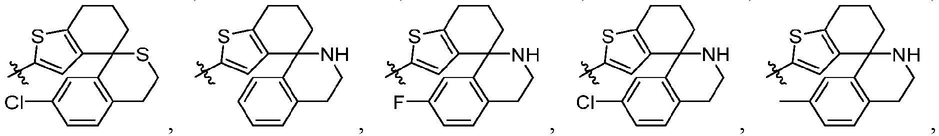 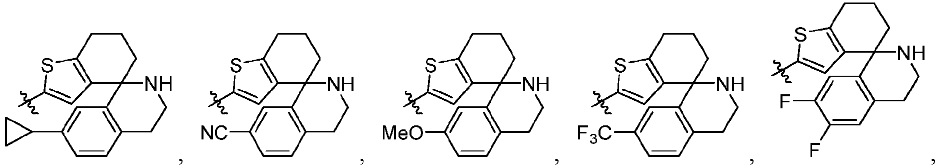 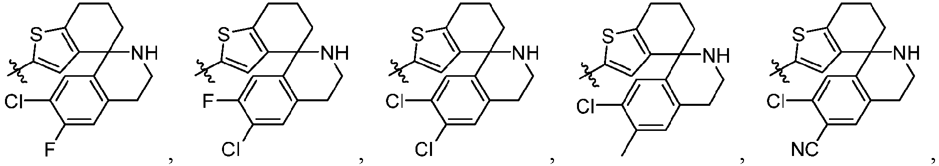 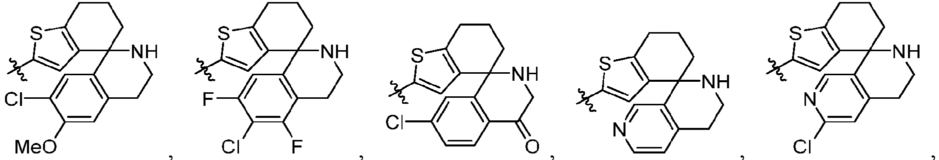 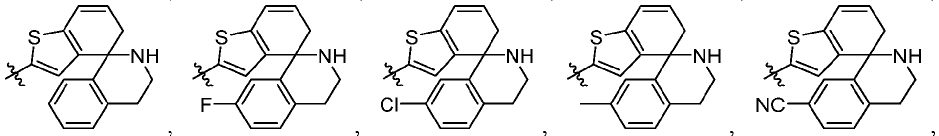 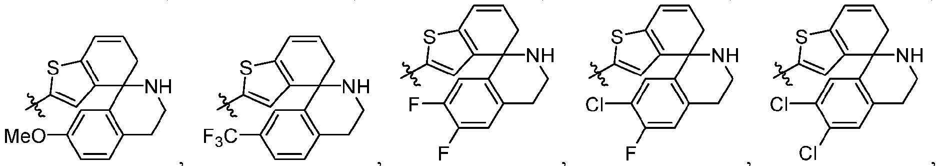 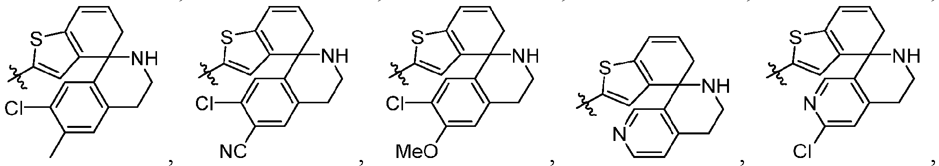 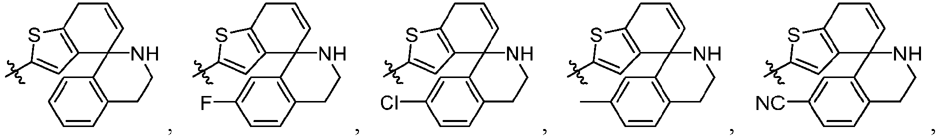 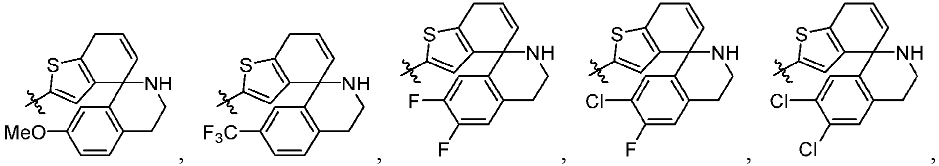 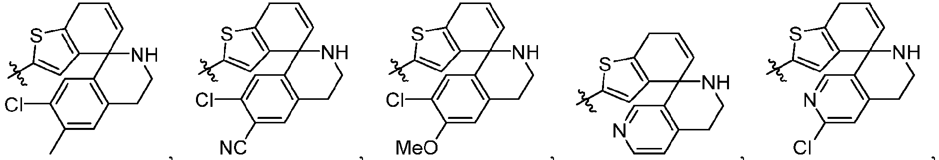 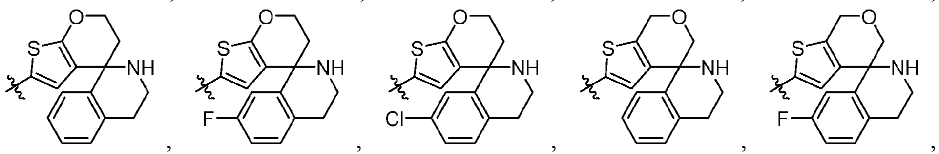 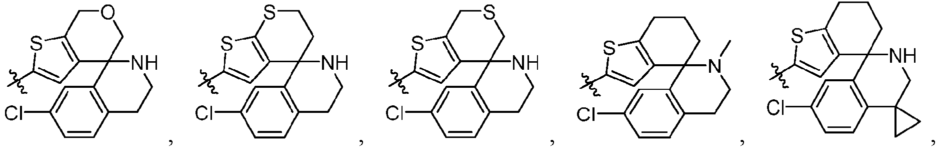 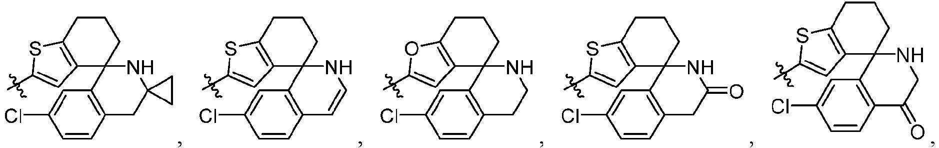 or

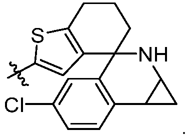

18. The compound or the isomer thereof, the crystalline form thereof, the pharmaceutically acceptable salt thereof, the hydrate thereof, or the solvate thereof according to any one of claims 1-16, wherein in the general formula (1), the structural unit 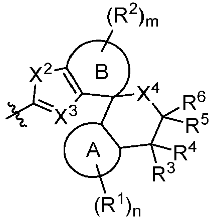 is:

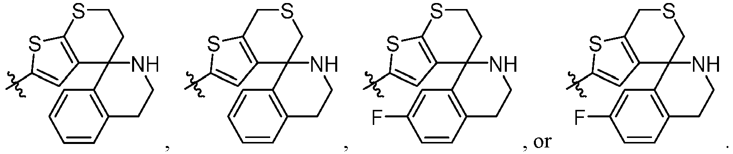

19. The compound or the isomer thereof, the crystalline form thereof, the pharmaceutically acceptable salt thereof, the hydrate thereof, or the solvate thereof according to any one of claims 1-16, wherein in the general formula (1), the structural unit 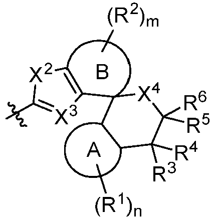 is:

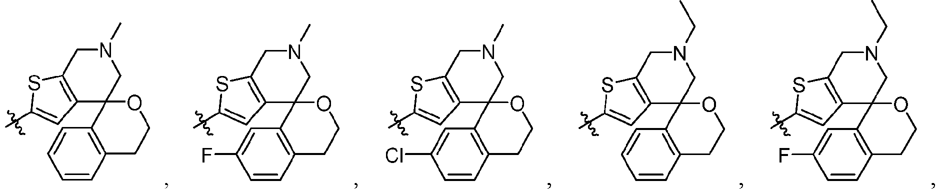

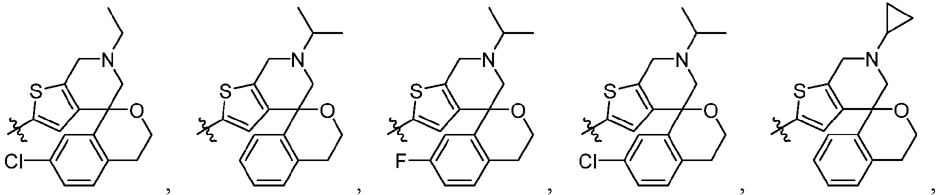

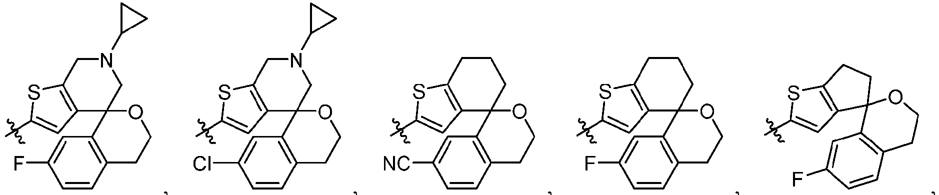

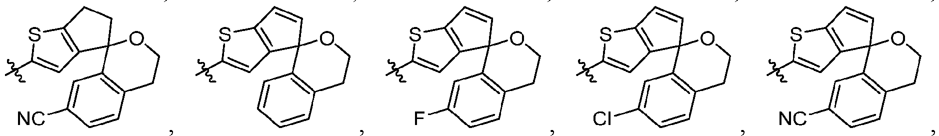

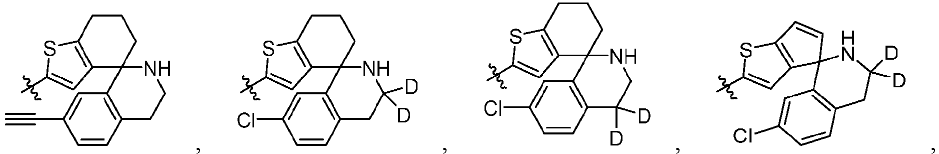

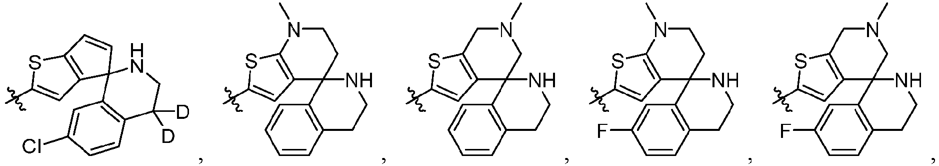

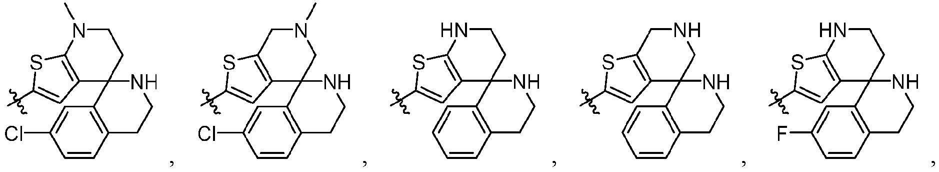

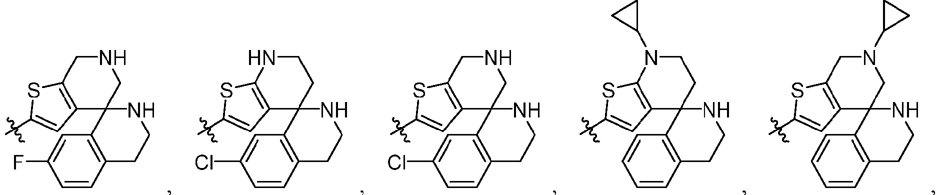

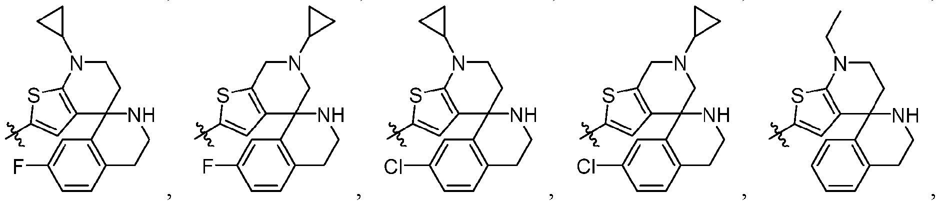

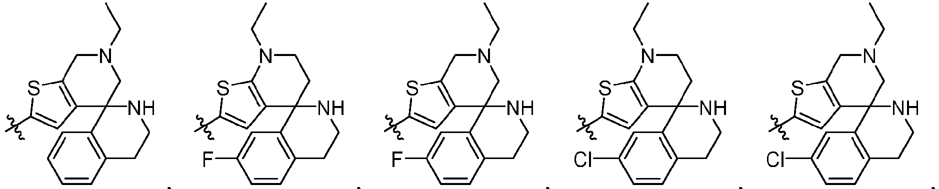

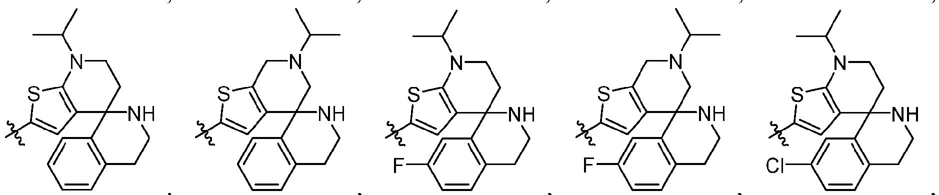

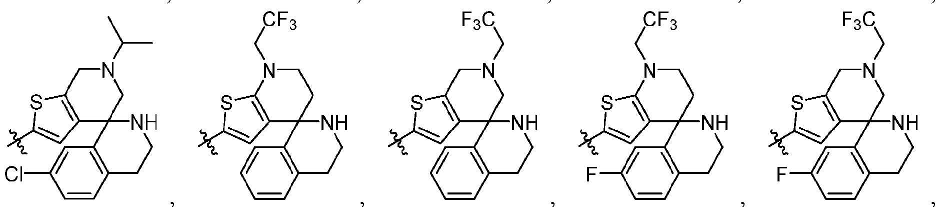

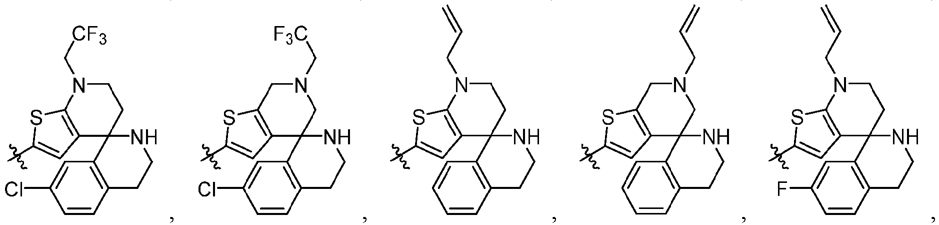

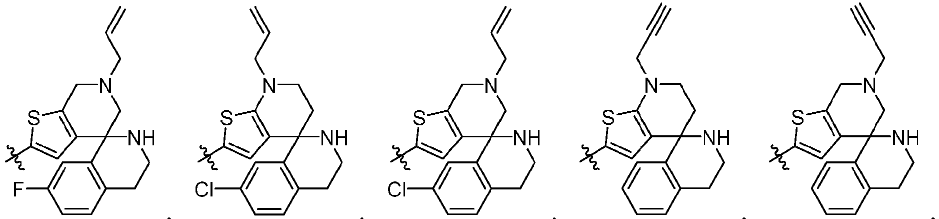

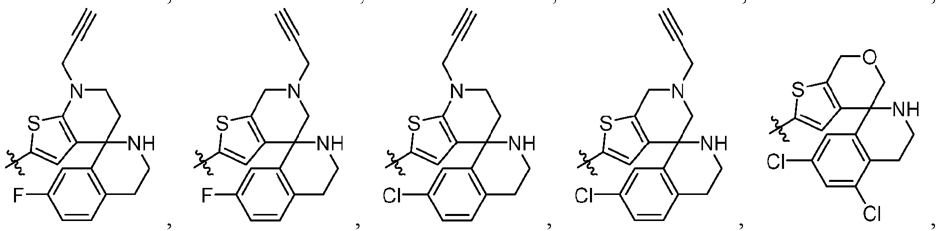

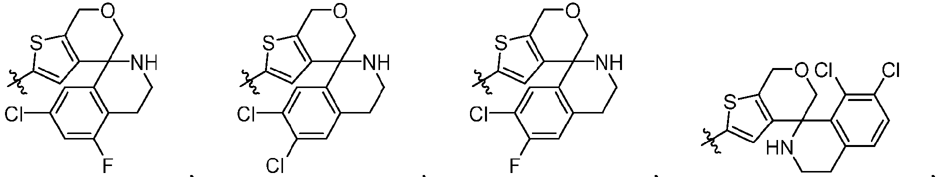

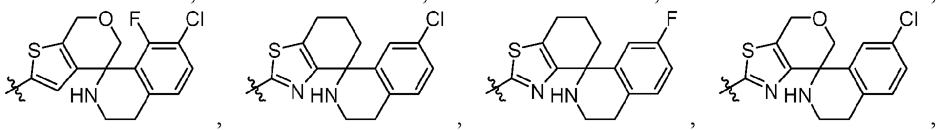

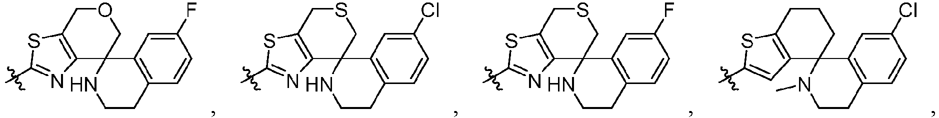

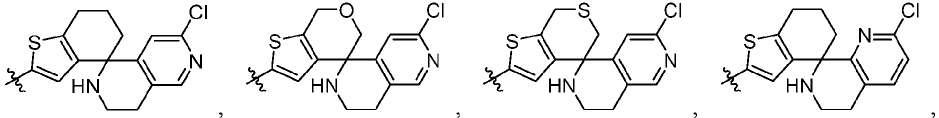

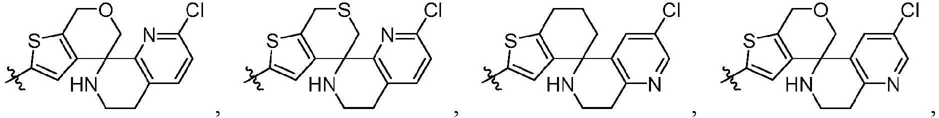

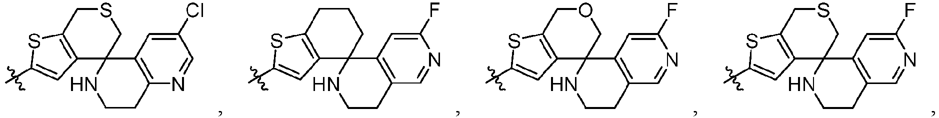

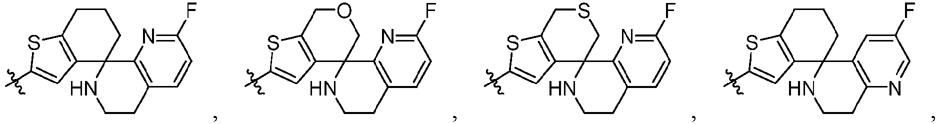

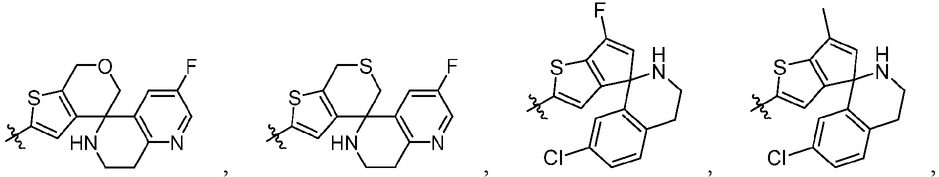

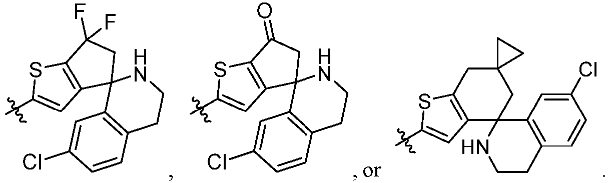

20. The compound or the isomer thereof, the crystalline form thereof, the pharmaceutically acceptable salt thereof, the hydrate thereof, or the solvate thereof according to any one of claims 1-19, wherein the compound has one of the following structures:

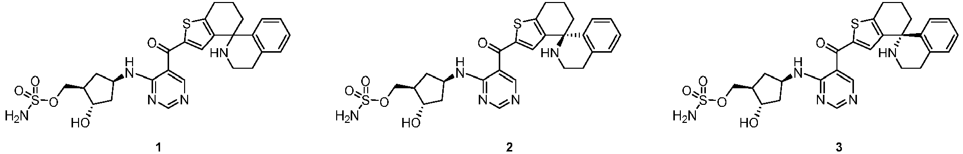

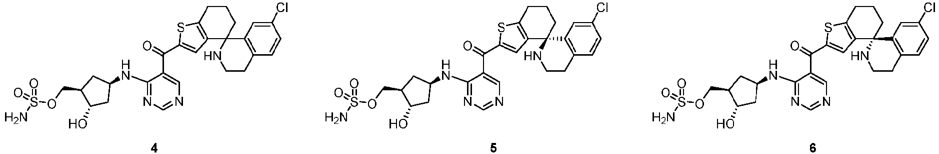

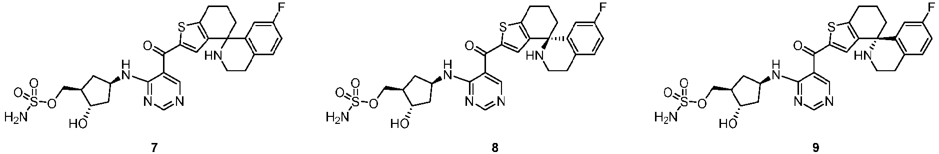

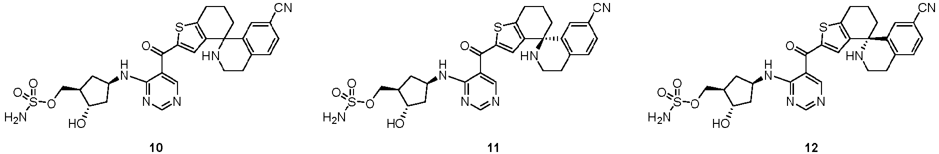

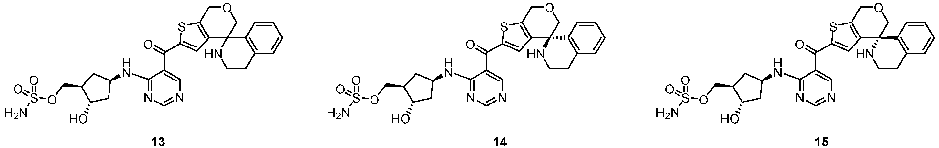

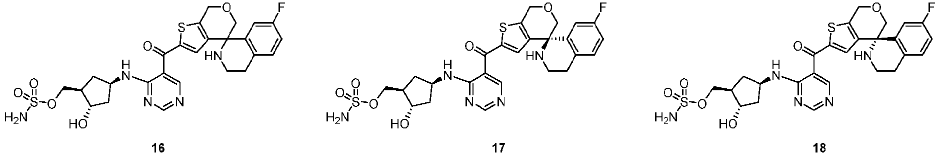

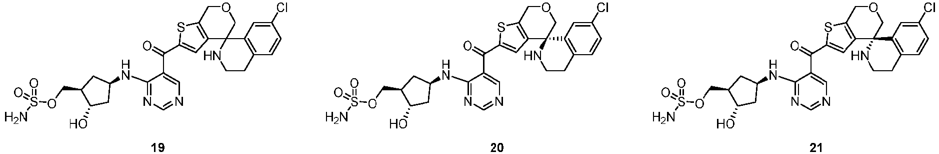

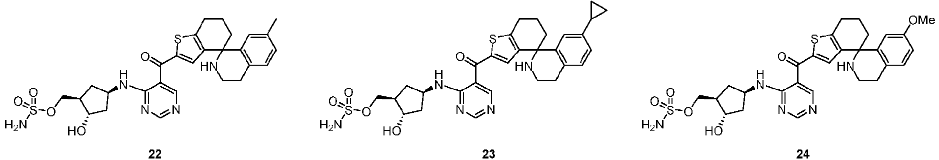

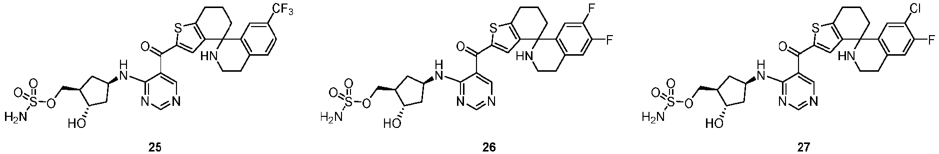

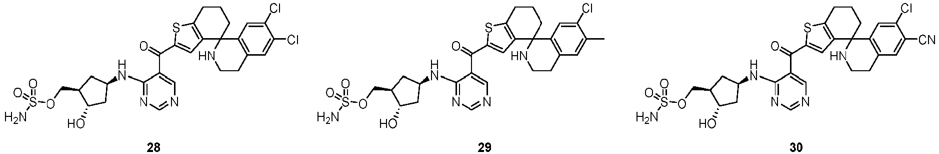

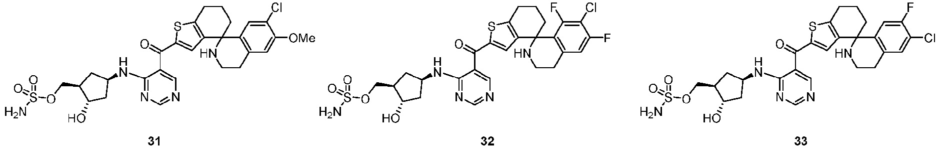

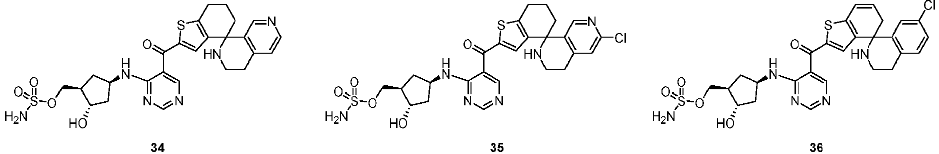

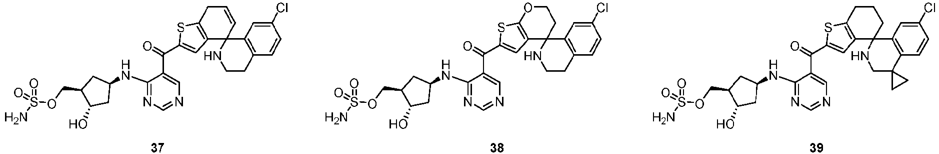

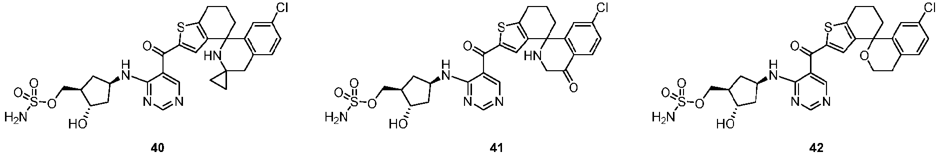

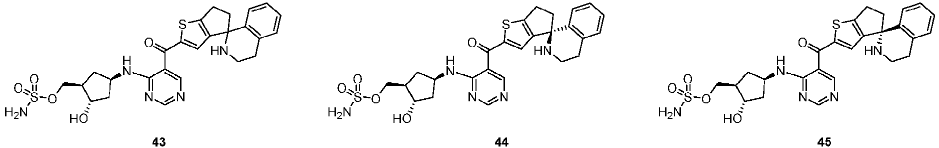

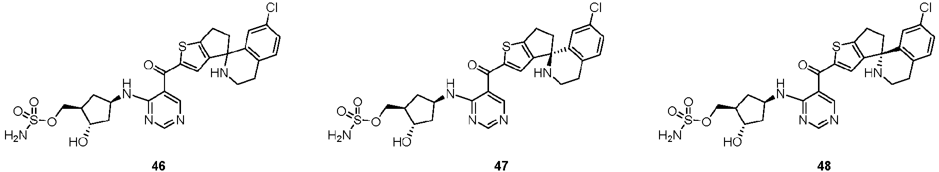

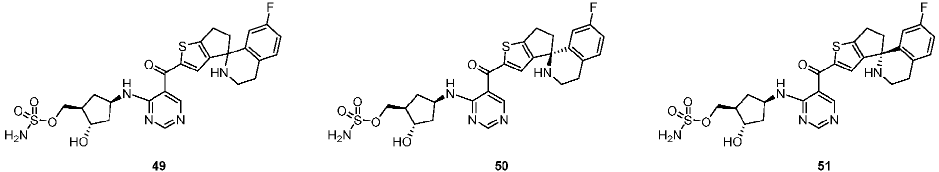

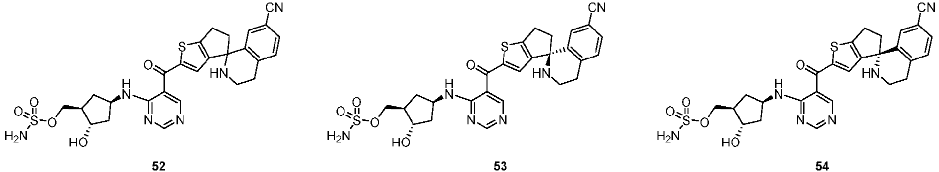

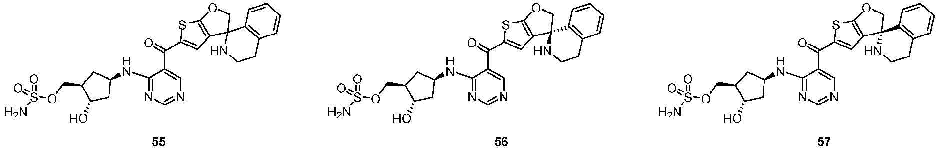

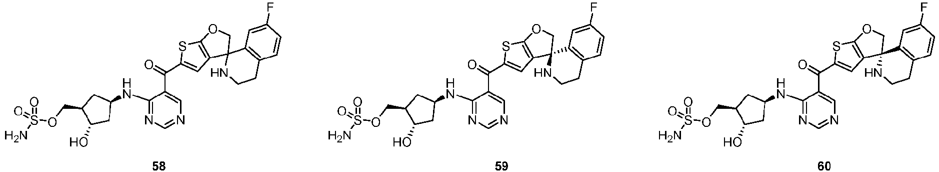

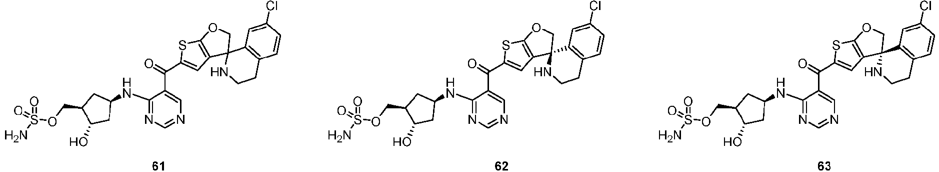

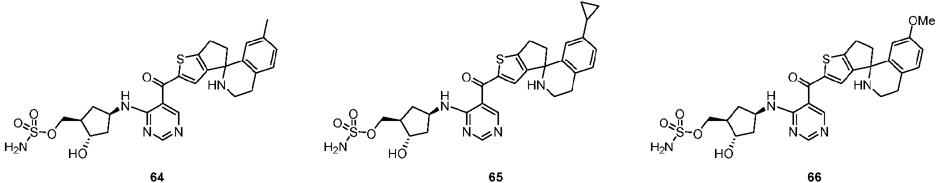

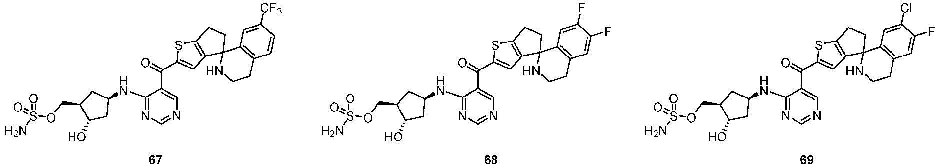

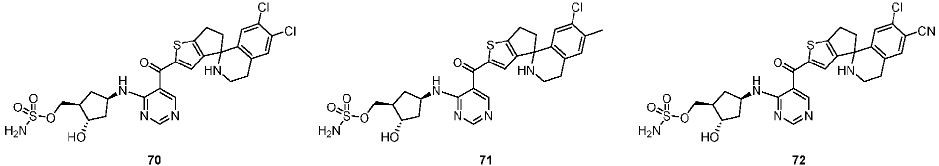

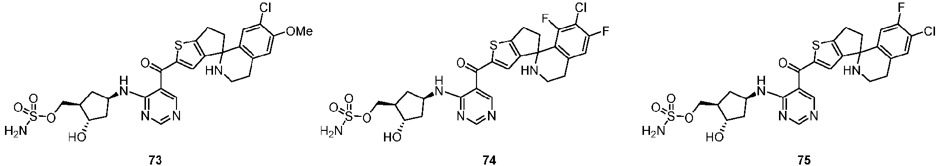

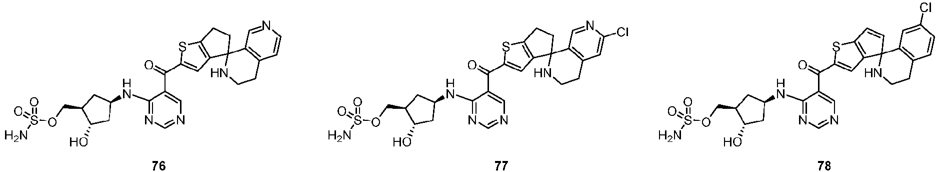

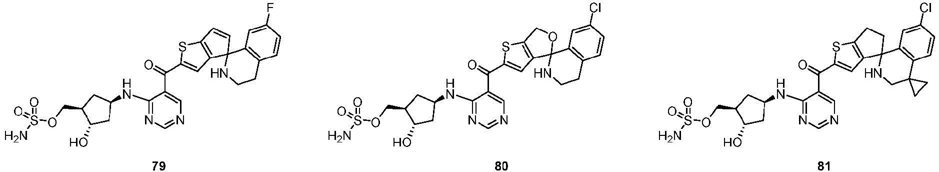

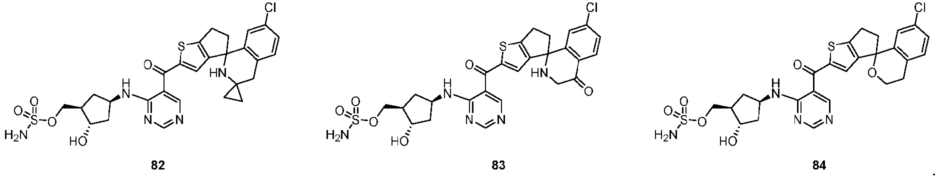

21. The compound or the isomer thereof, the crystalline form thereof, the pharmaceutically acceptable salt thereof, the hydrate thereof, or the solvate thereof according to any one of claims 1-19, wherein the compound has one of the following structures:

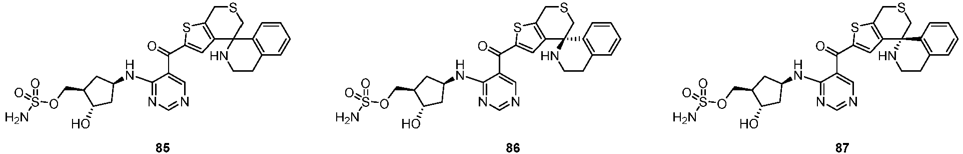

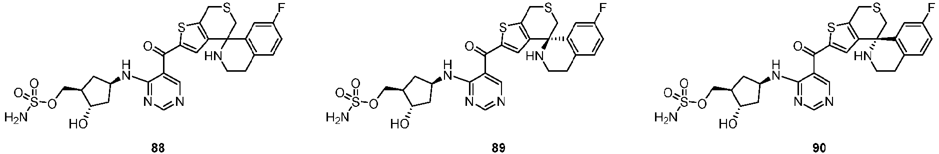

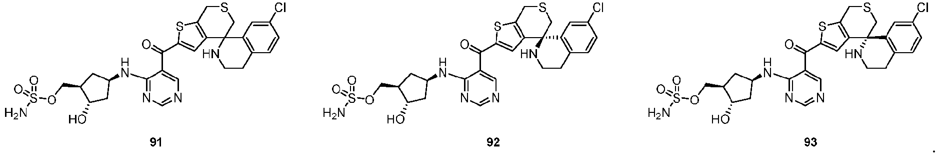

22. The compound or the isomer thereof, the crystalline form thereof, the pharmaceutically acceptable salt thereof, the hydrate thereof, or the solvate thereof according to any one of claims 1-19, wherein the compound has one of the following structures:

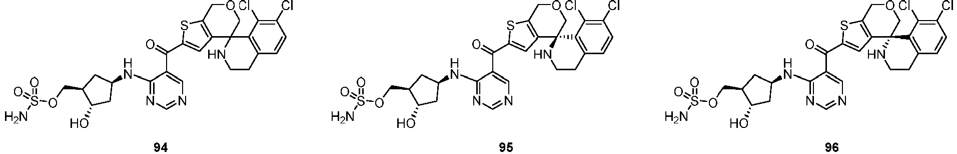

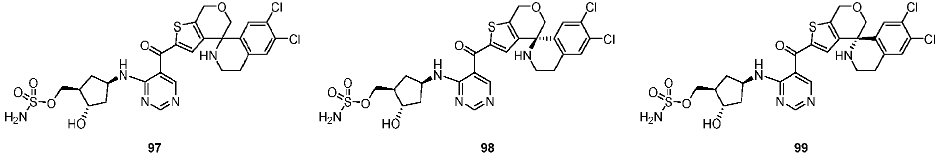

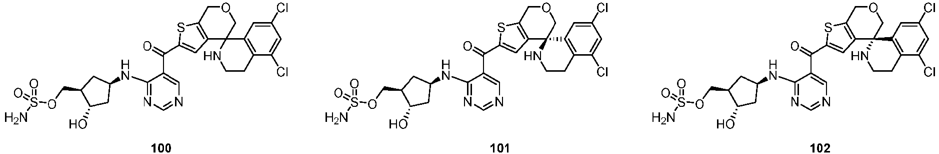

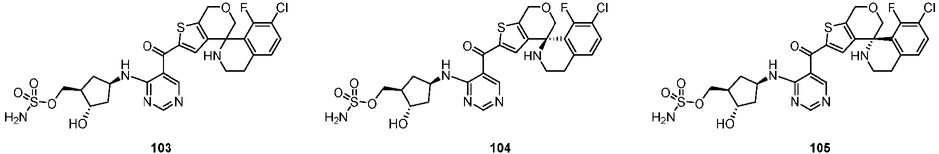

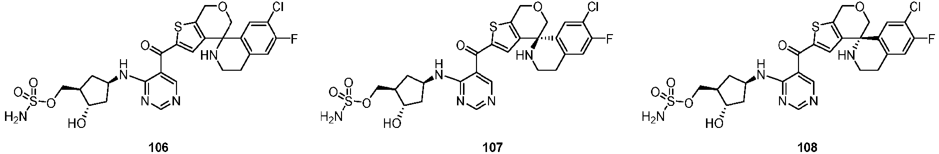

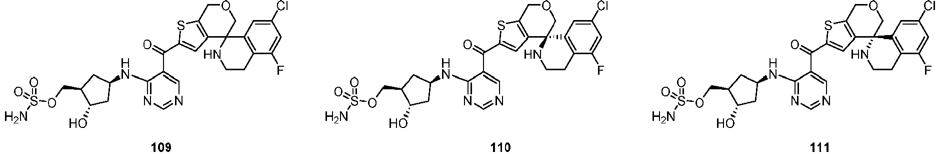

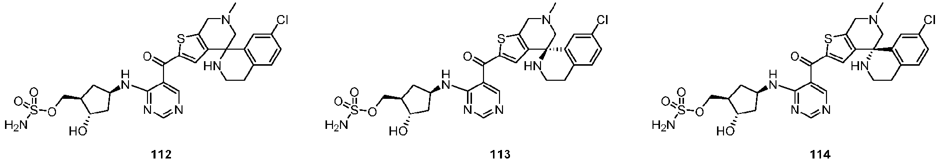

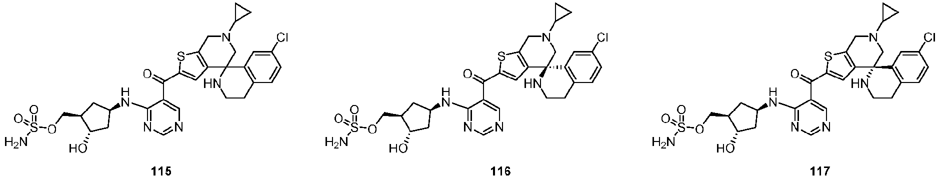

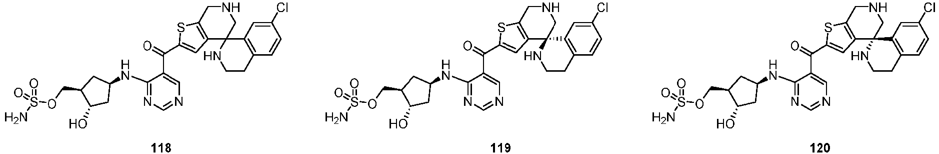

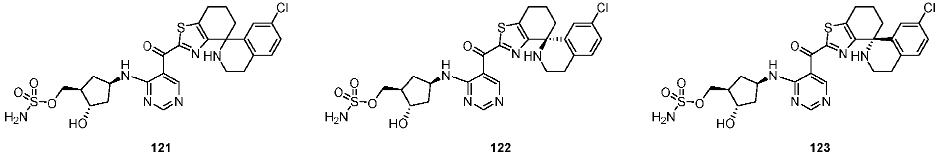

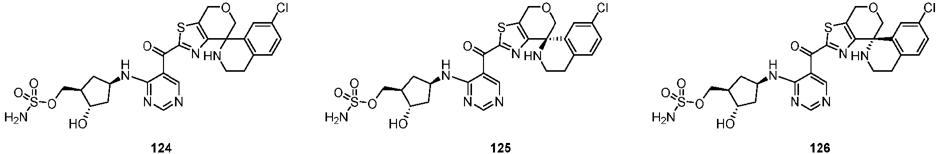

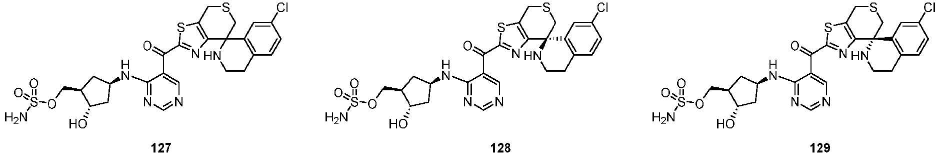

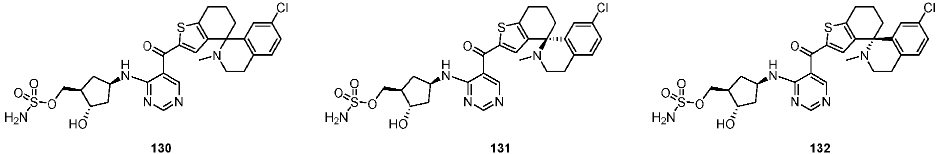

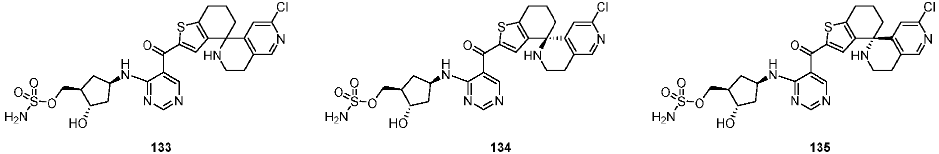

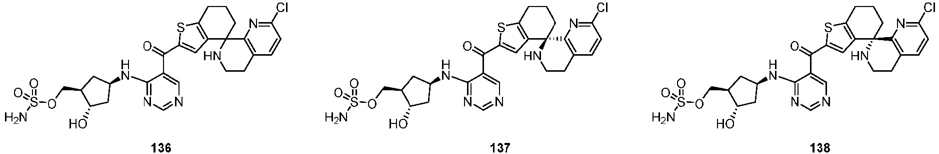

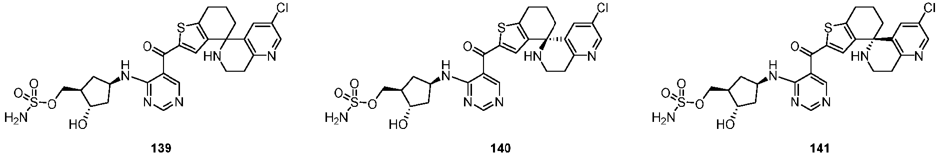

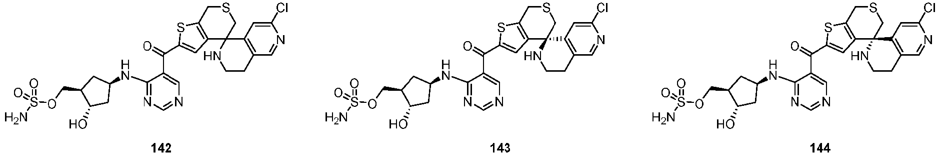

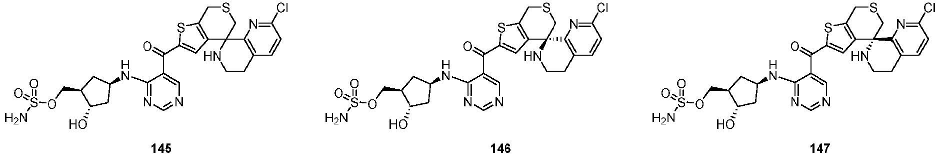

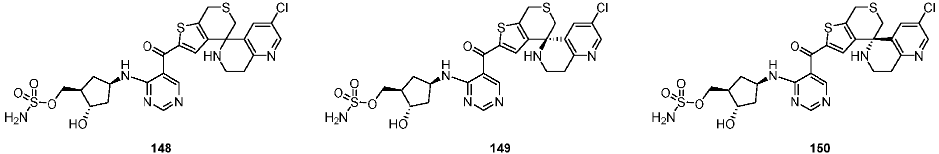

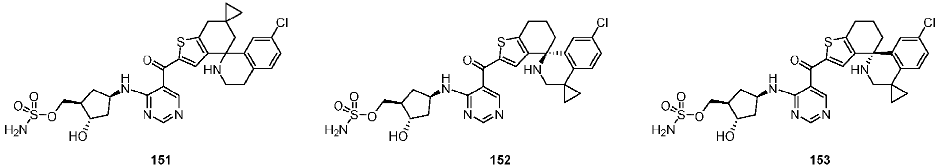

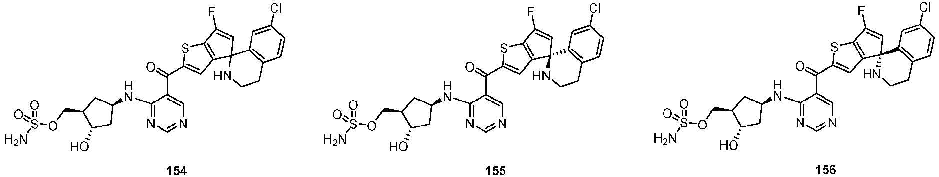

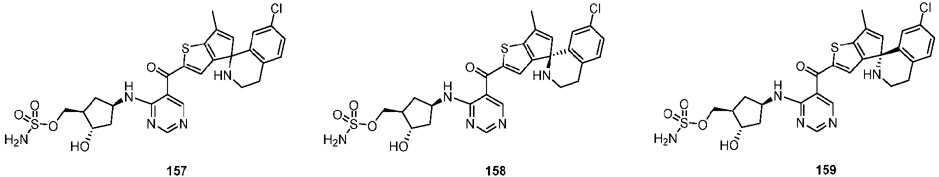

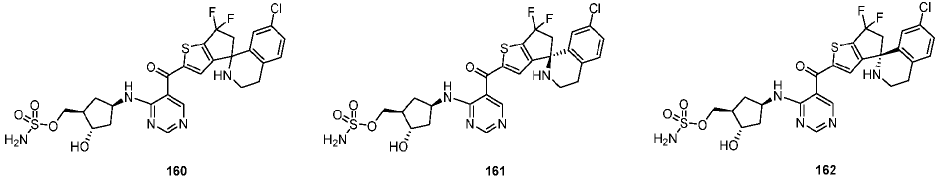

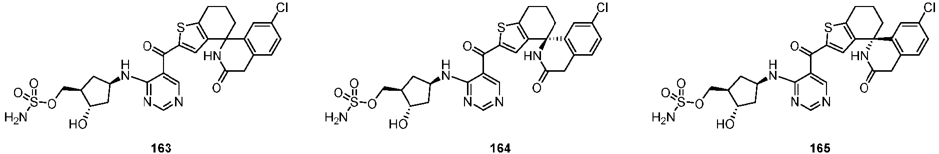

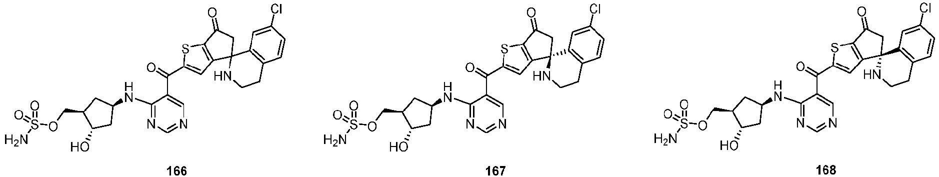

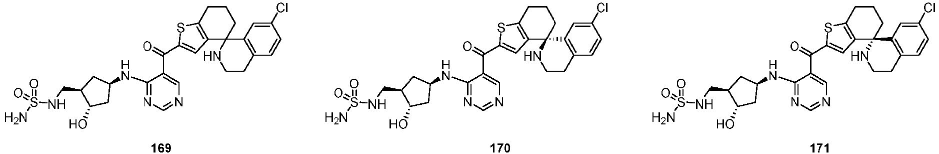

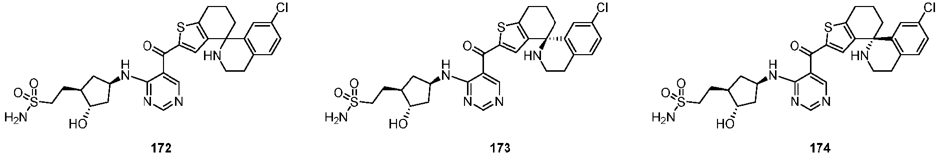

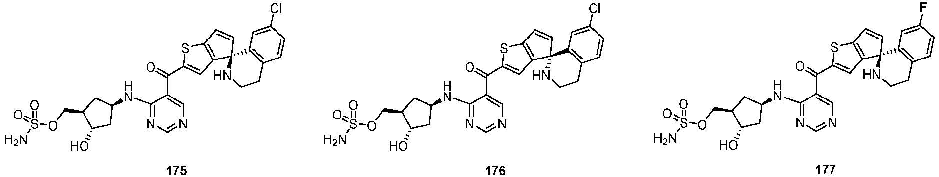

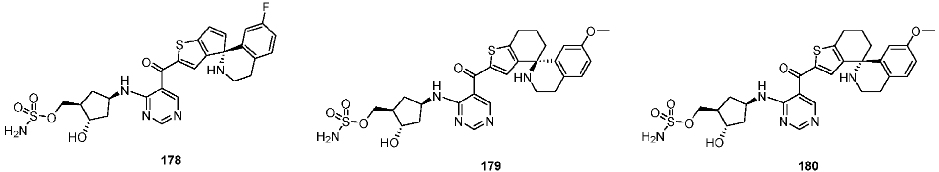

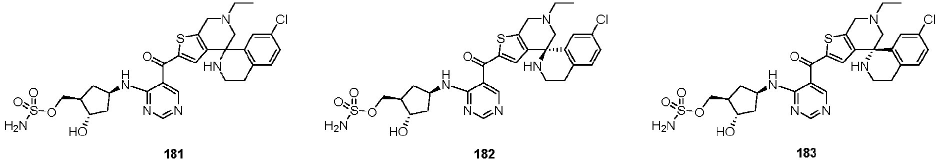

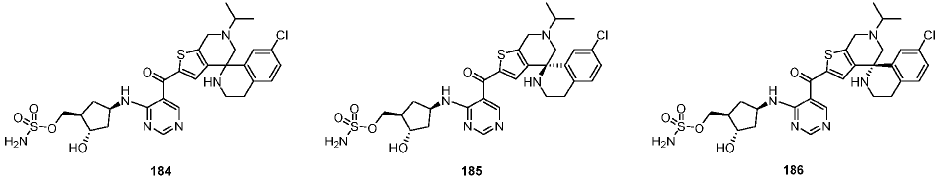

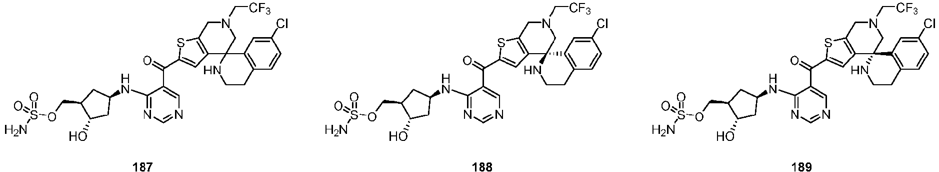

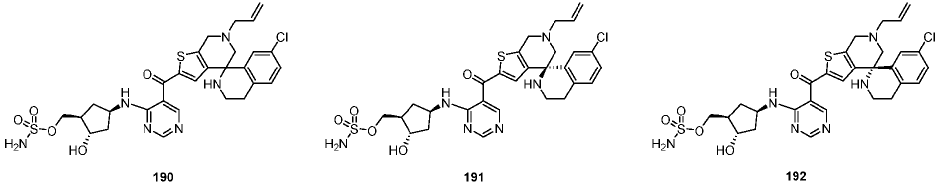

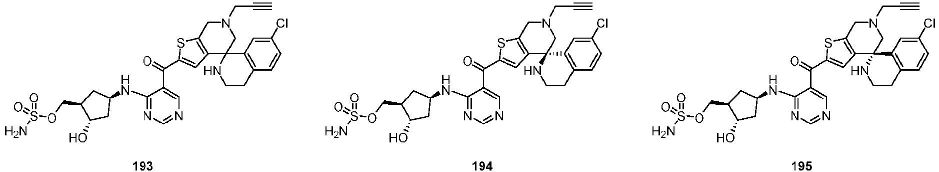

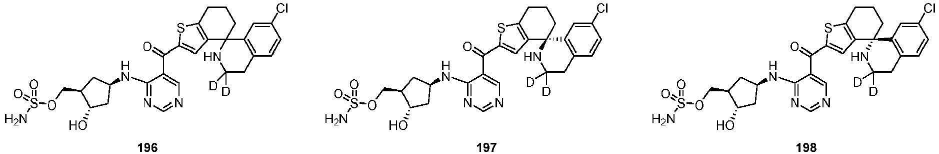

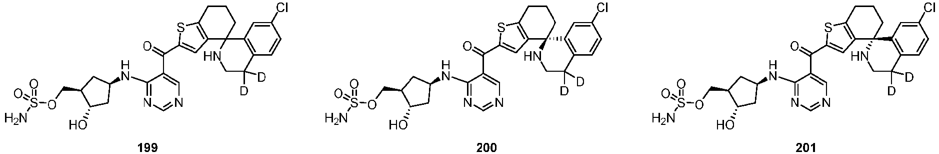

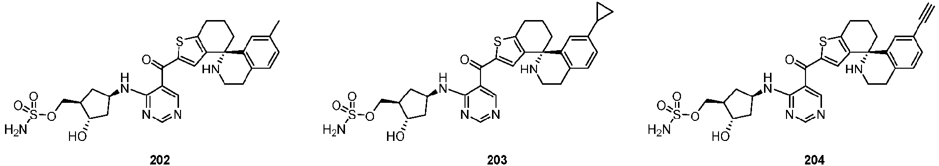

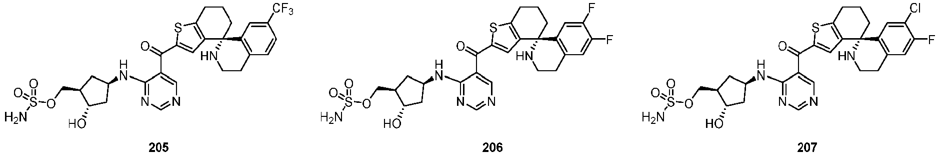

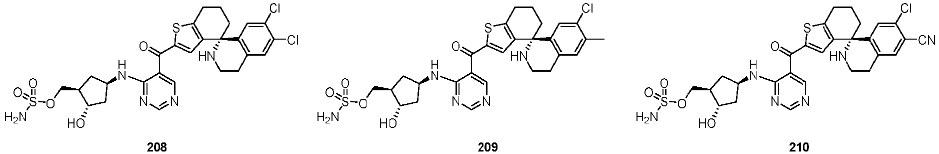

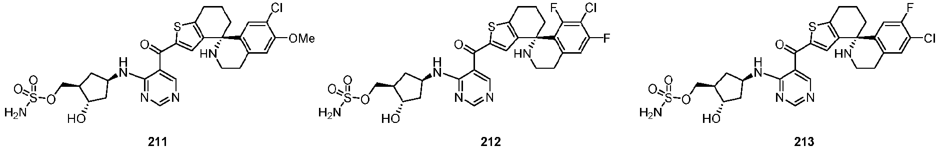

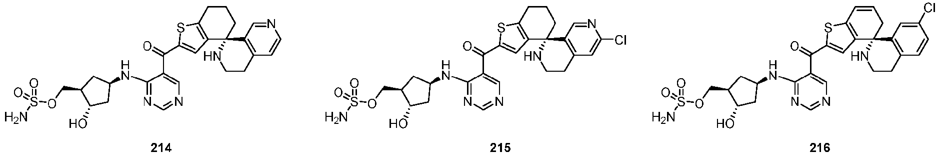

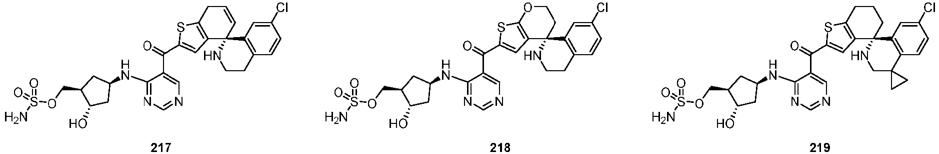

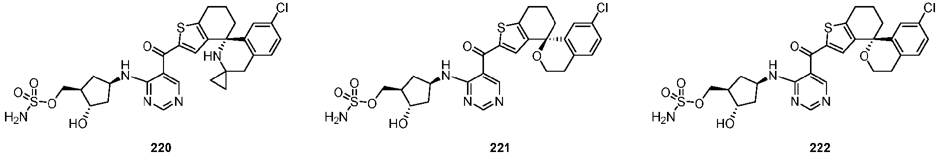

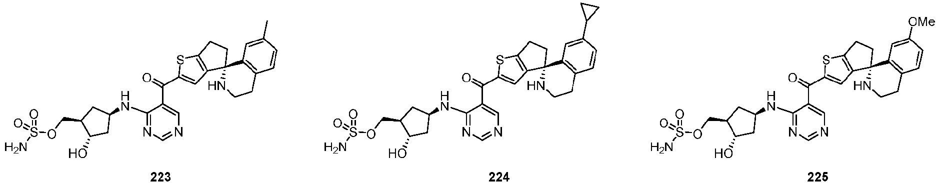

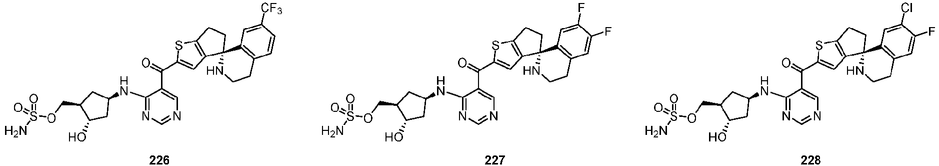

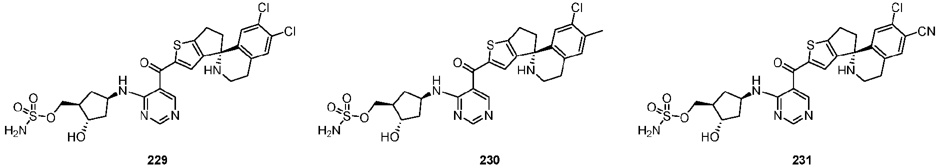

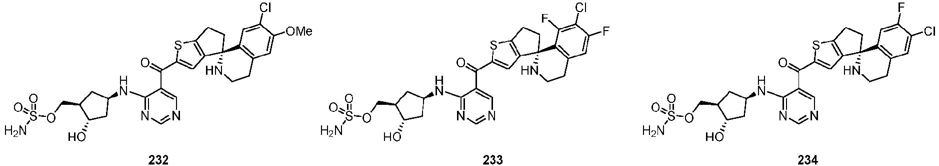

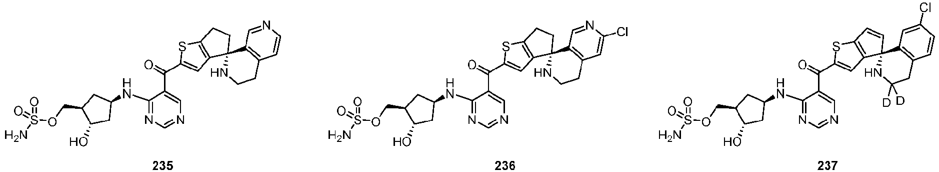

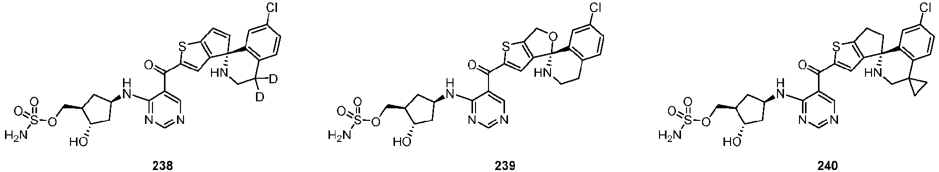

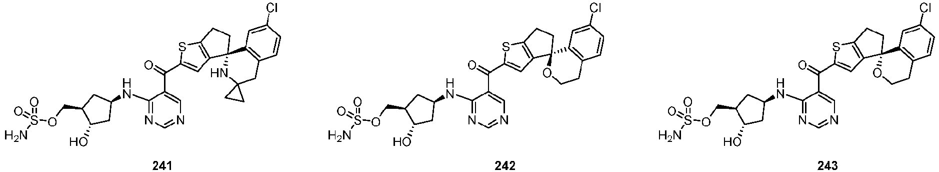

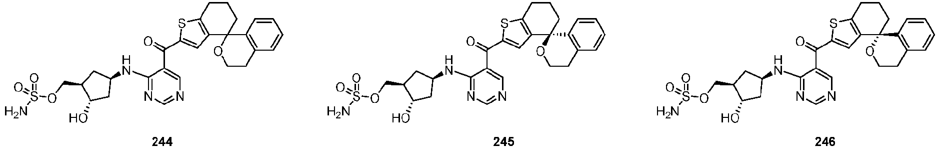

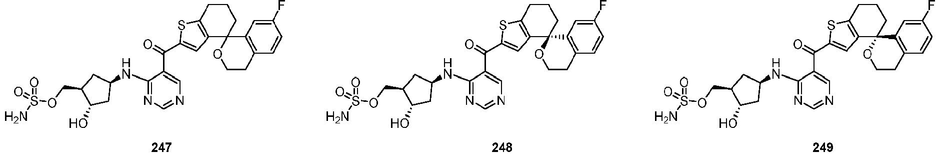

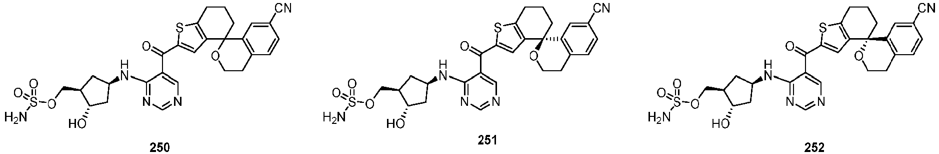

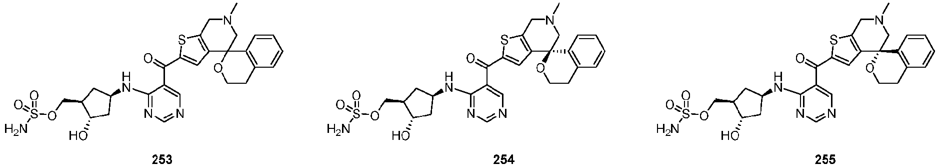

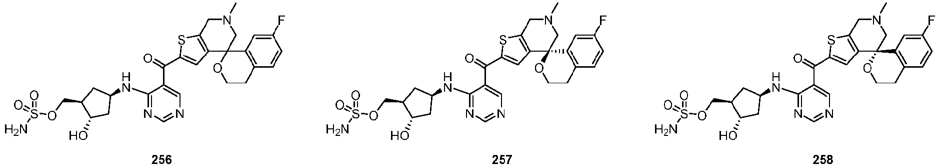

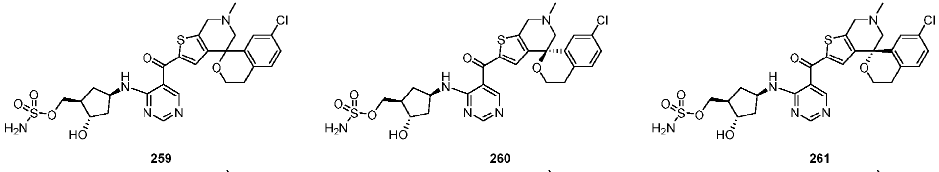

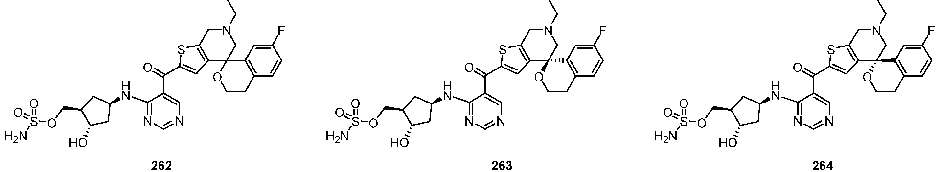

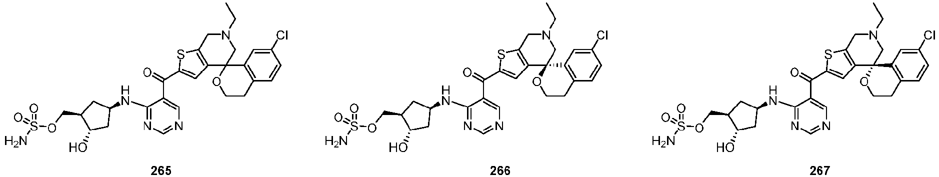

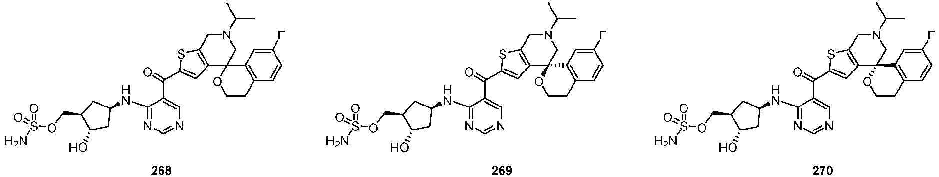

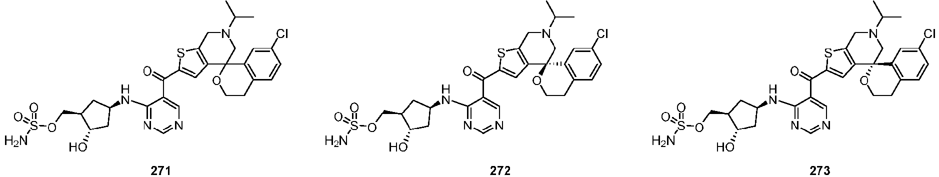

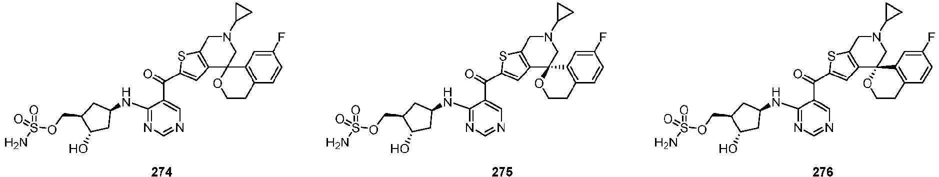

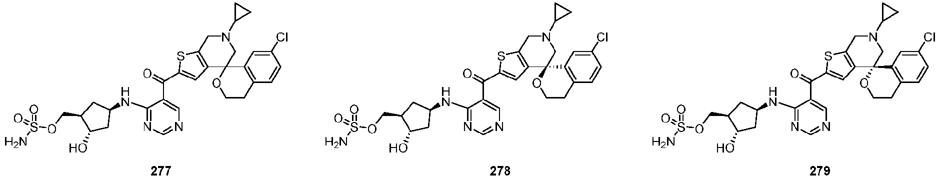

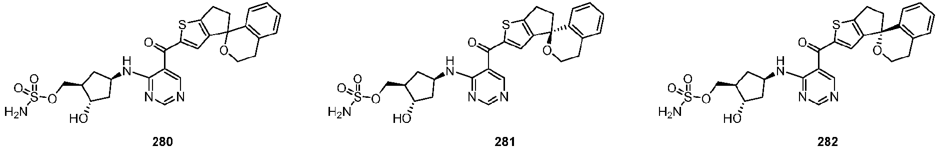

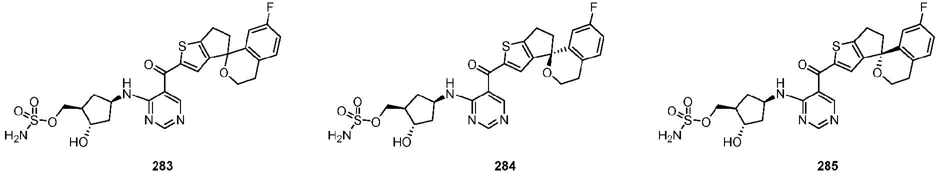

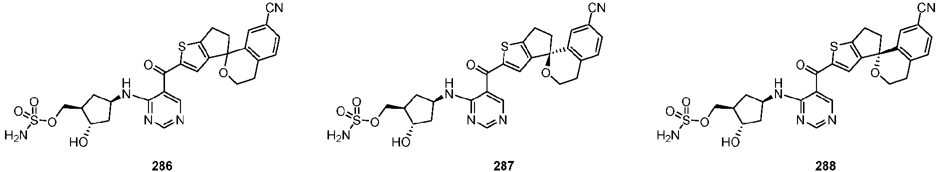

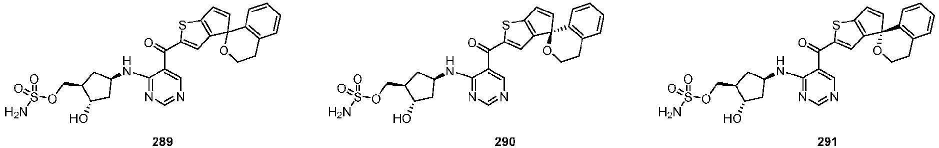

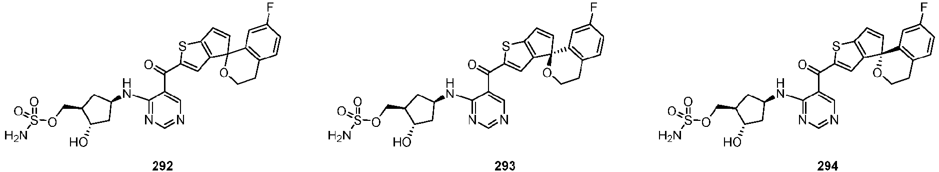

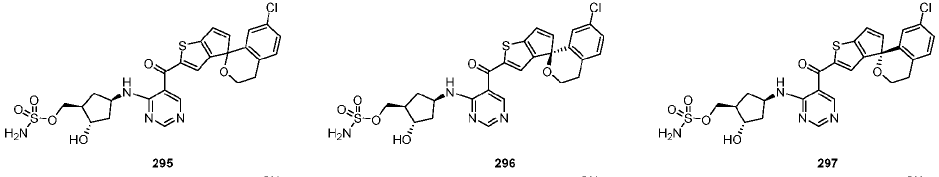

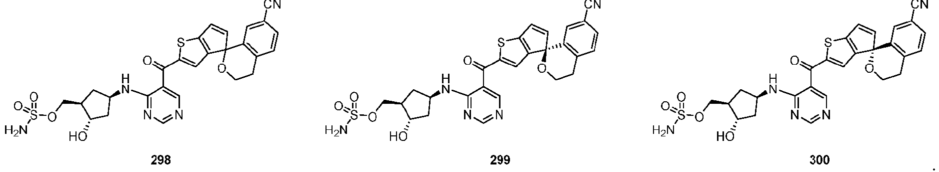

23. A pharmaceutical composition, comprising a pharmaceutically acceptable excipient or carrier, and the compound or the isomer thereof, the crystalline form thereof, the pharmaceutically acceptable salt thereof, the hydrate thereof, or the solvate thereof according to any one of claims 1-22 as an active ingredient.

24. Use of the compound or the isomer thereof, the crystalline form thereof, the pharmaceutically acceptable salt thereof, the hydrate thereof, or the solvate thereof according to any one of claims 1-22, or the pharmaceutical composition according to claim 23 in preparing a medicament for treating a related disease mediated by SAE.

25. The use according to claim 24, wherein the disease is cancer, and the cancer is a hematologic cancer or a solid tumor.
